# EUROPEAN PATENT APPLICATION

(11) **EP 4 365 288 A1**
(43) Date of publication of application: **08.05.2024**
(21) Application number: 22833314.2
(22) Date of filing: 30.06.2022
(51) Int. Cl.: C12N 15/11, A61K 31/7088, A61K 31/7115, A61K 31/712, A61K 31/7125, A61K 48/00, A61P 43/00, C07H 21/02, C07H 21/04, C12N 5/10, C12N 15/67, C12P 21/02

(54) **POLYNUCLEOTIDE AND MEDICINAL COMPOSITION**

(30) Priority: 30.06.2021 JP 2021109239; 15.10.2021 JP 2021169846
(71) Applicant: Kyowa Kirin Co., Ltd., Tokyo 100-0004 (JP); National University Corporation Tokai National Higher Education and Research System, Nagoya-shi, Aichi 464-8601 (JP)
(72) Inventor: IWAI, Hiroto, Tokyo 100-0004 (JP); HOMMA, Masakazu, Tokyo 100-0004 (JP); ATAGO, Takayuki, Tokyo 100-0004 (JP); YAMAMOTO, Junichiro, Tokyo 100-0004 (JP); ABE, Hiroshi, Nagoya-shi, Aichi 464-8601 (JP); KIMURA, Yasuaki, Nagoya-shi, Aichi 464-8601 (JP)
(74) Representative: D Young & Co LLP
(86) International application number: PCT/JP2022/026411
(87) International publication number: WO 2023/277168

(57) **Abstract**

The present invention relates to a polynucleotide, containing a translated region from a start codon to a stop codon, a 5' untranslated region, and a poly A chain, in which 65% or more of nucleotides contained in the poly A chain are sugar modified nucleotides.

## Description

### Technical Field

The present invention relates to a polynucleotide and a pharmaceutical composition containing the polynucleotide.

### Background Art

Genetic information in a cell is transferred by transcribing a messenger RNA (hereinafter, referred to as an "mRNA") by an RNA synthetic enzyme with a DNA used as a template, and by synthesizing a protein through translation by causing a ribosome to bind to the transcribed single-stranded mRNA. This transfer method is designated as "central dogma" in molecular biology, and is a basic principle common to both prokaryotes and eukaryotes.

An mRNA working as an intermediate in the genetic information transfer has base sequence information and structure directly recognized by a ribosome to be translated into a protein.

In recent years, a nucleic acid medicine is expected more and more as a next generation medicament. An artificial polynucleotide used as an mRNA (hereinafter referred to as an "artificial mRNA" in Background Art) produces desired peptide and protein through expression increase or expression acceleration, and can be used as a nucleic acid medicine for protein replacement therapy or a nucleic acid medicine for vaccine therapy.

It is, however, known that an artificial mRNA containing natural bases alone externally introduced into a cell binds to a Toll-like receptor (such as TLR3, TLR7, TLR8, or RIG-I) in the cell to rapidly cause an immune response, and cause an inflammatory reaction and decrease of protein translation level (Non Patent Literature 1). In order to express the protein in the cell, it is necessary to somehow reduce the immunoreactivity of the artificial mRNA itself, and at the same time, to prevent the decrease of the translation level. Besides, since an RNA containing natural bases alone is fragile against a nuclease, it is deemed that a modified nucleotide needs to be introduced also from the viewpoint of imparting stability (Non Patent Literature 2). It is described that a polynucleotide containing a sugar modified nucleotide such as a 2'-O-methyl-modified RNA, a 2'-F-modified RNA, a 2'-O-methoxyethyl-modified RNA, or a bridged nucleic acid of an LNA or the like among modified nucleotides is effective for both the decrease of the immunoreactivity of a nucleic acid medicine and the impartment of resistance against a nuclease (Non Patent Literature 3).

In recent years, movement to use an artificial mRNA produced by *in vitro* transcription (hereinafter referred to as "IVT") as a medicament has been actively promoted (Non Patent Literature 4).

For example, as reported in Non Patent Literature 5 that incidence of metastasis is greatly decreased in a clinical test of an artificial mRNA cancer vaccine on melanoma patients after administration of the cancer vaccine is started, given positive results have been reported regarding use of an artificial mRNA as a medicament.

The artificial mRNA thus clinically applied, however, is produced by IVT. The artificial mRNA produced by IVT has the following two problems. First, an introduction position of a modified nucleotide to be introduced for the purpose of the decrease of the immunoreactivity and the impartment of stability against a nuclease cannot be controlled. Patent Literature 1 discloses a case in which peptide translation potential is weakened/lost in an artificial mRNA having a 2'-F-modified RNA introduced therein by IVT. Secondly, it is impossible to introduce a modified nucleotide unless it is recognized as a substrate by an RNA synthetic enzyme used in IVT. Patent Literature 1 also discloses that it is difficult to prepare an artificial mRNA containing a 2'-O-methyl-modified RNA through an IVT reaction using a general RNA polymerase.

Accordingly, an artificial mRNA produced by introducing a modified nucleotide by IVT has not been sufficiently studied in the position and type of the modified nucleotide.

A method for artificially synthesizing an mRNA by a technique for chemically linking a plurality of RNAs has been reported (Non Patent Literatures 6 and 7). When this method is employed, a sugar modified nucleotide can be introduced into an optional position in an artificial mRNA containing a translated region and an untranslated region. Besides, Patent Literatures 2 and 3 disclose a concept of stabilization by introducing a sugar modified nucleotide into an untranslated region of an mRNA by a method for synthesizing an artificial mRNA by employing the technique for chemically linking a plurality of RNAs. Non Patent Literatures 6 and 7 disclose that the peptide translation potential of an artificial mRNA in which a 2'-O-methyl-modified RNA is introduced into one position in a translated region of the mRNA was found. On the other hand, it is also disclosed that the peptide translation potential is largely weakened depending on the introduction position of the sugar modified nucleotide (Non Patent Literatures 6 and 7).

Therefore, in order to realize sufficiently low immunoreactivity, high stability, and excellent translation potential as an artificial mRNA nucleic acid medicine, further knowledge about a modification rate, position and type of a modified nucleotide is required.

### Citation List

### Patent Literature

Patent Literature 1: International Publication No. WO2014/093574
Patent Literature 2: International Publication No. WO1999/014346
Patent Literature 3: International Publication No. WO2016/022914

### Non Patent Literature

Non Patent Literature 1: Nature Reviews Drug Discovery, Vol. 13, p. 759-780 (2014)
Non Patent Literature 2: Nature Biotechnology, Vol. 35, No. 3, p. 238-248 (2017)
Non Patent Literature 3: Drug Discovery Today, Vol. 13, No. 19/20, p. 842-855 (2008)
Non Patent Literature 4: Nature Biotechnology, Vol. 35, No. 3, p. 193-197 (2017)
Non Patent Literature 5: Nature, Vol. 547, No. 7662, p. 222-226 (2017)
Non Patent Literature 6: Nucleic Acids Research, Vol. 44, No. 2, p. 852-862 (2015)
Non Patent Literature 7: Genes, Vol. 10, No. 2, p. 84 (2019)

### Summary of Invention

### Technical Problem

An object of the present invention is to provide a polynucleotide having excellent translation potential.

### Solution to Problem

As a result of earnest studies made by the present inventors, it has been found that excellent translation potential is exhibited when 65% or more of nucleotides contained in a poly A chain in a 3' untranslated region are sugar modified nucleotides.

The present invention encompasses the following embodiments:
[1] A polynucleotide, comprising:
   a translated region from a start codon to a stop codon,
   a 5' untranslated region, and
   a poly A chain,
   wherein 65% or more of nucleotides contained in the poly A chain are sugar modified nucleotides.
[2] The polynucleotide according to [1], wherein all the nucleotides contained in the poly A chain are sugar modified nucleotides.
[3] The polynucleotide according to [1] or [2], wherein modified sugar portions of the sugar modified nucleotides are each independently selected from the following structures: 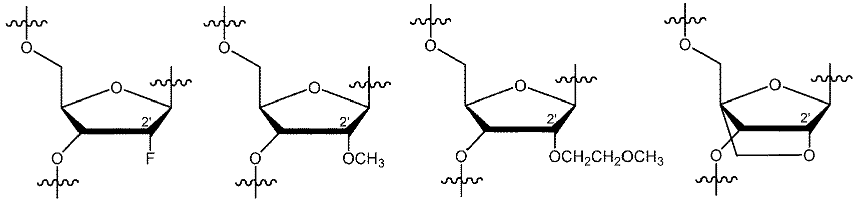
[4] The polynucleotide according to any one of [1] to [3], wherein modified sugar portions of the sugar modified nucleotides are each independently selected from the following structures: 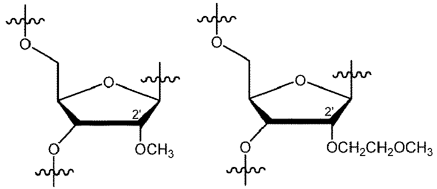
[5] The polynucleotide according to any one of [1] to [4], wherein the poly A chain contains at least one phosphate modified nucleotide.
[6] The polynucleotide according to any one of [1] to [5], wherein first to second nucleotides, first to third nucleotides, first to fourth nucleotides, or first to fifth nucleotides from the 3' end of the poly A chain are linked to one another via phosphorothioate.
[7] The polynucleotide according to any one of [1] to [6], wherein all the nucleotides contained in the poly A chain are linked to one another via phosphorothioate.
[8] The polynucleotide according to any one of [1] to [7], wherein the poly A chain has a length of 2 to 40 bases.
[9] The polynucleotide according to any one of [1] to [8], wherein nucleotides of the 5' untranslated region are each independently selected from a 2'-deoxyribonucleotide, and a spacer modified or sugar modified nucleotide.
[10] The polynucleotide according to any one of [1] to [9], wherein first to sixth nucleotides from the 5' end of the 5 untranslated region are sugar modified nucleotides, and modified sugar portions of the sugar modified nucleotides have the following structure: 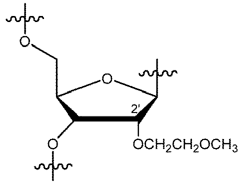
[11] The polynucleotide according to [10], further comprising, on the 5' side of the 5' end of the 5' untranslated region, a portion containing 1 to 10 non-sugar modified nucleotides.
[12] The polynucleotide according to any one of [1] to [11], wherein nucleotides excluding first to sixth nucleotides from the 5' end of the 5' untranslated region include a 2'-deoxyribonucleotide and/or spacer modification.
[13] The polynucleotide according to any one of [1] to [12], wherein the 5' untranslated region and/or a 3' untranslated region includes spacer modification, and preferably the 5' untranslated region and/or the 3' untranslated region includes spacer modifications, and the spacer modifications are each independently selected from the following structures: 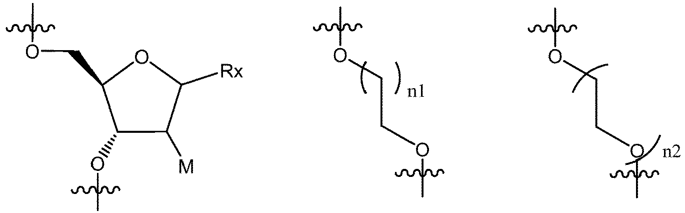
   wherein Rx is ethynyl, a hydrogen atom, or OH,
   M is a hydrogen atom or OH,
   n1 is 1, 2, or 5, and
   n2 is 1, 2, or 3.
   It is noted that the spacer modification of [13] may be the leftmost structure in which an oxygen atom in a five-membered ring is substituted with NH.
[14] The polynucleotide according to any one of [1] to [13],
   wherein first to second nucleotides, first to third nucleotides, first to fourth nucleotides, or first to fifth nucleotides from the 5' end of the 5' untranslated region are linked to one another via phosphorothioate.
[15] The polynucleotide according to any one of [1] to [14],
   wherein the 5' untranslated region contains a base modified nucleotide, and a modified base portion of the base modified nucleotide has the following structure: 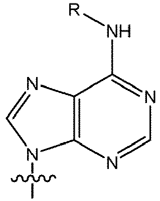
   wherein R is an alkyl group having 1 to 6 carbon atoms.
[16] The polynucleotide according to any one of [1] to [15], wherein the translated region contains at least two codons in which a first nucleotide is a sugar modified nucleotide.
[17] The polynucleotide according to any one of [1] to [16], wherein the translated region contains four or more codons, and first nucleotides of all the codons are sugar modified nucleotides.
[18] The polynucleotide according to any one of [1] to [17], wherein in the translated region, first nucleotides are sugar modified nucleotides in all codons excluding the stop codon, and modified sugar portions of the sugar modified nucleotides have the following structure: 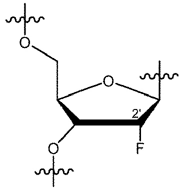
[19] The polynucleotide according to any one of [1] to [18], wherein the translated region contains 2000 or less codons.
   [19-1] The polynucleotide according to any one of [1] to [19], wherein the translated region contains four or more and 2000 or less (4 to 2000) codons.
[20] The polynucleotide according to any one of [1] to [19-1], wherein all nucleotides in the stop codon are sugar modified nucleotides.
[21] The polynucleotide according to any one of [1] to [20], comprising the following structure: 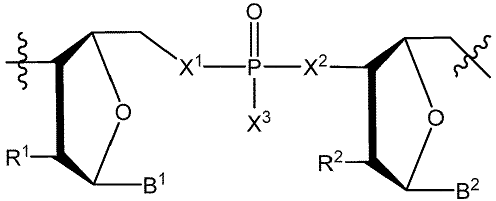
   wherein R¹ and R² are each independently H, OH, F, OCH₂CH₂OCH₃ or OCH₃,
   B¹ and B² are each independently a base portion,
   X¹ is O, S, or NH, and
   X² is O, S, NH, or the following structure: 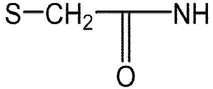
   wherein X³ is OH, SH, or a salt thereof, and
   X¹ and X² are not simultaneously O.
[22] A pharmaceutical composition, comprising the polynucleotide according to any one of [1] to [21].

The present invention further encompasses the following embodiments:
[101] A polynucleotide, comprising:
   a translated region from a start codon to a stop codon,
   a 5' untranslated region, and
   a poly A chain,
   wherein nucleotides of the 5' untranslated region are each independently selected from a 2'-deoxyribonucleotide, and a spacer modified or sugar modified nucleotide.
[101-1] The polynucleotide according to [101], wherein the nucleotides of the 5' untranslated region include at least one or more sugar modified nucleotides.
[102] The polynucleotide according to [101] or [101-1], wherein 65% or more of nucleotides contained in the poly A chain are sugar modified nucleotides, and all nucleotides contained in the poly A chain are preferably sugar modified nucleotides.
[103] The polynucleotide according to any one of [101] to [102], wherein modified sugar portions of the sugar modified nucleotides are each independently selected from the following structures: 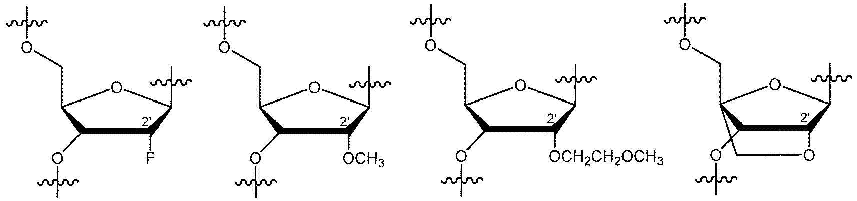
[104] The polynucleotide according to any one of [101] to [103], wherein modified sugar portions of the sugar modified nucleotides are each independently selected from the following structures: 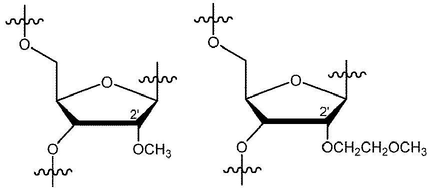
[105] The polynucleotide according to any one of [101] to [104], wherein the poly A chain contains at least one phosphate modified nucleotide.
[106] The polynucleotide according to any one of [101] to [105], wherein first to second nucleotides, first to third nucleotides, first to fourth nucleotides, or first to fifth nucleotides from the 3' end of the poly A chain are linked to one another via phosphorothioate.
[107] The polynucleotide according to any one of [101] to [106], wherein all nucleotides contained in the poly A chain are linked to one another via phosphorothioate.
[108] The polynucleotide according to any one of [101] to [107], wherein the poly A chain has a length of 2 to 40 bases.
[109] The polynucleotide according to any one of [101] to [108], wherein first to sixth nucleotides from the 5' end of the 5' untranslated region are sugar modified nucleotides, and modified sugar portions of the sugar modified nucleotides have the following structure: 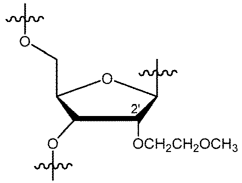
[110] The polynucleotide according to [109], further comprising a portion containing 1 to 10 non-sugar modified nucleotides on the 5' side of the 5' end of the 5' untranslated region.
[111] The polynucleotide according to any one of [101] to [110], wherein nucleotides excluding first to sixth nucleotides from the 5' end of the 5' untranslated region include a 2'-deoxyribonucleotide and/or spacer modification.
[112] The polynucleotide according to any one of [101] to [111], wherein the 5' untranslated region and/or a 3' untranslated region includes spacer modification, and preferably the 5' untranslated region and/or the 3' untranslated region includes spacer modifications, and the spacer modifications are each independently selected from the following structures: 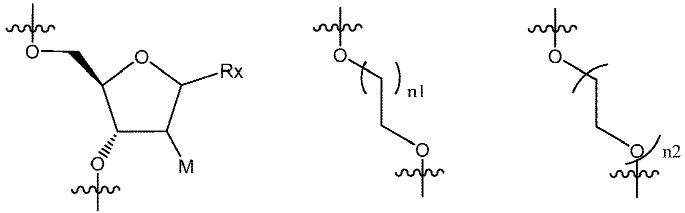
   wherein Rx is ethynyl, a hydrogen atom, or OH,
   M is a hydrogen atom or OH,
   n1 is 1, 2, or 5, and
   n2 is 1, 2, or 3.
   It is noted that the spacer modification of [112] may be the leftmost structure in which an oxygen atom in a five-membered ring is substituted with NH.
[113] The polynucleotide according to any one of [101] to [112], wherein first to second nucleotides, first to third nucleotides, first to fourth nucleotides, or first to fifth nucleotides from the 5' end of the 5' untranslated region are linked to one another via phosphorothioate.
[114] The polynucleotide according to any one of [101] to [113], wherein the 5' untranslated region contains a base modified nucleotide, and a modified base portion of the base modified nucleotide has the following structure: 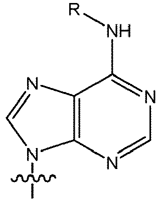 wherein R is an alkyl group having 1 to 6 carbon atoms.
[115] The polynucleotide according to any one of [101] to [114], wherein the translated region contains at least two codons in which the first nucleotide is a sugar modified nucleotide.
[116] The polynucleotide according to any one of [101] to [115], wherein the translated region contains four or more codons, and the first nucleotide is a sugar modified nucleotide in all the codons.
[116-1] The polynucleotide according to any one of [101] to [116], wherein the translated region contains four or more and 2000 or less (4 to 2000) codons.
[117] The polynucleotide according to any one of [101] to [116-1], wherein in the translated region, first nucleotides in all codons excluding the stop codon are sugar modified nucleotides, and modified sugar portions of the sugar modified nucleotides have the following structure: 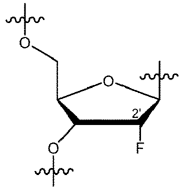
[118] The polynucleotide according to any one of [101] to [117], wherein the translated region contains 2000 or less codons.
[118-1] The polynucleotide according to any one of [101] to [118], wherein the translated region contains 4 to 2000 codons.
[119] The polynucleotide according to any one of [101] to [118-1], wherein all nucleotides in the stop codon are sugar modified nucleotides.
[120] The polynucleotide according to any one of [101] to [119], comprising the following structure: 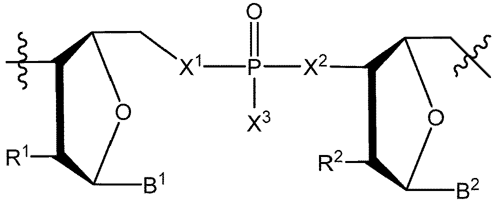
   wherein R¹ and R² are each independently H, OH, F, OCH₂CH₂OCH₃ or OCH₃,
   B¹ and B² are each independently a base portion,
   X¹ is O, S, or NH, and
   X² is O, S, NH, or the following structure: 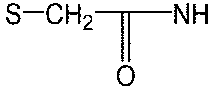
   wherein X³ is OH, SH, or a salt thereof, and
   X¹ and X² are not simultaneously O.
[121] A pharmaceutical composition, comprising the polynucleotide according to any one of [101] to [120].

The present invention further encompasses the following embodiments as aspects different from [1] to [22] described above:
[201] A polynucleotide, comprising:
   a translated region from a start codon to a stop codon,
   a 5' untranslated region, and
   a poly A chain,
   wherein nucleotides contained in the poly A chain are each independently selected from a 2'-deoxyribonucleotide, and a spacer modified or sugar modified nucleotide.
[201-1] The polynucleotide according to [201], wherein the nucleotides contained in the poly A chain include at least one or more sugar modified nucleotides.
[202] The polynucleotide according to [201] or [202-1], wherein 65% or more of nucleotides contained in the poly A chain are sugar modified nucleotides, and all nucleotides contained in the poly A chain are preferably sugar modified nucleotides.
[203] The polynucleotide according to any one of [201] to [202], wherein modified sugar portions of the sugar modified nucleotides are each independently selected from the following structures: 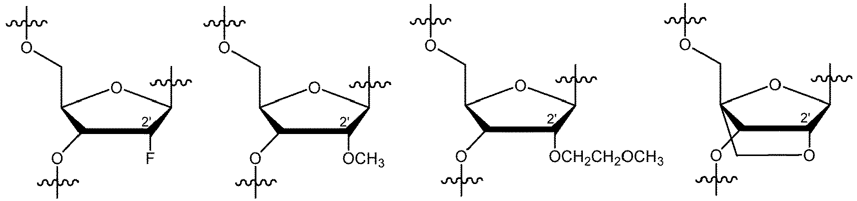
[204] The polynucleotide according to any one of [201] to [203], wherein modified sugar portions of the sugar modified nucleotides are each independently selected from the following structures: 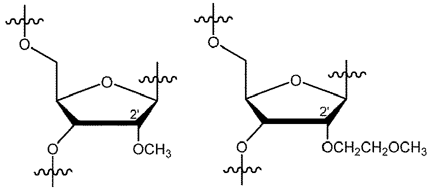
[205] The polynucleotide according to any one of [201] to [204], wherein the poly A chain contains at least one phosphate modified nucleotide.
[206] The polynucleotide according to any one of [201] to [205], wherein first to second nucleotides, first to third nucleotides, first to fourth nucleotides, or first to fifth nucleotides from the 3' end of the poly A chain are linked to one another via phosphorothioate.
[207] The polynucleotide according to any one of [201] to [206], wherein all nucleotides contained in the poly A chain are linked to one another via phosphorothioate.
[208] The polynucleotide according to any one of [201] to [207], wherein the poly A chain has a length of 2 to 40 bases.
[209] The polynucleotide according to any one of [201] to [208], wherein first to sixth nucleotides from the 5' end of the 5' untranslated region are sugar modified nucleotides, and modified sugar portions of the sugar modified nucleotides have the following structure: 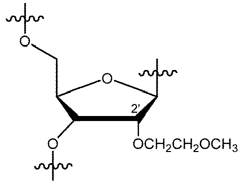
[210] The polynucleotide according to [209], further comprising a portion containing 1 to 10 non-sugar modified nucleotides on the 5' side of the 5' end of the 5' untranslated region.
[211] The polynucleotide according to any one of [201] to [210], wherein nucleotides excluding first to sixth nucleotides from the 5' end of the 5' untranslated region include a 2'-deoxyribonucleotide and/or spacer modification.
[212] The polynucleotide according to any one of [201] to [211], wherein the 5' untranslated region and/or a 3' untranslated region includes spacer modification, and preferably the 5' untranslated region and/or the 3' untranslated region includes spacer modifications, and the spacer modifications are each independently selected from the following structures: 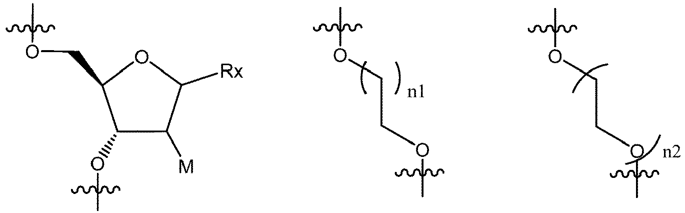
   wherein Rx is ethynyl, a hydrogen atom, or OH,
   M is a hydrogen atom or OH,
   n1 is 1, 2, or 5, and
   n2 is 1, 2, or 3.
   It is noted that the spacer modification of [212] may be the leftmost structure in which an oxygen atom in a five-membered ring is substituted with NH.
[213] The polynucleotide according to any one of [201] to [212], wherein first to second nucleotides, first to third nucleotides, first to fourth nucleotides, or first to fifth nucleotides from the 5' end of the 5' untranslated region are linked to one another via phosphorothioate.
[214] The polynucleotide according to any one of [201] to [213], wherein the 5' untranslated region contains a base modified nucleotide, and a modified base portion of the base modified nucleotide has the following structure: 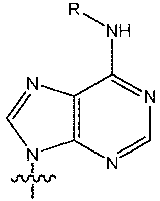 wherein R is an alkyl group having 1 to 6 carbon atoms.
[215] The polynucleotide according to any one of [201] to [214], wherein the translated region contains at least two codons in which the first nucleotide is a sugar modified nucleotide.
[216] The polynucleotide according to any one of [201] to [215], wherein the translated region contains four or more codons, and the first nucleotide is a sugar modified nucleotide in all the codons.
[216-1] The polynucleotide according to any one of [201] to [216], wherein the translated region contains four or more and 2000 or less (4 to 2000) codons.
[217] The polynucleotide according to any one of [201] to [216-1], wherein in the translated region, first nucleotides in all codons excluding the stop codon are sugar modified nucleotides, and modified sugar portions of the sugar modified nucleotides have the following structure: 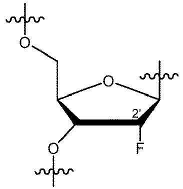
[218] The polynucleotide according to any one of [201] to [217], wherein the translated region contains 2000 or less codons.
[218-1] The polynucleotide according to any one of [201] to [218], wherein the translated region contains 4 to 2000 codons.
[219] The polynucleotide according to any one of [201] to [218-1], wherein all nucleotides in the stop codon are sugar modified nucleotides.
[220] The polynucleotide according to any one of [201] to [219], comprising the following structure: 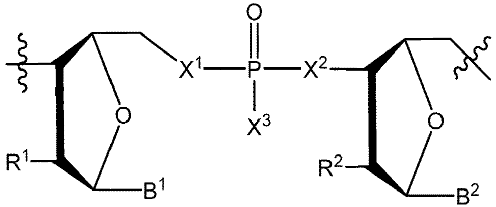
   wherein R¹ and R² are each independently H, OH, F, OCH₂CH₂OCH₃ or OCH₃,
   B¹ and B² are each independently a base portion,
   X¹ is O, S, or NH, and
   X² is O, S, NH, or the following structure: 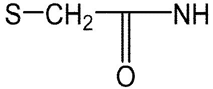
   wherein X³ is OH, SH, or a salt thereof, and
   X¹ and X² are not simultaneously O.
[221] A pharmaceutical composition, comprising the polynucleotide according to any one of [201] to [220].

The present invention further encompasses the following embodiments:
[1A] The polynucleotide according to any one of [1] to [21], [101] to [120], and [201] to [220] or the pharmaceutical composition according to any one of [22], [121], and [221] for use in treatment of a disease.
[1B] A method for treating a disease, including administering a therapeutically effective amount of the polynucleotide according to any one of [1] to [21], [101] to [120], and [201] to [220] or the pharmaceutical composition according to any one of [22], [121], and [221] to a patient requiring treatment.
[1C] Use of the polynucleotide according to any one of [1] to [21], [101] to [120], and [201] to [220] or the pharmaceutical composition according to any one of [22], [121], and [221] for treating a disease.
[1D] Use of the polynucleotide according to any one of [1] to [21], [101] to [120], and [201] to [220] in production of a medicament for treating a disease.
[1E] The polynucleotide according to any one of [1] to [21], [101] to [120], and [201] to [220], for use in production of a medicament for treating a disease.
[1F] A kit for use in treatment of a disease, including the polynucleotide according to any one of [1] to [21], [101] to [120], and [201] to [220] or the pharmaceutical composition according to any one of [22], [121], and [221], and an instruction manual.

### Description of Embodiments

### <Polynucleotide>

In one embodiment of the present invention, a polynucleotide comprises a translated region from a start codon to a stop codon, a 5' untranslated region, and a poly A chain, in which 65% or more of nucleotides contained in the poly A chain are sugar modified nucleotides.

In the present invention, the polynucleotide in which 65% or more of nucleotides contained in the poly A chain are sugar modified nucleotides exhibits excellent translation potential.

The polynucleotide of the present embodiment comprises a translated region, and a poly A chain, and a 5' untranslated region, and the translated region and the poly A chain are preferably arranged in the stated order in the 5' to 3' direction of the polynucleotide, the translated region and the poly A chain may be directly linked to each other, and another region or a sequence not contained in the poly A chain may be present therebetween. The translated region and the poly A chain being directly linked to each other means that the poly A chain is bonded subsequently to the stop codon of the translated region, and in this case, a 3' untranslated region corresponds to the poly A chain.

The poly A chain is present in the 3' untranslated region, and the polynucleotide comprises the 5' untranslated region, the translated region, and the 3' untranslated region. In this case, the poly A chain is present at the 3' end of the 3' untranslated region.

The polynucleotide of the present embodiment is understood as a polynucleotide having an equivalent function to, for example, an mRNA, a small open reading frame (smORF), a non-canonical open reading frame, a long noncoding RNA (lncRNA), or a pri-microRNA (pri-miRNA) in that the translated region is translated into a polypeptide (the term "polypeptide" used herein encompasses a protein).

The polynucleotide may be a single-stranded polynucleotide, or may be a cyclic polynucleotide having ends thereof mutually linked.

The polynucleotide of the present embodiment contains a plurality of nucleotides bonded to one another, and each nucleotide contained in the polynucleotide usually contains a sugar portion, a base portion, and a phosphate portion. A sugar portion is a portion corresponding to a sugar contained in the nucleotide, a base portion is a portion corresponding to a base contained in the nucleotide, and a phosphate portion is a portion corresponding to a phosphate contained in the nucleotide.

In general, a base portion of a nucleotide is selected from adenine (A), guanine (G), cytosine (C), uracil (U), and thymine (T), and a sugar portion is selected from a ribose, and a 2'-deoxyribose. Each of the ribose and the 2'-deoxyribose is preferably in a D-form.

The nucleotide contains a combination of the base portion and the sugar portion, and is preferably a ribonucleotide having adenine (A), guanine (G), cytosine (C), or uracil (U) as the base portion, and having a D-ribose as the sugar portion.

The nucleotide contained in the polynucleotide of the present embodiment may be a ribonucleotide (AUGC) that is an unmodified nucleotide, a deoxyribonucleotide (ATGC) that is an unmodified nucleotide, or a modified nucleotide having a structure not derived from an unmodified nucleotide in at least a part of the sugar portion, the base portion, and the phosphate portion.

Herein, a nucleotide having a sugar portion modified is designated as a "sugar modified nucleotide", a nucleotide having a base portion modified is designated as a "base modified nucleotide", and a nucleotide having a phosphate portion modified is designated as a "phosphate modified nucleotide". Herein, the term "modification" means change of the structure of the sugar portion, the base portion, or the phosphate portion. The structural change by modification is not especially limited. An example of the modification includes substitution in an optional site with an optional substituent.

A nucleotide having any one of a modified sugar portion, a modified base portion, and a modified phosphate portion is referred to as a modified nucleotide, and a nucleotide having modification in none of a sugar portion, a base portion, and a phosphate portion is an unmodified nucleotide.

A modified nucleotide may have one modified portion out of a modified sugar portion, a modified base portion, and a modified phosphate portion, may have an optional combination of two modified portions, or may have three modified portions.

An unmodified sugar portion is a sugar portion corresponding to a ribose or a 2'-deoxyribose, and is preferably a sugar portion corresponding to a ribose. In other words, in the polynucleotide of the present embodiment, nucleotides excluding a sugar modified nucleotide preferably contains a sugar portion corresponding to a ribose or a 2'-deoxyribose, and more preferably contains a sugar portion corresponding to a ribose.

### (Sugar Modified Nucleotide)

The sugar modified nucleotide is not especially limited as long as the sugar portion of the nucleotide is modified, and preferably contains a sugar portion modified at least in the 2' position. When the 2' position is modified, the stability against an enzyme can be improved. The sugar portion modified at least in the 2' position may be a sugar portion having the 2' position and the 4' position cross-linked.

An example of the modified sugar portion includes the following: 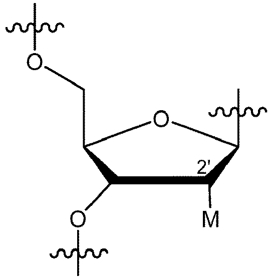 wherein M is R¹, OR¹, R²OR¹, OR²QR¹, SH, SR¹, NH₂, NHR¹, NR¹₂, N₃, CN, F, Cl, Br, or I, R¹ each independently is alkyl or aryl, preferably alkyl having 1 to 6 carbon atoms, and more preferably alkyl having 1 to 3 carbon atoms, and R² is alkylene, and preferably alkylene having 1 to 6 carbon atoms.

When M is H or OH, the portion is an unmodified sugar portion, a nucleotide having an unmodified sugar portion in which M is H is a 2'-deoxyribonucleotide, and a nucleotide having an unmodified sugar portion in which M is OH is a ribonucleotide.

Herein, an example of alkyl having 1 to 6 carbon atoms includes a linear or branched alkyl having 1 to 6 carbon atoms. Examples of the linear alkyl having 1 to 6 carbon atoms include methyl, ethyl, propyl, butyl, pentyl, and hexyl. Examples of the branched alkyl having 1 to 6 carbon atoms include isopropyl, isobutyl, sec-butyl, tert-butyl, and methyl-substituted pentyl.

Examples of alkyl having 1 to 3 carbon atoms include methyl, ethyl, propyl, and isopropyl.

Herein, examples of aryl include optionally substituted phenyl, and optionally substituted naphthyl.

Herein, alkylene having 1 to 6 carbon atoms is a group obtained by removing one hydrogen atom bonded to a carbon atom of alkyl having 1 to 6 carbon atoms.

Herein, the modified sugar portion refers to a modified sugar structure contained in the sugar modified nucleotide. Other examples of M in the modified sugar portion include 2-(methoxy)ethoxy, 3-aminopropoxy, 2-[(N,N-dimethylamino)oxy]ethoxy, 3-(N,N-dimethylamino)propoxy, 2-[2-(N,N-dimethylamino)ethoxy]ethoxy, 2-(methylamino)-2-oxoethoxy, 2-(N-methylcarbamoyl)ethoxy), and 2-cyanoethoxy.

Other examples of the modified sugar portion include sugar portions of the following nucleic acids:
- Locked Nucleic Acid (LNA) [Tetrahedron Letters, 38, 8735 (1997) and Tetrahedron, 54, 3607 (1998)];
- Ethylene bridged nucleic acid (ENA) [Nucleic Acids Research, 32, e175 (2004)];
- Constrained Ethyl (cEt) [The Journal of Organic Chemistry 75, 1569 (2010)];
- Amido-Bridged Nucleic Acid (AmNA) [Chem Bio Chem 13, 2513 (2012)];
- 2'-O,4'-c-Spirocyclopropylene bridged nucleic acid (scpBNA) [Chem. Commun., 51, 9737 (2015)];
- tricycloDNA (tcDNA) [Nat. Biotechnol., 35, 238 (2017)];
- Unlocked Nucleic Acid (UNA) [Mol. Ther. Nucleic Acids 2, e103 (2013)];
- 3'-fluoro hexitol nucleic acid (FHNA) [Nat. Biotechnol., 35, 238 (2017)];
- peptide nucleic acid (PNA) [Acc. Chem. Res., 32, 624 (1999)];
- oxy-peptide nucleic acid (OPNA) [J. Am. Chem. Soc., 123, 4653 (2001)]; and
- peptide ribonucleic acid (PRNA) [J. Am. Chem. Soc., 122, 6900 (2000)] .

The modified sugar portion is not especially limited, but is preferably selected from the following:

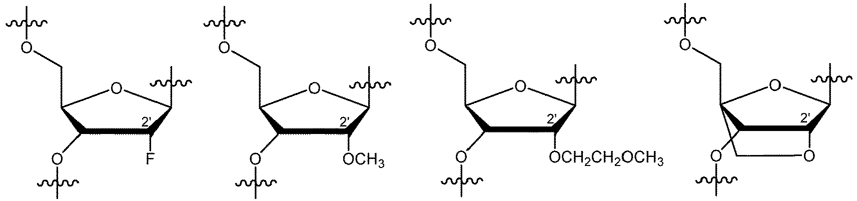

The sugar modified nucleotide preferably contains a base portion corresponding to a base selected from the group consisting of adenine (A), guanine (G), cytosine (C), and uracil (U), and the number of types of the base is preferably at least two. Here, the term "the number of types of the base being at least two" means, for example, that one sugar modified nucleotide contains a base portion corresponding to adenine and another sugar modified nucleotide contains a base portion corresponding to guanine.

The sugar modified nucleotide may be a base modified nucleotide and/or a phosphate modified nucleotide (in other words, the sugar modified nucleotide may further contain a modified base portion and/or a modified phosphate portion). At least one sugar modified nucleotide may contain a modified base portion.

### (Base Modified Nucleotide)

The base modified nucleotide is not especially limited as long as a base portion of a nucleotide is modified. Examples of an unmodified base portion include base portions corresponding to adenine, guanine, cytosine, and uracil. Examples of a modified base portion include a base portion in which oxygen atom of an unmodified base portion is substituted with sulfur atom, a base portion in which hydrogen atom of an unmodified base portion is substituted with alkyl having 1 to 6 carbon atoms, halogen or the like, a base portion in which methyl of an unmodified base portion is substituted with hydrogen atom, hydroxymethyl, alkyl having 2 to 6 carbon atoms or the like, and a base portion in which amino of an unmodified base portion is substituted with alkyl having 1 to 6 carbon atoms, alkanoyl having 1 to 6 carbon atoms, oxo, hydroxy or the like.

Specific examples of the modified base portion contained the base modified nucleotide include 5-methylcytosine (5-me-C), 5-hydroxymethylcytosine, xanthine, hypoxanthine, 2-aminoadenine, 6-methyladenine, 6-methylguanine, 2-propyladenine, 2-propylguanine, 2-thiouracil, 2-thiothymine, 2-thiocytosine, 5-propynyluracil, 5-propynylcytosine, 6-azouracil, 6-azocytosine, 6-azothimine, 5-pseudouracil, 4-thiouracil, 8-haloadenine, 8-haloguanine, 8-aminoadenine, 8-aminoguanine, 8-mercaptoadenine, 8-mercaptoguanine, 8-alkylthioadenine, 8-alkylthioguanine, 8-hydroxyadenine, 8-hydroxyguanine, 5-bromouracil, 5-bruomocytosine, 5-trifluoromethyluracil, 5-trifluoromethyluracil, 7-methylguanine, 7-methyladenine, 8-azaguanine, 8-azaadenine, 7-deazaguanine, 3-deazaguanine, 7-deazaadenine, 3-deazaadenine, pyrazolo[3,4-d]pyrimidine, imidazo[1,5-a]1,3,5 triazinone, 9-deazapurine, imidazo[4,5-d]pyrazine, thiazolo[4,5-d]pyrimidine, pyrazine-2-one, 1,2,4-triazine, pyridazine, and 1,3,5-triazine.

The base modified nucleotide may be a sugar modified nucleotide and/or a phosphate modified nucleotide (in other words, the base modified nucleotide may further contain a modified sugar portion and/or a modified phosphate portion).

### (Phosphate Modified Nucleotide)

The phosphate modified nucleotide is not especially limited as long as a phosphate portion (phosphodiester bond) of a nucleotide is modified. Examples of a modified phosphate portion include a phosphorothioate bond, a phosphorodithioate bond, an alkylphosphonate bond, and a phosphoramidate bond.

The translated region may contain a phosphate modified nucleotide having an optical isomer (Rp, Sp) in a modified phosphate portion. A method for selectively synthesizing an optical isomer of a phosphorothioate bond is disclosed in, for example, J. Am. Chem. Soc., 124, 4962 (2002), Nucleic Acids Research, 42, 13546 (2014), and Science 361, 1234 (2018) .

The phosphate modified nucleotide may be a sugar modified nucleotide and/or a base modified nucleotide (in other words, the phosphate modified nucleotide may further contain a modified sugar portion and/or a modified base portion).

### translated Region>

The polynucleotide of the present embodiment comprises a translated region. The translated region is also designated as a coding sequence (CDS). The translated region contains a plurality of codons from a start codon to a stop codon (or designated as a termination codon), and is a region to be translated to synthesize a polypeptide. A codon is a unit encoding each amino acid contained in a polypeptide, and the unit includes three nucleotides.

In the polynucleotide of the present embodiment, one polynucleotide may contain a plurality of translated regions, and in a polynucleotide containing a plurality of translated regions, a translated region portion of a polynucleotide containing one translated region may contain a plurality of translated regions.

Although not limited to a natural codon table, based on the natural codon table, a start codon can be, for example, AUG encoding methionine. Examples of an unusual start codon excluding AUG include CUG, GUG, UUG, ACG, AUC, AUU, AAG, AUA, and AGG. Examples of a stop codon include UAA, UAG and UGA. The types of codons contained in the translated region are not especially limited, and can be appropriately selected in accordance with a target polypeptide.

The number (n) of the codons contained in the translated region is preferably an integer of 2 to 2000, more preferably an integer of 2 to 1500, further preferably an integer of 2 to 1000, and most preferably an integer of 2 to 500. Alternatively, the lower limit of these numerical ranges may be changed to 5, 10, 50, 100, 200 or the like. When the lower limit is changed, the number (n) of the codons contained in the translated region is preferably an integer of 5 to 2000, 10 to 2000, 50 to 2000, 100 to 2000, or 200 to 2000, more preferably an integer of 5 to 1500, 10 to 1500, 50 to 1500, 100 to 1500, or 200 to 1500, further preferably an integer of 5 to 1000, 10 to 1000, 50 to 1000, 100 to 1000, or 200 to 1000, and most preferably an integer of 5 to 500, 10 to 500, 50 to 500, 100 to 500, or 200 to 500.

The number of the nucleotides contained in the translated region is a number three times as large as the number (n) of the codons.

Each codon contains the first, second and third nucleotides. For example, in the start codon (AUG), the first nucleotide is A, the second nucleotide is U, and the third nucleotide is G.

The translated region contains n codons, the number n is a positive integer of 2 or more, and when each of the n codons contains the first, second, and third nucleotides, it is preferable that the first nucleotides in at least two codons out of the n codons are sugar modified nucleotides.

In other words, it is preferable that the translated region contains, out of the codons, at least two codons in which the first nucleotide is a sugar modified nucleotide, and the at least two codons in which the first nucleotide is a sugar modified nucleotide may be codons in optional positions in the translated region.

Since translation activity is retained even when the sugar portion of the first nucleotide in the plurality of codons contained in the translated region is modified, the polynucleotide of the present embodiment retains the translation activity although it has a modification site in the translated region. Herein, the term "translation activity" means activity of translating an mRNA to synthesize a polypeptide. The polynucleotide of the present embodiment also has excellent stability against an enzyme (such as a nuclease).

The polynucleotide of the present embodiment exhibits excellent translation potential as long as the translated region retains the translation activity because 65% or more of nucleotides contained in the poly A chain are sugar modified nucleotides.

Herein, the term "translation activity being retained" refers to that the polynucleotide modified in the sugar portion of the first nucleotide in the plurality of codons has translation activity corresponding to 60% or more of that of an unmodified polynucleotide. The translation activity of the modified polynucleotide is preferably 70% or more, 80% or more, 90% or more, or 100% or more as compared with that of the unmodified polynucleotide.

In the polynucleotide of the present embodiment, at least two of the first nucleotides contained in the codons of the translated region may be sugar modified nucleotides. The position of each codon containing the sugar modified nucleotide is not especially limited. A ratio that the first nucleotides are sugar modified nucleotides is preferably 5% or more, 10% or more, 15% or more, 20% or more, 25% or more, 30% or more, 35% or more, 40% or more, 45% or more, 50% or more, 55% or more, 60% or more, 65% or more, 70% or more, 75% or more, 80% or more, 85% or more, 90% or more, 95% or more, or 100%. The ratio being 1000 means that all the first nucleotides are sugar modified nucleotides. As the ratio is higher, stability against an enzyme tends to be more excellent. All of the first nucleotides may be sugar modified nucleotides in the translated region. Although not especially limited, when the first nucleotide is a sugar modified nucleotide, a substituent in the 2' position of the sugar portion of the first nucleotide is preferably fluorine.

In the polynucleotide of the present embodiment, at least one of the second nucleotides contained in the codons of the translated region may be a sugar modified nucleotide, but the sugar portion of the second nucleotide may not be modified. A ratio that the second nucleotides are sugar modified nucleotides may be 50% or less, 45% or less, 40% or less, 35% or less, 30% or less, 25% or less, 20% or less, 15% or less, 10% or less, 5% or less, or 0%. The ratio being 0% means that none of the second nucleotides are sugar modified nucleotides. Although not especially limited, when the second nucleotide is a sugar modified nucleotide, a substituent in the 2' position of the sugar portion of the second nucleotide is preferably fluorine.

In the polynucleotide of the present embodiment, at least one of the third nucleotides contained in the codons of the translated region may be a sugar modified nucleotide. A ratio that the third nucleotides are sugar modified nucleotides may be 100%, 90% or less, 80% or less, 70% or less, 60% or less, 50% or less, 45% or less, 40% or less, 35% or less, 30% or less, 25% or less, 20% or less, 15% or less, 10% or less, 5% or less, or 0%.

In the polynucleotide of the present embodiment, from the viewpoint of improving translation activity, the first, second and third nucleotides of the stop codon may be sugar modified nucleotides. All of the first nucleotides, and all the nucleotides of the stop codon may be sugar modified nucleotides in the translated region.

In the polynucleotide of the present embodiment, from the viewpoint of improving the stability against a nuclease, the first, second and third nucleotides of the start codon may be sugar modified nucleotides. Although not especially limited, substituents in the 2' position of the sugar portions of the first, second and third nucleotides of the start codon are preferably all fluorine.

In the polynucleotide of the present embodiment, the first nucleotides of all the codons excluding the stop codon may be sugar modified nucleotide. Although not especially limited, substituents in the 2' position of the sugar portions of the first nucleotides of all the codons excluding the stop codon are preferably all fluorine.

The translated region may contain a base modified nucleotide. The position of the base modified nucleotide in the translated region is not especially limited.

The translated region may contain a phosphate modified nucleotide. The position of the phosphate modified nucleotide in the translated region is not especially limited, and a phosphate group linking between the first nucleotide and the second nucleotide of the codon is preferably a phosphorothioate bond.

### <5' Untranslated Region>

The polynucleotide of the present embodiment comprises a 5' untranslated region (5' UTR). The 5' untranslated region is a region that is present upstream (on the 5' end side) of the translated region, and is not translated for polypeptide synthesis. The number of nucleotides contained in the 5' untranslated region is preferably 1 or more, and may be 6 or more. The number of nucleotides contained in the 5' untranslated region is preferably 1000 or less, and may be 500 or less, 250 or less, or 100 or less.

The number of nucleotides contained in the 5' untranslated region may be a number within an optional range selected based on the upper and lower limits described above, and is preferably an integer of 1 to 1000, more preferably an integer of 1 to 500, further preferably an integer of 6 to 250, and particularly preferably an integer of 6 to 100.

In the polynucleotide of the present embodiment, the 5' untranslated region and the translated region are linked in the stated order.

The 5' untranslated region may contain a 2'-deoxyribonucleotide, and a spacer modified or sugar modified nucleotide.

The positions of these nucleotides are not especially limited in the 5' untranslated region.

From the viewpoint of improving translation activity, the first, second, and third nucleotides from the 5' end may be sugar modified nucleotide, and the first to sixth nucleotides from the 5' end are preferably all sugar modified nucleotides.

All the nucleotides of the 5' untranslated region may be sugar modified nucleotides. In the sugar modified nucleotide, a substituent in the 2' position of the sugar portion is preferably a methoxyethoxy group (OCH₂CH₂OCH₃) or fluorine (F) .

In one embodiment of the present invention, a polynucleotide comprises a translated region from a start codon to a stop codon, a 5' untranslated region, and a poly A chain, and nucleotides of the 5' untranslated region are each independently selected from a 2'-deoxyribonucleotide, and a spacer modified or sugar modified nucleotide.

In the present invention, the polynucleotide exhibits excellent translation potential because the nucleotides of the 5' untranslated region are each independently selected from a 2'-deoxyribonucleotide, and a spacer modified or sugar modified nucleotide.

When the nucleotides of the 5' untranslated region include a 2'-deoxyribonucleotide, and a spacer modified or sugar modified nucleotide, a sugar modified nucleotide is preferably included.

The polynucleotide of the present embodiment encompasses one in which an appropriate non-sugar modified nucleotide having a length of 1 to 10 bases is added to the original 5' end.

### (5' Cap Structure)

The polynucleotide of the present embodiment may further contain a 5' cap structure at the original 5' end. The 5' cap structure may be present in the form of being added to the 5' untranslated region. When the 5' cap structure is contained, translation activity tends to be improved.

The 5' cap structure of the present invention refers to the following structure in which a triphosphoric acid structure is added to 7-methylguanylic acid (m7G).

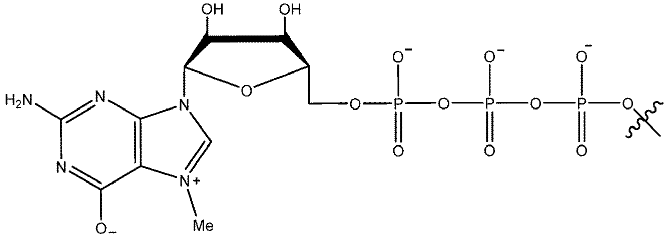

As the 5' cap structure, not only the above-described 7-methylguanylic acid (m7G) cap but also 5' cap analogues disclosed in the following papers can be used.
- ARCA: RNA, Vol. 7, p. 1486-1495 (2001), Cell Cycle, Vol. 17, No. 13, p. 1624-1636 (2018);
- LNA: Journal of American Chemical Society, Vol. 131, No. 18, p. 6364-6365 (2009);
- S-modified Cap: RNA, Vol. 14, p. 1119-1131 (2008); and
- Nature Reviews Drug Discovery, Vol. 13, p. 759-780 (2014).

The 5' untranslated region may contain a base modified nucleotide. The position of the base modified nucleotide in the 5' untranslated region is not especially limited. The base modified nucleotide may be a sugar modified nucleotide and/or a phosphate modified nucleotide (in other words, the base modified nucleotide may further contain a modified sugar portion and/or a modified phosphate portion).

Although not especially limited, from the viewpoint of improving translation activity, the 5' untranslated region preferably contains the following modified base portion: 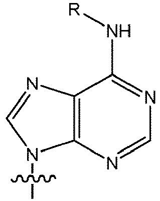 wherein R is an alkyl group having 1 to 6 carbon atoms.

The alkyl group R of the modified base portion is preferably methyl or ethyl.

The 5' untranslated region may contain a phosphate modified nucleotide. The position of the phosphate modified nucleotide in the 5' untranslated region is not especially limited. The phosphate modified nucleotide may be a sugar modified nucleotide and/or a base modified nucleotide (in other words, the phosphate modified nucleotide may further contain a modified sugar portion and/or a modified base portion).

The 5' untranslated region may contain a 2'-deoxyribonucleotide or spacer modification. The position of the 2'-deoxyribonucleotide or spacer modification in the 5' untranslated region is not especially limited, and it is preferable that a nucleotide in an optional position excluding the first to sixth nucleotides from the 5' end contains a 2'-deoxyribonucloetide or spacer modification.

In the present embodiment, it is preferable that spacer modification is not contained in the translated region.

### (Spacer Modification)

The spacer modification contained in the 5' untranslated region is not especially limited as long as it is a structure not containing a base portion and used as a replacement of a nucleotide, and examples include the following structures: 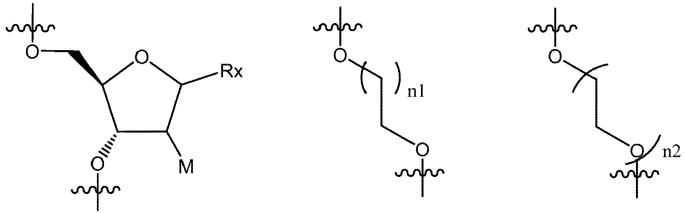 wherein Rx is alkyl having 1 to 6 carbon atoms, alkenyl having 1 to 6 carbon atoms, alkynyl having 1 to 6 carbon atoms, a hydrogen atom, or OH, M is R¹, OR¹, R²OR¹, OR²OR¹, SH, SR¹, NH₂, NHR¹, NR¹₂, N₃, a hydrogen atom, OH, CN, F, Cl, Br, or I, X is O, S, or NR¹, R¹ each independently is alkyl or aryl, preferably alkyl having 1 to 6 carbon atoms, and more preferably alkyl having 1 to 3 carbon atoms, R² is alkylene, and preferably alkylene having 1 to 6 carbon atoms, and each of n1 and n2 is an integer of 1 to 10.

As the spacer modification, in the leftmost structure, an oxygen atom of a five-membered ring may be substituted with NH.

Structures used as the spacer modification are disclosed in the following papers:
- M. Takeshita, C.N. Chang, F. Johnson, S. Will, and A.P. Grollman, J. Biol. Chem., 1987, 262, 10171-10179
- M.W. Kalnik, C.N. Chang, A.P. Grollman, and D.J. Patel, Biochemistry, 1988, 27, 924-931
- I.G. Shishkina and F. Johnson, Chem Res Toxicol, 2000, 13, 907-912
- K. Groebke, and C.J. Leumann, Helv Chim Acta, 1990, 73, 608-617
- T. Kuboyama, M. Nakahara, M. Yoshino, Y. Cui, T. Sako, Y. Wada, T. Imanishi, S. Obika, Y. Watanabe, M. Suzuki, H. Doi, Bioorg. Med. Chem. 2011, 19, 249-255
- M. Salunkhe, T.F. Wu, and R.L. Letsinger, J. Amer. Chem. Soc., 1992, 114, 8768-8772

The spacer modification is not especially limited, and is preferably any one of the following structures: 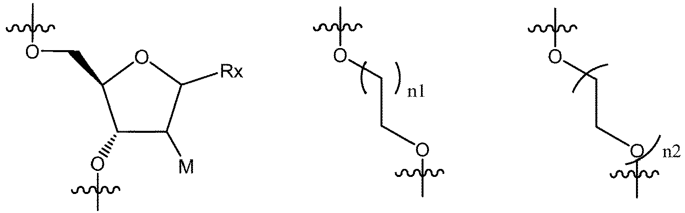 wherein Rx is ethynyl, a hydrogen atom, or OH, M is a hydrogen atom or OH, n1 is 1, 2, or 5, and n2 is 1, 2, or 3.

### <Poly A Chain>

The polynucleotide of the present embodiment comprises a poly A chain.

In the poly A chain in one aspect of the present embodiment, 65% or more of nucleotides contained therein are sugar modified nucleotides. The poly A chain is contained in the 3' untranslated region.

In the present embodiment, at least one or more poly A chains are contained in the 3' untranslated region.

A poly A chain is a polyadenylic acid containing two or more AMPs.

An AMP used in the present application encompasses a nucleotide corresponding to an AMP (including, for example, a sugar modified nucleotide of an AMP, a 2'-deoxyribonucloetide of an AMP, a phosphate modified nucleotide of an AMP, and a base modified nucleotide of an AMP). Hereinafter, in the present application, an AMP and a nucleotide corresponding to an AMP are together referred to as an AMP.

The poly A chain may contain a ribonucleotide except for an AMP (such as a CMP, a GMP, a UMP, or a nucleotide corresponding thereto) as long as it has a polyadenylic acid structure containing two or more AMPs. When the poly A chain contains a ribonucleotide except for an AMP, a nucleotide at the 5' end of the poly A chain is understood as an AMP corresponding to a starting point of a sequence in which the two or more AMPs are consecutively contained.

When the poly A chain contains a ribonucleotide except for an AMP, a ratio of the ribonucleotide except for an AMP among nucleotides contained in the poly A chain is 40% or less, 30% or less, 20% or less, or 10% or less, and is preferably 30% or less, more preferably 20% or less, and further preferably 10% or less.

In the polynucleotide of the present embodiment, 65% or more of nucleotides contained in the poly A chain are neither ribonucleotides nor 2'-deoxyribonucleotides.

A case in which a poly A chain contains a ribonucleotide except for an AMP is disclosed in, for example, Nature Medicine, Vol. 23, No. 7, p. 815-817 (2017), Science, Vol. 361, p. 701-704 (2018), and RNA, Vol. 25, p. 507-518 (2019).

As the poly A chain herein, a sequence in which regions including two or more consecutive AMPs present in two or more portions are linked to each other via an optional linker also means a poly A chain. Examples of the linker include a polyethylene glycol, a polypeptide, and an alkyl chain, but are not especially limited. For example, International Publication No. WO2016/011306 discloses a method for linking nucleotides via a specific linker.

In one aspect of the present embodiment, the poly A chain may contain a 2'-deoxyribonucleotide, or a spacer modified or sugar modified nucleotide.

The position of such a nucleotide in the 3' untranslated region is not especially limited.

The poly A chain of this aspect may not contain an AMP, but the description of the poly A chain of the above-described aspect is also applicable.

The poly A chain may contain a 2'-deoxyribonucleotide, or a spacer modified or sugar modified nucleotide in an amount of 65% or more, 70% or more, 80% or more, 90% or more, 95% or more, or 100%, and it is preferable that the poly A chain contains a 2'-deoxyribonucleotide, or a spacer modified or sugar modified nucleotide.

When the nucleotides of the poly A chain include a 2'-deoxyribonucleotide, or a spacer modified or sugar modified nucleotide, a sugar modified nucleotide is preferably included. When the poly A chain contains a 2'-deoxyribonucleotide, or a spacer modified or sugar modified nucleotide, the sugar modified nucleotide can occupy 65% or more of the nucleotides contained in the poly A chain.

### (3' Untranslated Region)

The 3' untranslated region (3' UTR) is a region that is present downstream (on the 3' end side) of the translated region, and is not translated for polypeptide synthesis. The number of nucleotides contained in the 3' untranslated region is preferably an integer of 2 to 6000, more preferably an integer of 2 to 3000, further preferably an integer of 2 to 1000, and particularly preferably an integer of 2 to 500.

A region except for the poly A chain in the 3' untranslated region may contain optional nucleotides, the respective nucleotides in the region except for the poly A chain in the 3' untranslated region may be unmodified nucleotides, or modified nucleotides.

In the polynucleotide of the present embodiment, the translated region and the 3' untranslated region are linked to each other in the stated order.

The poly A chain has a length of preferably 2 to 500 bases, more preferably 2 to 200 bases, further preferably 2 to 80 bases, further preferably 2 to 40 bases, further preferably 3 to 40 bases, further preferably 5 to 40 bases, further preferably 10 to 40 bases, and particularly preferably 20 to 40 bases.

In the poly A chain, 65% or more of nucleotides contained therein are sugar modified nucleotides. The position of the sugar modified nucleotide is not especially limited in the poly A chain.

The ratio of sugar modified nucleotides in the poly A chain is preferably 65% or more, 70% or more, 75% or more, 80% or more, 85% or more, 90% or more, 95% or more, or 100%. The ratio being 100% means that all the nucleotides in the poly A chain are sugar modified nucleotides.

When the poly A chain contains a 2'-deoxyribonucleotide, or a spacer modified or sugar modified nucleotide, a sugar modified nucleotide is preferably contained.

When the poly A chain contains a 2'-deoxyribonucleotide, or a spacer modified or sugar modified nucleotide, it is preferable that sugar modified nucleotides occupy 50% or more, 2'-deoxyribonucleotides occupy 30% or less, and spacer modification occupies 20% or less of nucleotides contained in the poly A chain.

When the poly A chain contains a 2'-deoxyribonucleotide, and a sugar modified nucleotide, it is preferable that sugar modified nucleotides occupy 50% or more and 2'-deoxyribonucleotides occupy 50% or less of the nucleotides contained in the poly A chain.

When the poly A chain contains spacer modified and sugar modified nucleotides, it is preferable that sugar modified nucleotides occupy 80% or more and spacer modification occupies 20% or less of the nucleotides contained in the poly A chain.

From the viewpoint of improving translation activity, the first, second and third nucleotides from the 3' end of the 3' untranslated region may be sugar modified nucleotides. Although not especially limited, a substituent in the 2' position of the sugar portion of the first, second and third nucleotides from the 3' end is preferably a methoxyethoxy group (OCH₂CH₂OCH₃).

Specific examples of modified sugar portions of the sugar modified nucleotides are preferably each independently selected, for example, from the following structures: 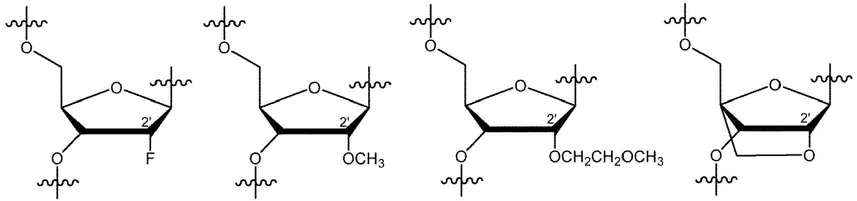 The modified sugar portions are preferably each independently selected from the following structures:

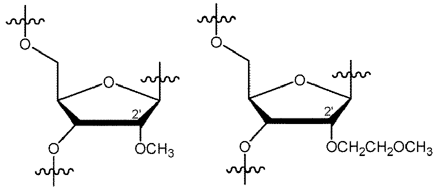

The poly A chain may contain a base modified nucleotide. The position of the base modified nucleotide in the poly A chain is not especially limited. The base modified nucleotide may be a sugar modified nucleotide and/or a phosphate modified nucleotide (in other words, the base modified nucleotide may further contain a modified sugar portion and/or a modified phosphate portion).

The 3' untranslated region may contain a 2'-deoxyribonucleotide or spacer modification preferably in the 3' untranslated region except for the poly A chain. Examples of a specific structure of the spacer modification include those described above in the section of (Spacer Modification) of (5' Untranslated Region).

The polynucleotide of the present embodiment encompasses one in which an appropriate non-sugar modified nucleotide having a length of 1 to 10 bases is added to the original 3' end.

The poly A chain may contain a phosphate modified nucleotide. The position of the phosphate modified nucleotide in the poly A chain is not especially limited. The phosphate modified nucleotide may be a sugar modified nucleotide and/or a base modified nucleotide (in other words, the phosphate modified nucleotide may contain a modified sugar portion and/or a modified base portion).

A modified phosphate portion contained in the poly A chain is preferably phosphorothioate. The position in the polymer A chain of a nucleotide linked via phosphorothioate is preferably consecutive from the 3' end side.

Among phosphate bonds in the poly A chain, a ratio of nucleotides linked via phosphorothioate is 10% or more, 20% or more, 30% or more, 40% or more, 50% or more, 60% or more, 70% or more, 80% or more, 90% or more, or 100% or more, and is preferably 50% or more, more preferably 80% or more, and particularly preferably 100%. The ratio being 100% means that all the nucleotides in the poly A chain are linked to one another via phosphorothioate.

Since the phosphate modified nucleotide can impart stability against endonuclease, that is, one of nucleases, two or more phosphate modified nucleotides are preferably consecutively contained from the 5' end and/or the 3' end of the polynucleotide of the present invention.

### (Linking Portion)

The polynucleotide of the present embodiment may contain the following linking portion: 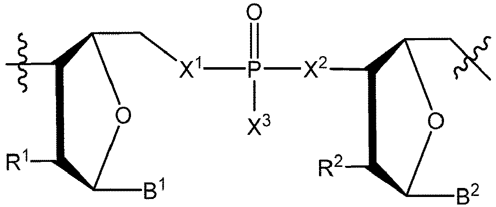 wherein R¹ and R² are each independently H, OH, F, OCH₂CH₂OCH₃, or OCH₃, B¹ and B² are each independently a base portion, X¹ is O, S or NH, and X² is O, S, NH or the following structure: 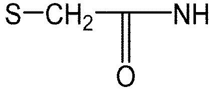 wherein X³ is OH, SH or a salt thereof (wherein OH and SH of X³ may be indicated respectively as O⁻ and S⁻), and X¹ and X² are not simultaneously O.

The base portion may be an unmodified base portion, or a modified base portion.

Nucleotides disposed on the left side and the right side of the linking portion are two nucleotides contained in the polynucleotide of the present embodiment. Even when the linking portion is contained, translation activity can be retained. A nucleotide A on the right side (5' end side) and a nucleotide B on the left side (3' end side) of the linking portion, and a nucleotide C adjacent to the nucleotide B on the 3' end side and a nucleotide D adjacent to the nucleotide C on the 3' end side may not be modified.

Examples of salts of OH and SH of X³ in the linking portion include pharmaceutically acceptable salts. Examples of the pharmaceutically acceptable salts include an alkali metal salt, an alkaline earth metal salt, an ammonium salt, an organic amine salt, and an amino acid salt. Examples of the alkali metal salt include a sodium salt, a lithium salt, and a potassium salt. Examples of the alkaline earth metal salt include a calcium salt and a magnesium salt.

Specific examples of the linking portion include the following: 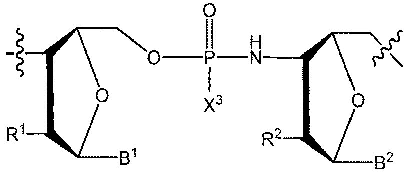 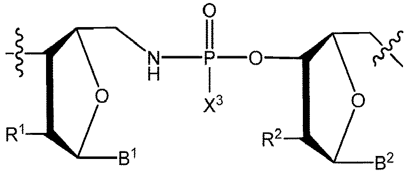 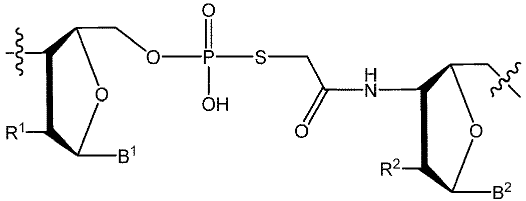 wherein R¹, R², B¹, B², and X³ are the same as those defined above.

The position of the linking portion is not especially limited. The linking portion may be present in any one of the translated region, the 5' untranslated region, and the 3' untranslated region (including the poly A chain), and when the linking portion is present, the linking portion is preferably present at least in the translated region.

The number of the linking portions is not especially limited, and can be appropriately selected in accordance with the length of the polynucleotide. The number of the linking portions can be, for example, 1 to 200, 1 to 100, 1 to 50, 1 to 20, 1 to 10, 1 to 8, 1 to 6, 1 to 4, 1 to 3, or 1 or 2.

In the polynucleotide of the present embodiment, the first nucleotide and the second nucleotide in at least one codon of the plurality of codons contained in the translated region may be linked to each other via phosphorothioate. The number of phosphorothioate bonds is not especially limited, and can be appropriately selected in accordance with the length of the polynucleotide. The number of phosphorothioate bonds can be, for example, 1 to 200, 1 to 100, 1 to 50, 1 to 20, 1 to 10, 1 to 8, 1 to 6, 1 to 4, 1 to 3, or 1 or 2.

From the viewpoint of improving translation activity, the first to second nucleotides, the first to third nucleotides, the first to fourth nucleotides, or the first to fifth nucleotides from the 5' end of the 5' untranslated region may be linked to one another via phosphorothioate. That the first to second nucleotides from the 5' end of the 5' untranslated region are linked to each other via phosphorothioate means that the first nucleotide and the second nucleotide from the 5' end of the 5' untranslated region are linked to each other via phosphorothioate, and for example, that the first to third nucleotides are linked to one another via phosphorothioate means that the first nucleotide and the second nucleotide are linked to each other via phosphorothioate, and in addition, the second nucleotide and the third nucleotide are linked to each other via phosphorothioate. When the first to third nucleotides are linked to one another via phosphorothioate, structures on the 5' side of the first nucleotide and the 3' side of the third nucleotide may be optional.

From the viewpoint of improving translation activity, the first to second nucleotides, the first to third nucleotides, the first to fourth nucleotides, or the first to fifth nucleotides from the 3' end of the 3' untranslated region may be linked to one another via phosphorothioate.

From the viewpoint of improving translation activity, the first to second nucleotides, the first to third nucleotides, the first to fourth nucleotides, or the first to fifth nucleotides from the 3' end of the poly A chain may be linked to one another via phosphorothioate. Besides, all the nucleotides of the poly A chain may be linked to one another via phosphorothioate.

Another embodiment of the present invention relates to a polynucleotide in which the first, second and third nucleotides from the 5' end of the 5' untranslated region are sugar modified nucleotides.

Another embodiment of the present invention relates to a polynucleotide in which the first, second and third nucleotides from the 3' end of a poly A chain are sugar modified nucleotides.

Another embodiment of the present invention relates to a polynucleotide in which the first, second and third nucleotides from the 5' end of the 5' untranslated region are sugar modified nucleotides, and the first, second and third nucleotides from the 3' end of the poly A chain are sugar modified nucleotides.

Another embodiment of the present invention relates to a polynucleotide in which the first to third nucleotides, the first to fourth nucleotides, or the first to fifth nucleotides from the 5' end of the 5' untranslated region are linked to one another via phosphorothioate.

Another embodiment of the present invention relates to a polynucleotide in which the first to third nucleotides, the first to fourth nucleotides, or the first to fifth nucleotides from the 3' end of the poly A chain are linked to one another via phosphorothioate.

Another embodiment of the present invention relates to a polynucleotide in which the first to third nucleotides, the first to fourth nucleotides, or the first to fifth nucleotides from the 5' end of the 5' untranslated region are linked to one another via phosphorothioate, and the first to third nucleotides, the first to fourth nucleotides, or the first to fifth nucleotides from the 3' end of the poly A chain are linked to one another via phosphorothioate.

Another embodiment of the present invention relates to a polynucleotide in which the first, second and third nucleotides from the 5' end of the 5' untranslated region are sugar modified nucleotides, and the first to third nucleotides, the first to fourth nucleotides, or the first to fifth nucleotides from the 5' end of the 5' untranslated region are linked to one another via phosphorothioate.

Another embodiment of the present invention relates to a polynucleotide in which the first, second and third nucleotides from the 3' end of the poly A chain are sugar modified nucleotides, and the first to third nucleotides, the first to fourth nucleotides, or the first to fifth nucleotides from the 3' end of the poly A chain are linked to one another via phosphorothioate.

Another embodiment of the present invention relates to a polynucleotide in which the first, second and third nucleotides from the 5' end of the 5' untranslated region are sugar modified nucleotides, the first to third nucleotides, the first to fourth nucleotides, or the first to fifth nucleotides from the 5' end of the 5' untranslated region are linked to one another via phosphorothioate, the first, second and third nucleotides from the 3' end of the poly A chain are sugar modified nucleotides, and the first to third nucleotides, the first to fourth nucleotides, or the first to fifth nucleotides from the 3' end of the poly A chain are linked to one another via phosphorothioate.

In the present invention, although exemplified aspects and favorable aspects are described in the description of each of the 5' untranslated region, the translated region, and the poly A chain, the 5' untranslated region, the translated region, and the poly A chain may be present in the form of any optional combination of these aspects, or any optional combination of the favorable aspects may be employed for any one or two of the 5' untranslated region, the translated region, and the poly A chain. Besides, for regions except for those described as the 5' untranslated region, the translated region, and the poly A chain, the exemplified aspects and the favorable aspects may be appropriately combined.

Specifically, herein, all combinations of the exemplified aspects and the favorable aspects given in each description of the 5' untranslated region, the translated region, and the poly A chain are described and exemplified as aspects herein.

### (Other Sequences)

The polynucleotide of the present embodiment may further contain a Kozak sequence and/or a ribosome binding sequence (RBS).

### <Method for Producing Polynucleotide>

The polynucleotide of the present embodiment can be produced by, for example, chemical synthesis. Specifically, the polynucleotide of the present embodiment can be produced by a known chemical synthesis method by introducing a prescribed sugar modified nucleotide into a prescribed position with elongating a polynucleotide chain. Examples of the known chemical synthesis method include a phosphoramidite method, a phosphorothioate method, a phosphotriester method, and a CEM method (see Nucleic Acids Research, 35, 3287 (2007)). In addition, an ABI3900 high-throughput nucleic acid synthesizer (manufactured by Applied Biosystems, Inc.) can be utilized.

More specifically, the known chemical synthesis method can be a method described in any of the following literatures:
- Tetrahedron, Vol. 48, No. 12, p. 2223-2311 (1992);
- Current Protocols in Nucleic Acids Chemistry, John Wiley & Sons (2000);
- Protocols for Oligonucleotides and Analogs, Human Press (1993);
- Chemistry and Biology of Artificial Nucleic Acids, Wiley-VCH (2012);
- Genome Chemistry Jinko Kakusan wo Katsuyo suru Kagakuteki Approach (Scientific approach for utilizing artificial nucleic acids), Kodansha Ltd. (2003); and
- New Trend of Nucleic Acid Chemistry, Kagaku-Dojin Publishing Company, Inc. (2011).

The polynucleotide of the present embodiment can be produced by chemically synthesizing a commercially unavailable phosphoramidite to be used as a raw material.

A method for synthesizing phosphoramidite (f) to be used as a raw material of a base modified nucleotide is as follows:

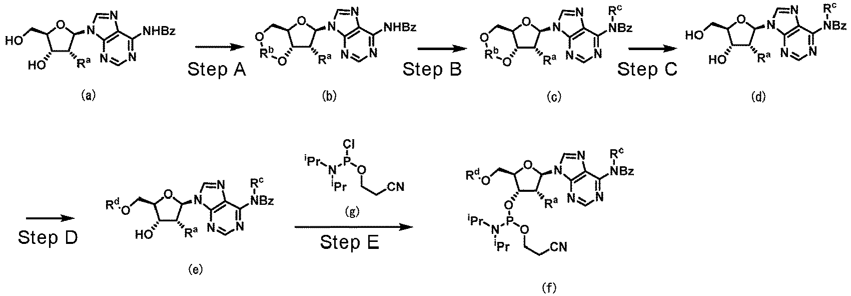

In this synthetic scheme, Ra is a hydrogen atom, F, OCH₂CH₂OCH₃, or OCH₃, Rb is a protecting group removable with a fluoride ion such as di-tert-butylsilyl, Rc is alkyl having 1 to 6 carbon atoms, and Rd is a protecting group used in nucleic acid solid phase synthesis, and is, for example, a p,p'-dimethoxytrityl group.

### (Step A)

A compound (b) can be produced by reacting a compound (a) and, for example, a corresponding silylating agent in a solvent in the presence of a base at a temperature between 0°C and 80°C for 10 minutes to 3 days.

Examples of the solvent include DMF, DMA, and NMP, and one of these or a mixture of these can be used.

Examples of the base include imidazole, triethylamine, and diisopropylethylamine.

An example of the silylating agent includes di-tert-butylsilyl bis(trifluoromethanesulfonate).

### (Step B)

A compound (c) can be produced by reacting the compound (b) and a corresponding alkylating agent in a solvent in the presence of a base at a temperature between 0°C and 150°C for 10 minutes to 3 days. The reaction can be accelerated by adding an adequate additive.

Examples of the solvent include DMF, pyridine, dichloromethane, THF, ethyl acetate, 1,4-dioxane, and NMP, and one of these or a mixture of these is used.

Examples of the base include a sodium hydroxide aqueous solution, potassium carbonate, pyridine, triethylamine, and N-ethyl-N,N-diisopropylamine.

Examples of the alkylating agent include methyl iodide, ethyl iodide, and methyl bromide.

An example of the additive includes tetrabutylammonium bromide.

### (Step C)

A compound (d) can be produced by reacting the compound (c) and a fluorine reagent in a solvent at a temperature between -80°C and 200°C for 10 seconds to 72 hours. At this point, a base can be also added.

Examples of the fluorine reagent include hydrogen fluoride, triethylamine hydrofluoride, and tetrabutylammonium fluoride (TBAF).

Examples of the base include triethylamine, and N,N-diisopropylethylamine.

Examples of the solvent include dichloromethane, chloroform, acetonitrile, toluene, ethyl acetate, THF, 1,4-dioxane, DMF, N,N-dimethylacetamide (DMA), NMP, and dimethylsulfoxide (DMSO).

### (Step D)

A compound (e) can be produced by reacting the compound (d) and a corresponding alkylating agent in a solvent in the presence of a base at a temperature between 0°C and 150°C for 10 minutes to 3 days. The reaction can be accelerated by an adequate activator.

Examples of the solvent include DMF, pyridine, dichloromethane, THF, ethyl acetate, 1,4-dioxane, and NMP, and one of these or a mixture of these is used.

Examples of the base include pyridine, triethylamine, N-ethyl-N,N-diisopropylamine, and 2,6-lutidine.

Examples of the alkylating agent include tritylchloride, and p,p'-dimethoxytritylchloride.

An example of the activator includes 4-dimethylaminopyridine.

### (Step E)

A compound (f) can be produced by reacting the compound (e) and a compound (g) in a solvent in the presence of a base at a temperature between 0°C and 100°C for 10 seconds to 24 hours.

Examples of the solvent include dichloromethane, acetonitrile, toluene, ethyl acetate, THF, 1,4-dioxane, DMF and NMP, and one of these or a mixture of these is used.

Examples of the base include triethylamine, N,N-diisopropylethylamine, and pyridine, and one of these or a mixture of these is used.

The 5' cap structure can be introduced by a known method (such as an enzymatic method or a chemical synthesis method). Examples of the known method include methods described in Top. Curr. Chem. (Z) (2017) 375:16 and Beilstein J. Org. Chem. 2017, 13, 2819-2832.

When the base length of the polynucleotide of the present embodiment is long, a plurality of polynucleotide units may be linked to one another. A linking method is not especially limited, and examples include an enzymatic method and a chemical synthesis method.

Linking by an enzymatic method can be, for example, linking with a ligase. Examples of the ligase include T4 DNA ligase, T4 RNA ligase 1, T4 RNA ligase 2, T4 RNA ligase 2, truncated T4 RNA ligase 2, truncated KQ, *E. Coli* DNA ligase, and Taq. DNA ligase, and one of these or a mixture of these can be used. In the enzymatic method, it is generally preferable that a nucleotide A at the 3' end of a polynucleotide unit contained on the 5' end side of a polynucleotide (hereinafter referred to as the "polynucleotide unit on the 5' end side"), a nucleotide B at the 5' end of a polynucleotide unit contained on the 3' end side of the polynucleotide (hereinafter referred to as the "polynucleotide unit on the 3' end side") (the nucleotides A and B being adjacent to each other in the linked polynucleotide), a nucleotide C adjacent to the nucleotide B, and a nucleotide D adjacent to the nucleotide C are not modified. On the other hand, the nucleotides A to D may be modified if T4 RNA ligase 2 or the like described in Molecular Cell, Vol. 16, 211-221, October 22, 2004 is used.

In the linking by the enzymatic method, polydisperse polyethylene glycol (PEG) may be used for accelerating the linking reaction by molecular crowding effect. Examples of the polydisperse PEG include PEG 4000, PEG 6000, PEG 8000, and PEG 10000, and one of these or a mixture of these can be used.

Linking by a chemical synthesis method (also referred to as "chemical ligation") can be, for example, the following method in which the 3' end (on the right side in the following) of a polynucleotide unit on the 5' end side and the 5' end (on the left side in the following) of a polynucleotide unit on the 3' end side are condensed in the presence of a condensing agent: 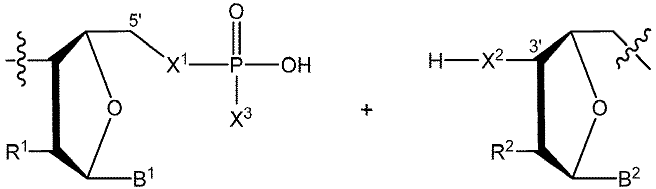 wherein R¹, R², B¹, B², and X³ are the same as those defined above, X¹ is O, S, or NH, and X² is O, S, NH, or the following structure:

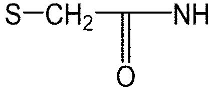

Examples of the condensing agent include 1,3-dicyclohexanecarbodiimide (DCC), 1-ethyl-3-(3-dimethylaminopropyl)carbodiimide hydrochloride (EDC), carbonyldiimidazole, benzotriazole-1-yloxytris(dimethylamino)phosphonium hexafluorophosphate, (benzotriazole-1-yloxy)tripyrrolidinophosphonium hexafluorophosphate, O-(7-azabenzotriazole-1-yl)-N,N,N',N'-tetramethyluronium hexafluorophosphate (HATU), O-(benzotriazole-1-yl)-N,N,N',N'-tetramethyluronium hexafluorophosphate (HBTU), 2-chloro-1-methylpyridinium iodide, 1H-imidazole-1-carbonitrile, 1-cyano-1H-benzimidazole, and 1-cyano-1H-benzotriazole.

The condensation reaction is performed preferably in the presence of a template DNA containing nucleotide chains complementary to a nucleotide chain on the 3' end side of the polynucleotide unit on the 5' end side and a nucleotide chain on the 5' end side of the polynucleotide unit on the 3' end side. The template DNA is preferably a nucleotide chain complementary to a nucleotide chain of preferably 2-50 base length, and more preferably 5-40 base length from the 3' end of the polynucleotide unit on the 5' end side, and to a nucleotide chain of preferably 2-50 base length, and more preferably 5-40 base length from the 5' end of the polynucleotide unit on the 3' end. Here, the term "complementary" means that base sequence identity is, for example, 50% or more, 60% or more, 70% or more, 80% or more, 90% or more, or 100%.

In the condensation reaction, an additive may be added. Examples of the additive include 1-hydroxybenzotriazole (HOBt), and 4-dimethylaminopyridine (DMAP).

In the condensation reaction, a metal salt may be added. Examples of the metal salt include zinc (II) chloride, zinc (II) bromide, zinc (II) acetate, nickel (II) chloride, and manganese (II) chloride.

The condensation reaction may be performed in the presence of a buffer. Examples of the buffer include acetate buffer, Tris buffer, citrate buffer, phosphate buffer, and water.

The temperature in the condensation reaction is not especially limited, and may be, for example, room temperature to 200°C. The time of the condensation reaction is not especially limited, and may be, for example, 5 minutes to 100 hours.

Specific examples of the condensation reaction between the 3' end (on the right side in the following) of the polynucleotide unit on the 5' end side and the 5' end (on the left side in the following) of the polynucleotide unit on the 3' end side include the following: 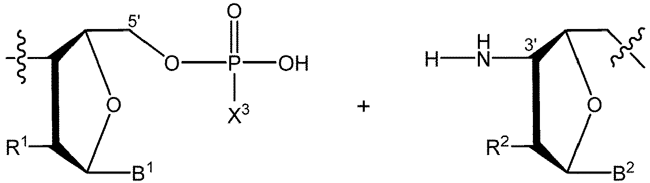 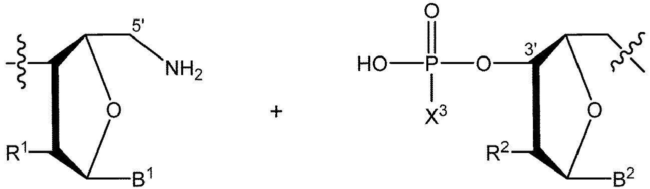 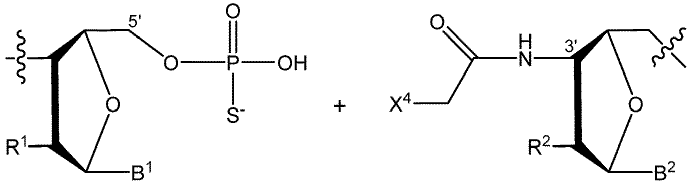 wherein R¹, R², B¹, B², and X³ are the same as those defined above, and X⁴ is a leaving group.

Specific examples of the leaving group include a chloro group, a bromo group, an iodo group, a methanesulfonyl group, a p-toluenesulfonyl group, and a trifluoromethanesulfonyl group. The leaving group is not especially limited, and is preferably a chloro group or a bromo group.

The linking of the polynucleotide units may be repeated a plurality of times in accordance with the length of the polynucleotide to be obtained. The number of times of the linking is not especially limited, and may be, for example, 1 to 200 times, 1 to 100 times, 1 to 50 times, 1 to 20 times, 1 to 10 times, 1 to 8 times, 1 to 6 times, 1 to 4 times, 1 to 3 times, or once or twice.

A method for producing a compound (M) and a compound (N), that is, the polynucleotide units on the 5' end side used in the linking is as follows: 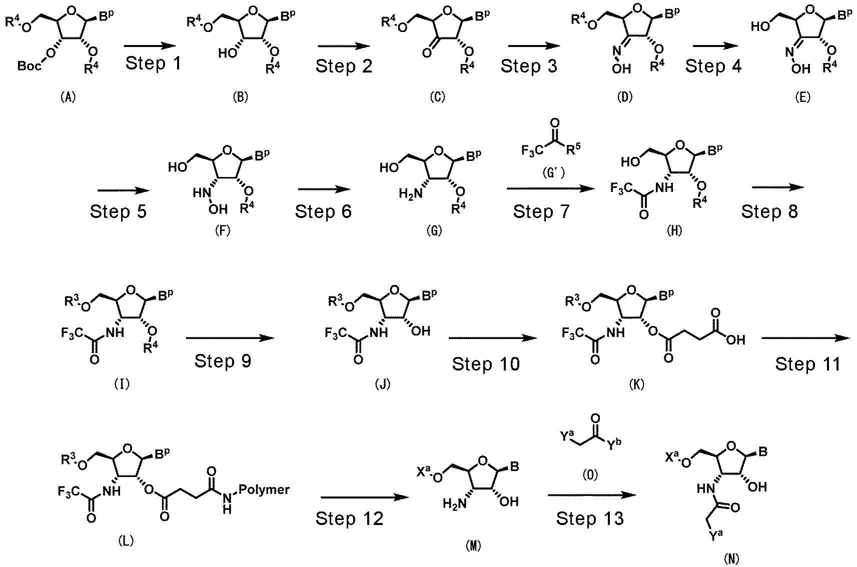 wherein BP is a base optionally protected by a protecting group, B is a base, and Polymer is a solid support. R⁴ is a protecting group selectively deprotectable, such as a tert-butyldimethylsilyl group or a triethylsilyl group, R³ is a protecting group used in nucleic acid solid phase synthesis, such as a p,p'-dimethoxytrityl group, X^{a} is a nucleic acid sequence, and Y^{a} and Y^{b} are each independently a leaving group, such as halogen, preferably a chlorine atom or a bromine atom. Herein, a nucleic acid sequence refers to a partial structure in a nucleic acid that forms the nucleic acid together with a compound bonded thereto. It is noted that if a plurality of Bs are contained in a molecule, these Bs may be the same or different.

### (Step 1)

A compound (B) can be produced by reacting a compound (A) in a solvent at a temperature between 60°C and a boiling point of the solvent to be used for 10 seconds to 3 days.

Examples of the solvent include toluene, xylene, 1,2-dichloroethane, 1,4-dioxane, N,N-dimethylformamide (DMF), N-methylpyrrolidone (NMP), 1,2-dichlorobenzene, and water, and one of these or a mixture of these can be used.

The compound (A) can be produced by a method described in, for example, J. Am. Chem. Soc. (1999), 121, 5661-5665.

BP in the compound (A) is not especially limited, and preferably has any one of the following structures: 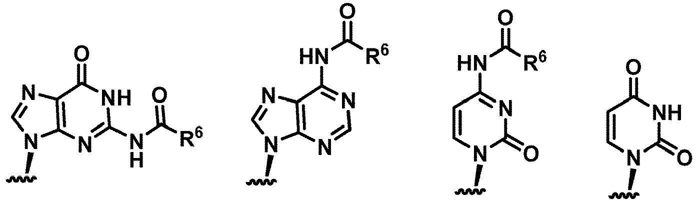 R⁶ is a group constituting a part of a protecting group of a base, and represents, for example, a methyl group, an isopropyl group, or a phenyl group optionally having a substituent. Examples of the substituent in the phenyl group optionally having a substituent include a methyl group, an isopropyl group, and a tert-butyl group.

### (Step 2)

A compound (C) can be produced by reacting the compound (B) in a solvent in the presence of 1 to 100 equivalents of an oxidant at a temperature between 0° and a boiling point of the solvent to be used for 10 seconds to 3 days preferably with 1 to 100 equivalents of an additive.

Examples of the solvent include aprotic solvents such as chloroform and dichloromethane, and one of these or a mixture of these can be used.

Examples of the oxidant include organic oxidants such as Jones reagent, chromic acid, pyridinium dichromate, ruthenium tetroxide, sodium chlorite, and Dess-Martin reagent, and inorganic oxidants such as pyridinium chlorochromate, and one of these or a mixture of these can be used.

Examples of the additive include pyridine, triethylamine, and N,N-diisopropylethylamine, and one of these or a mixture of these can be used.

### (Step 3)

A compound (D) can be produced by reacting the compound (C) in a solvent such as pyridine in the presence of hydroxylamine chloride at a temperature between 0°C and a boiling point of the solvent to be used for 10 seconds to 3 days.

### (Step 4)

A compound (E) can be produced by reacting the compound (D) in a solvent in the presence of 1 to 100000 equivalents of a deprotecting agent at a temperature between 0°C and a boiling point of the solvent to be used for 10 seconds to 3 days.

Examples of the solvent include toluene, xylene, and water, and one of these or a mixture of these can be used.

Examples of the deprotecting agent include trifluoroacetic acid, trichloroacetic acid, acetic acid, and hydrochloric acid, and one of these or a mixture of these can be used.

### (Step 5)

A compound (F) can be produced by reacting the compound (E) in a solvent in the presence of a reductant at a temperature between 0°C and a boiling point of the solvent to be used for 10 seconds to 3 days.

Examples of the solvent include trifluoroacetic acid, trichloroacetic acid, acetic acid, hydrochloric acid, toluene, xylene, toluene, xylene, tetrahydrofuran, methanol, ethanol, 1,4-dioxane, and water, and one of these or a mixture of these can be used.

Examples of the reductant include sodium borohydride, sodium cyanoborohydride, lithium borohydride, and sodium triacetoxyborohydride.

### (Step 6)

A compound (G) can be produced by reacting the compound (F) in a solvent in the presence of a catalyst under a hydrogen atmosphere at a temperature between 0°C and a boiling point of the solvent to be used for 10 seconds to 3 days.

Examples of the solvent include trifluoroacetic acid, acetic acid, dilute hydrochloric acid, methanol, ethanol, isopropanol, and water, and one of these or a mixture of these can be used.

Examples of the catalyst include palladium carbon and ruthenium carbon.

The compound (G) can be produced also by, for example, a method described in International Publication No. WO2017/123669.

### (Step 7)

A compound (H) can be produced by reacting the compound (G) in a solvent in the presence of 1 to 100 equivalents of a compound (G') and a base at a temperature between 0°C and a boiling point of the solvent to be used for 10 seconds to 3 days preferably with 1 to 1000 equivalents of the base.

Examples of the solvent include methanol, ethanol, isopropanol, dichloromethane, acetonitrile, toluene, ethyl acetate, tetrahydrofuran (THF), 1,4-dioxane, N,N-dimethylformamide (DMF), N-methylpyrrolidone (NMP), and water, and one of these or a mixture of these can be used.

Examples of the base include pyridine, triethylamine, N-ethyl-N,N-diisopropylamine, and 2,6-lutidine, and one of these or a mixture of these can be used.

As the compound (G'), a commercially available product can be used.

### (Step 8)

A compound (I) can be produced by reacting the compound (H) and p,p'-dimethoxytritylchloride in a solvent such as pyridine in the presence of a cosolvent if necessary at a temperature between 0°C and 100°C for 5 minutes to 100 hours.

Examples of the cosolvent include methanol, ethanol, dichloromethane, chloroform, 1,2-dichloroethane, toluene, ethyl acetate, acetonitrile, diethyl ether, tetrahydrofuran, 1,2-dimethoxyethane, dioxane, N,N-dimethylformamide (DMF), N,N-dimethylacetamide, N-methylpyrrolidone, triethylamine, N,N-diisopropylethylamine, and water, and one of these or a mixture of these can be used.

### (Step 9)

A compound (J) can be produced by reacting the compound (I) in a solvent at a temperature between 0°C and a boiling point of the solvent to be used for 10 minutes to 10 days with 1 to 10 equivalents of an additive.

Examples of the solvent include dichloromethane, acetonitrile, toluene, ethyl acetate, THF, 1,4-dioxane, DMF, DMA, and NMP, and one of these or a mixture of these can be used.

Examples of the additive include tetrabutylammonium fluoride and triethylamine trihydrofluoride, and one of these or a mixture of these can be used.

### (Step 10)

A compound (K) can be produced by reacting the compound (J) and succinic anhydride in a solvent in the presence of 1 to 30 equivalents of a base at a temperature between room temperature and 200°C for 5 minutes to 100 hours.

Examples of the solvent include methanol, ethanol, dichloromethane, chloroform, 1,2-dichloroethane, toluene, ethyl acetate, acetonitrile, diethyl ether, tetrahydrofuran, 1,2-dimethoxyethane, dioxane, N,N-dimethylformamide (DMF), N,N-dimethylacetamide, N-methylpyrrolidone, pyridine, and water, and one of these or a mixture of these can be used.

Examples of the base include cesium carbonate, potassium carbonate, potassium hydroxide, sodium hydroxide, sodium methoxide, potassium tert-butoxide, triethylamine, diisopropylethylamine, N-methylmorpholine, pyridine, 1,8-diazabicyclo[5.4.0]-7-undecene (DBU), and N,N-dimethyl-4-aminopyridine (DMAP), and one of these or a mixture of these can be used.

### (Step 11)

A compound (L) can be produced by reacting the compound (K) and a solid support having an aminized end in the absence of a solvent or in a solvent in the presence of 1 to 30 equivalents of a base, a condensing agent, and 0.01 to 30 equivalents of an additive if necessary at a temperature between room temperature and 200°C for 5 minutes to 100 hours, and then reacting the resultant in an acetic anhydride/pyridine solution at a temperature between room temperature and 200°C for 5 minutes to 100 hours.

Examples of the solvent include those mentioned as the examples in Step 4.

Examples of the base include cesium carbonate, potassium carbonate, potassium hydroxide, sodium hydroxide, sodium methoxide, potassium tert-butoxide, triethylamine, diisopropylethylamine, N-methylmorpholine, pyridine, 1,8-diazabicyclo[5.4.0]-7-undecene (DBU), and N,N-dimethyl-4-aminopyridine (DMAP), and one of these or a mixture of these can be used.

Examples of the condensing agent include 1,3-dicyclohexanecarbodiimide (DCC), 1-ethyl-3-(3-dimethylaminopropyl)carbodiimide hydrochloride (EDC), carbonyldiimidazole, benzotriazole-1-yloxytris(dimethylamino)phosphonium hexafluorophosphate, (benzotriazole-1-yloxy)tripyrrolidinophosphonium hexafluorophosphate, O-(7-azabenzotriazole-1-yl)-N,N,N',N'-tetramethyluronium hexafluorophosphate (HATU), O-(benzotriazole-1-yl)-N,N,N',N'-tetramethyluronium hexafluorophosphate (HBTU), and 2-chloro-1-methylpyridinium iodide.

Examples of the additive include 1-hydroxybenzotriazole (HOBt) and 4-dimethylaminopyridine (DMAP), and one of these or a mixture of these can be used.

The solid support is not especially limited as long as an aminized solid support known to be used in solid phase synthesis is used, and examples include solid supports such as CPG (controlled pore glass) modified with a long chain alkylamino group, and PS (polystyrene resin) .

For example, as long chain alkylamine controlled pore glass (LCAA-CPG), a commercially available product can be used.

### (Step 12)

A compound (M) can be produced by elongating a corresponding nucleotide chain with the compound (L) used by a known oligonucleotide chemical synthesis method, and then performing removal from a solid phase, deprotection of a protecting group, and purification.

For performing the removal from a solid phase and deprotection, after the oligonucleotide chemical synthesis, the resultant is treated with a base in a solvent or in the absence of a solvent at a temperature between -80°C and 200°C for 10 seconds to 72 hours.

Examples of the base include ammonia, methylamine, dimethylamine, ethylamine, diethylamine, isopropylamine, diisopropylamine, piperidine, triethylamine, ethylenediamine, 1,8-diazabicyclo[5.4.0]-7-undecene (DBU), and potassium carbonate, and one of these or a mixture of these can be used.

Examples of the solvent include water, methanol, ethanol, and THF, and one of these or a mixture of these can be used.

The purification of the oligonucleotide can be performed with a C18 reverse phase column or an anion exchange column, and preferably with a combination of the two methods described above.

A concentration of a nucleic acid complex obtained after the purification is preferably 90% or more, and more preferably 95% or more.

### (Step 13)

A compound (N) can be produced by causing a reaction using the compound (M) in a buffer in the presence of 1 to 1000 equivalents of a compound (O) at a temperature between room temperature and 100°C for 5 minutes to 100 hours.

Examples of the buffer include acetate buffer, Tris buffer, citrate buffer, phosphate buffer, and water, and one of these or a mixture of these can be used.

As the compound (O), a commercially available product can be used.

A method for producing a compound (W), that is, a polynucleotide unit on the 3' end side, to be used in the linking is as follows: 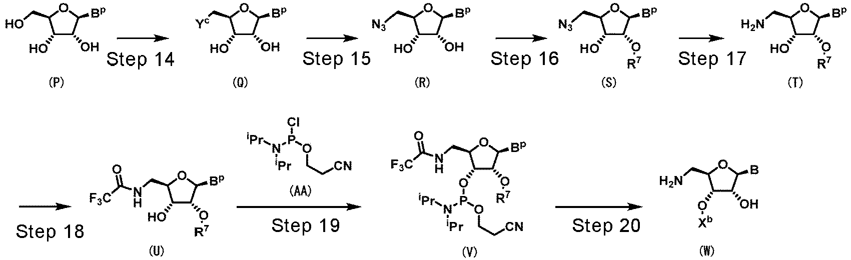 wherein BP is a base optionally protected by a protecting group, B is a base, R⁷ is a protecting group, such as a tert-butyldimethylsilyl group, or a triethylsilyl group, Yc is, for example, a chlorine atom, a bromine atom, or a tosylate group, and X^{b} is a nucleic acid sequence. If a plurality of B are contained in a molecule, these B may be the same or different.

### (Step 14)

A compound (Q) can be produced by reacting a compound (P) in a solvent in the presence of an additive and a base at a temperature between 0°C and a boiling point of the solvent to be used for 10 seconds to 3 days.

Examples of the solvent include a dichloromethane, acetonitrile, toluene, ethyl acetate, THF, 1,4-dioxane, DMF, DMA, and NMP, and one of these or a mixture of these can be used.

Examples of the additive include p-toluenesulfonic acid anhydride, tosyl chloride, thionyl chloride, and oxalyl chloride, and one of these or a mixture of these can be used.

Examples of the base include pyridine, triethylamine, N-ethyl-N,N-diisopropylamine, and potassium carbonate, and one of these or a mixture of these can be used.

As the compound (P), a commercially available product can be used.

### (Step 15)

A compound (R) can be produced by reacting the compound (Q) in a solvent in the presence of an azidizing agent, and a base if necessary, at a temperature between room temperature and a boiling point of the solvent to be used for 10 seconds to 3 days.

Examples of the solvent include dichloromethane, acetonitrile, toluene, ethyl acetate, THF, 1,4-dioxane, DMF, DMA, and NMP, and one of these or a mixture of these can be used.

An example of the azidizing agent includes sodium azide.

Examples of the base include pyridine, triethylamine, N-ethyl-N,N-diisopropylamine, and potassium carbonate, and one of these or a mixture of these can be used.

### (Step 16)

A compound (S) can be produced by reacting the compound (R) in a solvent in the presence of a silylating agent and a base at a temperature between room temperature and a boiling point of the solvent to be used for 10 seconds to 3 days.

Examples of the solvent include dichloromethane, acetonitrile, toluene, ethyl acetate, THF, 1,4-dioxane, DMF, DMA, and NMP, and one of these or a mixture of these can be used.

Examples of the silylating agent include tert-butyldimethylsilyl chloride, tert-butyldimethylsilyl triflate, and triethylsilyl chloride.

Examples of the base include pyridine, triethylamine, N-ethyl-N,N-diisopropylamine, potassium carbonate, potassium hydroxide, sodium hydroxide, sodium methoxide, potassium tert-butoxide, triethylamine, diisopropylethylamine, N-methylmorpholine, pyridine, 1,8-diazabicyclo[5.4.0]-7-undecene (DBU), and N,N-dimethyl-4-aminopyridine (DMAP), and one of these or a mixture of these can be used.

### (Step 17)

A compound (T) can be produced by reacting the compound (S) in a solvent with a reductant added at a temperature between room temperature and a boiling point of the solvent to be used for 10 seconds to 3 days.

Examples of the solvent include methanol, ethanol, dichloromethane, chloroform, 1,2-dichloroethane, toluene, ethyl acetate, acetonitrile, diethyl ether, tetrahydrofuran, 1,2-dimethoxyethane, dioxane, N,N-dimethylformamide (DMF), N,N-dimethylacetamide, N-methylpyrrolidone, triethylamine, N,N-diisopropylethylamine, acetic acid, and water, and one of these or a mixture of these can be used.

Examples of the reductant include sodium borohydride, sodium cyanoborohydride, lithium borohydride, sodium triacetoxyborohydride, and palladium carbon used in a hydrogen atmosphere.

### (Step 18)

A compound (U) can be produced with the compound (T) used in the same manner as in Step 7.

### (Step 19)

A compound (V) can be produced by reacting the compound (U) and a compound (AA) in a solvent in the presence of a base at a temperature between 0°C and 100°C for 10 seconds to 24 hours.

Examples of the solvent include dichloromethane, acetonitrile, toluene, ethyl acetate, THF, 1,4-dioxane, DMF and NMP, and one of these or a mixture of these can be used.

Examples of the base include triethylamine, N,N-diisopropylethylamine, and pyridine, and one of these or a mixture of these can be used.

As the compound (AA), a commercially available product can be used.

### (Step 20)

A compound (W) can be produced with the compound (V) used in the same manner as in Step 12.

When the polynucleotide of the present embodiment is produced by linking a plurality of polynucleotide units, some of the polynucleotide units may be a polynucleotide produced by IVT. A method for linking polynucleotides produced by IVT is not especially limited, and examples include the enzymatic method and the chemical synthesis method described above. An example of a method for producing a polynucleotide unit by IVT includes a method in which an RNA is transcribed from a template DNA having a promoter sequence by using an RNA polymerase. More specific examples of known IVT include methods described in the following literatures:
- RNA, Methods in Molecular Biology (Methods and Protocols), Vol. 703, Chapter 3 (2011);
- Cardiac Gene Therapy: Methods in Molecular Biology (Methods and Protocols), Vol. 1521, Chapter 8 (2016); and
- Journal of Molecular Biology, Vol. 249, p. 398-408 (1995).

Examples of the template DNA to be used in IVT include one produced by chemical synthesis, one produced by polymerase chain reaction, a plasmid DNA, and one produced by linearizing a plasmid DNA with a restriction enzyme, and one of these or a mixture of these can be used. Examples of the RNA polymerase include T3RNA polymerase, T7RNA polymerase, and SP6RNA polymerase, and one of these or a mixture of these can be used. Ribonucleoside triphosphate used in the transcription may be modified, or a mixture of a plurality of ribonucleoside triphosphates can be used. As described in Cardiac Gene Therapy: Methods in Molecular Biology (Methods and Protocols), Vol. 1521, Chapter 8 (2016), a compound such as m7G(5')ppp(5')G (manufactured by TriLink Biotechnologies, Catalog No. S1404) or Anti Reverse Cap Analog, 3'-O-Mem7G(5')ppp(5')G (manufactured by TriLink Biotechnologies, Catalog No. N-7003) can be used for imparting the 5' cap structure. As described in Journal of Molecular Biology, Vol. 249, p. 398-408 (1995), the 5' end or the 3' end of an RNA can be cut after the transcription by inserting a sequence of Hepatitis delta virus (HDV) ribosome or the like into the template DNA.

### <Pharmaceutical Composition>

One embodiment of the present invention relates to a pharmaceutical composition containing the polynucleotide. When the pharmaceutical composition of the present embodiment is administered to a patient having a disease, the polynucleotide is translated to synthesize a polypeptide encoded by the polynucleotide, and thus, the disease is treated.

Although not especially limited, a method for treating a disease characterized in that the function or activity of a specific protein is lost or abnormal by compensating the function or activity by the polypeptide translated from the polynucleotide is provided. Alternatively, a treatment method for artificially controlling immune response by causing a foreign antigen peptide and an analog thereof to express in a living body by the polypeptide translated from the polynucleotide is provided. Besides, the function, the differentiation, the growth and the like of a cell can be artificially controlled and modified by causing, by the polypeptide translated from the polynucleotide, a specific protein present in a living body such as a transcription factor, or a polypeptide essentially not present in a living body to express in a living body, and thus, a treatment method, for a disease characterized in that a tissue or a cell is damaged, or is deteriorated or becomes abnormal in the function or activity, for recovering the function of the tissue or cell is also provided.

The disease is not especially limited, and examples include cancers and proliferative diseases, infectious diseases and parasitic diseases, diseases of blood and hematopoietic organs, autoimmune disease, diseases of internal secretion, nutrient, and metabolism (including inborn error of metabolism), mental and nervous system diseases, diseases of the skin and subcutaneous tissues, eye disease, ear disease, respiratory system diseases, digestive system diseases, diseases of the kidney, the urinary tract and the reproductive system, cardiovascular diseases, cerebrovascular diseases, diseases of the musculoskeletal system and connective tissues, spontaneous abortion, perinatal disorders, congenital malformation abnormality, acquired injuries, and addiction.

The pharmaceutical composition may be administered in a prescribed formulation form. An example of the formulation includes a liquid dosage form for oral administration or parenteral administration, and examples of the liquid dosage form include a pharmaceutically acceptable emulsion, a microemulsion, a solution, a suspension, a syrup, and an elixir. The liquid dosage form may contain, in addition to the active ingredient, an inactive diluent (such as water or another solvent) generally used in this technical field, a solubilizing agent and an emulsifier (such as ethyl alcohol, isopropyl alcohol, ethyl carbonate, ethyl acetate, benzyl alcohol, benzyl benzoate, propylene glycol, 1,3-butylene glycol, dimethylformamide, an oil (particularly, an oil of cottonseed, peanuts, corn, germ, olive, castor-oil plant, or sesame), glycerol, tetrahydrofurfuryl alcohol, polyethylene glycol, and a sorbitan fatty acid ester, and a mixture of these). A formulation for oral administration may contain at least any one of an adjuvant (such as a humectant, an emulsifier, or a suspending agent), a sweetening agent, a flavor and a flavoring agent. A formulation for parenteral administration may contain a solubilizing agent (such as Cremophor(R), an alcohol, an oil, a modified oil, glycol, polysorbate, cyclodextrin, a polymer, or a combination of these).

Examples of a method for administering the pharmaceutical composition include lymph node topical administration, intratumoral topical administration, intramuscular administration, intradermal administration, subcutaneous administration, intratracheal administration, intrathecal administration, intraventricular administration, intraocular administration, intratympanic administration, catheter administration to the coronary artery, catheter administration to the hepatic portal vein, catheter administration to the heart muscle, transurethral catheter administration, and intravenous administration.

The pharmaceutical composition may contain, in addition to the polynucleotide, an optional component. Examples of the optional component include one or more pharmaceutically acceptable additives selected from a solvent, an aqueous solvent, a nonaqueous solvent, a dispersion medium, a diluent, a dispersion, a suspension aid, a surfactant, a tonicity agent, a thickener, an emulsifier, a preservative, a lipid, a lipidoid liposome, a lipid nanoparticle, a core-shell nanoparticle, a polymer, a lipoplexe, a peptide, a protein, a cell, a hyaluronidase, and a mixture of these.

### Examples

Now, the present invention will be described in more detail with reference to examples and reference examples, and it is noted that the technical field of the present invention is not limited to these.

As reagents used in synthesis of compounds, those purchased from Sigma Aldrich Co., Tokyo Chemical Industry Co., Ltd., Wako Pure Chemical Industries Ltd., and Kanto Chemical Co., Inc. were used without purification. An anhydrous solvent was prepared by drying a solvent on activated molecular sieve 4 Angstrom for 12 hours, or a commercially available anhydrous grade solvent was used. A reaction was tracked by thin layer silica gel chromatography (silica gel 70F254 TLC plate-Wako, Wako Pure Chemical Industries Ltd.). For purification of a compound, silica gel 60 N for flash chromatography (spherical, neutral, particle size: 40 to 50 µm) (Kanto Chemical Co., Inc.) was used. NMR was measured with JEOL ECS 400 MHz (JEOL Ltd.) with a deuteration solvent (CDCl₃, CD₃OD, DMSO-d₆) (Kanto Chemical Co., Inc.) used as a measurement solvent. Data of NMR thus obtained was analyzed with software of JEOL Delta (JEOL Ltd.), and a chemical shift value was corrected by a residual signal (CDCl₃: 7.26, CD₃OD: 3.31, DMSO-d₆: 2.50) (Organometallics 2010, 29, 2176-2179) in the deuteration solvent. Data of ¹H NMR was shown as a chemical shift value (δ), an integrated value (H), a signal splitting pattern, and a coupling constant (Hz) (s: singlet, d: doublet, t: triplet, sept.: septet, m: multiplet, br.: broad). High resolution mass spectrometry was measured with micrOTOF-QII ESI (Bruker Daltonics), and an accurate mass was corrected with ESI TUNING MIX (Agilent Technologies) used as internal standard.

Synthesis of a compound 12 used as a raw material of a polynucleotide was performed in accordance with the following scheme:

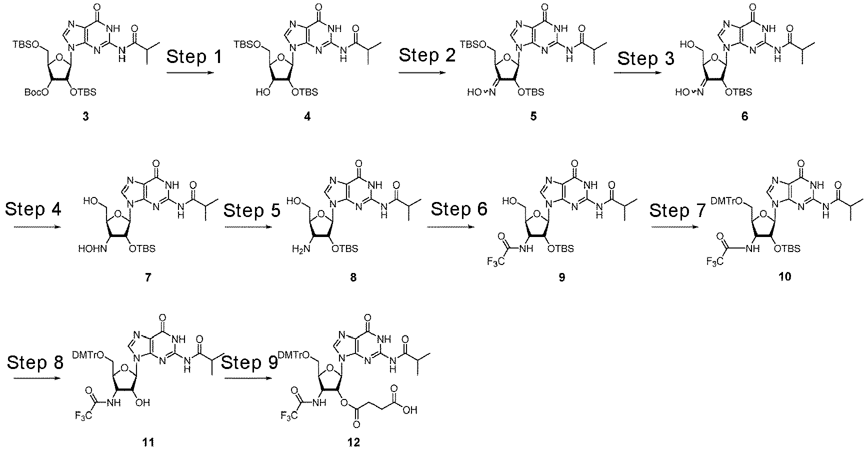

### Step 1 Synthesis of Compound 4

### N-(9-((2R,3S,4S,5R)-3-(tert-butyldimethylsilyloxy)-5-((tert-butyldimethylsilyloxy)methyl)-4-hydroxy-tetrahydrofuran-2-yl)-6-oxo-6,9-dihydro-1H-purin-2-yl)isobutyramide

Step A compound Step 3 obtained by a method described in a literature (J. Am. Chem. Soc., 1999, 121, 5661-5665) or the like was used to be dissolved in 1,2-dichlorobenzene (2.0 mL), and the resultant was stirred on an oil bath (160°C) for 4 hours. The resultant reaction solution was returned to room temperature, and purified, without concentration, by flash column chromatography (neutral silica gel, dichloromethane/methanol = 40:1) to obtain a compound 4 in the form of a white solid (0.31 g, yield: 53%).
¹H NMR (400 MHz, CDCl₃) δ 12.01 (1H, s), 8.50 (1H, s), 8.07 (1H, s), 5.86 (1H, d, J = 6.0 Hz), 4.47 (1H, s), 4.24 - 4.23 (1H, m), 4.22 - 4.21 (1H, m), 3.93 (1H, dd, J = 11.6, 2.0 Hz), 3.82 (1H, dd, J = 11.6, 2.0 Hz), 2.66 (1H, sept., J = 6.8 Hz), 1.27 (3H, d, J = 6.8 Hz), 1.25 (3H, d, J = 6.8 Hz), 0.93 (9H, s), 0.82 (9H, s), 0.13 (3H, s), 0.12 (3H, s), -0.07 (3H, s), - 0.20 (3H, s)
¹³C NMR (100 MHz, CDCl₃) δ 179.0, 155.7, 148.5, 148.7, 147.8, 136.8, 121.0, 87.4, 85.4, 77.6, 71.8, 63.6, 36.2, 25.9, 25.4, 19.1, 18.7, 18.3, 17.8, -5.3, -5.4, -5.5, -5.6
ESI-HRMS: calcd for C₂₆H₄₈N₅O₆Si₂ 582.31[M+H]⁺, found : 582.31[M+H]⁺

### Step 2 Synthesis of Compound 5

### N-(9-((2R,3S,5S)-3-(tert-butyldimethylsilyloxy)-5-((tert-butyldimethylsilyloxy)methyl)-4-(hydroxyimino)-tetrahydrofuran-2-yl)-6-oxo-6,9-dihydro-1H-purin-2-yl)isobutyramide

Molecular sieve 3 Angstrom (in the shape of a powder) (258 mg) was added to a solution of chromic acid (129 mg, 1.29 mmol) in anhydrous dichloromethane (2.0 mL), followed by cooling on an ice bath. Anhydrous pyridine (207 µL, 1.29 mmol) was added in a dropwise manner to the resultant solution under stirring, followed by stirring on an ice bath. After 30 minutes, acetic anhydride (122 µL, 1.29 mmol) was added thereto in a dropwise manner, followed by stirring on an ice bath. After 30 minutes, a solution of the compound 4 (250 mg, 0.43 mmol) in dichloromethane (1.3 mL) was added thereto in a dropwise manner, followed by stirring at room temperature for 2 hours. After confirming disappearance of the raw material by thin layer chromatography, the resultant reaction solution was diluted with ethyl acetate, and filtered through a silica pad (with a thickness of 2 cm), and the resultant filtrate was concentrated under reduced pressure to obtain a colorless solid. The thus obtained crude product 4' was directly used in the following reaction.

Hydroxylamine hydrochloride (299 mg, 4.30 mmol) was added to a solution of the crude product 4' (as 0.43 mmol) in pyridine (4 mL), followed by stirring at room temperature. After 24 hours, the resultant reaction solution was concentrated under reduced pressure, and water was added to the resultant residue, followed by extraction with ethyl acetate. An organic layer was washed with a saturated saline solution, and dried over anhydrous sodium sulfate. The organic layer was concentrated under reduced pressure, and the residue was purified by flash column chromatography (neutral silica gel, dichloromethane/methanol = 40:1) to obtain a compound 5 in the form of a white solid (255 mg, yield for two steps: 68%).
¹H NMR (400 MHz, CDCl₃) δ 12.14 (1H, s), 9.27 (1H, s), 8.78 (1H, s), 8.11 (1H, s), 5.78 (1H, d, J = 7.6 Hz), 5.09 (1H, s), 4.92 (1H, d, J = 7.2 Hz), 4.14 (1H, d, J = 11.4 Hz), 3.92 (1H, d, J = 11.4 Hz), 2.79 - 2.74 (1H, m), 1.27 - 1.21 (6H, m), 0.91 (9H, s), 0.71 (9H, s), 0.10 (3H, s), 0.07 (3H, s), -0.10 (3H, s), -0.23 (3H, s)
¹³C NMR (100 MHz, CDCl₃) δ 178.9, 157.8, 155.6, 148.7, 147.8, 136.8, 120.8, 87.5, 86.5, 62.2, 36.3, 25.9, 25.5, 25.2, 19.1, 18.8, 18.3, 18.0, -5.0, -5.5, -5.6, -5.7
ESI-HRMS : calcd for C₂₆H₄₇N₆O₆Si₂ 595.31[M+H]⁺, found : 595.31[M+H]⁺

### Step 3 Synthesis of Compound 6

### N-(9-((2R,3S,5S)-3-(tert-butyldimethylsilyloxy)-4-(hydroxyimino)-5-(hydroxymethyl)-tetrahydrofuran-2-yl)-6-oxo-6,9-dihydro-1H-purin-2-yl)isobutyramide

A 90% trifluoroacetic acid aqueous solution (1.0 mL) cooled on ice was added to the compound 5 (129 mg, 0.22 mmol), followed by stirring on an ice bath for 30 minutes. The resultant reaction solution was concentrated under reduced pressure, and the thus obtained residue was azeotroped with toluene and water (1:1, v/v) three times under reduced pressure. The thus obtained residue was purified by flash column chromatography (neutral silica gel, dichloromethane/methanol = 50:1 to 40:1) to obtain a compound 6 in the form of a white solid (96 mg, yield: 92%).
¹H NMR (400 MHz, CD₃OD) δ 8.36 (1H, s), 5.87 (1H, d, J = 7.6 Hz), 5.18 (1H, dd, J = 7.6, 2.0 Hz), 5.02 (1H, d, J = 2.0 Hz), 4.11 (1H, dd, J = 12.0, 2.0 Hz), 3.92 (1H, d, J = 12.0, 2.0 Hz), 2.71 (1H, sept., J = 7.2 Hz), 1.21 (6H, d, J = 7.2 Hz), 0.72 (9H, s), 0.00 (3H, s), -0.16 (3H, s)
¹³C NMR (100 MHz, CD₃OD) δ 181.8, 157.4, 156.8, 151.0, 150.0, 139.8, 121.3, 88.4, 79.7, 76.5, 61.6, 36.9, 25.9, 19.4, 19.2, -4.5, -5.5
ESI-HRMS : calcd for C₂₀H₃₂N₆NaO₆Si 503.21[M+Na]⁺, found : 503.20[M+Na]⁺

### Step 4 Synthesis of Compound 7

### N-(9-((2R,3S,4S,5S)-4-amino-3-(tert-butyldimethylsilyloxy)-5-(hydroxymethyl)-tetrahydrofuran-2-yl)-6-oxo-6,9-dihydro-1H-purin-2-yl)isobutyramide

Sodium borohydride (15 mg, 0.38 mmol) was added to a solution of the compound 6 (93 mg, 0.19 mmol) in acetic acid (1.9 mL), followed by stirring at room temperature for 1 hour. After confirming disappearance of the raw material by thin layer chromatography, the resultant reaction solution was concentrated under reduced pressure, and the thus obtained residue was dissolved in ethyl acetate, washed with a saturated saline solution, and dried over anhydrous sodium sulfate. An organic layer was concentrated under reduced pressure, and the thus obtained residue was purified by flash column chromatography (neutral silica gel, dichloromethane/methanol: 20:1) to obtain a compound 7 in the form of a white solid (51 mg, yield: 55%).
¹H NMR (400 MHz, CD₃OD) δ 8.34 (1H, s), 6.06 (1H, d, J = 6.0 Hz), 4.75 (1H, t, J = 6.4 Hz), 4.27 (1H, d, J = 2.8 Hz), 3.86 (1H, dd, J = 12.4, 2.0 Hz), 3.73 (1H, d, J = 12.4, 2.0 Hz), 3.62 - 3.60 (1H, m), 2.71 (1H, sept., J = 6.8 Hz), 1.21 (6H, d, J = 6.8 Hz), 0.82 (9H, s), -0.02 (3H, s), -0.23 (3H, s)
¹³C NMR (100 MHz, CD₃OD) δ 181.8, 157.4, 150.8, 149.8, 139.6, 139.4, 121.1, 89.7, 84.5, 77.7, 65.4, 65.2, 36.9, 26.0, -5.2, -5.3
ESI-HRMS : calcd for C₂₀H₃₅N₆O₆Si 483.24[M+H]⁺, found : 483.23[M+H]⁺

### Step 5 Synthesis of Compound 8

### N-(9-((2R,3S,4S,5S)-4-amino-3-(tert-butyldimethylsilyloxy)-5-(hydroxymethyl)-tetrahydrofuran-2-yl)-6-oxo-6,9-dihydro-1H-purin-2-yl)isobutyramide

10% Palladium carbon (20 mg) was added to a 90% acetic acid aqueous solution (1.5 mL) of the compound 7 (50 mg, 0.10 mmol), followed by stirring at room temperature under a hydrogen atmosphere for 18 hours. After confirming disappearance of the raw material by thin layer chromatography, the resultant reaction solution was diluted with methanol, and palladium carbon was removed by celite filtration. The resultant filtrate was concentrated under reduced pressure, and the thus obtained residue was purified by flash column chromatography (neutral silica gel, dichloromethane/methanol = 15:1 to 10:1) to obtain a compound 8 in the form of a white solid (41 mg in terms of acetate, yield: 75%).
¹H NMR (400 MHz, CD₃OD) δ 8.32 (1H, s), 5.99 (1H, s), 4.60 (1H, s), 3.95 - 3.68 (4H, m), 2.73 (1H, br. s), 1.22 (6H, br. s), 0.05 (3H, s), -0.06 (3H, s)
ESI-HRMS : calcd for C₂₀H₃₄N₆NaO₅Si 489.2258[M+Na]⁺, found : 489.2231[M+Na]⁺

### Step 6 Synthesis of Compound 9

### N-(9-((2R,3S,4R,5S)-3-(tert-butyldimethylsilyloxy)-5-(hydroxymethyl)-4-(2,2,2-trifluoroacetamido)-tetrahydrofuran-2-yl)-6-oxo-6,9-dihydro-1H-purin-2-yl)isobutyramide

Ethyl trifluoroacetate (0.76 mL) was added to a methanol solution (0.76 mL) of the compound 8 (40 mg, 0.076 mmol) of known literature (WO2017/123669) and triethylamine (45 L, 0.38 mmol), followed by stirring at room temperature for 24 hours. After confirming disappearance of the raw material by thin layer chromatography, the resultant reaction solution was concentrated under reduced pressure, and the thus obtained residue was purified by flash column chromatography (neutral silica gel, dichloromethane/methanol = 20:1 to 12:1) to obtain a compound 9 in the form of a white solid (12 mg, yield: 28%).
¹H NMR (400 MHz, CDCl₃) δ 12.26 (1H, s), 10.11 (1H, s), 7.76 (1H, s), 7.26 (1H, d, J = 3.6 Hz), 5.71 (1H, d, J = 3.6 Hz), 4.98 (1H, dd, J = 6.8 Hz), 4.78 (1H, dd, J = 6.8, 3.6 Hz), 4.21 (1H, d, J = 6.8 Hz), 4.03 (1H, dd, J =11.2 Hz), 3.82 (1H, dd, J =11.2 Hz), 2.79 (1H, sept., J = 6.8 Hz), 1.26 (3H, d, J = 6.8 Hz), 1.24 (3H, d, J = 6.8 Hz), 0.85 (9H, s), -0.01 (3H, s), - 0.11 (3H, s)
¹³C NMR (100 MHz, CDCl₃) δ 179.8, 158.0, 157.7, 157.3, 156.9, 155.2, 148.3, 147.3, 138.6, 122.0, 120.0, 117.1, 114.2, 111.3, 91.6, 83.7, 74.5, 61.3, 51.0, 36.1, 25.2, 18.9, 17.7, -5.0, -5.4
ESI-HRMS : calcd for C₂₂H₃₃F₃N₆NaO₆Si 585.21[M+Na]⁺, found : 585.21[M+Na]⁺

### Step 7 Synthesis of Compound 10

### N-(9-((2R,3S,4R,5S)-5-((bis(4-methoxyphenyl)(phenyl)methoxy)methyl)-3-(tert-butyldimethylsilyloxy)-4-(2,2,2-trifluoroacetamido)-tetrahydrofuran-2-yl)-6-oxo-6,9-dihydro-1H-purin-2-yl)isobutyramide

Dimethoxytrityl chloride (18 mg, 0.053 mmol) was added to a solution of the compound 9 (10 mg, 0.017 mmol) in anhydrous pyridine (1 mL), followed by stirring at room temperature for 1.5 hours. Thereafter, dimethoxytrityl chloride (18 mg, 0.053 mmol) was further added thereto, followed by stirring at room temperature for 30 minutes. After confirming disappearance of the raw material by thin layer chromatography, methanol (1 mL) was added to the resultant reaction solution, followed by concentration under reduced pressure. The resultant residue was dissolved in ethyl acetate, and was washed with water, and then with a saturated saline solution. An organic layer was dried over anhydrous sodium sulfate, and a residue obtained by concentration under reduced pressure of the resultant was purified by flash column chromatography (neutral silica gel, hexane/ethyl acetate = 5:1 to 2:1) to obtain a compound 10 in the form of a white solid (15.2 mg, yield: 99%).
¹H NMR (400 MHz, CDCl₃) δ 11.99 (1H, s), 10.11 (1H, s), 8.07 (1H, s), 7.81 (1H, s), 7.45 (2H, dd, J = 8.2, 2.0 Hz), 7.32 (4H, dd, J = 9.2, 3.6 Hz), 7.24 - 7.29 (3H, m), 7.01 (1H, d, J = 7.2 Hz), 6.76 (4H, J = 9.2, 3.6 Hz), 5.71 (1H, d, J = 4.2 Hz), 5.16 (1H, dd, J = 6.4, 4.2 Hz), 4.20 - 4.17 (1H, m), 3.76 (3H, s), 3.75 (3H, s), 3.56 (1H, dd, J = 11.2, 2.8 Hz), 3.22 (1H, dd, J = 11.2, 2.8 Hz), 1.82 (1H, d, J = 6.8 Hz), 0.97 (3H, d, J = 6.8 Hz), 0.68 (9H, s), 0.79 (3H, d, J = 6.8 Hz), 0.04 (3H, s), -0.06 (3H, s)
¹³C NMR (100 MHz, CDCl₃) δ 171.2, 158.7, 158.0, 157.6, 157.2, 156.8, 155.4, 147.6, 147.2, 144.8, 139.2, 135.9, 135.4, 130.0, 127.9, 127.1, 122.6, 120.0, 117.0, 114.1, 111.2, 90.1, 86.3, 81.7, 73.4, 62.4, 60.4, 55.2, 51.4, 36.1, 25.4, 18.4, 17.8, -5.0, -5.3
ESI-HRMS : calcd for C₄₃H₅₂F₃N₆O₈Si 865.36[M+H]⁺, found : 865.35[M+H]⁺

### Step 8 Synthesis of Compound 11

### N-(9-((2R,3S,4S,5S)-5-((bis(4-methoxyphenyl)(phenyl)methoxy)methyl)-3-hydroxy-4-(2,2,2-trifluoroacetamido)-tetrahydrofuran-2-yl)-6-oxo-6,9-dihydro-1H-purin-2-yl)isobutyramide

Tetrabutylammonium fluoride (1M tetrahydrofuran solution, 19 µL, 0.019 mmol) was added to a solution of the compound 10 (14 mg, 0.016 mmol) in tetrahydrofuran (1 mL), followed by stirring at room temperature for 1 hour. After confirming disappearance of the raw material by thin layer chromatography, the resultant reaction solution was concentrated under reduced pressure. The thus obtained residue was purified by flash column chromatography (neutral silica gel, dichloromethane/methanol = 30:1 to 15:1) to obtain a compound 11 in the form of a white solid (10.8 mg, yield: 83%).
¹H NMR (400 MHz, CDCl₃) δ 12.12 (1H, br. s), 8.76 (1H, br. s), 7.74 (1H, s), 7.81 (1H, s), 7.68 (1H, d, J = 5.4 Hz), 7.48 (2H, d, J = 7.6 Hz), 7.37 (2H, d, J = 9.2 Hz), 7.34 (2H, d, J = 9.2 Hz), 7.25 - 7.21 (2H, m), 7.17 (1H, t, J = 7.2 Hz), 6.81 (2H, d, J = 9.2 Hz), 6.78 (2H, d, J = 9.2 Hz), 5.80 (1H, d, J = 4.0 Hz), 5.35 (1H, br. s), 5.08 (1H, dd, J = 12.4, 6.4 Hz), 4.30 - 4.29 (1H, m), 3.76 (3H, s), 3.74 (3H, s), 3.57 - 3.53 (1H, m), 3.29 - 3.26 (1H, m), 1.84 - 1.57 (1H, m), 0.94 (3H, d, J = 6.8 Hz), 0.68 (3H, d, J = 6.8 Hz)
¹³C NMR (100 MHz, CDCl₃) δ 179.4, 158.6, 158.4, 158.0, 157.6, 157.3, 147.8, 147.2, 144.8, 139.4, 136.3, 135.7, 130.1, 129.9, 128.1, 128.0, 127.0, 121.0, 120.0, 117.1, 114.2, 111.3, 91.2, 86.1, 82.5, 71.5, 62.7, 55.1, 51.2, 35.9, 18.5, 18.2ESI-HRMS : calcd for C₃₇H₃₈F₃N₆O₈ 751.27[M+H]⁺, found : 751.27[M+H]⁺

### Step 9 Synthesis of Compound 12

Succinic anhydride (0.24 g, 2.40 mmol) and dimethylaminopyridine (29 mg, 0.24 mmol) were added to a solution of the compound 11 (0.90 g, 1.20 mmol) and triethylamine (0.42 mL, 3.0 mmol) in acetonitrile (12 mL), followed by stirring at room temperature for 1 hour. After confirming disappearance of the raw material by thin layer chromatography, the resultant reaction solution was concentrated under reduced pressure. The resultant residue was dissolved in ethyl acetate, and was washed with a saturated sodium bicarbonate aqueous solution twice, and then with a saturated saline solution. An organic layer was dried over anhydrous sodium sulfate, and was concentrated under reduced pressure. The thus obtained residue was subjected to an azeotropic operation through concentration under reduced pressure with dichloromethane/methanol solution (1:1, v/v) to obtain a white foamy solid (1.11 g in terms of triethylamine salt, 97%). The thus obtained compound 12 was directly used in the following reaction.

The compound 12 can be synthesized by obtaining an intermediate 6 from the following starting material 13:

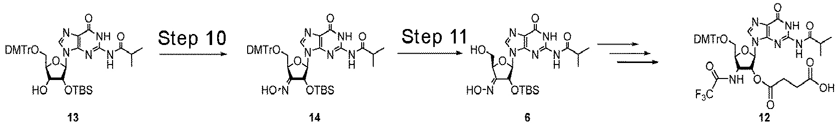

### Step 10 Synthesis of Compound 14

### N-(9-((2R,3R,5S)-5-((bis(4-methoxyphenyl)(phenyl)methoxy)methyl)-3-((tertbutyldimethylsilyl)oxy)-4-(hydroxyimino)tetrahydrofuran-2-yl)-6-oxo-6,9-dihydro-1H-purin-2-yl)isobutyramide

Under an argon atmosphere, a compound 13 (manufactured by ChemGenes Corp., 5.0 g, 6.5 mmol) was dissolved in dehydrated dichloromethane (50 mL), followed by stirring with cooling on an ice bath. With cooling the resultant reaction solution, sodium bicarbonate (8.2 g, 97.3 mmol) and nor-AZADO (36 mg, 0.260 mmol) were added thereto, and iodo benzene diacetate (3.14 g, 9.73 mmol) was added thereto dividedly with attention paid to internal temperature increase, followed by stirring for 21 hours and 10 minutes with increasing the temperature up to room temperature. After confirming disappearance of the raw material, isopropyl alcohol (7.5 mL) was added to the reaction solution, followed by stirring for 4 hours (for quenching an excessive portion of the oxidant). The resultant reaction solution was added to ice water, chloroform was further added thereto for separation, and an aqueous layer was extracted again with chloroform. An organic layer was combined, the resultant was washed with water once and with a saturated saline solution once, and was dehydrated with anhydrous sodium sulfate. The desiccant was filtered, and the resultant filtrate was concentrated to obtain a crude product (9.01 g, containing a compound having a DMTr group partially deprotected) in the form of an orange solid.

Under an argon atmosphere, the crude product (9.01 g) was dissolved in anhydrous pyridine (40 mL), followed by stirring with cooling on an ice bath. With cooling the resultant reaction solution, hydroxylamine hydrochloride (4.06 g, 58.7 mmol) was added thereto, followed by stirring for 17 hours and 25 minutes with increasing the temperature up to room temperature. After confirming disappearance of the raw material, the resultant reaction solution was transferred to an eggplant flask with washing with chloroform (containing 1% triethylamine) to be concentrated. The thus obtained residue was added to a saturated sodium bicarbonate solution, and the resultant was stirred for 15 minutes, followed by extraction with chloroform twice. After combining an organic layer, the resultant was washed with a saturated saline solution once, and then was dehydrated with anhydrous sodium sulfate. After filtering the desiccant, the resultant filtrate was concentrated to obtain a compound 14 (4.13 g, mixture with diastereomer, yield for two steps: 81%) in the form of an orange foamy substance.
¹H NMR (400MHz, CDCl₃) δ: 12.04 (1H, d, J = 23.3 Hz), 9.23 (1H, s), 8.49 (1H, s), 7.89 (1H, s), 7.79 (1H, s), 7.66-7.58 (2H, m), 7.49-7.39 (4H, m), 7.31-7.14 (5H, m), 6.81-6.76 (2H, m), 6.73-6.68 (2H, m), 5.92 (1H, dd, J = 8.0, 1.6 Hz), 5.83 (1H, d, J = 3.7 Hz), 5.64 (1H, d, J = 8.2 Hz), 5.54 (1H, dd, J = 3.9, 1.1 Hz), 5.01 (1H, t, J = 7.3 Hz), 3.80-3.73 (6H, m), 3.54-3.46 (2H, m), 1.28 (1H, m), 1.08 (1H, d, J = 6.9 Hz), 0.99 (1H, d, J = 6.9 Hz), 0.86-0.76 (9H, m), 0.47 (2H, m), 0.11 (1H, s), 0.02--0.02 (3H, m), -0.07 (2H, s) [mixture with diastereomer]
ESI-HRMS : calcd for C₄₁H₅₀N₆O₈Si 781.97[M-H]⁻, found : 781.84[M-H]⁻

### Step 11 Synthesis of Compound 6 from Compound 14

The compound 14 (3.80 g) obtained in Step 10 was used to obtain the compound 6 (2.12 g, 4.41 mmol, yield: 91%) in the same manner as in Step 3.

It is noted that detailed data of the compound 6 is the same as that described regarding Step 3.

### Synthesis of Compound 15

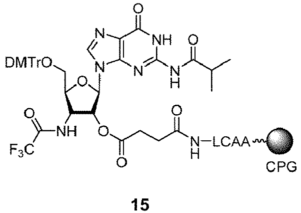

To a solution of the compound 12 (380 mg, 0.50 mmol) in N,N-dimethylaminoformamide (2.5 mL), Native amino lcaa CPG (1000 angstrom, ChemGenes Corp.) (84 µmol/g, 1.20 g, 0.10 mmol) and subsequently a solution of HOBt (136 mg, 1.01 mmol) and EDC-HCl (193 mg, 1.01 mmol) in DMF (2.5 mL) were added, followed by shaking at room temperature. After 20 hours, the resultant reaction solution was discarded, and the solid phase support was washed with N,N-dimethylaminoformamide (5 mL, four times) and subsequently with dichloromethane (5 mL, four times). An unreacted amino group remaining on the solid phase support was capped with a 10% acetic anhydride/pyridine solution (5 mL) (room temperature, shaking for 16 hours). The resultant reaction solution was discarded, and the solid phase support was washed with pyridine (5 mL, once) and subsequently with dichloromethane (5 mL, four times), and then dried under vacuum to obtain a compound 15 (1.20 g) in which the compound 12 is supported on the solid phase support.

An amount of the compound 12 supported on the solid phase was calculated as follows: A prescribed amount of the obtained solid phase support was taken, and color development of 4,4'-dimethoxytrityl cation caused by adding thereto a deblocking reagent (3 w/v% trichloroacetic acid/dichloromethane solution) was measured by ultraviolet visible spectrophotometry (quartz cell, cell length: 10 mm). Based on an absorbance at 504 nm and a molar extinction coefficient of 4,4'-dimethoxytrityl cation (wavelength of 504 mm: 76,000), the amount of the compound 12 supported on the solid phase was calculated by Lambert-Beer method. Specifically, the obtained solid phase support (2.0 mg) was weighed in a 2 mL volumetric flask, the deblocking reagent was added thereto to obtain a total amount of 2 mL, and the resultant was mixed by inverting to obtain a measurement sample. After performing blank measurement using a 3 w/v% trichloroacetic acid/dichloromethane solution, the measurement was performed on the measurement sample. Based on an absorbance at 504 nm of 0.377, the supported amount: 24. 8 µmol/g)

Synthesis of a compound 24 was performed in accordance with the following scheme:

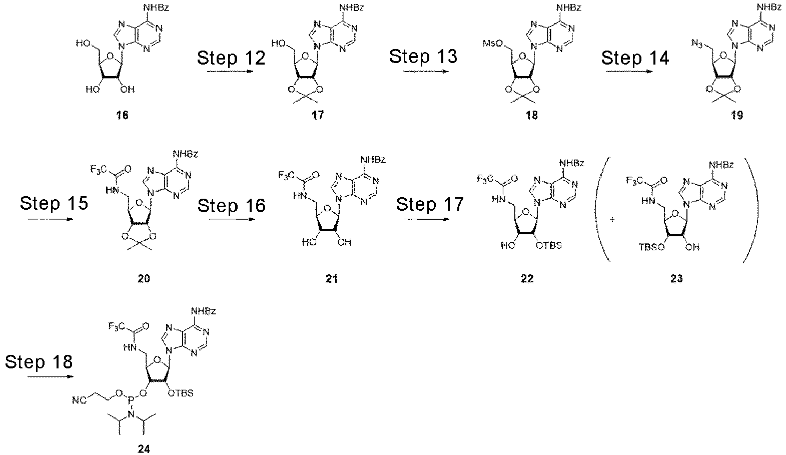

### Step 12 Synthesis of Compound 17

### N-(9-((3aR,4R,6R,6aR)-6-(hydroxymethyl)-2,2-dimethyltetrahydrofuro[3,4-d][1,3]dioxol-4-yl)-9H-purin-6-yl)benzamide

Under an argon atmosphere, commercially available N6-benzyladenosine (compound 16) (100 g, 269 mmol, 1.0 eq.), acetone (2.70 L), and dimethoxypropane (166 mL, 1.35 mol, 5.0 eq.) were successively added to a 10 L four-neck flask. Concentrated sulfuric acid (1.44 mL, 26.9 mmol, 0.10 eq.) was added to the resultant reaction solution, followed by stirring at room temperature for 15 hours. Since the raw material was found to still remain, concentrated sulfuric acid (1.44 mL, 26.9 mmol, 0.10 eq.) was further added thereto, followed by stirring for 24 hours. Since the raw material was found to still remain, concentrated sulfuric acid (1.44 mL, 26.9 mmol, 0.10 eq.) was further added thereto, followed by stirring for 1 hour and 30 minutes, and then, concentrated sulfuric acid (2.87 mL, 53.8 mmol, 0.20 eq.) was added thereto, followed by stirring for 4 hours.

After checking progress of the reaction by LC/MS, the resultant reaction solution was cooled on an ice bath, and a saturated sodium bicarbonate aqueous solution (400 mL) was added thereto in a dropwise manner over 5 minutes to obtain an internal temperature of 3 to 5°C to neutralize the resultant solution. The reaction solution was concentrated under reduced pressure, and distilled water (2.0 L) was added to the resultant residue. The resultant solution was extracted with chloroform (1.0 L) three times, and an organic layer was dehydrated with anhydrous sodium sulfate. After filtration, the solvent was distilled off under reduced pressure to obtain a compound 17 (222 g). The thus obtained compound 17 was used in the following step without being subjected to further purification operation.

### Step 13 Synthesis of Compound 18

### ((3aR,4R,6R,6aR)-6-(6-benzamido-9H-purin-9-yl)-2,2-dimethyltetrahydrofuro[3,4-d][1,3]dioxol-4-yl)methyl methanesulfonate

Under an argon atmosphere, the compound 17 (222 g) obtained in Step 12 and pyridine (520 mL) were added to a 2 L four-neck flask, the resultant reaction solution was cooled on an ice bath, and methanesulfonyl chloride (25.0 mL, 321 mmol, 1.2 eq.) was added thereto in a dropwise manner over 15 minutes to obtain an internal temperature of 4°C to 9°C, followed by stirring for 2 hours.

After checking progress of the reaction by LC/MS, distilled water (500 mL) was added to the reaction solution, the resultant solution was extracted with ethyl acetate (1.0 L) three times, and then, an organic layer was washed successively with 1N hydrochloric acid (1.0 L x 1,500 mL x 2), with a saturated sodium bicarbonate aqueous solution (500 mL x 2), and with a saturated saline solution (500 mL x 2), and the resultant was dehydrated with anhydrous sodium sulfate. After filtration, the solvent was distilled off under reduced pressure, and the thus obtained residue was azeotroped with toluene to obtain a compound 18 (150 g, containing 17.6 wt% of toluene). The thus obtained compound 18 was used in the following step without being subjected to further purification operation.

### Step 14 Synthesis of Compound 19

### N-(9-((3aR,4R,6R,6aR)-6-(azidomethyl)-2,2-dimethyltetrahydrofuro[3,4-d][1,3]dioxol-4-yl)-9H-purin-6-yl)benzamide

Under an argon atmosphere, the compound 18 (150 g) obtained in Step 13 and dehydrated DMF (1.26 L) were added to a 3 L four-neck flask. To the resultant reaction solution, sodium azide (82.8 g, 1.26 mol, 5.0 eq.) was added, and the temperature was increased up to 60°C over 30 minutes, followed by stirring for 3 hours and 30 minutes at 60°C.

After checking progress of the reaction by LC/MS, the resultant reaction solution was gradually cooled to room temperature, and distilled water (1.0 L) and ethyl acetate (600 mL) were added thereto. To the thus obtained solution, distilled water (3.0 L) was added, and an aqueous layer was extracted with ethyl acetate (500 mL) six times. An organic layer was washed with distilled water (800 mL) twice and with a saturated saline solution (800 mL) twice, and was dehydrated with anhydrous sodium sulfate. After filtration, the solvent was distilled off under reduced pressure, and the thus obtained residue was purified by silica gel column chromatography (SiO₂ 700 g, ethyl acetate) to obtain a compound 19 (55.7 g, 128 mmol, yield: 48% (through three steps from the compound 16)).

### Step 15 Synthesis of Compound 20

### N-(9-((3aR,4R,6R,6aR)-2,2-dimethyl-6-((2,2,2-trifluoroacetamido)methyl)tetrahydrofuro[3,4-d][1,3]dioxol-4-yl)-9H-purin-6-yl)benzamide

Under an argon atmosphere, the compound 19 (55.7 g, 128 mmol, 1.0 eq.) obtained in Step 14 and methanol (1.28 L) were added to a 3 L four-neck flask. To the resultant reaction solution, 10% Pd/C (76.8 g, 21.2 mmol, 0.17 eq.) was added, and the inside of the reaction solution was replaced with hydrogen, followed by stirring at room temperature for 16 hours.

After checking progress of the reaction by LC/MS, the inside of the resultant reaction solution was replaced with an argon gas, and the reaction solution was subjected to celite filtration. The resultant filtrate was concentrated under reduced pressure, the thus obtained residue was dissolved in methanol (985 mL), and the resultant was transferred to a 3 L four-neck flask. The solution was cooled on an ice bath, and 1-(trifluoroacetyl)imidazole (17.0 mL, 149 mmol, 1.2 eq.) was added thereto in a dropwise manner over 15 minutes to obtain an internal temperature of 2 to 4°C, followed by stirring at 4°C for 2 hours. After checking progress of the reaction by LC/MS, the reaction solution was concentrated under reduced pressure. The thus obtained residue was purified by silica gel column chromatography (SiO₂ 800 g, heptane/ethyl acetate = 1:4) to obtain a compound 20 (21.4 g, 42.2 mmol, yield: 33%).

### Step 16 Synthesis of Compound 21

### N-(9-((2R,3R,4S,5R)-3,4-dihydroxy-5-((2,2,2-trifluoroacetamido)methyl)tetrahydrofuran-2-yl)-9H-purin-6-yl)benzamide

To a 1 L eggplant flask, the compound 20 (10.0 g, 19.8 mmol, 10 eq.) obtained in Step 15 and distilled water (50.0 mL) were added, and the resultant solution was cooled on an ice bath. Under ice cooling, trifluoroacetic acid (50.0 mL, 640 mmol, 32.4 eq.) was added thereto in a dropwise manner over 5 minutes, and the temperature of the resultant reaction solution was increased up to room temperature, followed by stirring for 4 hours and 30 minutes.

After checking progress of the reaction by LC/MS, the reaction solution was concentrated under reduced pressure, and the resultant residue was azeotroped with toluene. To the thus obtained residue, isopropyl ether was added to precipitate a solid, which was taken out by filtration. The thus obtained solid was dried under reduced pressure at room temperature to obtain a compound 21 (8.86 g, 19.0 mmol, yield: 96%).

### Step 17 Synthesis of Compound 22

### N-(9-((2R,3R,4R,5R)-3-((tertbutyldimethylsilyl)oxy)-4-hydroxy-5-((2,2,2-trifluoroacetamido)methyl)tetrahydrofuran-2-yl)-9H-purin-6-yl)benzamide

Under an argon atmosphere, the compound 21 (15.6 g, 33.6 mmol, 1.0 eq.) obtained in Step 16 and dehydrated DMF (111 mL) were added to a 500 mL eggplant flask, and the resultant solution was cooled on an ice bath. Under ice cooling, imidazole (9.16 g, 134 mmol, 4.0 eq.) and t-butyldimethylsilyl chloride (15.2 g, 101 mmol, 3.0 eq.) were added thereto to obtain an internal temperature less than 6°C, followed by stirring for 30 minutes at the same temperature.

After checking progress of the reaction by LC/MS, ice water was added to the resultant reaction solution. An aqueous layer was extracted with ethyl acetate three times, washed with a saturated saline solution, and dehydrated with anhydrous sodium sulfate. After filtration, the solvent was distilled off under reduced pressure, and the thus obtained residue was purified by silica gel column chromatography (SiO₂ 800 g, chloroform/2-butanone = 100:0 to 85:15) to obtain a mixture (10.9 g) of a compound 22 and a compound 23. The thus obtained mixture of the compound 22 and the compound 23 was used in the following step without being subjected to further purification operation.

### Step 18 Synthesis of Compound 24

### (2R,3R,4R,5R)-5-(6-benzamido-9H-purin-9-yl)-4-((tert-butyldimethylsilyl)oxy)-2-((2,2,2-trifluoroacetamido)methyl)tetrahydrofuran-3-yl(2-cyanoethyl)diisopropylphosphoramidite

Under an argon atmosphere, the mixture of the compound 22 and the compound 23 (1.19 g, 2.05 mmol, Compound 22:Compound 23 = 9:1) obtained in Step 17 and dehydrated dichloromethane (6.83 mL) were added to a 200 mL eggplant flask, and the resultant solution was cooled on an ice bath. Under ice cooling, a mixed solution of diisopropylethylamine (0.537 mL, 3.07 mmol, 1.5 eq.) and 3-((chloro(diisopropylamino)phosphanyl)oxy)propanenitril e (0.857 mL, 3.07 mmol, 1.5 eq.) in dichloromethane was added thereto in a dropwise manner, and the temperature of the resultant reaction solution was increased up to room temperature, followed by stirring at room temperature for 2 hours.

After confirming disappearance of the raw material by TLC, distilled water was added to the resultant reaction solution, the resultant was extracted with chloroform (50 mL) twice, and an organic layer was dehydrated with anhydrous sodium sulfate. After filtration, the solvent was distilled off under reduced pressure, and the thus obtained residue was purified several times by silica gel column chromatography (heptane/ethyl acetate = 50:50 to 30:70, containing 0.5% triethylamine) to obtain a target compound 24 (608 mg, 0.779 mmol, yield: 38%) in the form of a pale yellow amorphous substance.
¹H NMR (400 MHz, CDCl₃) δ: 9.82 (1H, d, J = 8.8 Hz), 9.07 (1H, s), 8.83-8.80 (1H, m), 8.06-8.01 (3H, m), 7.66-7.52 (3H, m), 5.86 (1H, d, J = 7.8 Hz), 4.88 (1H, dd, J = 7.8, 5.2 Hz), 4.50 (1H, br s), 4.39-4.29 (1H, m), 4.21-3.88 (3H, m), 3.74-3.63 (1H, m), 3.48-3.35 (1H, m), 2.73-2.66 (2H, m), 1.28-1.23 (12H, m), 1.08-1.04 (1H, m), 0.72-0.68 (9H, m), -0.17 (3H, s), -0.45 (3H, s).
³¹P NMR(CDCl3) δ: 149.95

Synthesis of a compound 6a to be used as a raw material of the polynucleotide was performed in accordance with the following scheme:

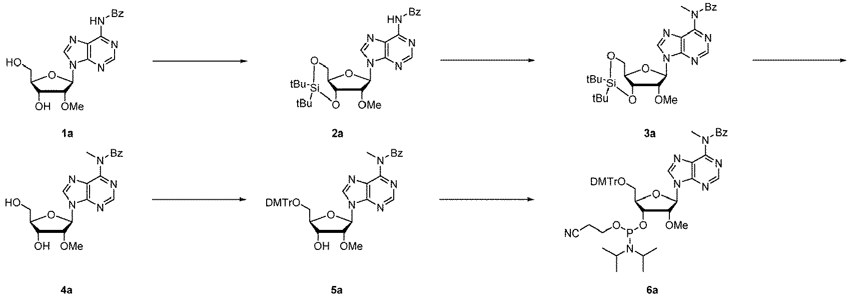

### Step 1 Synthesis of Compound 2a

### N-(9-((3aR,5R,6R,6aS)-2,2-di-tert-butyl-6-methoxytetrahydrofuro[2,3-d][1,3,2]dioxasilol-5-yl)-9H-purin-6-yl)benzamide

To a solution of a commercially available compound 1a (30.0 g, 78.0 mmol) in DMF (300 mL), di-t-butylsilylbis(trifluoromethanesulfonate) (68.6 g, 156 mmol) was slowly added under ice cooling. After stirring the resultant for 1 hour under ice cooling, the resultant reaction solution was added to a saturated sodium bicarbonate aqueous solution, and a mixed solvent of heptane/ethyl acetate was added thereto for performing extraction twice. An organic layer was washed with water twice, and with a saturated saline solution once, and was dried over anhydrous sodium sulfate. After filtration, the resultant concentrated residue was purified by silica gel column chromatography (heptane/ethyl acetate = 7/3 to 3/7) to obtain a compound 2a (38.7 g, 73.7 mmol) in the form of a colorless solid (yield: 95%).
ESI-MS: calcd: 524.23 [M-H]⁻, found: 524.5 [M-H]⁻¹H-NMR (CDCl₃, 400 MHz) δ: 9.32 (s, 1H), 8.76 (s, 1H), 8.05 (s, 1H), 8.03 (t, J = 6.6 Hz, 2H), 7.60 (t, J = 7.3 Hz, 1H), 7.51 (t, J = 7.8 Hz, 2H), 6.01 (s, 1H), 4.66 (dd, J = 9.6, 5.0 Hz, 1H), 4.48 (dd, J = 8.9, 4.8 Hz, 1H), 4.31 (d, J = 4.6 Hz, 1H), 4.20 (ddd, J = 10.1, 5.0, 5.0 Hz, 1H), 4.03 (t, J = 9.8 Hz, 1H), 3.70 (s, 3H), 1.10 (s, 9H), 1.06 (s, 9H).

### Step 2 Synthesis of Compound 3a

### N-(9-((3aR,5R,6R,6aS)-2,2-di-tert-butyl-6-methoxytetrahydrofuro[2,3-d][1,3,2]dioxasilol-5-yl)-9H-purin-6-yl)-N-methylbenzamide

The compound 2a (10.0 g, 19.0 mmol) was dissolved in dichloromethane (50 mL), and tetrabutylammonium bromide (9.20 g, 28.5 mmol) and a 1 M sodium hydroxide aqueous solution (50 ml) were added to the resultant. Methyl iodide (4.76 ml, 76.0 mmol) was slowly added thereto in a dropwise manner. Thereafter, the resultant was stirred at room temperature for 1 hour and 10 minutes. After confirming disappearance of the raw material, the resultant reaction solution was added to ice cooled water/chloroform = 1/1 for quenching. An organic layer was washed with water twice, and was dehydrated with anhydrous sodium sulfate, the desiccant was filtered out, and the resultant filtrate was concentrated. The thus obtained concentrated residue was purified by silica gel column chromatography (heptane/ethyl acetate = 90/10 to 50/50) to obtain a compound 3a (6.25 g, 11.6 mmol) in the form of a colorless amorphous (yield: 61%).
ESI-MS: calcd: 540.26 [M+H]⁺, found: 540.4 [M+H]^{+ 1}H-NMR (CDCl₃, 400 MHz) δ: 8.56 (s, 1H), 7.94 (s, 1H), 7.49-7.46 (m, 2H), 7.34-7.29 (m, 1H), 7.21 (t, J = 7.6 Hz, 2H), 5.94 (s, 1H), 4.61 (dd, J = 9.6, 5.0 Hz, 1H), 4.46 (dd, J = 9.2, 5.0 Hz, 1H), 4.22 (d, J = 4.6 Hz, 1H), 4.17 (ddd, J = 10.0, 5.2, 5.0 Hz, 1H), 4.00 (dd, J = 10.5, 9.2 Hz, 1H), 3.79 (s, 3H), 3.67 (s, 3H), 1.08 (s, 9H), 1.05 (s, 9H).

### Step 3 Synthesis of Compound 4a

### N-(9-((2R,3R,4S,5S)-4,5-dihydroxy-3-methoxytetrahydrofuran-2-yl)-9H-purin-6-yl)-N-methylbenzamide

The compound 3a (6.25 g, 11.6 mmol) was dissolved in tetrahydrofuran (63 mL), and the resultant was cooled on an ice bath. Triethylamine (8.07 ml, 57.9 mmol) and triethylamine trihydrofluoride (1.89 ml, 11.6 mmol) were added thereto, followed by stirring for 1 hour and 5 minutes with cooling on an ice bath. After confirming disappearance of the raw material, triethylamine (10 ml, 76.0 mmol) was added thereto for quenching, the resultant was diluted with chloroform, and the resultant reaction solution was concentrated. The thus obtained concentrated residue was purified by silica gel column chromatography (chloroform/methanol = 100/0 to 90/10) to obtain a compound 4a (4.25 g, 10.6 mmol) in the form of a colorless amorphous (yield: quant.).
ESI-MS: calcd: 400.16 [M+H]⁺, found: 400.3 [M+H]^{+ 1}H-NMR (CDCl₃, 400 MHz) δ: 8.56 (s, 1H), 7.97 (s, 1H), 7.50-7.48 (m, 2H), 7.35-7.31 (m, 1H), 7.22 (t, J = 7.8 Hz, 2H), 5.87 (d, J = 7.3 Hz, 1H), 5.86 (dd, J = 11.4, 2.3 Hz, 1H), 4.63 (dd, J = 7.3, 4.6 Hz, 1H), 4.57-4.56 (m, 1H), 4.36-4.34 (m, 1H), 3.99-3.94 (m, 1H), 3.81 (s, 3H), 3.77 (td, J = 12.3, 1.7 Hz, 1H), 3.31 (s, 3H), 2.77 (d, J = 1.4 Hz, 1H).

### Step 4 Synthesis of Compound 5a

### N-(9-((2R,3R,4S,5S)-5-(bis(4-methoxyphenyl)(phenyl)methoxy)-4-hydroxy-3-methoxytetrahydrofuran-2-yl)-9H-purin-6-yl)-N-methylbenzamide

The compound 4a (4.25 g, 10.6 mmol) was dissolved in pyridine (43 mL), and the resultant was stirred on an ice bath. To the resultant reaction solution, 4,4'-dimethoxytrityl chloride (5.41 g, 20.0 mmol) was added, followed by stirring at room temperature for 2 hours and 25 minutes. After confirming disappearance of the raw material, the resultant reaction solution was added to ice cooled sodium bicarbonate water for quenching, and the resultant was extracted with ethyl acetate. An organic layer was washed with a saturated saline solution, dried over anhydrous sodium sulfate, and was filtered, and then, the thus obtained filtrate was concentrated. The thus obtained concentrated residue was purified by silica gel column chromatography (heptane/ethyl acetate (containing 1% triethylamine) = 70/30 to 50/50) to obtain a compound 5a (5.35 g, 7.62 mmol) in the form of a colorless amorphous. (yield 71%)
ESI-MS: calcd: 702.29 [M+H]⁺, found: 702.6 [M+H]^{+ 1}H-NMR (CDCl₃, 400 MHz) δ: 8.50 (s, 1H), 8.14 (s, 1H), 7.45-7.40 (m, 4H), 7.33-7.22 (m, 8H), 7.16 (t, J = 7.6 Hz, 2H), 6.81 (dd, J = 8.9, 1.1 Hz, 4H), 6.15 (d, J = 3.7 Hz, 1H), 4.48 (dd, J = 11.9, 5.0 Hz, 1H), 4.35 (dd, J = 5.3, 3.9 Hz, 1H), 4.21-4.19 (m, 1H), 3.80 (s, 3H), 3.79 (s, 6H), 3.53 (s, 3H), 3.50 (dd, J = 10.8, 3.0 Hz, 1H), 3.40 (dd, J = 10.8, 4.4 Hz, 1H), 2.66 (d, J = 6.4 Hz, 1H).

### Step 5 Synthesis of Amidite 6a

### (2S,3S,4R,5R)-2-(bis(4-methoxyphenyl)(phenyl)methoxy)-4-methoxy-5-(6-(N-methylbenzamido)-9H-purin-9-yl)tetrahydrofuran-3-yl(2-cyanoethyl)diisopropylphosphoramidite

The compound 5a (5.30 g, 7.55 mmol) was dissolved in dichloromethane (48 mL), diisopropylethylamine (2.64 mL, 15.1 mmol) was added thereto, and the resultant was cooled on an ice bath. To the resultant, 2-cyanoethyl diisopropylchlorophosphoramidite (2.68 g, 11.3 mmol) dissolved in dichloromethane (5 mL) was added thereto in a dropwise manner over 5 minutes. Thereafter, the resultant was stirred for 1 hour and 10 minutes with increasing the temperature up to room temperature. After confirming disappearance of the raw material, the resultant reaction solution was added to ice cooled saturated sodium bicarbonate water for quenching. Ethyl acetate was added to the resultant for extraction. An organic layer was washed with a saturated saline solution, and was dried over anhydrous sodium sulfate, the desiccant was removed by filtration, and the thus obtained filtrate was concentrated. The thus obtained concentrated residue was purified by silica gel column chromatography (heptane/ethyl acetate (containing 1% triethylamine) = 70/30 to 50/50) to obtain an amidite 6a (6.22 g, 6.90 mmol) in the form of a colorless amorphous. (yield: 91%)
ESI-MS: calcd: 902.40 [M+H]⁺, found: 902.5 [M+H]^{+ 1}H-NMR (CDCl₃, 400 MHz) δ: 8.47 (s, 0.35H), 8.47 (s, 0.65H), 8.14 (s, 0.35H), 8.09 (s, 0.65H), 7.44-7.39 (m, 4H), 7.33-7.21 (m, 8H), 7.16-7.12 (m, 2H), 6.93-6.78 (m, 4H), 6.12 (d, J = 5.5, 0.65H), 6.10 (d, J = 5.0, 0.35H), 4.66-4.53 (m, 2H), 4.41-4.38 (m, 0.35H), 4.34-4.32 (m, 0.65H), 3.97-3.78 (m, 10H), 3.70-3.44 (m, 7H), 3.36-3.30 (m, 1H), 2.64 (t, J = 6.2 Hz, 1.3H), 2.38 (t, J = 6.4 Hz, 0.70H), 1.22-1.17 (m, 8H), 1.06 (d, J = 6.9 Hz, 4H).
³¹P-NMR(CDCl₃, 162 MHz) δ: 150.70, 150.94.

Synthesis of a compound 6b to be used as a raw material of the polynucleotide was performed in accordance with the following scheme:

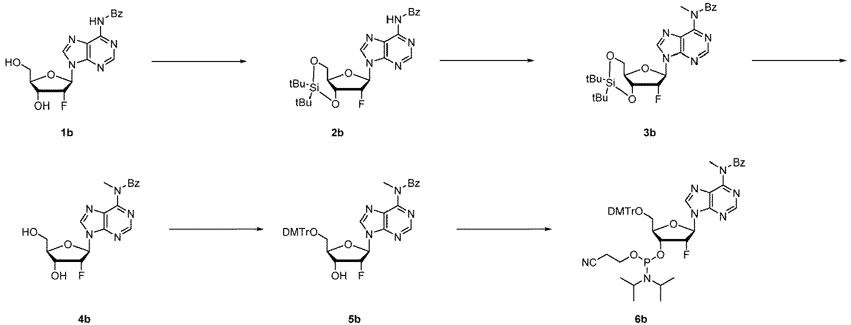

### Step 1 Synthesis of Compound 2b

### N-(9-((4aR,6R,7R,7aS)-2,2-di-tert-butyl-7-fluorotetrahydro-4H-furo[3,2-d][1,3,2]dioxasilin-6-yl)-9H-purin-6-yl)benzamide

To a solution of a commercially available compound 1b (30.0 g, 80.4 mmol) in DMF (300 mL), di-t-butylsilylbis(trifluoromethanesulfonate) (70.8 g, 161 mmol) was slowly added under ice cooling. After stirring the resultant for 1 hour under ice cooling, the resultant reaction solution was added to a saturated sodium bicarbonate aqueous solution, a mixed solvent of heptane/ethyl acetate was added thereto, and the resultant was extracted twice. An organic layer was washed with water twice, and with a saturated saline solution once, and was dried over anhydrous sodium sulfate. After filtration, the thus obtained concentrated residue was subjected to slurry purification with heptane/ethyl acetate = 9/1 to obtain a compound 2b (38.7 g, 75.4 mmol) in the form of a colorless solid (yield: 94%).
ESI-MS: calcd: 514.23 [M+H]⁺, found: 514.5 [M+H]^{+ 1}H-NMR (DMSO-d₆, 400 MHz) δ: 11.27 (s, 1H), 8.74 (s, 1H), 8.65 (s, 1H), 8.04 (d, J = 8.7 Hz, 2H), 7.65 (t, J = 7.5 Hz, 1H), 7.55 (t, J = 7.5 Hz, 2H), 6.45 (d, J = 23 Hz, 1H), 5.71 (dd, J = 54.5, 4.1 Hz, 1H), 5.03 (m, 1H), 4.44 (q, J = 3.7 Hz, 1H), 4.09 (m, 2H), 1.11 (s, 9H), 1.02 (s, 9H).

### Step 2 Synthesis of Compound 3b

### N-(9-((4aR,6R,7R,7aS)-2,2-di-tert-butyl-7-fluorotetrahydro-4H-furo[3,2-d][1,3,2]dioxasilin-6-yl)-9H-purin-6-yl)-N-methylbenzamide

The compound 2b (10.0 g, 19.5 mmol) was dissolved in dichloromethane (50 mL), and tetrabutylammonium bromide (9.41 g, 29.2 mmol) and a 1 M sodium hydroxide aqueous solution (50 ml) were added to the resultant. Methyl iodide (1.83 ml, 29.2 mmol) was slowly added thereto in a dropwise manner. Thereafter, the resultant was stirred at room temperature for 1 hour. After confirming disappearance of the raw material, the resultant reaction solution was added to ice cooled water/chloroform = 1/1 for quenching. An organic layer was washed with water twice, and was dehydrated with anhydrous sodium sulfate, the desiccant was filtered out, and the thus obtained filtrate was concentrated. The thus obtained concentrated residue was purified by silica gel column chromatography (heptane/ethyl acetate = 90/10 to 50/50) to obtain a compound 3b (6.86 g, 12.8 mmol) in the form of a colorless amorphous. (yield: 65%)
ESI-MS: calcd: 528.24 [M+H]⁺, found: 538.6 [M+H]^{+ 1}H-NMR (CDCl₃, 400 MHz) δ: 8.54 (s, 1H), 7.94 (s, 1H), 7.47 (d, J = 8.1 Hz, 2H), 7.32 (t, J = 7.3 Hz, 2H), 7.21 (t, J = 7.6 Hz, 2H), 6.10 (d, J = 22.0 Hz, 1H), 5.46 (dd, J = 54.5, 4.1 Hz, 1H), 4.86 (ddd, J = 27.2, 9.8, 4.1 Hz, 1H), 4.47 (dd, J = 9.2, 5.0 Hz, 1H), 4.14 (m, 1H), 4.03 (t, J = 9.8 Hz, 1H), 3.78 (s, 3H), 1.11 (s, 9H), 1.05 (s, 9H).

### Step 3 Synthesis of Compound 4b

### N-(9-((2R,3R,4R,5R)-3-fluoro-4-hydroxy-5-(hydroxymethyl)tetrahydrofuran-2-yl)-9H-purin-6-yl)-N-methylbenzamide

The compound 3b (6.67 g, 12.6 mmol) was dissolved in tetrahydrofuran (66 mL), and the resultant was cooled on an ice bath. To the resultant, triethylamine (8.81 ml, 63.2 mmol) and triethylamine trihydrofluoride (2.05 ml, 12.6 mmol) were added, followed by stirring for 1 hour and 5 minutes with cooling the resultant on an ice bath. After confirming disappearance of the raw material, triethylamine (10.6 ml, 76.0 mmol) was added to the resultant for quenching, the resultant was diluted with chloroform, and then, the resultant reaction solution was concentrated. The thus obtained concentrated residue was purified by silica gel column chromatography (chloroform/methanol = 100/0 to 90/10) to obtain a compound 4b (4.98 g, 12.9 mmol) in the form of a colorless amorphous. (yield: quant.) ESI-MS: calcd: 388.14 [M+H]⁺, found: 388.4 [M+H]^{+ 1}H-NMR (DMSO-d₆, 400 MHz) δ: 8.70 (s, 1H), 8.58 (s, 1H), 7.30 (m, 5H), 6.31 (dd J = 16.9, 2.3 Hz, 1H), 5.75 (d, J = 6.4 Hz, 1H), 5.41 (m, 1H), 5.15 (t, J = 5.3 Hz, 1H), 4.46 (m, 1H), 3.98 (m, 1H), 3.75 (dq, J = 12.4, 2.6 Hz, 1H), 3.67 (s, 3H), 3.61 - 3.56 (m, 1H).

### Step 4 Synthesis of Compound 5b

### N-(9-((2R,3R,4R,5R)-5-((bis(4-methoxyphenyl)(phenyl)methoxy)methyl)-3-fluoro-4-hydroxytetrahydrofuran-2-yl)-9H-purin-6-yl)-N-methylbenzamide

The compound 4b (4.93 g, 12.7 mmol) was dissolved in pyridine (49 mL), and the resultant was stirred on an ice bath. To the resultant reaction solution, 4,4'-dimethoxytrityl chloride (6.47 g, 29.2 mmol) was added, followed by stirring at room temperature for 1 hour and 20 minutes. After confirming disappearance of the raw material, the resultant reaction solution was added to ice cooled sodium bicarbonate water for quenching, followed by extraction with ethyl acetate. An organic layer was washed with a saturated saline solution, was dried over anhydrous sodium sulfate, and was filtered, and the thus obtained filtrate was concentrated. The thus obtained concentrated residue was purified by silica gel column chromatography (heptane/ethyl acetate (containing 1% triethylamine) = 70/30 to 50/50) to obtain a compound 5b (8.34 g, 12.1 mmol) in the form of a colorless amorphous. (yield: 95%)
ESI-MS: calcd: 690.27 [M+H]⁺, found: 690.7 [M+H]^{+ 1}H-NMR (CDCl₃, 400 MHz) δ: 8.51 (s, 1H), 8.10 (s, 1H), 7.43 (dd, J = 8.2, 1.4 Hz, 2H), 7.37 (dd, 8.2, 1.4 Hz, 2H), 7.28-7.20 (m, 8H), 7.12 (t, J = 7.5 Hz, 2H), 6.79 (d, J = 8.7 Hz), 6.23 (dd, J = 17.1, 2.5 Hz, 1H), 5.58 (dq, J = 52.9, 2.3 Hz, 1H), 4.78 (m, 1H), 4.19 (m, 1H), 3.78 (s, 6H), 3.47 (ddd, J = 57.9, 10.6, 3.5 Hz, 2H), 2.44 (dd, J = 7.5, 2.5 Hz, 1H).

### Step 5 Synthesis of Amidite 6b

### (2R,3R,4R,5R)-2-((bis(4-methoxyphenyl)(phenyl)methoxy)methyl)-4-fluoro-5-(6-(N-methylbenzamido)-9H-purin-9-yl)tetrahydrofuran-3-yl(2-cyanoethyl)diisopropylphosphoramidite

The compound 5b (10.0 g, 14.6 mmol) was dissolved in dichloromethane (80 mL), diisopropylethylamine (5.08 mL, 29.1 mmol) was added thereto, and the resultant was cooled on an ice bath. To the resultant, 2-cyanoethyl diisopropylchlorophosphoramidite (4.18 g, 21.8 mmol) dissolved in dichloromethane (15 mL) was added in a dropwise manner over 5 minutes. Thereafter, the resultant was stirred for 1 hour with increasing the temperature up to room temperature. After confirming disappearance of the raw material, the resultant reaction solution was added to ice cooled sodium bicarbonate water for quenching. To the resultant, ethyl acetate was added for extraction. An organic layer was washed with a saturated saline solution, and was dried over anhydrous sodium sulfate, the desiccant was filtered out, and the resultant filtrate was concentrated. The thus obtained concentrated residue was purified by silica gel column chromatography (heptane/ethyl acetate (containing 1% triethylamine) = 70/30 to 50/50) to obtain an amidite 6b (12.1 g, 13.6 mmol) in the form of a colorless amorphous. (yield: 93%)
ESI-MS: calcd: 890.38 [M+H]⁺, found: 890.8 [M+H]^{+ 1}H-NMR (CDCl₃, 400 MHz) δ: 8.53 (s, 0.49H), 8.50 (s, 0.51H), 8.14 (s, 1H), 7.43-7.39 (m, 2H), 7.37-7.33 (m, 2H), 7.27-7.20 (m, 8H), 7.09-7.04 (m, 2H), 6.77 (t, J = 9.1 Hz, 4H), 6.28-6.19 (m, 1H), 5.74 (dq, J = 18.5, 2.2 Hz, 0.50H), 5.61 (dq, J = 19.2, 2.3 Hz, 0.50H), 5.10-5.00 (m, 0.47H), 4.94-4.85 (m, 0.53H), 4.31 (m, 1H), 3.97-3.82 (m, 1H), 3.79 (s, 3H), 3.79 (s, 3H), 3.63-3.53 (m, 4H), 3.31-3.27 (m, 1H), 2.59 (t, J = 6.2 Hz, 1H), 2.41 (t, J = 6.4 Hz, 1H), 1.20-1.15 (m, 9H), 1.04 (d, J = 6.4 Hz, 3H).
³¹P-NMR(CDCl₃, 162 MHz) δ: 151.97, 151.92, 151.19, 151.11.

Synthesis of a compound 6c to be used as a raw material of the polynucleotide was performed in accordance with the following scheme:

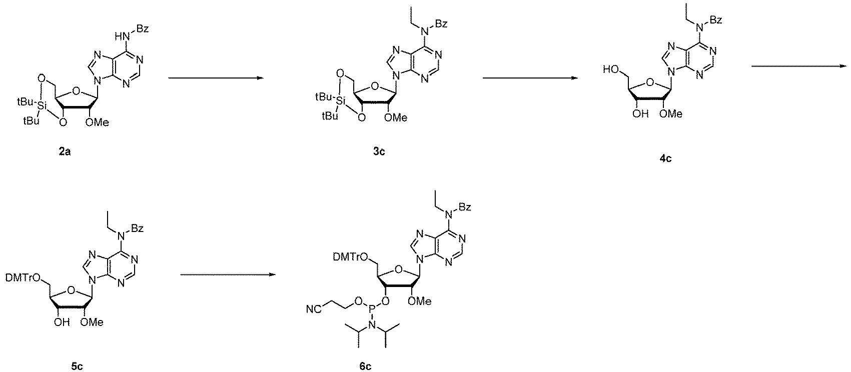

### Step 1 Synthesis of Compound 3c

### N-(9-((4aR,6R,7R,7aS)-2,2-di-tert-butyl-7-methoxytetrahydro-4H-furo[3,2-d][1,3,2]dioxasilin-6-yl)-9H-purin-6-yl)-N-ethylbenzamide

The compound 2a (11.7 g, 22.3 mmol) was dissolved in dichloromethane (58.5 mL), and tetrabutylammonium bromide (10.8 g, 33.4 mmol) and a 1 M sodium hydroxide aqueous solution (58.5 ml) were added thereto. Ethyl iodide (10.8 ml, 134 mmol) was slowly added to the resultant in a dropwise manner. Thereafter, the resultant was stirred at room temperature for 2 hours. After confirming disappearance of the raw material, the resultant reaction solution was added to ice cooled water/chloroform = 1/1 for quenching. An organic layer was washed with water twice, and was dried over anhydrous sodium sulfate. After filtration, the resultant filtrate was concentrated. The thus obtained concentrated residue was subjected to slurry purification with toluene, and the resultant filtrate was concentrated again. The resultant concentrated residue was purified by silica gel column chromatography (heptane/ethyl acetate = 90/10 to 70/30) to obtain a compound 3c (6.14 g, 11.1 mmol) in the form of a colorless amorphous. (yield: 49%) ESI-MS: calcd: 554.28 [M+H]⁺, found: 554.6 [M+H]^{+ 1}H-NMR (CDCl₃, 400 MHz) δ: 8.56 (s, 1H), 7.91 (s, 1H), 7.47-7.45 (m, 2H), 7.31-7.27 (m, 1H), 7.19 (t, J = 7.5 Hz, 2H), 5.94 (s, 1H), 4.61 (dd, J = 9.6, 4.6 Hz, 1H), 4.46 (dd, J = 9.1, 5.0 Hz, 1H), 4.40 (q, J = 7.0 Hz, 2H), 4.22 (d, J = 4.6 Hz, 1H), 4.16 (ddd, J = 10.1, 4.9, 4.8 Hz, 1H), 4.00 (dd, J = 10.5, 9.6 Hz, 1H), 3.67 (s, 3H), 1.34 (t, J = 7.1 Hz, 3H), 1.09 (s, 9H), 1.05 (s, 9H).

### Step 2 Synthesis of Compound 4c

### N-ethyl-N-(9-((2R,3R,4R,5R)-4-hydroxy-5-(hydroxymethyl)-3-methoxytetrahydrofuran-2-yl)-9H-purin-6-yl)benzamide

The compound 3c (6.14 g, 11.1 mmol) was dissolved in tetrahydrofuran (61.4 mL), followed by cooling on an ice bath. Triethylamine (7.73 ml, 55.4 mmol) and triethylamine trihydrofluoride (1.81 ml, 11.1 mmol) were added to the resultant, followed by stirring for 2 hours with cooling on an ice bath. After confirming disappearance of the raw material, triethylamine (10 ml, 76.0 mmol) was added to the resultant for quenching, the resultant was diluted with chloroform, and the resultant reaction solution was concentrated. The thus obtained concentrated residue was purified by silica gel column chromatography (chloroform/methanol = 100/0 to 90/10) to obtain a compound 4c (4.60 g, 11.1 mmol) in the form of a colorless amorphous. (yield: quant.) ESI-MS: calcd: 414.18 [M+H]⁺, found: 414.3 [M+H]^{+ 1}H-NMR (CDCl₃, 400 MHz) δ: 8.56 (s, 1H), 7.92 (s, 1H), 7.50-7.46 (m, 2H), 7.32-7.28 (m, 1H), 7.20 (t, J = 7.5 Hz, 2H), 5.89 (dd, J = 11.6, 2.1 Hz, 1H), 5.85 (d, J = 7.3 Hz, 1H), 4.62 (dd, J = 7.3, 4.6 Hz, 1H), 4.57-4.56 (m, 1H), 4.45-4.39 (m, 2H), 4.36-4.34 (m, 1H), 3.96 (dt, J = 12.9, 1.9, 1H), 3.80-3.73 (m, 1H), 3.29 (s, 3H), 2.70 (d, J = 1.4 Hz, 1H), 1.37 (t, J = 7.1 Hz, 3H).

### Step 3 Synthesis of Compound 5c

### N-(9-((2R,3R,4R,5R)-5-((bis(4-methoxyphenyl)(phenyl)methoxy)methyl)-4-hydroxy-3-methoxytetrahydrofuran-2-yl)-9H-purin-6-yl)-N-ethylbenzamide

The compound 4c (4.58 g, 11.1 mmol) was dissolved in pyridine (46 mL), followed by stirring on an ice bath. To the resultant reaction solution, 4,4'-dimethoxytrityl chloride (5.63 g, 16.6 mmol) was added, followed by stirring at room temperature for 2 hours. After confirming disappearance of the raw material, the resultant reaction solution was added to ice cooled sodium bicarbonate water for quenching, and the resultant was extracted with ethyl acetate. An organic layer was washed with a saturated saline solution, and was dried over anhydrous sodium sulfate. After filtration, the resultant filtrate was concentrated. The thus obtained concentrated residue was purified by silica gel column chromatography (heptane/ethyl acetate (containing 1% triethylamine) = 70/30 to 50/50) to obtain a compound 5c (7.55 g, 10.6 mmol) in the form of a colorless amorphous. (yield: 95%)
ESI-MS: calcd: 716.31 [M+H]⁺, found: 716.2 [M+H]^{+ 1}H-NMR (CDCl₃, 400 MHz) δ: 8.51 (s, 1H), 8.11 (s, 1H), 7.43-7.40 (m, 4H), 7.32-7.22 (m, 8H), 7.13 (t, J = 7.5 Hz, 2H), 6.81 (dd, J = 9.1, 1.4 Hz, 4H), 6.14 (d, J = 3.7 Hz, 1H), 4.47 (dd, J = 5.7, 5.6 Hz, 1H), 4.41 (q, J = 7.0 Hz, 2H), 4.34 (dd, J = 8.4, 4.1 Hz, 1H), 4.21-4.17 (m, 1H), 3.79 (s, 6H), 3.53 (s, 3H), 3.50 (dd, J = 10.5, 3.2 Hz, 1H), 3.39 (dd, J = 10.7, 4.3 Hz, 1H), 2.64 (d, J = 6.4 Hz, 1H), 1.34 (t, J = 7.1 Hz, 3H).

### Step 4 Synthesis of Amidite 6c

### (2R,3R,4R,5R)-2-((bis(4-methoxyphenyl) (phenyl)methoxy)methyl)-5-(6-(N-ethylbenzamido)-9H-purin-9-yl)-4-methoxytetrahydrofuran-3-yl(2-cyanoethyl)diisopropylphosphoramidite

The compound 5c (8.83 g, 12.3 mmol) was dissolved in dichloromethane (74 mL), and diisopropylethylamine (4.31 mL, 24.7 mmol) was added to the resultant, followed by cooling on an ice bath. To the resultant, 2-cyanoethyldiisopropylchlorophosphoramidite (4.40 g, 18.6 mmol) dissolved in dehydrated dichloromethane (14 mL) was added in a dropwise manner over 9 minutes. Thereafter, the resultant was stirred for 1 hour with increasing the temperature up to room temperature. After confirming disappearance of the raw material, the resultant reaction solution was added to ice cooled saturated sodium bicarbonate water for quenching. Ethyl acetate was added to the resultant for extraction. An organic layer was washed with a saturated saline solution, and was dried over anhydrous sodium sulfate. After filtration, the resultant filtrate was concentrated. The thus obtained concentrated residue was purified by silica gel column chromatography (heptane/ethyl acetate (containing 1% triethylamine) = 70/30 to 50/50) to obtain an amidite 6c (10.4 g, 11.3 mmol) in the form of a colorless amorphous. (yield: 92%)
ESI-MS: calcd: 916.42 [M+H]⁺, found: 917.3 [M+H]^{+ 1}H-NMR (CDCl₃, 400 MHz) δ: 8.49 (s, 0.37H), 8.48 (s, 0.63H), 8.11 (s, 0.34H), 8.05 (s, 0.66H), 7.43-7.38 (m, 4H), 7.32-7.21 (m, 8H), 7.11 (t, J = 7.8 Hz, 2H), 6.80 (m, 4H), 6.10 (m, 1H), 4.64-4.52 (m, 2H), 4.43-4.32 (m, 3H), 3.94-3.83 (m, 1H), 3.79-3.78 (m, 6H), 3.67 -3.46 (m, 4H), 3.35-3.29 (m, 1H), 2.64 (t, J = 6.4 Hz, 1.3H), 2.37 (t, J = 6.4 Hz, 0.70H), 1.33 (t, J = 7.1 Hz, 3H), 1.18 (m, 8H), 1.06 (d, J = 6.9 Hz, 4H). ³¹P-NMR(CDCl₃, 162 MHz) δ: 151.67, 150.92.

Synthesis of a compound 6d to be used as a raw material of the polynucleotide was performed in accordance with the following scheme:

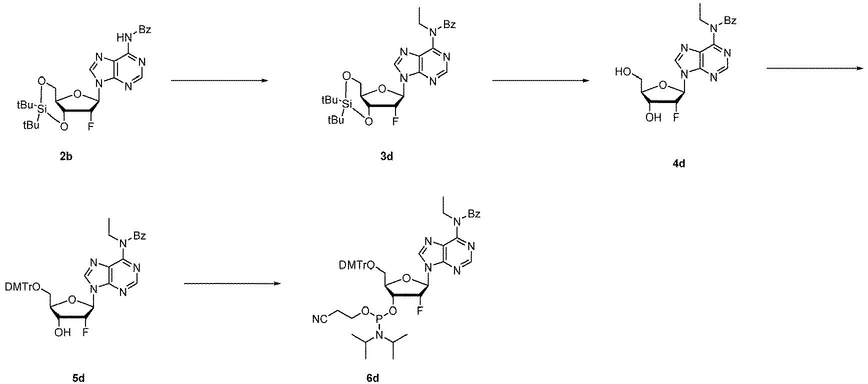

### Step 1 Synthesis of Compound 3d

### N-(9-((4aR,6R,7R,7aS)-2,2-di-tert-butyl-7-fluorotetrahydro-4H-furo[3,2-d][1,3,2]dioxasilin-6-yl)-9H-purin-6-yl)-N-ethylbenzamide

The compound 2b (1.00 g, 1.95 mmol) was dissolved in dichloromethane (5.0 mL), and tetrabutylammonium bromide (0.942 g, 2.92 mmol) and a 1 M sodium hydroxide aqueous solution (5.0 ml) were added thereto. Methyl iodide (0.942 ml, 11.7 mmol) was slowly added thereto in a dropwise manner. Thereafter, the resultant was stirred at room temperature for 2 hours. After confirming disappearance of the raw material, the resultant reaction solution was added to ice cooled water/chloroform = 1/1 for quenching. An organic layer was washed with water twice, and was dehydrated with anhydrous sodium sulfate, the desiccant was filtered out, and the resultant filtrate was concentrated. The thus obtained concentrated residue was purified by silica gel column chromatography (heptane/ethyl acetate = 80/20 to 70/30) to obtain a compound 3d (629 mg, 1.16 mmol) in the form of a colorless amorphous. (yield 60%)
ESI-MS: calcd: 542.26 [M+H]⁺, found: 542.6 [M+H]^{+ 1}H-NMR (CDCl₃, 400 MHz) δ: 8.55 (s, 1H), 7.91 (s, 1H), 7.46 (d, J = 7.3 Hz, 2H), 7.30 (t, J = 7.1 Hz, 1H), 7.19 (t, J = 7.8 hz, 2H), 6.09 (d, J = 22.4 Hz, 1H), 5.45 (dd, J = 54.1, 3.9 Hz, 1H), 4.86 (ddd, J = 27.2, 9.8, 4.1 Hz, 1H), 4.48 (dd, J = 9.1, 5.0, 1H), 4.40 (q, J = 7.2 Hz, 2H), 4.04 (t, J = 9.8 Hz, 1H), 1.34 (t, J = 7.1 Hz, 3H), 1.11 (s, 9H), 1.05 (s, 9H).

### Step 2 Synthesis of Compound 4d

### N-ethyl-N-(9-((2R,3R,4R,5R)-3-fluoro-4-hydroxy-5-(hydroxymethyl)tetrahydrofuran-2-yl)-9H-purin-6-yl)benzamide

A compound 4d was obtained in the same manner as in the procedures for obtaining the compound 4c. ESI-MS: calcd: 401.40 [M+H]⁺, found: 402.1 [M+H]⁺

### Step 3 Synthesis of Compound 5d

### N-(9-((2R,3R,4R,5R)-5-((bis(4-methoxyphenyl)(phenyl)methoxy)methyl)-3-fluoro-4-hydroxytetrahydrofuran-2-yl)-9H-purin-6-yl)-N-ethylbenzamide

A compound 5d was obtained in the same manner as in the procedures for obtaining the compound 5c. ESI-MS: calcd: 738.25 [M+Cl]⁻, found: 738.7 [M+Cl]⁻¹H-NMR (CDCl₃, 400 MHz) δ: 8.52 (s, 1H), 8.07 (s, 1H), 7.43-7.41 (m, 2H), 7.38-7.35 (m, 2H), 7.30-7.20 (m, 8H), 7.10 (t, J = 7.8 Hz, 2H), 6.79 (d, J = 8.2 Hz, 4H), 6.22 (dd, J = 17.4, 2.3 Hz, 1H), 5.58 (ddd, J = 53.0, 2.4, 1.2 Hz, 1H), 4.93-4.74 (m, 1H), 4.40 (q, J = 7.2 Hz, 2H), 4.19-4.16 (m, 1H), 3.78 (s, 6H), 3.54 (dd, J = 11.0, 3.2 Hz, 1H), 3.40 (dd, J = 10.5, 3.1 Hz, 1H), 2.23 (dd, J = 6.9, 2.3 Hz, 1H), 1.33 (t, J = 7.1 Hz, 3H).

### Step 4 Synthesis of Amidite 6d

### (2R,3R,4R,5R)-2-((bis(4-methoxyphenyl)(phenyl)methoxy)methyl)-5-(6-(N-ethylbenzamido)-9H-purin-9-yl)-4-fluorotetrahydrofuran-3-yl(2-cyanoethyl)diisopropylphosphoramidite

The compound 5d (1.93 g, 2.74 mmol) was dissolved in dichloromethane (16 mL), and diisopropylethylamine (0.958 mL, 5.48 mmol) was added thereto, followed by cooling on an ice bath. To the resultant, 2-cyanoethyldiisopropylchlorophosphoramidite (970 mg, 4.11 mmol) dissolved in dehydrated dichloromethane (3.8 mL) was added in a dropwise manner. Thereafter, the resultant was stirred for 1 hour with increasing the temperature up to room temperature. After confirming disappearance of the raw material, the resultant reaction solution was added to ice cooled saturated sodium bicarbonate water for quenching. To the resultant, ethyl acetate was added for extraction. An organic layer was washed with a saturated saline solution, and was dried over anhydrous sodium sulfate. After filtration, the resultant filtrate was concentrated. The thus obtained concentrated residue was purified by silica gel column chromatography (heptane/ethyl acetate (containing 1% triethylamine) = 90/10 to 60/40) to obtain an amidite 6d (2.29 g, 2.53 mmol) in the form of a colorless amorphous. (yield: 92%)
ESI-MS: calcd: 938.36 [M+Cl]⁻, found: 938.7 [M+Cl]⁻¹H-NMR (CDCl₃, 400 MHz) δ: 8.53 (s, 0.5H), 8.51 (s, 0.5H), 8.11 (s, 1H), 7.41-7.33 (m, 4H), 7.27-7.16 (m, 8H), 7.03 (t, J = 7.8 Hz, 2H), 6.79-6.75 (m, 4H), 6.27-6.18 (m, 1H), 5.78-5.58 (m, 1H), 5.10-4.85 (m, 1H), 4.42-4.38 (m, 2H), 4.31-4.30 (m, 1H), 3.96-3.72 (m, 7H), 3.68-3.51 (m, 4H), 3.29-3.27 (m, 1H), 2.59 (t, J = 6.2 Hz, 1H), 2.40 (t, J = 6.4 Hz, 1H), 1.33-1.31 (m, 3H), 1.19-1.15 (m, 9H), 1.04 (d, J = 6.9 Hz, 3H).
³¹P-NMR(CDCl₃, 162 MHz) δ: 151.94, 151.89, 151.20, 151.11.

For an RNA oligonucleotide, 2'-TOM (triisopropylsilyloxymethyl) protected β-cyanoethyl phosphoramidite (DMT-2'-O-TOM-rA(Ac), DMT-2'-O-TOM-rG(Ac), DMT-2'-O-TOM-rC(Ac), or DMT-2'-O-TOM-rU) (each Glen Research Corporation or ChemGenes Corp.) was used, and for a DNA oligonucleotide, β-cyanoethyl phosphoramidite (DMT-dA(Bz), DMT-dG(iBu), DMT-dC(Ac), or DMT-T) was used. Each phosphoramidite monomer was prepared in the form of a 0.05 mol/L acetonitrile solution, and was synthesized with a DNA/RNA solid phase synthesizer (NTS M-2-MX, Nihon Techno Service Co., Ltd.) using 0.2 µmol or 0.8 µmol of a solid phase support.

For obtaining the DNA oligonucleotide, CPG 1000 Angstrom (dA-CPG, dG-CPG, Ac-dC-CPG, or dT-CPG) (Glen Research Corporation) was used as a solid phase support, and a condensation time was set to 2 minutes.

For obtaining an RNA having a phosphate group at the 5' end (5'-monophosphate RNA), Universal UnyLinker Support 2000 Angstrom (ChemGenes Corp.) was used as a solid phase support, and a condensation time for the first base was set to 15 minutes, and for following bases was set to 3 minutes each. Phosphorylation of a hydroxyl group at the 5' end was performed with a chemical phosphorylation reagent (0.05 mol/L acetonitrile solution) (Glen Research Corporation or ChemGenes Corp.).

Solid phase synthesis of an RNA oligonucleotide having a 3'-aminoguanosine monomer introduced to the 3' end was performed using the compound 15. A condensation time for the first base was set to 15 minutes, and for the following bases was set to 3 minutes each.

Reagents used in the solid phase synthesizer were as follows: Removal of a dimethoxytrityl group of a 5' end hydroxyl group was performed using a commercially available deblocking reagent (Deblocking Solution-1, 3 w/v% trichloroacetic acid/dichloromethane solution) (Wako Pure Chemical Industries Ltd.) by causing a reaction for 10 seconds. As an activator of a phosphoramidite, a commercially available activator solution (activator solution 3) (Wako Pure Chemical Industries Ltd.) was used. Capping of an unreacted 5' end hydroxyl group was performed using a commercially available capping solution (Cap A solution-2 and Cap B solution-2) (Wako Pure Chemical Industries Ltd.) by causing a reaction for 10 seconds. As an oxidant used in producing a phosphoric acid ester, a solution containing pyridine, THF, water and iodine (Oxidizer, 0.01 M iodine, 29.2% water, 6.3% pyridine, 64.5% acetonitrile), Honeywell Inc.) was used, and a reaction was performed for 10 seconds. After solid phase synthesis, the dimethoxytrityl group of the 5' end hydroxyl group of the RNA oligonucleotide was deprotected on the solid phase support. The synthesized DNA and RNA oligonucleotides were all deresined/deprotected by an ordinary method (concentrated ammonia water, 55°C, 12 hours). The DNA oligonucleotide was purified with a cartridge column (MicroPure II Column, LGC Biosearch Technologies Inc.) in accordance with product protocol. For the RNA oligonucleotide, a solution obtained by deresination was completely dried and hardened by concentration with a centrifugal evaporator, and thereafter, the TOM protected group of the 2' hydroxyl group was removed with tetrabutylammonium fluoride (1 M tetrahydrofuran solution) (1 mL) (at 50°C for 10 minutes, and subsequently at room temperature for 12 hours, or at 50°C for 10 minutes, and subsequently at 35°C for 6 hours). A Tris-hydrochloric acid buffer (hereinafter referred to as Tris-HCl) (1 M, pH 7.4) (1 mL) was added to and mixed with the resultant solution, and tetrahydrofuran was removed by concentration with a centrifugal evaporator. The thus obtained solution was treated with a gel filtration column (NAP-25, GE Healthcare Ltd.) equilibrated with ultrapure water in accordance with product protocol. The thus obtained fraction containing the RNA oligonucleotide was concentrated with a centrifugal evaporator, followed by purification with modified polyacrylamide gel (hereinafter referred to as dPAGE).

### (Purification of RNA Fragment with dPAGE)

To an acrylamide gel solution (containing 7M urea as a modifier), an aqueous solution of ammonium persulfate (hereinafter referred to as APS) and N,N,N',N'-tetramethylethylenediamine (hereinafter referred to as TEMED) were added as a polymerizing agent, and the resultant was solidified (room temperature, 6 to 12 hours) to produce a gel. An RNA sample was mixed with a gel loading buffer (80% formamide, TBE), and the resultant mixture was heated at 90°C for 3 minutes, and then loaded on the gel. After electrophoresis, a band of the RNA was detected with UV light irradiation (254 nm), and was cut out from the gel with a razor blade. The thus cut gel piece was finely crushed, and extracted from the gel with ultrapure water (shaking at room temperature for 6 to 12 hours). The RNA extract thus obtained was desalted/concentrated with Amicon Ultra 10K (Millipore Corp.), and subjected to ethanol precipitation (0.3 M sodium acetate (pH 5.2)/70% ethanol) to obtain an RNA pellet. The RNA pellet was rinsed with 80% ethanol, and was air-dried at room temperature for 1 hour. The resultant RNA pellet was dissolved in ultrapure water, the resultant was diluted to an appropriate concentration, and was measured for an absorbance at 260 nm by ultraviolet visible spectrophotometry (NanoDrop, Thermo Scientific), and the concentration was determined based on a molar extinction coefficient of each RNA sequence (with the following numerical values used as molar extinction coefficients of respective bases: A = 15300, G = 11800, C = 7400, T = 9300, and U = 9900).

The structure of the purified oligonucleotide was determined by mass spectrometry with MALDI-TOF MS (Ultraflex III, Bruker Daltonics) (matrix: 3-hydroxypicolinic acid) or through analysis by modified polyacrylamide gel electrophoresis.

### (Analysis of Chemical Ligation Reaction with dPAGE)

In analysis of a chemical ligation reaction, a reaction solution appropriately diluted with ultrapure water was used as a sample. The diluted sample was mixed with a gel loading buffer (80% formamide/TBE), and the resultant mixture was heated at 90°C for 3 minutes, and then loaded on a gel. After electrophoresis, gel staining (room temperature, 15 minutes) was performed with SYBR(R) Green II Nucleic Acid Stain (Lonza) diluted 10,000-fold with ultrapure water, and thus a band of the RNA was detected (used device: ChemiDoc, BIORAD).

A yield in a chemical ligation reaction was calculated through comparison of band intensity of an RNA ligation product with a ligation product isolated and purified with dPAGE used as a reference substance.

### (Purification of Chemical Ligation Product with dPAGE)

An RNA ligation product obtained by a chemical ligation reaction was collected as an RNA pellet from a reaction solution by ethanol precipitation (0.3 M sodium acetate (pH 5.2)/70% ethanol), and then purified with dPAGE.

Each nucleotide N (upper case) in the following Tables 1 to 27-5 indicates an RNA, each nucleotide n (lower case) indicates a DNA, N(M) indicates a 2'-O-methyl modified RNA, N(F) indicates a 2'-F modified RNA, N(L) indicates an LNA, and N(MOE) indicates a 2'-O-methoxyethyl modified RNA. Am6 indicates that a base portion is N6-methyladenine. It is noted that a DNA may be referred to as a dN. p indicates that the 3' or 5' end is phosphorylated. A sign ^ indicates that a phosphate group linking between sugar portions is phosphorothioate. Underlined "AUG" indicates a start codon, and underlined "UGA" or "TGA" indicates a stop codon.

Sequence information of compounds (polynucleotides) used in Example 1 and Example 2 are shown in the following Table 1:

### Table 1:

**[Table 1]**

| Compound Name | Sequence (5' to 3') | SEQ ID NO: |
|---|---|---|
| E1 | GG(F)GAG(F)AAU(F)ACA(F)AGC(F)UAC(F)UUG(F)UUC(F)UUU(F)UUG(F)CAG(F) CCA(F)CCA(F)UGG(F)ACU(F)ACA(F)AGG(F)ACG(F)ACG(F)ACG(F)ACA(F)AGA( F)UCA(F)UCG(F)ACU(F)AUA(F)AAGACGACG(F)ACG(F)AUA(F)AAG(F)GUG(F)G CG(F)ACU(F)AUA(F)AGG(F)ACG(F)ACG(F)ACG(F)ACA(F)AAC(F)ACC(F)ACC(F) ACC(F)ACC(F)ACC(F)ACU(F)GA | 1 |
| E1-1 | GG(F)GAG(F)AAU(F)ACA(F)AGC(F)UAC(F)UUG(F)UUC(F)UUU(F)UUG(F)CAG(F) CCA(F)CCA(F)UGG(F)ACU(F)ACA(F)AGG(F)ACG(F)ACG(F)ACG(F)ACA(F)AGA(F )UCA(F)UCG(F)ACU(F)AUA(F)AAG | 2 |
| E1-2 | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACU(F)GA | 3 |
| Template DNA1 | cctttatcgtcgtcgtctttatagtcgatg | 4 |
| E2 | G(MOE)^G(MOE)^G(MOE)^A(MOE)^G(MOE)^A(MOE)^A(F)^U(M)^A(F)^C(M)^A(F)^ A(M)^G(F)C(M)U(F)A(M)C(F)U(M)U(F)G(M)U(F)U(M)C(F)U(M)U(F)U(M)U(F)U(M)G (F)C(M)A(F)G(M)C(F)C(M)A(F)C(M)C(F)AUGG(F)ACU(F)ACA(F)AGG(F)ACG(F)A CG(F)ACG(F)ACA(F)AGA(F)UCA(F)UCG(F)ACU(F)AUA(F)AAG(F)ACG(F)ACG(F) ACG(F)AUA(F)AAG(F)GUG(F)GCG(F)ACU(F)AUA(F)AGG(F)ACG(F)ACG(F)ACG( F)ACA(F)AAC(F)ACC(F)ACC(F)ACC(F)ACC(F)ACC(F)ACT(MOE)^G(MOE)^A(MO E)^A(F)^A(M)^A(F)^A(M)^A(F)^A(M)^A(F)^A(M)^A(F)^A(M)^A(F)^A(M)^A(F)^A(M)^ A(F)^A(M)^A(F)^A(MOE)^A(MOE)^A(MOE) | 5 |
| E2-1 | G(MOE)^G(MOE)^G(MOE)^A(MOE)^G(MOE)^A(MOE)^A(F)^U(M)^A(F)^C(M)^A(F)^ A(M)^G(F)C(M)U(F)A(M)C(F)U(M)U(F)G(M)U(F)U(M)C(F)U(M)U(F)U(M)U(F)U(M)G (F)C(M)A(F)G(M)C(F)C(M)A(F)C(M)C(F)AUGG(F)ACU(F)ACA(F)AGG(F)ACG(F)A CG(F)ACG(F)ACA(F)AGA(F)UCA(F)UCG(F)ACU(F)AUA(F)AAG(F)p | 6 |
| E2-2 | ACG(F)ACG(F)ACG(F)AUA(F)AAG(F)GUG(F)GCG(F)ACU(F)AUA(F)AGG(F)ACG( F)ACG(F)ACG(F)ACA(F)AAC(F)ACC(F)ACC(F)ACC(F)ACC(F)ACC(F)ACT(MOE)^ G(MOE)^A(MOE)^A(F)^A(M)^A(F)^A(M)^A(F)^A(M)^A(F)^A(M)^A(F)^A(M)^A(F)^A( M)^A(F)^A(M)^A(F)^A(M)^A(F)^A(MOE)^A(MOE)^A(MOE) | 7 |

### Example 1 (Linking with Enzyme)

Ultrapure water solutions (200 µL, nucleic acid final concentration: 50 µM) of an RNA fragment E1-1 (10 nmol and a 5' phosphate RNA fragment E1-2 (10 nmol) obtained by solid phase synthesis, and a template DNA1 (10 nmol) were prepared in 3 batches. To each of the prepared solutions, 100 µL of T4 RNA Ligase 2 Reaction Buffer (10X) (manufactured by New England BioLabs, Inc.) and 440 µL of ultrapure water were added, and the resultant was heated at 90°C for 5 minutes, and was returned to room temperature over 30 minutes or more. To each of the resultant solutions, a 60% PEG 6000 aqueous solution was added to a final concentration of 15%. To the resultant solution, T4 RNA Ligase 2 (manufactured by New England BioLabs, Inc.) (10 units/µL) (10 µL) was added to be mixed, and the resultant was allowed to stand still on a temperature controlled heat block (37°C, 16 hours). To the resultant reaction solution, chloroform in the same volume was added to be mixed by vortex, the resultant was centrifuged, and then, an upper layer was taken out and subjected to alcohol precipitation (0.3 M sodium acetate aqueous solution (pH 5.2)/70% ethanol), and thus, an RNA pellet was obtained. RNAs obtained in the respective batches were collected, and the resultant was purified with a 7.5% modified polyacrylamide gel to obtain an RNA ligation product E1 (9.7 nmol, yield: 32%).

### Example 2 (Linking by Chemical Reaction)

To a nucleic acid mixed solution of a 3' phosphate RNA fragment E2-1 (10 nmol) obtained by solid phase synthesis and an RNA fragment E2-2 (10 nmol) obtained as a sequence 7, and a template DNA1 (20 nmol), a 1 M sodium chloride aqueous solution and ultrapure water were added to prepare a 100 mM sodium chloride aqueous solution (180 µL). The thus prepared solution was heated at 90°C for 5 minutes, and was returned to room temperature over 30 minutes or more. To the resultant solution, a 100 mM zinc (II) chloride aqueous solution was added to a final concentration of 5 mM. After adding a 100 mM 1H-imidazole-1-carbonitrile (manufactured by Apollo Scientific)/DMSO solution to the resultant solution to a final concentration of 5 mM to be mixed, the resultant was allowed to stand still on a temperature controlled heat block (30°C, 20 hours). To the resultant reaction solution, 50 µL of a 2 M triethylamine/acetic acid (TEAA) solution was added, the resultant was subjected to simple purification with NAP-10 Columns (manufactured by Cytiva), and a fraction having an absorption wavelength of A260 was centrifugally concentrated or freeze-dried. Crude product RNAs obtained by the simple purification were collected, and purified with a 7.5% modified polyacrylamide gel to obtain an RNA ligation product E2 (2.5 nmol, yield: 25%).

### Example 3

In Tables 2-1 to 2-60 below, rSpacer, dSpacer, Pyrrolidine, Ethynyl-dSpacer, C3, C2, and Spacer9 are spacer modifications used instead of sugar portions of nucleotides, and have the following structures:

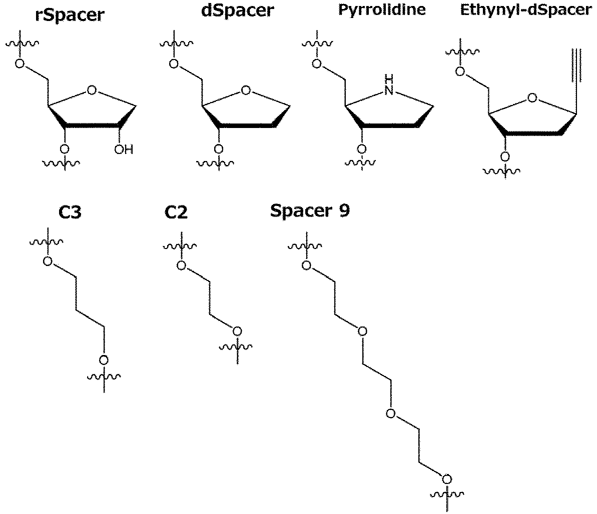

Tables 2-1 to 2-60 below show sequence information and synthesis methods of compounds (polynucleotides) used in Example 3. Tables 3-1 to 3-14 below show yields (%) and MS (found values) of the compounds (polynucleotides) of Example 3.

It is noted that MS (found value) was measured with LC (1260 Infinity II)/MSD XT (G6135B) available from Agilent Technologies.

Table 2-1 to Table 2-60:

**[Table 2-1]**

| Compound Name | Synthesis Method | Sequence (5' to 3') | SEQ ID NO: |
|---|---|---|---|
| E3 | Same as Example 1 | GGGAGAAUACAAGCUACUUGUUCUUUUUGCAGCCACCAUGGACUACAAGGAC GACGACGACAAGAUCAUCGACUAUAAAGACGACGACGAUAAAGGUGGCGACU AUAAGGACGACGACGACAAACACCACCACCACCACCACUGA | 8 |
| E3-1 | Solid Phase Synthesis | GGGAGAAUACAAGCUACUUGUUCUUUUUGCAGCCACCAUGGACUACAAGGAC GACGACGACAAGAUCAUCGACUAUAAAG | 9 |
| E3-2 | Solid Phase Synthesis | pACGACGACGAUAAAGGUGGCGACUAUAAGGACGACGACGACAAACACCACCA CCACCACCACUGA | 10 |
| E4 | Same as Example 1 | GGGAGAAUACAAGCUACUUGUUCUUUUUGCAGCCACCAUGGACUACAAGGAC GACGACGACAAGAUCAUCGACUAUAAAGACGACGAUAAAGGUGGCGACU AUAAGGACGACGACGACAAACACCACCACCACCACCACUGAAAAAAAAAAAA AAAAAAAA | 11 |
| E4-1 | Solid Phase Synthesis | GGGAGAAUACAAGCUACUUGUUCUUUUUGCAGCCACCAUGGACUACAAGGAC GACGACGACAAGAUCAUCGACUAUAAAG | 12 |
| E4-2 | Solid Phase Synthesis | pACGACGACGAUAAAGGUGGCGACUAUAAGGACGACGACGACAAACACCACCA CCACCACCACUGAAAAAAAAAAAAAAAAAAAAAA | 13 |
| E5 | Same as Example 1 | G(M)G(M)G(M)A(M)G(M)A(M)AUACAAGCUACUUGUUCUUUUUGCAGCCACCAUG G(F)ACU(F)ACA(F)AGG(F)ACG(F)ACG(F)ACG(F)ACA(F)AGA(F)UCA(F)UCG(F)A CU(F)AUA(F)AAGACGACG(F)ACG(F)AUA(F)AAG(F)GUG(F)GCG(F)ACU(F)AUA( F)AGG(F)ACG(F)ACG(F)ACG(F)ACA(F)AAC(F)ACC(F)ACC(F)ACC(F)ACC(F)AC C(F)ACU(M)G(M)A(M) | 14 |
| E5-1 | Solid Phase Synthesis | G(M)G(M)G(M)A(M)G(M)A(M)AUACAAGCUACUUGUUCUUUUUGCAGCCACCAUG G(F)ACU(F)ACA(F)AGG(F)ACG(F)ACG(F)ACG(F)ACA(F)AGA(F)UCA(F)UCG(F)A CU(F)AUA(F)AAG | 15 |
| E5-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACU(M)G(M) A(M) | 16 |
| E6 | Same as Example 1 | G(F)G(M)G(F)A(M)G(F)A(M)A(F)U(M)A(F)C(M)A(F)A(M)G(F)C(M)U(F)A(M)C(F)U( M)U(F)G(M)U(F)U(M)C(F)U(M)U(F)U(M)U(F)U(M)G(F)C(M)A(F)G(M)C(F)C(M)A(F) C(M)C(F)AUGGACUACAAGGACGACGACGACAAGAUCAUCGACUAUAAAGACG ACGACGAUAAAGGUGGCGACUAUAAGGACGACGACGACAAACACCACCACCA CCACCACUGA | 17 |
| E6-1 | Solid Phase Synthesis | G(F)G(M)G(F)A(M)G(F)A(M)A(F)U(M)A(F)C(M)A(F)A(M)G(F)C(M)U(F)A(M)C(F)U( M)U(F)G(M)U(F)U(M)C(F)U(M)U(F)U(M)U(F)U(M)G(F)C(M)A(F)G(M)C(F)C(M)A(F) C(M)C(F)AUGGACUACAAGGACGACGACGACAAGAUCAUCGACUAUAAAG | 18 |

**[Table 2-2]**

| | | | |
|---|---|---|---|
| E6-2 | Solid Phase Synthesis | pACGACGACGAUAAAGGUGGCGACUAUAAGGACGACGACGACAAACACCACCA CCACCACCACUGA | 19 |
| E7 | Same as Example 1 | G(M)G(M)G(M)A(M)G(M)A(M)AUACAAGCUACUUGUUCUUUUUGCAGCCACCAUG GACUACAAGGACGACGACGACAAGAUCAUCGACUAUAAAGACGACGACGAUA AAGGUGGCGACUAUAAGGACGACGACGACAAACACCACCACCACCACCACU(M )G(M)A(M)AAAAAAAAAAAAAAAAAAAA | 20 |
| E7-1 | Solid Phase Synthesis | G(M)G(M)G(M)A(M)G(M)A(M)AUACAAGCUACUUGUUCUUUUUGCAGCCACCAUG GACUACAAGGACGACGACGACAAGAUCAUCGACUAUAAAG | 21 |
| E7-2 | Solid Phase Synthesis | pACGACGACGAUAAAGGUGGCGACUAUAAGGACGACGACGACAAACACCACCA CCACCACCACU(M)G(M)A(M)AAAAAAAAAAAAAAAAAAA | 22 |
| E8 | Same as Example 1 | G(M)G(M)G(M)A(M)G(M)A(M)AUACAAGCUACUUGUUCUUUUUGCAGCCACCAUG GACUACAAGGACGACGACGACAAGAUCAUCGACUAUAAAGACGACGACGAUA AAGGUGGCGACUAUAAGGACGACGACGACAAACACCACCACCACCACCACU(M )G(M)A(M)A(M)A(M)AA(M)A(M)AA(M)AA(M)A(F)AA(M)AAA(F)AA(M)AAA(F) | 23 |
| E8-1 | Solid Phase Synthesis | G(M)G(M)G(M)A(M)G(M)A(M)AUACAAGCUACUUGUUCUUUUUGCAGCCACCAUG GACUACAAGGACGACGACGACAAGAUCAUCGACUAUAAAG | 24 |
| E8-2 | Solid Phase Synthesis | pACGACGACGAUAAAGGUGGCGACUAUAAGGACGACGACGACAAACACCACCA CCACCACCACU(M)G(M)A(M)A(M)A(M)AA(M)A(M)AA(M)AA(M)A(F)AA(M)AAA(F) AA(M)AAA(F) | 25 |
| E9 | Same as Example 1 | G(M)G(M)G(M)A(M)G(M)A(M)AUACAAGCUACUUGUUCUUUUUGCAGCCACCAUG GACUACAAGGACGACGACGACAAGAUCAUCGACUAUAAAGACGACGACGAUA AAGGUGGCGACUAUAAGGACGACGACGACAAACACCACCACCACCACCACU(M )G(M)A(M)AAA(M)AA(F)A(F)AAA(M)A(F)A(F)A(F)A(M)AA(F)A(M)A(M)A(F)A(F)A( M) | 26 |
| E9-1 | Solid Phase Synthesis | G(M)G(M)G(M)A(M)G(M)A(M)AUACAAGCUACUUGUUCUUUUUGCAGCCACCAUG GACUACAAGGACGACGACGACAAGAUCAUCGACUAUAAAG | 27 |
| E9-2 | Solid Phase Synthesis | pACGACGACGAUAAAGGUGGCGACUAUAAGGACGACGACGACAAACACCACCA CCACCACCACU(M)G(M)A(M)AAA(M)AA(F)A(F)AAA(M)A(F)A(F)A(F)A(M)AA(F)A (M)A(M)A(F)A(F)A(M) | 28 |
| E10 | Same as Example 1 | G(MOE)G(MOE)G(MOE)A(MOE)G(MOE)A(MOE)AUACAAGCUACUUGUUCUUUUU GCAGCCACCAUGG(F)ACU(F)ACA(F)AGG(F)ACG(F)ACG(F)ACG(F)ACA(F)AGA( F)UCA(F)UCG(F)ACU(F)AUA(F)AAGACGACG(F)ACG(F)AUA(F)AAG(F)GUG(F)G CG(F)ACU(F)AUA(F)AGG(F)ACG(F)ACG(F)ACG(F)ACA(F)AAC(F)ACC(F)ACC(F) ACC(F)ACC(F)ACC(F)ACU(MOE)G(MOE)A(MOE) | 29 |

**[Table 2-3]**

| | | | |
|---|---|---|---|
| E10-1 | Solid Phase Synthesis | G(MOE)G(MOE)G(MOE)A(MOE)G(MOE)A(MOE)AUACAAGCUACUUGUUCUUUUU GCAGCCACCAUGG(F)ACU(F)ACA(F)AGG(F)ACG(F)ACG(F)ACG(F)ACA(F)AGA( F)UCA(F)UCG(F)ACU(F)AUA(F)AAG | 30 |
| E10-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACU(MOE)G( MOE)A(MOE) | 31 |
| E11 | Same as Example 1 | G(MOE)G(MOE)G(MOE)A(MOE)G(MOE)A(MOE)AUACAAGCUACUUGUUCUUUUU GCAGCCACCAUGG(F)ACU(F)ACA(F)AGG(F)ACG(F)ACG(F)ACG(F)ACA(F)AGA( F)UCA(F)UCG(F)ACU(F)AUA(F)AAGACGACG(F)ACG(F)AUA(F)AAG(F)GUG(F)G CG(F)ACU(F)AUA(F)AGG(F)ACG(F)ACG(F)ACG(F)ACA(F)AAC(F)ACC(F)ACC(F) ACC(F)ACC(F)ACC(F)ACU(MOE)G(MOE)A(MOE)AAAAAAAAAAAAAAAAAAAA | 32 |
| E11-1 | Solid Phase Synthesis | G(MOE)G(MOE)G(MOE)A(MOE)G(MOE)A(MOE)AUACAAGCUACUUGUUCUUUUU GCAGCCACCAUGG(F)ACU(F)ACA(F)AGG(F)ACG(F)ACG(F)ACG(F)ACA(F)AGA( F)UCA(F)UCG(F)ACU(F)AUA(F)AAG | 33 |
| E11-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACU(MOE)G( MOE)A(MOE)AAAAAAAAAAAAAAAAAAAA | 34 |
| E12 | Same as Example 1 | G(MOE)^G(MOE)^G(MOE)A(MOE)G(MOE)A(MOE)AUACAAGCUACUUGUUCUUUU UGCAGCCACCAUGG(F)ACU(F)ACA(F)AGG(F)ACG(F)ACG(F)ACG(F)ACA(F)AG A(F)UCA(F)UCG(F)ACU(F)AUA(F)AAGACGACG(F)ACG(F)AUA(F)AAG(F)GUG(F) GCG(F)ACU(F)AUA(F)AGG(F)ACG(F)ACG(F)ACG(F)ACA(F)AAC(F)ACC(F)ACC( F)ACC(F)ACC(F)ACC(F)ACU(MOE)^G(MOE)^A(MOE)A(F)AAA(F)AAA(F)AAA(F)A AA(F)AAA(F)AA(MOE)^A(MOE)^A(MOE) | 35 |
| E12-1 | Solid Phase Synthesis | G(MOE)^G(MOE)^G(MOE)A(MOE)G(MOE)A(MOE)AUACAAGCUACUUGUUCUUUU UGCAGCCACCAUGG(F)ACU(F)ACA(F)AGG(F)ACG(F)ACG(F)ACG(F)ACA(F)AG A(F)UCA(F)UCG(F)ACU(F)AUA(F)AAG | 36 |
| E12-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACU(MOE)^G (MOE)^A(MOE)A(F)AAA(F)AAA(F)AAA(F)AAA(F)AAA(F)AA(MOE)^A(MOE)^A(MO E) | 37 |
| E13 | Same as Example 1 | G(M)G(M)G(M)A(M)G(M)A(M)AUACAAGCUACUUGUUCUUUUUGCAGCCACCAUG G(F)ACU(F)ACA(F)AGG(F)ACG(F)ACG(F)ACG(F)ACA(F)AGA(F)UCA(F)UCG(F)A CU(F)AUA(F)AAGACGACG(F)ACG(F)AUA(F)AAG(F)GUG(F)GCG(F)ACU(F)AUA( F)AGG(F)ACG(F)ACG(F)ACG(F)ACA(F)AAC(F)ACC(F)ACC(F)ACC(F)ACC(F)AC C(F)ACU(M)G(M)A(M)AAAAAAAAAAAAAAAAAAAA | 38 |

**[Table 2-4]**

| | | | |
|---|---|---|---|
| E13-1 | Solid Phase Synthesis | G(M)G(M)G(M)A(M)G(M)A(M)AUACAAGCUACUUGUUCUUUUUGCAGCCACCAUG G(F)ACU(F)ACA(F)AGG(F)ACG(F)ACG(F)ACG(F)ACA(F)AGA(F)UCA(F)UCG(F)A CU(F)AUA(F)AAG | 39 |
| E13-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACU(M)G(M) A(M)AAAAAAAAAAAAAAAAAAAA | 40 |
| E14 | Same as Example 1 | G(M)G(M)G(M)A(M)G(M)A(M)AUACAAGCUACUUGUUCUUUUUGCAGCCACCAUG G(F)ACU(F)ACA(F)AGG(F)ACG(F)ACG(F)ACG(F)ACA(F)AGA(F)UCA(F)UCG(F)A CU(F)AUA(F)AAGACGACG(F)ACG(F)AUA(F)AAG(F)GUG(F)GCG(F)ACU(F)AUA( F)AGG(F)ACG(F)ACG(F)ACG(F)ACA(F)AAC(F)ACC(F)ACC(F)ACC(F)ACC(F)AC C(F)ACU(M)G(M)A(M)A(F)A(F)A(F)A(F)A(F)A(F)A(F)A(F)A(F)A(F)A(F)A(F)A(F)A( F)A(F)A(F)A(F)A(F)A(F)A(F)A(F) | 41 |
| E14-1 | Solid Phase Synthesis | G(M)G(M)G(M)A(M)G(M)A(M)AUACAAGCUACUUGUUCUUUUUGCAGCCACCAUG G(F)ACU(F)ACA(F)AGG(F)ACG(F)ACG(F)ACG(F)ACA(F)AGA(F)UCA(F)UCG(F)A CU(F)AUA(F)AAG | 42 |
| E14-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACU(M)G(M) A(M)A(F)A(F)A(F)A(F)A(F)A(F)A(F)A(F)A(F)A(F)A(F)A(F)A(F)A(F)A(F)A(F)A(F)A( F)A(F)A(F) | 43 |
| E15 | Same as Example 1 | G(M)G(M)G(M)A(M)G(M)A(M)AUACAAGCUACUUGUUCUUUUUGCAGCCACCAUG G(F)ACU(F)ACA(F)AGG(F)ACG(F)ACG(F)ACG(F)ACA(F)AGA(F)UCA(F)UCG(F)A CU(F)AUA(F)AAGACGACG(F)ACG(F)AUA(F)AAG(F)GUG(F)GCG(F)ACU(F)AUA( F)AGG(F)ACG(F)ACG(F)ACG(F)ACA(F)AAC(F)ACC(F)ACC(F)ACC(F)ACC(F)AC C(F)ACU(M)G(M)A(M)A(M)A(M)A(M)A(M)A(M)A(M)A(M)A(M)A(M)A(M)A(M)A(M)A (M)A(M)A(M)A(M)A(M)A(M)A(M)A(M) | 44 |
| E15-1 | Solid Phase Synthesis | G(M)G(M)G(M)A(M)G(M)A(M)AUACAAGCUACUUGUUCUUUUUGCAGCCACCAUG G(F)ACU(F)ACA(F)AGG(F)ACG(F)ACG(F)ACG(F)ACA(F)AGA(F)UCA(F)UCG(F)A CU(F)AUA(F)AAG | 45 |
| E15-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACU(M)G(M) A(M)A(M)A(M)A(M)A(M)A(M)A(M)A(M)A(M)A(M)A(M)A(M)A(M)A(M)A(M)A(M)A(M) A(M)A(M)A(M)A(M) | 46 |

**[Table 2-5]**

| | | | |
|---|---|---|---|
| E16 | Same as Example 1 | G(M)G(M)G(M)A(M)G(M)A(M)AUACAAGCUACUUGUUCUUUUUGCAGCCACCAUG G(F)ACU(F)ACA(F)AGG(F)ACG(F)ACG(F)ACG(F)ACA(F)AGA(F)UCA(F)UCG(F)A CU(F)AUA(F)AAGACGACG(F)ACG(F)AUA(F)AAG(F)GUG(F)GCG(F)ACU(F)AUA( F)AGG(F)ACG(F)ACG(F)ACG(F)ACA(F)AAC(F)ACC(F)ACC(F)ACC(F)ACC(F)AC C(F)ACU(M)G(M)A(M)A(F)A(M)A(F)A(M)A(F)A(M)A(F)A(M)A(F)A(M)A(F)A(M)A(F) A(M)A(F)A(M)A(F)A(M)A(F)A(M) | 47 |
| E16-1 | Solid Phase Synthesis | G(M)G(M)G(M)A(M)G(M)A(M)AUACAAGCUACUUGUUCUUUUUGCAGCCACCAUG G(F)ACU(F)ACA(F)AGG(F)ACG(F)ACG(F)ACG(F)ACA(F)AGA(F)UCA(F)UCG(F)A CU(F)AUA(F)AAG | 48 |
| E16-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACU(M)G(M) A(M)A(F)A(M)A(F)A(M)A(F)A(M)A(F)A(M)A(F)A(M)A(F)A(M)A(F)A(M)A(F)A(M)A( F)A(M)A(F)A(M) | 49 |
| E17 | Same as Example 1 | G(M)G(M)G(M)A(M)G(M)A(M)AUACAAGCUACUUGUUCUUUUUGCAGCCACCAUG G(F)ACU(F)ACA(F)AGG(F)ACG(F)ACG(F)ACG(F)ACA(F)AGA(F)UCA(F)UCG(F)A CU(F)AUA(F)AAGACGACG(F)ACG(F)AUA(F)AAG(F)GUG(F)GCG(F)ACU(F)AUA( F)AGG(F)ACG(F)ACG(F)ACG(F)ACA(F)AAC(F)ACC(F)ACC(F)ACC(F)ACC(F)AC C(F)ACU(M)G(M)A(M)^A(F)^A(M)^A(F)^A(M)^A(F)^A(M)^A(F)^A(M)^A(F)^A(M)^A( F)^A(M)^A(F)^A(M)^A(F)^A(M)^A(F)^A(M)^A(F)^A(M) | 50 |
| E17-1 | Solid Phase Synthesis | G(M)G(M)G(M)A(M)G(M)A(M)AUACAAGCUACUUGUUCUUUUUGCAGCCACCAUG G(F)ACU(F)ACA(F)AGG(F)ACG(F)ACG(F)ACG(F)ACA(F)AGA(F)UCA(F)UCG(F)A CU(F)AUA(F)AAG | 51 |
| E17-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACU(M)G(M) A(M)^A(F)^A(M)^A(F)^A(M)^A(F)^A(M)^A(F)^A(M)^A(F)^A(M)^A(F)^A(M)^A(F)^A( M)^A(F)^A(M)^A(F)^A(M)^A(F)^A(M) | 52 |
| E18 | Same as Example 1 | G(M)G(M)G(M)A(M)G(M)A(M)A(M)U(M)A(M)C(M)A(M)A(M)G(M)C(M)U(M)A(M)C(M )U(M)U(M)G(M)U(M)U(M)C(M)U(M)U(M)U(M)U(M)G(M)C(M)A(M)GCCACCAU GG(F)ACU(F)ACA(F)AGG(F)ACG(F)ACG(F)ACG(F)ACA(F)AGA(F)UCA(F)UCG(F) ACU(F)AUA(F)AAGACGACG(F)ACG(F)AUA(F)AAG(F)GUG(F)GCG(F)ACU(F)AUA (F)AGG(F)ACG(F)ACG(F)ACG(F)ACA(F)AAC(F)ACC(F)ACC(F)ACC(F)ACC(F)AC C(F)ACU(M)G(M)A(M) | 53 |

**[Table 2-6]**

| | | | |
|---|---|---|---|
| E18-1 | Solid Phase Synthesis | G(M)G(M)G(M)A(M)G(M)A(M)A(M)U(M)A(M)C(M)A(M)A(M)G(M)C(M)U(M)A(M)C(M )U(M)U(M)G(M)U(M)U(M)C(M)U(M)U(M)U(M)U(M)G(M)C(M)A(M)GCCACCAU GG(F)ACU(F)ACA(F)AGG(F)ACG(F)ACG(F)ACG(F)ACA(F)AGA(F)UCA(F)UCG(F) ACU(F)AUA(F)AAG | 54 |
| E18-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACU(M)G(M) A(M) | 55 |
| E19 | Same as Example 1 | G(M)G(M)G(M)Am6(M)G(M)Am6(M)Am6(M)U(M)Am6(M)C(M)Am6(M)Am6(M)G(M) C(M)U(M)Am6(M)C(M)U(M)U(M)G(M)U(M)U(M)C(M)U(M)U(M)U(M)U(M)G(M) C(M)Am6(M)GCCACCAUGG(F)ACU(F)ACA(F)AGG(F)ACG(F)ACG(F)ACG(F)ACA( F)AGA(F)UCA(F)UCG(F)ACU(F)AUA(F)AAGACGACG(F)ACG(F)AUA(F)AAG(F)G UG(F)GCG(F)ACU(F)AUA(F)AGG(F)ACG(F)ACG(F)ACG(F)ACA(F)AAC(F)ACC(F) ACC(F)ACC(F)ACC(F)ACC(F)ACU(M)G(M)A(M) | 56 |
| E19-1 | Solid Phase Synthesis | G(M)G(M)G(M)Am6(M)G(M)Am6(M)Am6(M)U(M)Am6(M)C(M)Am6(M)Am6(M)G(M) C(M)U(M)Am6(M)C(M)U(M)U(M)G(M)U(M)U(M)C(M)U(M)U(M)U(M)U(M)G(M) C(M)Am6(M)GCCACCAUGG(F)ACU(F)ACA(F)AGG(F)ACG(F)ACG(F)ACG(F)ACA( F)AGA(F)UCA(F)UCG(F)ACU(F)AUA(F)AAG | 57 |
| E19-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACU(M)G(M) A(M) | 58 |
| E20 | Same as Example 1 | G(M)G(M)G(M)Am6(F)G(M)Am6(F)Am6(F)U(M)Am6(F)C(M)Am6(F)Am6(F)G(M)C( M)U(M)Am6(F)C(M)U(M)U(M)G(M)U(M)U(M)C(M)U(M)U(M)U(M)U(M)G(M)C(M )Am6(F)GCCACCAUGG(F)ACU(F)ACA(F)AGG(F)ACG(F)ACG(F)ACG(F)ACA(F)A GA(F)UCA(F)UCG(F)ACU(F)AUA(F)AAGACGACG(F)ACG(F)AUA(F)AAG(F)GUG( F)GCG(F)ACU(F)AUA(F)AGG(F)ACG(F)ACG(F)ACG(F)ACA(F)AAC(F)ACC(F)AC C(F)ACC(F)ACC(F)ACC(F)ACU(M)G(M)A(M) | 59 |
| E20-1 | Solid Phase Synthesis | G(M)G(M)G(M)Am6(F)G(M)Am6(F)Am6(F)U(M)Am6(F)C(M)Am6(F)Am6(F)G(M)C( M)U(M)Am6(F)C(M)U(M)U(M)G(M)U(M)U(M)C(M)U(M)U(M)U(M)U(M)G(M)C(M )Am6(F)GCCACCAUGG(F)ACU(F)ACA(F)AGG(F)ACG(F)ACG(F)ACG(F)ACA(F)A GA(F)UCA(F)UCG(F)ACU(F)AUA(F)AAG | 60 |
| E20-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACU(M)G(M) A(M) | 61 |

**[Table 2-7]**

| | | | |
|---|---|---|---|
| E21 | Same as Example 1 | G(M)G(M)G(M)Am6(F)G(M)Am6(F)Am6(F)UAm6(F)CAm6(F)Am6(F)GCUAm6(F)CU UGUUCUUUUUGCAm6(F)GCCAm6(F)CCAUGG(F)ACU(F)ACA(F)AGG(F)ACG(F)A CG(F)ACG(F)ACA(F)AGA(F)UCA(F)UCG(F)ACU(F)AUA(F)AAGACGACG(F)ACG( F)AUA(F)AAG(F)GUG(F)GCG(F)ACU(F)AUA(F)AGG(F)ACG(F)ACG(F)ACG(F)AC A(F)AAC(F)ACC(F)ACC(F)ACC(F)ACC(F)ACC(F)ACU(M)G(M)A(M) | 62 |
| E21-1 | Solid Phase Synthesis | G(M)G(M)G(M)Am6(F)G(M)Am6(F)Am6(F)UAm6(F)CAm6(F)Am6(F)GCUAm6(F)CU UGUUCUUUUUGCAm6(F)GCCAm6(F)CCAUGG(F)ACU(F)ACA(F)AGG(F)ACG(F)A CG(F)ACG(F)ACA(F)AGA(F)UCA(F)UCG(F)ACU(F)AUA(F)AAG | 63 |
| E21-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACU(M)G(M) A(M) | 64 |
| E22 | Same as Example 1 | G(M)G(M)G(M)Am6(F)G(M)Am6(F)Am6(F)U(F)Am6(F)CAm6(F)Am6(F)GC(F)UAm6 (F)C(F)UUG(F)UUC(F)UUU(F)UUG(F)CAm6(F)G(F)CCAm6(F)CCAUGG(F)ACU(F) ACA(F)AGG(F)ACG(F)ACG(F)ACG(F)ACA(F)AGA(F)UCA(F)UCG(F)ACU(F)AUA( F)AAGACGACG(F)ACG(F)AUA(F)AAG(F)GUG(F)GCG(F)ACU(F)AUA(F)AGG(F)A CG(F)ACG(F)ACG(F)ACA(F)AAC(F)ACC(F)ACC(F)ACC(F)ACC(F)ACC(F)ACU(M) G(M)A(M) | 65 |
| E22-1 | Solid Phase Synthesis | G(M)G(M)G(M)Am6(F)G(M)Am6(F)Am6(F)U(F)Am6(F)CAm6(F)Am6(F)GC(F)UAm6 (F)C(F)UUG(F)UUC(F)UUU(F)UUG(F)CAm6(F)G(F)CCAm6(F)CCAUGG(F)ACU(F) ACA(F)AGG(F)ACG(F)ACG(F)ACG(F)ACA(F)AGA(F)UCA(F)UCG(F)ACU(F)AUA( F)AAG | 66 |
| E22-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACU(M)G(M) A(M) | 67 |
| E23 | Same as Example 1 | G(M)G(M)G(M)A(M)G(M)A(M)A(M)U(M)A(M)C(M)A(M)A(M)G(M)C(M)U(M)A(M)C(M )U(M)U(M)G(M)U(M)U(M)C(M)U(M)U(M)U(M)U(M)G(M)C(M)A(M)G(M)C(M)C( M)A(M)C(M)C(M)AUGG(F)ACU(F)ACA(F)AGG(F)ACG(F)ACG(F)ACG(F)ACA(F)A GA(F)UCA(F)UCG(F)ACU(F)AUA(F)AAGACGACG(F)ACG(F)AUA(F)AAG(F)GUG( F)GCG(F)ACU(F)AUA(F)AGG(F)ACG(F)ACG(F)ACG(F)ACA(F)AAC(F)ACC(F)AC C(F)ACC(F)ACC(F)ACC(F)ACU(M)G(M)A(M) | 68 |
| E23-1 | Solid Phase Synthesis | G(M)G(M)G(M)A(M)G(M)A(M)A(M)U(M)A(M)C(M)A(M)A(M)G(M)C(M)U(M)A(M)C(M )U(M)U(M)G(M)U(M)U(M)C(M)U(M)U(M)U(M)U(M)G(M)C(M)A(M)G(M)C(M)C( M)A(M)C(M)C(M)AUGG(F)ACU(F)ACA(F)AGG(F)ACG(F)ACG(F)ACG(F)ACA(F)A GA(F)UCA(F)UCG(F)ACU(F)AUA(F)AAG | 69 |
| E23-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACU(M)G(M) A(M) | 70 |

**[Table 2-8]**

| | | | |
|---|---|---|---|
| E24 | Same as Example 1 | G(M)G(M)G(M)Am6(M)G(M)Am6(M)Am6(M)U(M)Am6(M)C(M)Am6(M)Am6(M)G(M) C(M)U(M)Am6(M)C(M)U(M)U(M)G(M)U(M)U(M)C(M)U(M)U(M)U(M)U(M)G(M) C(M)Am6(M)G(M)C(M)C(M)Am6(M)C(M)C(M)AUGG(F)ACU(F)ACA(F)AGG(F)ACG (F)ACG(F)ACG(F)ACA(F)AGA(F)UCA(F)UCG(F)ACU(F)AUA(F)AAGACGACG(F)A CG(F)AUA(F)AAG(F)GUG(F)GCG(F)ACU(F)AUA(F)AGG(F)ACG(F)ACG(F)ACG(F) ACA(F)AAC(F)ACC(F)ACC(F)ACC(F)ACC(F)ACC(F)ACU(M)G(M)A(M) | 71 |
| E24-1 | Solid Phase Synthesis | G(M)G(M)G(M)Am6(M)G(M)Am6(M)Am6(M)U(M)Am6(M)C(M)Am6(M)Am6(M)G(M) C(M)U(M)Am6(M)C(M)U(M)U(M)G(M)U(M)U(M)C(M)U(M)U(M)U(M)U(M)U(M)G(M) C(M)Am6(M)G(M)C(M)C(M)Am6(M)C(M)C(M)AUGG(F)ACU(F)ACA(F)AGG(F)ACG (F)ACG(F)ACG(F)ACA(F)AGA(F)UCA(F)UCG(F)ACU(F)AUA(F)AAG | 72 |
| E24-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACU(M)G(M) A(M) | 73 |
| E25 | Same as Example 1 | G(M)G(M)G(M)Am6(F)G(M)Am6(F)Am6(F)U(M)Am6(F)C(M)Am6(F)Am6(F)G(M)C( M)U(M)Am6(F)C(M)U(M)U(M)G(M)U(M)U(M)C(M)U(M)U(M)U(M)U(M)G(M)C(M )Am6(F)G(M)C(M)C(M)Am6(F)C(M)C(M)AUGG(F)ACU(F)ACA(F)AGG(F)ACG(F)A CG(F)ACG(F)ACA(F)AGA(F)UCA(F)UCG(F)ACU(F)AUA(F)AAGACGACG(F)ACG( F)AUA(F)AAG(F)GUG(F)GCG(F)ACU(F)AUA(F)AGG(F)ACG(F)ACG(F)ACG(F)AC A(F)AAC(F)ACC(F)ACC(F)ACC(F)ACC(F)ACC(F)ACU(M)G(M)A(M) | 74 |
| E25-1 | Solid Phase Synthesis | G(M)G(M)G(M)Am6(F)G(M)Am6(F)Am6(F)U(M)Am6(F)C(M)Am6(F)Am6(F)G(M)C( M)U(M)Am6(F)C(M)U(M)U(M)G(M)U(M)U(M)C(M)U(M)U(M)U(M)U(M)G(M)C(M )Am6(F)G(M)C(M)C(M)Am6(F)C(M)C(M)AUGG(F)ACU(F)ACA(F)AGG(F)ACG(F)A CG(F)ACG(F)ACA(F)AGA(F)UCA(F)UCG(F)ACU(F)AUA(F)AAG | 75 |
| E25-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACU(M)G(M) A(M) | 76 |
| E26 | Same as Example 1 | G(M)G(M)G(M)A(M)G(M)A(M)AUACAAGCUACUUGUUCUUUUUGCAGCCACCAUG GACUACAAGGACGACGACGACAAGAUCAUCGACUAUAAAGACGACGACGAUA AAGGUGGCGACUAUAAGGACGACGACGACAAACACCACCACCACCACCACU(M )G(M)A(M)A(F)A(M)AA(F)AA(F)A(F)A(M)AA(F)AA(M)AAA(M)A(M)A(M)A(F)A(F)A | 77 |
| E26-1 | Solid Phase Synthesis | G(M)G(M)G(M)A(M)G(M)A(M)AUACAAGCUACUUGUUCUUUUUGCAGCCACCAUG GACUACAAGGACGACGACGACAAGAUCAUCGACUAUAAAG | 78 |
| E26-2 | Solid Phase Synthesis | pACGACGACGAUAAAGGUGGCGACUAUAAGGACGACGACGACAAACACCACCA CCACCACCACU(M)G(M)A(M)A(F)A(M)AA(F)AA(F)A(F)A(M)AA(F)AA(M)AAA(M)A (M)A(M)A(F)A(F)A | 79 |

**[Table 2-9]**

| | | | |
|---|---|---|---|
| E27 | Same as Example 1 | G(M)G(M)G(M)A(M)G(M)A(M)AUACAAGCUACUUGUUCUUUUUGCAGCCACCAUG GACUACAAGGACGACGACGACAAGAUCAUCGACUAUAAAGACGACGACGAUA AAGGUGGCGACUAUAAGGACGACGACGACAAACACCACCACCACCACCACU(M )G(M)A(M)AA(M)A(M)A(F)A(F)AA(F)A(F)AA(F)A(F)A(M)A(F)A(F)AA(F)AAA(M)A( M) | 80 |
| E27-1 | Solid Phase Synthesis | G(M)G(M)G(M)A(M)G(M)A(M)AUACAAGCUACUUGUUCUUUUUGCAGCCACCAUG GACUACAAGGACGACGACGACAAGAUCAUCGACUAUAAAG | 81 |
| E27-2 | Solid Phase Synthesis | pACGACGACGAUAAAGGUGGCGACUAUAAGGACGACGACGACAAACACCACCA CCACCACCACU(M)G(M)A(M)AA(M)A(M)A(F)A(F)AA(F)A(F)AA(F)A(F)A(M)A(F)A (F)AA(F)AAA(M)A(M) | 82 |
| E28 | Same as Example 1 | G(M)G(M)G(M)A(M)G(M)A(M)AUACAAGCUACUUGUUCUUUUUGCAGCCACCAUG GACUACAAGGACGACGACGACAAGAUCAUCGACUAUAAAGACGACGACGAUA AAGGUGGCGACUAUAAGGACGACGACGACAAACACCACCACCACCACCACU(M )G(M)A(M)A(F)A(F)A(F)A(F)A(F)AA(F)A(M)A(M)AAA(F)A(F)AA(F)AA(M)A(F)AA(M ) | 83 |
| E28-1 | Solid Phase Synthesis | G(M)G(M)G(M)A(M)G(M)A(M)AUACAAGCUACUUGUUCUUUUUGCAGCCACCAUG GACUACAAGGACGACGACGACAAGAUCAUCGACUAUAAAG | 84 |
| E28-2 | Solid Phase Synthesis | pACGACGACGAUAAAGGUGGCGACUAUAAGGACGACGACGACAAACACCACCA CCACCACCACU(M)G(M)A(M)A(F)A(F)A(F)A(F)A(F)AA(F)A(M)A(M)AAA(F)A(F)A A(F)AA(M)A(F)AA(M) | 85 |
| E29 | Same as Example 1 | G(M)G(M)G(M)A(M)G(M)A(M)AUACAAGCUACUUGUUCUUUUUGCAGCCACCAUG GACUACAAGGACGACGACGACAAGAUCAUCGACUAUAAAGACGACGACGAUA AAGGUGGCGACUAUAAGGACGACGACGACAAACACCACCACCACCACCACU(M )G(M)A(M)AAA(M)A(M)A(M)AA(F)A(M)AA(F)A(M)AA(M)AAAAA(M)A(M)A | 86 |
| E29-1 | Solid Phase Synthesis | G(M)G(M)G(M)A(M)G(M)A(M)AUACAAGCUACUUGUUCUUUUUGCAGCCACCAUG GACUACAAGGACGACGACGACAAGAUCAUCGACUAUAAAG | 87 |
| E29-2 | Solid Phase Synthesis | pACGACGACGAUAAAGGUGGCGACUAUAAGGACGACGACGACAAACACCACCA CCACCACCACU(M)G(M)A(M)AAA(M)A(M)A(M)AA(F)A(M)AA(F)A(M)AA(M)AAAA A(M)A(M)A | 88 |
| E30 | Solid Phase Synthesis | GGGAGAAUACAAGCUACUUGUUCUUUUUGCAGCCACCAUGGACUAUAAGGAC GACGACGACAAAGGUGGCCACCACCACCACCACCACUGA | 89 |
| E31 | Solid Phase Synthesis | G(MOE)G(MOE)G(MOE)A(MOE)G(MOE)A(MOE)AUACAAGCUACUUGUUCUUUUU GCAGCCACCAUGGACUAUAAGGACGACGACGACAAAGGUGGCCACCACCACC ACCACCACU(MOE)G(MOE)A(MOE) | 90 |

**[Table 2-10]**

| | | | |
|---|---|---|---|
| E32 | Same as Example 1 | G(M)G(M)G(M)A(M)G(M)A(M)A(F)U(M)A(F)C(M)A(F)A(M)G(F)C(M)U(F)A(M)C(F)U (M)U(F)G(M)U(F)U(M)C(F)U(M)U(F)U(M)G(F)C(M)A(F)G(M)C(F)C(M)A(F )C(M)C(F)AUGG(F)ACU(F)ACA(F)AGG(F)ACG(F)ACG(F)ACG(F)ACA(F)AGA(F)U CA(F)UCG(F)ACU(F)AUA(F)AAGACGACG(F)ACG(F)AUA(F)AAG(F)GUG(F)GCG( F)ACU(F)AUA(F)AGG(F)ACG(F)ACG(F)ACG(F)ACA(F)AAC(F)ACC(F)ACC(F)AC C(F)ACC(F)ACC(F)ACU(M)G(M)A(M) | 91 |
| E32-1 | Solid Phase Synthesis | G(M)G(M)G(M)A(M)G(M)A(M)A(F)U(M)A(F)C(M)A(F)A(M)G(F)C(M)U(F)A(M)C(F)U (M)U(F)G(M)U(F)U(M)C(F)U(M)U(F)U(M)G(F)C(M)A(F)G(M)C(F)C(M)A(F )C(M)C(F)AUGG(F)ACU(F)ACA(F)AGG(F)ACG(F)ACG(F)ACG(F)ACA(F)AGA(F)U CA(F)UCG(F)ACU(F)AUA(F)AAG | 92 |
| E32-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACU(M)G(M) A(M) | 93 |
| E33 | Same as Example 1 | G(M)G(M)G(M)A(M)G(M)A(M)A(M)U(M)A(M)C(M)A(M)A(M)G(F)C(M)U(F)A(M)C(F) U(M)U(F)G(M)U(F)U(M)C(F)U(M)U(F)U(M)U(F)U(M)G(F)C(M)A(F)G(M)C(F)C(M)A( F)C(M)C(F)AUGG(F)ACU(F)ACA(F)AGG(F)ACG(F)ACG(F)ACG(F)ACA(F)AGA(F) UCA(F)UCG(F)ACU(F)AUA(F)AAGACGACG(F)ACG(F)AUA(F)AAG(F)GUG(F)GCG (F)ACU(F)AUA(F)AGG(F)ACG(F)ACG(F)ACG(F)ACA(F)AAC(F)ACC(F)ACC(F)AC C(F)ACC(F)ACC(F)ACU(M)G(M)A(M) | 94 |
| E33-1 | Solid Phase Synthesis | G(M)G(M)G(M)A(M)G(M)A(M)A(M)U(M)A(M)C(M)A(M)A(M)G(F)C(M)U(F)A(M)C(F) U(M)U(F)G(M)U(F)U(M)C(F)U(M)U(F)U(F)U(M)G(F)C(M)A(F)G(M)C(F)C(M)A( F)C(M)C(F)AUGG(F)ACU(F)ACA(F)AGG(F)ACG(F)ACG(F)ACG(F)ACA(F)AGA(F) UCA(F)UCG(F)ACU(F)AUA(F)AAG | 95 |
| E33-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACU(M)G(M) A(M) | 96 |
| E34 | Same as Example 1 | G(M)G(M)G(M)A(M)G(M)A(M)A(M)U(M)A(M)C(M)A(M)A(M)G(M)C(M)U(M)A(M)C(M )U(M)U(M)G(M)U(M)U(M)C(M)U(M)U(F)U(M)U(F)U(M)G(F)C(M)A(F)G(M)C(F)C(M) A(F)C(M)C(F)AUGG(F)ACU(F)ACA(F)AGG(F)ACG(F)ACG(F)ACG(F)ACA(F)AGA( F)UCA(F)UCG(F)ACU(F)AUA(F)AAGACGACG(F)ACG(F)AUA(F)AAG(F)GUG(F)G CG(F)ACU(F)AUA(F)AGG(F)ACG(F)ACG(F)ACG(F)ACA(F)AAC(F)ACC(F)ACC(F) ACC(F)ACC(F)ACC(F)ACU(M)G(M)A(M) | 97 |
| E34-1 | Solid Phase Synthesis | G(M)G(M)G(M)A(M)G(M)A(M)A(M)U(M)A(M)C(M)A(M)A(M)G(M)C(M)U(M)A(M)C(M )U(M)U(M)G(M)U(M)U(M)C(M)U(M)U(F)U(M)U(F)U(M)G(F)C(M)A(F)G(M)C(F)C(M) A(F)C(M)C(F)AUGG(F)ACU(F)ACA(F)AGG(F)ACG(F)ACG(F)ACG(F)ACA(F)AGA( F)UCA(F)UCG(F)ACU(F)AUA(F)AAG | 98 |

**[Table 2-11]**

| | | | |
|---|---|---|---|
| E34-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACU(M)G(M) A(M) | 99 |
| E35 | Same as Example 1 | G(M)G(M)G(M)A(M)G(M)A(M)A(M)U(M)A(M)C(M)A(M)A(M)G(M)C(M)U(M)A(M)C(M )U(M)U(M)G(M)U(M)U(M)C(M)U(M)U(M)U(M)U(M)G(M)C(M)A(M)G(M)C(F)C( M)A(F)C(M)C(F)AUGG(F)ACU(F)ACA(F)AGG(F)ACG(F)ACG(F)ACG(F)ACA(F)AG A(F)UCA(F)UCG(F)ACU(F)AUA(F)AAGACGACG(F)ACG(F)AUA(F)AAG(F)GUG(F) | 100 |
| | | GCG(F)ACU(F)AUA(F)AGG(F)ACG(F)ACG(F)ACG(F)ACA(F)AAC(F)ACC(F)ACC( F)ACC(F)ACC(F)ACC(F)ACU(M)G(M)A(M) | |
| E35-1 | Solid Phase Synthesis | G(M)G(M)G(M)A(M)G(M)A(M)A(M)U(M)A(M)C(M)A(M)A(M)G(M)C(M)U(M)A(M)C(M )U(M)U(M)G(M)U(M)U(M)C(M)U(M)U(M)U(M)U(M)G(M)C(M)A(M)G(M)C(F)C( M)A(F)C(M)C(F)AUGG(F)ACU(F)ACA(F)AGG(F)ACG(F)ACG(F)ACG(F)ACA(F)AG A(F)UCA(F)UCG(F)ACU(F)AUA(F)AAG | 101 |
| E35-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACU(M)G(M) A(M) | 102 |
| E36 | Same as Example 1 | G(MOE)G(MOE)G(MOE)A(MOE)G(MOE)A(MOE)A(F)U(M)A(F)C(M)A(F)A(M)G(F)C (M)U(F)A(M)C(F)U(M)U(F)G(M)U(F)U(M)C(F)U(M)U(F)U(M)U(F)U(M)G(F)C(M)A(F) G(M)C(F)C(M)A(F)C(M)C(F)AUGG(F)ACU(F)ACA(F)AGG(F)ACG(F)ACG(F)ACG(F )ACA(F)AGA(F)UCA(F)UCG(F)ACU(F)AUA(F)AAGACGACG(F)ACG(F)AUA(F)AA G(F)GUG(F)GCG(F)ACU(F)AUA(F)AGG(F)ACG(F)ACG(F)ACG(F)ACA(F)AAC(F)A CC(F)ACC(F)ACC(F)ACC(F)ACC(F)ACU(MOE)G(MOE)A(MOE) | 103 |
| E36-1 | Solid Phase Synthesis | G(MOE)G(MOE)G(MOE)A(MOE)G(MOE)A(MOE)A(F)U(M)A(F)C(M)A(F)A(M)G(F)C (M)U(F)A(M)C(F)U(M)U(F)G(M)U(F)U(M)C(F)U(M)U(M)U(F)U(M)G(F)C(M)A(F) G(M)C(F)C(M)A(F)C(M)C(F)AUGG(F)ACU(F)ACA(F)AGG(F)ACG(F)ACG(F)ACG(F )ACA(F)AGA(F)UCA(F)UCG(F)ACU(F)AUA(F)AAG | 104 |
| E36-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACU(MOE)G( MOE)A(MOE) | 105 |
| E37 | Same as Example 1 | G(MOE)G(MOE)G(MOE)A(MOE)G(MOE)A(MOE)A(MOE)U(MOE)A(MOE)C(MOE)A( MOE)A(MOE)G(F)C(M)U(F)A(M)C(F)U(M)U(F)G(M)U(F)U(M)C(F)U(M)U(F)U(M)U( F)U(M)G(F)C(M)A(F)G(M)C(F)C(M)A(F)C(M)C(F)AUGG(F)ACU(F)ACA(F)AGG(F)A CG(F)ACG(F)ACG(F)ACA(F)AGA(F)UCA(F)UCG(F)ACU(F)AUA(F)AAGACGACG( F)ACG(F)AUA(F)AAG(F)GUG(F)GCG(F)ACU(F)AUA(F)AGG(F)ACG(F)ACG(F)AC G(F)ACA(F)AAC(F)ACC(F)ACC(F)ACC(F)ACC(F)ACC(F)ACU(MOE)G(MOE)A(MO E) | 106 |

**[Table 2-12]**

| | | | |
|---|---|---|---|
| E37-1 | Solid Phase Synthesis | G(MOE)G(MOE)G(MOE)A(MOE)G(MOE)A(MOE)A(MOE)U(MOE)A(MOE)C(MOE)A( MOE)A(MOE)G(F)C(M)U(F)A(M)C(F)U(M)U(F)G(M)U(F)U(M)C(F)U(M)U(F)U(M)U( F)U(M)G(F)C(M)A(F)G(M)C(F)C(M)A(F)C(M)C(F)AUGG(F)ACU(F)ACA(F)AGG(F)A CG(F)ACG(F)ACG(F)ACA(F)AGA(F)UCA(F)UCG(F)ACU(F)AUA(F)AAG | 107 |
| E37-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACU(MOE)G( MOE)A(MOE) | 108 |
| E38 | Same as Example 1 | G(MOE)G(MOE)G(MOE)A(MOE)G(MOE)A(MOE)A(MOE)U(MOE)A(MOE)C(MOE)A( MOE)A(MOE)G(MOE)C(MOE)U(MOE)A(MOE)C(MOE)U(MOE)U(MOE)G(MOE)U(M OE)U(MOE)C(MOE)U(MOE)U(F)U(M)U(F)U(M)G(F)C(M)A(F)G(M)C(F)C(M)A(F)C( M)C(F)AUGG(F)ACU(F)ACA(F)AGG(F)ACG(F)ACG(F)ACG(F)ACA(F)AGA(F)UCA( F)UCG(F)ACU(F)AUA(F)AAGACGACG(F)ACG(F)AUA(F)AAG(F)GUG(F)GCG(F)A CU(F)AUA(F)AGG(F)ACG(F)ACG(F)ACG(F)ACA(F)AAC(F)ACC(F)ACC(F)ACC(F) ACC(F)ACC(F)ACU(MOE)G(MOE)A(MOE) | 109 |
| E38-1 | Solid Phase Synthesis | G(MOE)G(MOE)G(MOE)A(MOE)G(MOE)A(MOE)A(MOE)U(MOE)A(MOE)C(MOE)A( MOE)A(MOE)G(MOE)C(MOE)U(MOE)A(MOE)C(MOE)U(MOE)U(MOE)G(MOE)U(M OE)U(MOE)C(MOE)U(MOE)U(F)U(M)U(F)U(M)G(F)C(M)A(F)G(M)C(F)C(M)A(F)C( M)C(F)AUGG(F)ACU(F)ACA(F)AGG(F)ACG(F)ACG(F)ACG(F)ACA(F)AGA(F)UCA( F)UCG(F)ACU(F)AUA(F)AAG | 110 |
| E38-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACU(MOE)G( MOE)A(MOE) | 111 |
| E39 | Same as Example 1 | G(MOE)G(MOE)G(MOE)A(MOE)G(MOE)A(MOE)A(MOE)U(MOE)A(MOE)C(MOE)A( MOE)A(MOE)G(MOE)C(MOE)U(MOE)A(MOE)C(MOE)U(MOE)U(MOE)G(MOE)U(M OE)U(MOE)C(MOE)U(MOE)U(MOE)U(MOE)U(MOE)U(MOE)G(MOE)C(MOE)A(MOE )G(M)C(F)C(M)A(F)C(M)C(F)AUGG(F)ACU(F)ACA(F)AGG(F)ACG(F)ACG(F)ACG( F)ACA(F)AGA(F)UCA(F)UCG(F)ACU(F)AUA(F)AAGACGACG(F)ACG(F)AUA(F)A AG(F)GUG(F)GCG(F)ACU(F)AUA(F)AGG(F)ACG(F)ACG(F)ACG(F)ACA(F)AAC(F) ACC(F)ACC(F)ACC(F)ACC(F)ACC(F)ACU(MOE)G(MOE)A(MOE) | 112 |
| E39-1 | Solid Phase Synthesis | G(MOE)G(MOE)G(MOE)A(MOE)G(MOE)A(MOE)A(MOE)U(MOE)A(MOE)C(MOE)A( MOE)A(MOE)G(MOE)C(MOE)U(MOE)A(MOE)C(MOE)U(MOE)U(MOE)G(MOE)U(M OE)U(MOE)C(MOE)U(MOE)U(MOE)U(MOE)U(MOE)U(MOE)G(MOE)C(MOE)A(MOE )G(M)C(F)C(M)A(F)C(M)C(F)AUGG(F)ACU(F)ACA(F)AGG(F)ACG(F)ACG(F)ACG( F)ACA(F)AGA(F)UCA(F)UCG(F)ACU(F)AUA(F)AAG | 113 |

**[Table 2-13]**

| | | | |
|---|---|---|---|
| E39-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACU(MOE)G( MOE)A(MOE) | 114 |
| E40 | Same as Example 1 | G(F)G(MOE)G(F)A(MOE)G(F)A(MOE)A(F)U(MOE)A(F)C(MOE)A(F)A(MOE)G(F)C( MOE)U(F)A(MOE)C(F)U(MOE)U(F)G(MOE)U(F)U(MOE)C(F)U(MOE)U(F)U(MOE)U( F)U(MOE)G(F)C(MOE)A(F)G(MOE)C(F)C(MOE)A(F)C(MOE)C(F)AUGG(F)ACU(F) ACA(F)AGG(F)ACG(F)ACG(F)ACG(F)ACA(F)AGA(F)UCA(F)UCG(F)ACU(F)AUA( F)AAGACGACG(F)ACG(F)AUA(F)AAG(F)GUG(F)GCG(F)ACU(F)AUA(F)AGG(F)A CG(F)ACG(F)ACG(F)ACA(F)AAC(F)ACC(F)ACC(F)ACC(F)ACC(F)ACC(F)ACU(M) G(M)A(M) | 115 |
| E40-1 | Solid Phase Synthesis | G(F)G(MOE)G(F)A(MOE)G(F)A(MOE)A(F)U(MOE)A(F)C(MOE)A(F)A(MOE)G(F)C( MOE)U(F)A(MOE)C(F)U(MOE)U(F)G(MOE)U(F)U(MOE)C(F)U(MOE)U(F)U(MOE)U( F)U(MOE)G(F)C(MOE)A(F)G(MOE)C(F)C(MOE)A(F)C(MOE)C(F)AUGG(F)ACU(F) ACA(F)AGG(F)ACG(F)ACG(F)ACG(F)ACA(F)AGA(F)UCA(F)UCG(F)ACU(F)AUA( F)AAG | 116 |
| E40-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACU(M)G(M) A(M) | 117 |
| E41 | Same as Example 1 | G(MOE)G(M)G(MOE)A(M)G(MOE)A(M)A(MOE)U(M)A(MOE)C(M)A(MOE)A(M)G(M OE)C(M)U(MOE)A(M)C(MOE)U(M)U(MOE)G(M)U(MOE)U(M)C(MOE)U(M)U(MOE)U (M)U(MOE)U(M)G(MOE)C(M)A(MOE)G(M)C(MOE)C(M)A(MOE)C(M)C(MOE)AUGG (F)ACU(F)ACA(F)AGG(F)ACG(F)ACG(F)ACG(F)ACA(F)AGA(F)UCA(F)UCG(F)AC U(F)AUA(F)AAGACGACG(F)ACG(F)AUA(F)AAG(F)GUG(F)GCG(F)ACU(F)AUA(F) AGG(F)ACG(F)ACG(F)ACG(F)ACA(F)AAC(F)ACC(F)ACC(F)ACC(F)ACC(F)ACC( F)ACU(M)G(M)A(M) | 118 |
| E41-1 | Solid Phase Synthesis | G(MOE)G(M)G(MOE)A(M)G(MOE)A(M)A(MOE)U(M)A(MOE)C(M)A(MOE)A(M)G(M OE)C(M)U(MOE)A(M)C(MOE)U(M)U(MOE)G(M)U(MOE)U(M)C(MOE)U(M)U(MOE)U (M)U(MOE)U(M)G(MOE)C(M)A(MOE)G(M)C(MOE)C(M)A(MOE)C(M)C(MOE)AUGG (F)ACU(F)ACA(F)AGG(F)ACG(F)ACG(F)ACG(F)ACA(F)AGA(F)UCA(F)UCG(F)AC U(F)AUA(F)AAG | 119 |
| E41-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACU(M)G(M) A(M) | 120 |

**[Table 2-14]**

| | | | |
|---|---|---|---|
| E42 | Same as Example 1 | G(F)G(M)G(F)A(M)G(F)A(M)A(F)U(M)A(F)C(M)A(F)A(M)G(F)C(M)U(F)A(M)C(F)U( M)U(F)G(M)U(F)U(M)C(F)U(M)U(F)U(M)U(F)U(M)G(F)C(M)A(F)G(M)C(F)C(M)A(F) C(M)C(F)AUGG(F)ACU(F)ACA(F)AGG(F)ACG(F)ACG(F)ACG(F)ACA(F)AGA(F)UC A(F)UCG(F)ACU(F)AUA(F)AAGACGACG(F)ACG(F)AUA(F)AAG(F)GUG(F)GCG(F) ACU(F)AUA(F)AGG(F)ACG(F)ACG(F)ACG(F)ACA(F)AAC(F)ACC(F)ACC(F)ACC( F)ACC(F)ACC(F)ACU(M)G(M)A(M) | 121 |
| E42-1 | Solid Phase Synthesis | G(F)G(M)G(F)A(M)G(F)A(M)A(F)U(M)A(F)C(M)A(F)A(M)G(F)C(M)U(F)A(M)C(F)U( M)U(F)G(M)U(F)U(M)C(F)U(M)U(F)U(M)U(F)U(M)G(F)C(M)A(F)G(M)C(F)C(M)A(F) C(M)C(F)AUGG(F)ACU(F)ACA(F)AGG(F)ACG(F)ACG(F)ACG(F)ACA(F)AGA(F)UC A(F)UCG(F)ACU(F)AUA(F)AAG | 122 |
| E42-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACU(M)G(M) A(M) | 123 |
| E43 | Same as Example 1 | G(F)^G(M)^G(F)^A(M)^G(F)^A(M)^A(F)U(M)A(F)C(M)A(F)A(M)G(F)C(M)U(F)A(M) C(F)U(M)U(F)G(M)U(F)U(M)C(F)U(M)U(F)U(M)U(F)U(M)G(F)C(M)A(F)G(M)C(F)C( M)A(F)C(M)C(F)AUGG(F)ACU(F)ACA(F)AGG(F)ACG(F)ACG(F)ACG(F)ACA(F)AG A(F)UCA(F)UCG(F)ACU(F)AUA(F)AAGACGACG(F)ACG(F)AUA(F)AAG(F)GUG(F) GCG(F)ACU(F)AUA(F)AGG(F)ACG(F)ACG(F)ACG(F)ACA(F)AAC(F)ACC(F)ACC( F)ACC(F)ACC(F)ACC(F)ACU(M)G(M)A(M) | 124 |
| E43-1 | Solid Phase Synthesis | G(F)^G(M)^G(F)^A(M)^G(F)^A(M)^A(F)U(M)A(F)C(M)A(F)A(M)G(F)C(M)U(F)A(M) C(F)U(M)U(F)G(M)U(F)U(M)C(F)U(M)U(F)U(M)U(F)U(M)G(F)C(M)A(F)G(M)C(F)C( M)A(F)C(M)C(F)AUGG(F)ACU(F)ACA(F)AGG(F)ACG(F)ACG(F)ACG(F)ACA(F)AG A(F)UCA(F)UCG(F)ACU(F)AUA(F)AAG | 125 |
| E43-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACU(M)G(M) A(M) | 126 |
| E44 | Same as Example 1 | G(F)^G(M)^G(F)^A(M)^G(F)^A(M)^A(F)^U(M)^A(F)^C(M)^A(F)^A(M)^G(F)C(M)U(F) A(M)C(F)U(M)U(F)G(M)U(F)U(M)C(F)U(M)U(F)U(M)U(F)U(M)G(F)C(M)A(F)G(M)C( F)C(M)A(F)C(M)C(F)AUGG(F)ACU(F)ACA(F)AGG(F)ACG(F)ACG(F)ACG(F)ACA(F )AGA(F)UCA(F)UCG(F)ACU(F)AUA(F)AAGACGACG(F)ACG(F)AUA(F)AAG(F)GU G(F)GCG(F)ACU(F)AUA(F)AGG(F)ACG(F)ACG(F)ACG(F)ACA(F)AAC(F)ACC(F)A CC(F)ACC(F)ACC(F)ACC(F)ACU(M)G(M)A(M) | 127 |
| E44-1 | Solid Phase Synthesis | G(F)^G(M)^G(F)^A(M)^G(F)^A(M)^A(F)^U(M)^A(F)^C(M)^A(F)^A(M)^G(F)C(M)U(F) A(M)C(F)U(M)U(F)G(M)U(F)U(M)C(F)U(M)U(F)U(M)U(F)U(M)G(F)C(M)A(F)G(M)C( F)C(M)A(F)C(M)C(F)AUGG(F)ACU(F)ACA(F)AGG(F)ACG(F)ACG(F)ACG(F)ACA(F )AGA(F)UCA(F)UCG(F)ACU(F)AUA(F)AAG | 128 |

**[Table 2-15]**

| | | | |
|---|---|---|---|
| E44-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACU(M)G(M) A(M) | 129 |
| E45 | Same as Example 1 | G(M)G(M)G(M)A(M)G(M)A(M)AUACAAGCUACUUGUUCUUUUUGCAGCCACCAUG G(F)ACU(F)ACA(F)AGG(F)ACG(F)ACG(F)ACG(F)ACA(F)AGA(F)UCA(F)UCG(F)A CU(F)AUA(F)AAGACGACG(F)ACG(F)AUA(F)AAG(F)GUG(F)GCG(F)ACU(F)AUA( F)AGG(F)ACG(F)ACG(F)ACG(F)ACA(F)AAC(F)ACC(F)ACC(F)ACC(F)ACC(F)AC C(F)ACU(M)G(M)A(M)AAAAAAAAAAAAAAA(L)A(L)A(L)A(L)A(L)A(L) | 130 |
| E45-1 | Solid Phase Synthesis | G(M)G(M)G(M)A(M)G(M)A(M)AUACAAGCUACUUGUUCUUUUUGCAGCCACCAUG G(F)ACU(F)ACA(F)AGG(F)ACG(F)ACG(F)ACG(F)ACA(F)AGA(F)UCA(F)UCG(F)A CU(F)AUA(F)AAG | 131 |
| E45-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACU(M)G(M) A(M)AAAAAAAAAAAAAAA(L)A(L)A(L)A(L)A(L)A(L) | 132 |
| E46 | Same as Example 1 | G(M)G(M)G(M)A(M)G(M)A(M)AUACAAGCUACUUGUUCUUUUUGCAGCCACCAUG G(F)ACU(F)ACA(F)AGG(F)ACG(F)ACG(F)ACG(F)ACA(F)AGA(F)UCA(F)UCG(F)A CU(F)AUA(F)AAGACGACG(F)ACG(F)AUA(F)AAG(F)GUG(F)GCG(F)ACU(F)AUA( F)AGG(F)ACG(F)ACG(F)ACG(F)ACA(F)AAC(F)ACC(F)ACC(F)ACC(F)ACC(F)AC C(F)ACU(M)G(M)A(M)A(MOE)A(MOE)A(MOE)A(MOE)A(MOE)A(MOE)A(MOE)A(M OE)A(MOE)A(MOE)A(MOE)A(MOE)A(MOE)A(MOE)A(MOE)A(MOE)A(MOE)A(MOE )A(MOE)A(MOE) | 133 |
| E46-1 | Solid Phase Synthesis | G(M)G(M)G(M)A(M)G(M)A(M)AUACAAGCUACUUGUUCUUUUUGCAGCCACCAUG G(F)ACU(F)ACA(F)AGG(F)ACG(F)ACG(F)ACG(F)ACA(F)AGA(F)UCA(F)UCG(F)A CU(F)AUA(F)AAG | 134 |
| E46-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACU(M)G(M) A(M)A(MOE)A(MOE)A(MOE)A(MOE)A(MOE)A(MOE)A(MOE)A(MOE)A(MOE)A(MO E)A(MOE)A(MOE)A(MOE)A(MOE)A(MOE)A(MOE)A(MOE)A(MOE)A(MOE)A(MOE) | 135 |
| E47 | Purchased from GeneDesign | G(MOE)G(MOE)G(MOE)A(MOE)G(MOE)A(MOE)A(M)U(M)A(M)C(M)A(M)A(M)G(M) C(M)U(M)A(M)C(M)UUGUUCUUU(M)U(M)U(M)G(M)C(M)A(M)GCCACCAUGG(F)A CU(F)ACA(F)AGG(F)ACG(F)ACG(F)ACG(F)ACA(F)AGA(F)UCA(F)UCG(F)ACU(F) AUA(F)AAGACGACG(F)ACG(F)AUA(F)AAG(F)GUG(F)GCG(F)ACU(F)AUA(F)AGG (F)ACG(F)ACG(F)ACG(F)ACA(F)AAC(F)ACC(F)ACC(F)ACC(F)ACC(F)ACC(F)AC T(MOE)G(MOE)A(MOE) | 136 |

**[Table 2-16]**

| | | | |
|---|---|---|---|
| E48 | Purchased from GeneDesign | G(MOE)G(MOE)G(MOE)A(MOE)G(MOE)A(MOE)A(M)U(M)A(M)C(M)A(M)A(M)G(M) C(M)U(M)A(M)C(M)UUGUUCUUU(M)U(M)U(M)G(M)C(M)A(M)G(F)C(M)C(F)A(M)C( F)C(M)AUGG(F)ACU(F)ACA(F)AGG(F)ACG(F)ACG(F)ACG(F)ACA(F)AGA(F)UCA( F)UCG(F)ACU(F)AUA(F)AAGACGACG(F)ACG(F)AUA(F)AAG(F)GUG(F)GCG(F)A CU(F)AUA(F)AGG(F)ACG(F)ACG(F)ACG(F)ACA(F)AAC(F)ACC(F)ACC(F)ACC(F) ACC(F)ACC(F)ACT(MOE)G(MOE)A(MOE) | 137 |
| E49 | Purchased from GeneDesign | G(MOE)G(MOE)G(MOE)A(MOE)G(MOE)A(MOE)A(M)U(M)A(M)C(M)A(M)A(M)G(M) C(M)U(M)A(M)C(M)U(F)U(M)G(F)U(M)U(F)C(M)U(F)U(M)U(M)U(M)G(M)C(M) A(M)GCCA(M)CCAUGG(F)ACU(F)ACA(F)AGG(F)ACG(F)ACG(F)ACG(F)ACA(F)A GA(F)UCA(F)UCG(F)ACU(F)AUA(F)AAGACGACG(F)ACG(F)AUA(F)AAG(F)GUG( F)GCG(F)ACU(F)AUA(F)AGG(F)ACG(F)ACG(F)ACG(F)ACA(F)AAC(F)ACC(F)AC C(F)ACC(F)ACC(F)ACC(F)ACT(MOE)G(MOE)A(MOE) | 138 |
| E50 | Purchased from GeneDesign | G(MOE)G(MOE)G(MOE)A(MOE)G(MOE)A(MOE)A(M)U(M)A(M)C(M)A(M)A(M)G(M) C(M)U(M)A(M)C(M)U(F)U(M)G(F)U(M)U(F)C(M)U(F)U(M)U(M)U(M)G(M)C(M) A(M)G(F)C(M)C(F)A(M)C(F)C(M)AUGG(F)ACU(F)ACA(F)AGG(F)ACG(F)ACG(F)A CG(F)ACA(F)AGA(F)UCA(F)UCG(F)ACU(F)AUA(F)AAGACGACG(F)ACG(F)AUA( F)AAG(F)GUG(F)GCG(F)ACU(F)AUA(F)AGG(F)ACG(F)ACG(F)ACG(F)ACA(F)AA | 139 |
| | | C(F)ACC(F)ACC(F)ACC(F)ACC(F)ACC(F)ACT(MOE)G(MOE)A(MOE) | |
| E51 | Same as Example 1 | G(MOE)G(MOE)G(MOE)A(MOE)G(MOE)A(MOE)A(M)U(M)A(M)C(M)A(M)A(M)G(M) C(M)U(M)A(M)C(M)U(F)U(rSpacer)G(F)U(rSpacer)U(F)C(rSpacer)U(F)U(rSpacer) U(M)U(M)U(M)G(M)C(M)A(M)GCCA(M)CCAUGG(F)ACU(F)ACA(F)AGG(F)ACG(F) ACG(F)ACG(F)ACA(F)AGA(F)UCA(F)UCG(F)ACU(F)AUA(F)AAGACGACG(F)ACG (F)AUA(F)AAG(F)GUG(F)GCG(F)ACU(F)AUA(F)AGG(F)ACG(F)ACG(F)ACG(F)AC A(F)AAC(F)ACC(F)ACC(F)ACC(F)ACC(F)ACC(F)ACT(MOE)G(MOE)A(MOE) | 140 |
| E51-1 | Solid Phase Synthesis | G(MOE)G(MOE)G(MOE)A(MOE)G(MOE)A(MOE)A(M)U(M)A(M)C(M)A(M)A(M)G(M) C(M)U(M)A(M)C(M)U(F)U(rSpacer)G(F)U(rSpacer)U(F)C(rSpacer)U(F)U(rSpacer) U(M)U(M)U(M)G(M)C(M)A(M)GCCA(M)CCAUGG(F)ACU(F)ACA(F)AGG(F)ACG(F) ACG(F)ACG(F)ACA(F)AGA(F)UCA(F)UCG(F)ACU(F)AUA(F)AAG | 141 |
| E51-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACT(MOE)G( MOE)A(MOE) | 142 |

**[Table 2-17]**

| | | | |
|---|---|---|---|
| E52 | Same as Example 1 | G(MOE)G(MOE)G(MOE)A(MOE)G(MOE)A(MOE)A(M)U(M)A(M)C(M)A(M)A(M)G(M) C(M)U(M)A(M)C(M)UUGUUCUUU(M)U(M)U(M)G(M)C(M)A(M)G(MOE)C(MOE)C(M OE)A(M)C(MOE)C(MOE)AUGG(F)ACU(F)ACA(F)AGG(F)ACG(F)ACG(F)ACG(F)A CA(F)AGA(F)UCA(F)UCG(F)ACU(F)AUA(F)AAGACGACG(F)ACG(F)AUA(F)AAG( F)GUG(F)GCG(F)ACU(F)AUA(F)AGG(F)ACG(F)ACG(F)ACG(F)ACA(F)AAC(F)AC C(F)ACC(F)ACC(F)ACC(F)ACC(F)ACT(MOE)G(MOE)A(MOE) | 143 |
| E52-1 | Solid Phase Synthesis | G(MOE)G(MOE)G(MOE)A(MOE)G(MOE)A(MOE)A(M)U(M)A(M)C(M)A(M)A(M)G(M) C(M)U(M)A(M)C(M)UUGUUCUUU(M)U(M)U(M)G(M)C(M)A(M)G(MOE)C(MOE)C(M OE)A(M)C(MOE)C(MOE)AUGG(F)ACU(F)ACA(F)AGG(F)ACG(F)ACG(F)ACG(F)A CA(F)AGA(F)UCA(F)UCG(F)ACU(F)AUA(F)AAG | 144 |
| E52-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACT(MOE)G( MOE)A(MOE) | 145 |
| E53 | Same as Example 1 | G(MOE)^G(MOE)^G(MOE)A(MOE)G(MOE)A(MOE)AUACAAGCUACUUGUUCUUUU UGCAGCCACCAUGG(F)ACU(F)ACA(F)AGG(F)ACG(F)ACG(F)ACG(F)ACA(F)AG A(F)UCA(F)UCG(F)ACU(F)AUA(F)AAGACGACG(F)ACG(F)AUA(F)AAG(F)GUG(F) GCG(F)ACU(F)AUA(F)AGG(F)ACG(F)ACG(F)ACG(F)ACA(F)AAC(F)ACC(F)ACC( F)ACC(F)ACC(F)ACC(F)ACT(MOE)^G(MOE)^A(MOE)^A(MOE)^A(MOE)^A(MOE)^ A(MOE)^A(MOE)^A(MOE)^A(MOE)^A(MOE)^A(MOE)^A(MOE)^A(MOE)^A(MOE)^A (MOE)^A(MOE)^A(MOE)^A(MOE)^A(MOE)^A(MOE)^A(MOE)^A(MOE)^A(MOE) | 146 |
| E53-1 | Solid Phase Synthesis | G(MOE)^G(MOE)^G(MOE)A(MOE)G(MOE)A(MOE)AUACAAGCUACUUGUUCUUUU UGCAGCCACCAUGG(F)ACU(F)ACA(F)AGG(F)ACG(F)ACG(F)ACG(F)ACA(F)AG A(F)UCA(F)UCG(F)ACU(F)AUA(F)AAG | 147 |
| E53-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACT(MOE)^G (MOE)^A(MOE)^A(MOE)^A(MOE)^A(MOE)^A(MOE)^A(MOE)^A(MOE)^A(MOE)^A( MOE)^A(MOE)^A(MOE)^A(MOE)^A(MOE)^A(MOE)^A(MOE)^A(MOE)^A(MOE)^A(M OE)^A(MOE)^A(MOE)^A(MOE) | 148 |

**[Table 2-18]**

| | | | |
|---|---|---|---|
| E54 | Same as Example 1 | G(MOE)^G(MOE)^G(MOE)A(MOE)G(MOE)A(MOE)AUACAAGCUACUUGUUCUUUU UGCAGCCACCAUGG(F)ACU(F)ACA(F)AGG(F)ACG(F)ACG(F)ACG(F)ACA(F)AG A(F)UCA(F)UCG(F)ACU(F)AUA(F)AAGACGACG(F)ACG(F)AUA(F)AAG(F)GUG(F) GCG(F)ACU(F)AUA(F)AGG(F)ACG(F)ACG(F)ACG(F)ACA(F)AAC(F)ACC(F)ACC( F)ACC(F)ACC(F)ACC(F)ACT(MOE)^G(MOE)^A(MOE)^A(M)^A(M)^A(M)^A(M)^A(M )^A(M)^A(M)^A(M)^A(M)^A(M)^A(M)^A(M)^A(M)^A(M)^A(M)^A(M)^A(MOE)^ A(MOE)^A(MOE) | 149 |
| E54-1 | Solid Phase Synthesis | G(MOE)^G(MOE)^G(MOE)A(MOE)G(MOE)A(MOE)AUACAAGCUACUUGUUCUUUU UGCAGCCACCAUGG(F)ACU(F)ACA(F)AGG(F)ACG(F)ACG(F)ACG(F)ACA(F)AG A(F)UCA(F)UCG(F)ACU(F)AUA(F)AAG | 150 |
| E54-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACT(MOE)^G (MOE)^A(MOE)^A(M)^A(M)^A(M)^A(M)^A(M)^A(M)^A(M)^A(M)^A(M)^A(M)^A(M)^A (M)^A(M)^A(M)^A(M)^A(M)^A(M)^A(MOE)^A(MOE)^A(MOE) | 151 |
| E55 | Same as Example 1 | G(MOE)^G(MOE)^G(MOE)A(MOE)G(MOE)A(MOE)AUACAAGCUACUUGUUCUUUU UGCAGCCACCAUGG(F)ACU(F)ACA(F)AGG(F)ACG(F)ACG(F)ACG(F)ACA(F)AG A(F)UCA(F)UCG(F)ACU(F)AUA(F)AAGACGACG(F)ACG(F)AUA(F)AAG(F)GUG(F) GCG(F)ACU(F)AUA(F)AGG(F)ACG(F)ACG(F)ACG(F)ACA(F)AAC(F)ACC(F)ACC( F)ACC(F)ACC(F)ACC(F)ACT(MOE)^G(MOE)^A(MOE)^A(F)^A(M)^A(F)^A(M)^A(F) ^A(M)^A(F)^A(M)^A(F)^A(M)^A(F)^A(M)^A(F)^A(M)^A(F)^A(M)^A(F)^A(MOE)^A(M OE)^A(MOE) | 152 |
| E55-1 | Solid Phase Synthesis | G(MOE)^G(MOE)^G(MOE)A(MOE)G(MOE)A(MOE)AUACAAGCUACUUGUUCUUUU UGCAGCCACCAUGG(F)ACU(F)ACA(F)AGG(F)ACG(F)ACG(F)ACG(F)ACA(F)AG A(F)UCA(F)UCG(F)ACU(F)AUA(F)AAG | 153 |
| E55-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACT(MOE)^G (MOE)^A(MOE)^A(F)^A(M)^A(F)^A(M)^A(F)^A(M)^A(F)^A(M)^A(F)^A(M)^A(F)^A(M )^A(F)^A(M)^A(F)^A(M)^A(F)^A(MOE)^A(MOE)^A(MOE) | 154 |

**[Table 2-19]**

| | | | |
|---|---|---|---|
| E56 | Same as Example 1 | G(MOE)^G(MOE)^G(MOE)A(MOE)G(MOE)A(MOE)A(F)U(M)A(F)C(M)A(F)A(M)G(F) C(M)U(F)A(M)C(F)U(M)U(F)G(M)U(F)U(M)C(F)U(M)U(F)U(M)U(F)U(M)G(F)C(M)A( F)G(M)C(F)C(M)A(F)C(M)C(F)AUGG(F)ACU(F)ACA(F)AGG(F)ACG(F)ACG(F)ACG (F)ACA(F)AGA(F)UCA(F)UCG(F)ACU(F)AUA(F)AAGACGACG(F)ACG(F)AUA(F)A AG(F)GUG(F)GCG(F)ACU(F)AUA(F)AGG(F)ACG(F)ACG(F)ACG(F)ACA(F)AAC(F) ACC(F)ACC(F)ACC(F)ACC(F)ACC(F)ACT(MOE)^G(MOE)^A(MOE)^A(F)^A(M)^A( F)^A(M)^A(F)^A(M)^A(F)^A(M)^A(F)^A(M)^A(F)^A(M)^A(F)^A(M)^A(F)^A(M)^A(F)^ A(MOE)^A(MOE)^A(MOE) | 155 |
| E56-1 | Solid Phase Synthesis | G(MOE)^G(MOE)^G(MOE)A(MOE)G(MOE)A(MOE)A(F)U(M)A(F)C(M)A(F)A(M)G(F) C(M)U(F)A(M)C(F)U(M)U(F)G(M)U(F)U(M)C(F)U(M)U(F)U(M)U(F)U(M)G(F)C(M)A( F)G(M)C(F)C(M)A(F)C(M)C(F)AUGG(F)ACU(F)ACA(F)AGG(F)ACG(F)ACG(F)ACG (F)ACA(F)AGA(F)UCA(F)UCG(F)ACU(F)AUA(F)AAG | 156 |
| E56-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACT(MOE)^G (MOE)^A(MOE)^A(F)^A(M)^A(F)^A(M)^A(F)^A(M)^A(F)^A(M)^A(F)^A(M)^A(F)^A(M )^A(F)^A(M)^A(F)^A(M)^A(F)^A(MOE)^A(MOE)^A(MOE) | 157 |
| E57 | Same as Example 1 | G(MOE)^G(MOE)^G(MOE)^A(MOE)^G(MOE)^A(MOE)^A(M)^U(M)^A(M)^C(M)^A(M) ^A(M)^G(M)^C(M)^U(M)^A(M)^C(M)UUGUUCUUU(M)^U(M)^U(M)^G(M)^C(M)^A(M) GCCACCAUGG(F)ACU(F)ACA(F)AGG(F)ACG(F)ACG(F)ACG(F)ACA(F)AGA(F)UC A(F)UCG(F)ACU(F)AUA(F)AAGACGACG(F)ACG(F)AUA(F)AAG(F)GUG(F)GCG(F) ACU(F)AUA(F)AGG(F)ACG(F)ACG(F)ACG(F)ACA(F)AAC(F)ACC(F)ACC(F)ACC( F)ACC(F)ACC(F)ACT(MOE)^G(MOE)^A(MOE)^A(F)^A(M)^A(F)^A(M)^A(F)^A(M)^ A(F)^A(M)^A(F)^A(M)^A(F)^A(M)^A(F)^A(M)^A(F)^A(M)^A(F)^A(MOE)^A(MOE)^A( MOE) | 158 |
| E57-1 | Solid Phase Synthesis | G(MOE)^G(MOE)^G(MOE)^A(MOE)^G(MOE)^A(MOE)^A(M)^U(M)^A(M)^C(M)^A(M) ^A(M)^G(M)^C(M)^U(M)^A(M)^C(M)UUGUUCUUU(M)^U(M)^U(M)^G(M)^C(M)^A(M) GCCACCAUGG(F)ACU(F)ACA(F)AGG(F)ACG(F)ACG(F)ACG(F)ACA(F)AGA(F)UC A(F)UCG(F)ACU(F)AUA(F)AAG | 159 |
| E57-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACT(MOE)^G (MOE)^A(MOE)^A(F)^A(M)^A(F)^A(M)^A(F)^A(M)^A(F)^A(M)^A(F)^A(M)^A(F)^A(M )^A(F)^A(M)^A(F)^A(M)^A(F)^A(MOE)^A(MOE)^A(MOE) | 160 |

**[Table 2-20]**

| | | | |
|---|---|---|---|
| E58 | Same as Example 1 | G(MOE)G(MOE)G(MOE)A(MOE)G(MOE)A(MOE)AUACAAGCUACUUGUUCUUUUU GCAGCCACCAUGG(F)ACU(F)ACA(F)AGG(F)ACG(F)ACG(F)ACG(F)ACA(F)AGA( F)UCA(F)UCG(F)ACU(F)AUA(F)AAGACGACG(F)ACG(F)AUA(F)AAG(F)GUG(F)G CG(F)ACU(F)AUA(F)AGG(F)ACG(F)ACG(F)ACG(F)ACA(F)AAC(F)ACC(F)ACC(F) ACC(F)ACC(F)ACC(F)ACT(MOE)G(MOE)A(MOE)AAAAAAAAAAAAAAAAAAAA AAAAAAAAAAAAAAAAAAAA | 161 |
| E58-1 | Solid Phase Synthesis | G(MOE)G(MOE)G(MOE)A(MOE)G(MOE)A(MOE)AUACAAGCUACUUGUUCUUUUU GCAGCCACCAUGG(F)ACU(F)ACA(F)AGG(F)ACG(F)ACG(F)ACG(F)ACA(F)AGA( F)UCA(F)UCG(F)ACU(F)AUA(F)AAG | 162 |
| E58-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACT(MOE)G( MOE)A(MOE)AAAAAAAAAAAAAAAAAAAAAAAAAAAAAAAAAAAAAAAA | 163 |
| E59 | Same as Example 1 | G(MOE)^G(MOE)^G(MOE)A(MOE)G(MOE)A(MOE)AUACAAGCUACUUGUUCUUUU UGCAGCCACCAUGG(F)ACU(F)ACA(F)AGG(F)ACG(F)ACG(F)ACG(F)ACA(F)AG A(F)UCA(F)UCG(F)ACU(F)AUA(F)AAGACGACG(F)ACG(F)AUA(F)AAG(F)GUG(F) GCG(F)ACU(F)AUA(F)AGG(F)ACG(F)ACG(F)ACG(F)ACA(F)AAC(F)ACC(F)ACC( F)ACC(F)ACC(F)ACC(F)ACT(MOE)^G(MOE)^A(MOE)^A(F)^A(M)^A(F)^A(M)^A(F) ^A(M)^A(F)^A(M)^A(F)^A(M)^A(F)^A(M)^A(F)^A(M)^A(F)^A(M)^A(F)^A(M)^A(F)^A (M)^A(F)^A(M)^A(F)^A(M)^A(F)^A(M)^A(F)^A(M)^A(F)^A(M)^A(F)^A(M)^A(F)^A(M )^A(F)^A(M)^A(F)^A(MOE)^A(MOE)^A(MOE) | 164 |
| E59-1 | Solid Phase Synthesis | G(MOE)^G(MOE)^G(MOE)A(MOE)G(MOE)A(MOE)AUACAAGCUACUUGUUCUUUU UGCAGCCACCAUGG(F)ACU(F)ACA(F)AGG(F)ACG(F)ACG(F)ACG(F)ACA(F)AG A(F)UCA(F)UCG(F)ACU(F)AUA(F)AAG | 165 |
| E59-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACT(MOE)^G (MOE)^A(MOE)^A(F)^A(M)^A(F)^A(M)^A(F)^A(M)^A(F)^A(M)^A(F)^A(M)^A(F)^A(M )^A(F)^A(M)^A(F)^A(M)^A(F)^A(M)^A(F)^A(M)^A(F)^A(M)^A(F)^A(M)^A(F)^A(M)^ A(F)^A(M)^A(F)^A(M)^A(F)^A(M)^A(F)^A(M)^A(F)^A(M)^A(F)^A(MOE)^A(MOE)^A( MOE) | 166 |

**[Table 2-21]**

| | | | |
|---|---|---|---|
| E60 | Same as Example 1 | G(MOE)^G(MOE)^G(MOE)A(MOE)G(MOE)A(MOE)A(M)U(M)A(M)C(M)A(M)A(M)G( M)C(M)U(M)A(M)C(M)UUGUUCUUU(M)U(M)U(M)G(M)C(M)A(M)GCCACCAUGG(F) ^ACU(F)^ACA(F)^AGG(F)^ACG(F)^ACG(F)^ACG(F)^ACA(F)^AGA(F)^UCA(F)^UC G(F)^ACU(F)^AUA(F)^AAGACGACG(F)^ACG(F)^AUA(F)^AAG(F)^GUG(F)^GCG(F )^ACU(F)^AUA(F)^AGG(F)^ACG(F)^ACG(F)^ACG(F)^ACA(F)^AAC(F)^ACC(F)^AC C(F)^ACC(F)^ACC(F)^ACC(F)^ACT(MOE)^G(MOE)^A(MOE)^A(F)^A(M)^A(F)^A(M )^A(F)^A(M)^A(F)^A(M)^A(F)^A(M)^A(F)^A(M)^A(F)^A(M)^A(F)^A(M)^A(F)^A(MOE )^A(MOE)^A(MOE) | 167 |
| E60-1 | Solid Phase Synthesis | G(MOE)^G(MOE)^G(MOE)A(MOE)G(MOE)A(MOE)A(M)U(M)A(M)C(M)A(M)A(M)G( M)C(M)U(M)A(M)C(M)UUGUUCUUU(M)U(M)U(M)G(M)C(M)A(M)GCCACCAUGG(F) ^ACU(F)^ACA(F)^AGG(F)^ACG(F)^ACG(F)^ACG(F)^ACA(F)^AGA(F)^UCA(F)^UC G(F)^ACU(F)^AUA(F)^AAG | 168 |
| E60-2 | Solid Phase Synthesis | pACGACG(F)^ACG(F)^AUA(F)^AAG(F)^GUG(F)^GCG(F)^ACU(F)^AUA(F)^AGG(F )^ACG(F)^ACG(F)^ACG(F)^ACA(F)^AAC(F)^ACC(F)^ACC(F)^ACC(F)^ACC(F)^AC C(F)^ACT(MOE)^G(MOE)^A(MOE)^A(F)^A(M)^A(F)^A(M)^A(F)^A(M)^A(F)^A(M)^A (F)^A(M)^A(F)^A(M)^A(F)^A(M)^A(F)^A(M)^A(F)^A(MOE)^A(MOE)^A(MOE) | 169 |
| E61 | Solid Phase Synthesis | G(MOE)G(MOE)G(MOE)A(MOE)G(MOE)A(MOE)AUACAAGCUACUUGUUCUUUUU GCAGCCACCA(F)UGG(F)ACU(F)AUA(F)AGG(F)ACG(F)ACG(F)ACG(F)ACA(F)AA G(F)GUG(F)GCC(F)ACC(F)ACC(F)ACC(F)ACC(F)ACC(F)ACU(MOE)G(MOE)A(M OE) | 170 |
| E62 | Same as Example 1 | G(MOE)G(MOE)G(MOE)A(MOE)G(MOE)A(MOE)A(F)U(M)A(F)C(M)A(F)A(M)G(F)C (M)U(F)A(M)C(F)U(M)U(F)G(M)U(F)U(M)C(F)U(M)U(F)U(M)G(F)C(M)A(F) G(M)C(F)C(M)A(F)C(M)C(F)AUGGACUACAAGGACGACGACGACAAGAUCAUCG ACUAUAAAGACGACGACGAUAAAGGUGGCGACUAUAAGGACGACGACGACAA ACACCACCACCACCACCACT(MOE)G(MOE)A(MOE)A(F)A(M)A(F)A(M)A(F)A(M) A(F)A(M)A(F)A(M)A(F)A(M)A(F)A(M)A(F)A(M)A(F)A(MOE)A(MOE)A(MOE) | 171 |
| E62-1 | Solid Phase Synthesis | G(MOE)G(MOE)G(MOE)A(MOE)G(MOE)A(MOE)A(F)U(M)A(F)C(M)A(F)A(M)G(F)C (M)U(F)A(M)C(F)U(M)U(F)G(M)U(F)U(M)C(F)U(M)U(F)U(M)U(F)U(M)G(F)C(M)A(F) G(M)C(F)C(M)A(F)C(M)C(F)AUGGACUACAAGGACGACGACGACAAGAUCAUCG ACUAUAAAG | 172 |
| E62-2 | Solid Phase Synthesis | pACGACGACGAUAAAGGUGGCGACUAUAAGGACGACGACGACAAACACCACCA CCACCACCACT(MOE)G(MOE)A(MOE)A(F)A(M)A(F)A(M)A(F)A(M)A(F)A(M)A(F)A (M)A(F)A(M)A(F)A(M)A(F)A(M)A(F)A(MOE)A(MOE)A(MOE) | 173 |

**[Table 2-22]**

| | | | |
|---|---|---|---|
| E63 | Same as Example 1 | G(MOE)^G(MOE)^G(MOE)^A(MOE)^G(MOE)^A(MOE)^A(F)^U(M)^A(F)^C(M)^A(F)^ A(M)^G(F)C(M)U(F)A(M)C(F)U(M)U(F)G(M)U(F)U(M)C(F)U(M)U(F)U(M)U(F)U(M)G (F)C(M)A(F)G(M)C(F)C(M)A(F)C(M)C(F)AUGGACUACAAGGACGACGACGACAA GAUCAUCGACUAUAAAGACGACGACGAUAAAGGUGGCGACUAUAAGGACGAC GACGACAAACACCACCACCACCACCACT(MOE)^G(MOE)^A(MOE)^A(F)^A(M)^A (F)^A(M)^A(F)^A(M)^A(F)^A(M)^A(F)^A(M)^A(F)^A(M)^A(F)^A(M)^A(F)^A(M)^A(F) ^A(MOE)^A(MOE)^A(MOE) | 174 |
| E63-1 | Solid Phase Synthesis | G(MOE)^G(MOE)^G(MOE)^A(MOE)^G(MOE)^A(MOE)^A(F)^U(M)^A(F)^C(M)^A(F)^ A(M)^G(F)C(M)U(F)A(M)C(F)U(M)U(F)G(M)U(F)U(M)C(F)U(M)U(F)U(M)U(F)U(M)G (F)C(M)A(F)G(M)C(F)C(M)A(F)C(M)C(F)AUGGACUACAAGGACGACGACGACAA GAUCAUCGACUAUAAAG | 175 |
| E63-2 | Solid Phase Synthesis | pACGACGACGAUAAAGGUGGCGACUAUAAGGACGACGACGACAAACACCACCA CCACCACCACT(MOE)^G(MOE)^A(MOE)^A(F)^A(M)^A(F)^A(M)^A(F)^A(M)^A(F)^ A(M)^A(F)^A(M)^A(F)^A(M)^A(F)^A(M)^A(F)^A(M)^A(F)^A(MOE)^A(MOE)^A(MOE) | 176 |
| E64 | Same as Example 2 | G(MOE)G(MOE)G(MOE)A(MOE)G(MOE)A(MOE)A(F)U(M)A(F)C(M)A(F)A(M)G(F)C (M)U(F)A(M)C(F)U(M)U(F)G(M)U(F)U(M)C(F)U(M)U(F)U(M)U(F)U(M)G(F)C(M)A(F) G(M)C(F)C(M)A(F)C(M)C(F)AUGG(F)ACU(F)ACA(F)AGG(F)ACG(F)ACG(F)ACG(F )ACA(F)AGA(F)UCA(F)UCG(F)ACU(F)AUA(F)AAG(F)ACG(F)ACG(F)ACG(F)AUA( F)AAG(F)GUG(F)GCG(F)ACU(F)AUA(F)AGG(F)ACG(F)ACG(F)ACG(F)ACA(F)AA C(F)ACC(F)ACC(F)ACC(F)ACC(F)ACC(F)ACT(MOE)G(MOE)A(MOE)A(F)A(M)A(F )A(M)A(F)A(M)A(F)A(M)A(F)A(M)A(F)A(M)A(F)A(M)A(F)A(M)A(F)A(MOE)A(MOE) A(MOE) | 177 |
| E64-1 | Solid Phase Synthesis | G(MOE)G(MOE)G(MOE)A(MOE)G(MOE)A(MOE)A(F)U(M)A(F)C(M)A(F)A(M)G(F)C (M)U(F)A(M)C(F)U(M)U(F)G(M)U(F)U(M)C(F)U(M)U(F)U(M)U(F)U(M)G(F)C(M)A(F) G(M)C(F)C(M)A(F)C(M)C(F)AUGG(F)ACU(F)ACA(F)AGG(F)ACG(F)ACG(F)ACG(F )ACA(F)AGA(F)UCA(F)UCG(F)ACU(F)AUA(F)AAG(F)p | 178 |
| E64-2 | Solid Phase Synthesis | ACG(F)ACG(F)ACG(F)AUA(F)AAG(F)GUG(F)GCG(F)ACU(F)AUA(F)AGG(F)ACG( F)ACG(F)ACG(F)ACA(F)AAC(F)ACC(F)ACC(F)ACC(F)ACC(F)ACC(F)ACT(MOE) G(MOE)A(MOE)A(F)A(M)A(F)A(M)A(F)A(M)A(F)A(M)A(F)A(M)A(F)A(M)A(F)A(M)A (F)A(M)A(F)A(MOE)A(MOE)A(MOE) | 179 |

**[Table 2-23]**

| | | | |
|---|---|---|---|
| E65 | Same as Example 2 | G(MOE)G(MOE)G(MOE)A(MOE)G(MOE)A(MOE)A(F)U(M)A(F)C(M)A(F)A(M)G(F)C (M)U(F)A(M)C(F)U(M)U(F)G(M)U(F)U(M)C(F)U(M)U(F)U(M)U(F)U(M)G(F)C(M)A(F) G(M)C(F)C(M)A(F)C(M)C(F)A(F)UGG(F)ACU(F)ACA(F)AGG(F)ACG(F)ACG(F)AC G(F)ACA(F)AGA(F)UCA(F)UCG(F)ACU(F)AUA(F)AAG(F)ACG(F)ACG(F)ACG(F)A UA(F)AAG(F)GUG(F)GCG(F)ACU(F)AUA(F)AGG(F)ACG(F)ACG(F)ACG(F)ACA(F) AAC(F)ACC(F)ACC(F)ACC(F)ACC(F)ACC(F)ACT(MOE)G(MOE)A(MOE)A(F)A(M) A(F)A(M)A(F)A(M)A(F)A(M)A(F)A(M)A(F)A(M)A(F)A(M)A(F)A(M)A(F)A(MOE)A(M OE)A(MOE) | 180 |
| E65-1 | Solid Phase Synthesis | G(MOE)G(MOE)G(MOE)A(MOE)G(MOE)A(MOE)A(F)U(M)A(F)C(M)A(F)A(M)G(F)C (M)U(F)A(M)C(F)U(M)U(F)G(M)U(F)U(M)C(F)U(M)U(M)U(F)U(M)G(F)C(M)A(F) G(M)C(F)C(M)A(F)C(M)C(F)A(F)UGG(F)ACU(F)ACA(F)AGG(F)ACG(F)ACG(F)AC G(F)ACA(F)AGA(F)UCA(F)UCG(F)ACU(F)AUA(F)AAG(F)p | 181 |
| E65-2 | Solid Phase Synthesis | ACG(F)ACG(F)ACG(F)AUA(F)AAG(F)GUG(F)GCG(F)ACU(F)AUA(F)AGG(F)ACG( F)ACG(F)ACG(F)ACA(F)AAC(F)ACC(F)ACC(F)ACC(F)ACC(F)ACC(F)ACT(MOE) G(MOE)A(MOE)A(F)A(M)A(F)A(M)A(F)A(M)A(F)A(M)A(F)A(M)A(F)A(M)A(F)A(M)A (F)A(M)A(F)A(MOE)A(MOE)A(MOE) | 182 |
| E66 | Same as Example 2 | G(MOE)G(MOE)G(MOE)A(MOE)G(MOE)A(MOE)A(F)U(M)A(F)C(M)A(F)A(M)G(F)C (M)U(F)A(M)C(F)U(M)U(F)G(M)U(F)U(M)C(F)U(M)U(F)U(M)U(F)U(M)G(F)C(M)A(F) G(M)C(F)C(M)A(F)C(M)C(F)A(F)UGG(F)ACU(F)ACA(F)AG(M)G(F)ACG(F)ACG(F) ACG(F)ACA(F)AG(M)A(F)UCA(F)UCG(F)ACU(F)AU(M)A(F)AAG(F)ACG(F)ACG(F) ACG(F)AU(M)A(F)AAG(F)GU(M)G(F)GCG(F)ACU(F)AU(M)A(F)AG(M)G(F)ACG(F) ACG(F)ACG(F)ACA(F)AAC(F)ACC(F)ACC(F)ACC(F)ACC(F)ACC(F)ACT(MOE)G( MOE)A(MOE)A(F)A(M)A(F)A(M)A(F)A(M)A(F)A(M)A(F)A(M)A(F)A(M)A(F)A(M)A(F )A(M)A(F)A(MOE)A(MOE)A(MOE) | 183 |
| E66-1 | Solid Phase Synthesis | G(MOE)G(MOE)G(MOE)A(MOE)G(MOE)A(MOE)A(F)U(M)A(F)C(M)A(F)A(M)G(F)C (M)U(F)A(M)C(F)U(M)U(F)G(M)U(F)U(M)C(F)U(M)U(F)U(M)G(F)C(M)A(F) G(M)C(F)C(M)A(F)C(M)C(F)A(F)UGG(F)ACU(F)ACA(F)AG(M)G(F)ACG(F)ACG(F) ACG(F)ACA(F)AG(M)A(F)UCA(F)UCG(F)ACU(F)AU(M)A(F)AAG(F)p | 184 |
| E66-2 | Solid Phase Synthesis | ACG(F)ACG(F)ACG(F)AU(M)A(F)AAG(F)GU(M)G(F)GCG(F)ACU(F)AU(M)A(F)AG( M)G(F)ACG(F)ACG(F)ACG(F)ACA(F)AAC(F)ACC(F)ACC(F)ACC(F)ACC(F)ACC(F )ACT(MOE)G(MOE)A(MOE)A(F)A(M)A(F)A(M)A(F)A(M)A(F)A(M)A(F)A(M)A(F)A(M )A(F)A(M)A(F)A(M)A(F)A(MOE)A(MOE)A(MOE) | 185 |

**[Table 2-24]**

| | | | |
|---|---|---|---|
| E67 | Same as Example 2 | G(MOE)^G(MOE)^G(MOE)^A(MOE)^G(MOE)^A(MOE)^A(F)^U(M)^A(F)^C(M)^A(F)^ A(M)^G(F)C(M)U(F)A(M)C(F)U(M)U(F)G(M)U(F)U(M)C(F)U(M)U(F)U(M)U(F)U(M)G (F)C(M)A(F)G(M)C(F)C(M)A(F)C(M)C(F)A(F)UGG(F)ACU(F)ACA(F)AGG(F)ACG(F )ACG(F)ACG(F)ACA(F)AGA(F)UCA(F)UCG(F)ACU(F)AUA(F)AAG(F)ACG(F)ACG( F)ACG(F)AUA(F)AAG(F)GUG(F)GCG(F)ACU(F)AUA(F)AGG(F)ACG(F)ACG(F)AC G(F)ACA(F)AAC(F)ACC(F)ACC(F)ACC(F)ACC(F)ACC(F)ACT(MOE)^G(MOE)^A( MOE)^A(F)^A(M)^A(F)^A(M)^A(F)^A(M)^A(F)^A(M)^A(F)^A(M)^A(F)^A(M)^A(F)^A( M)^A(F)^A(M)^A(F)^A(MOE)^A(MOE)^A(MOE) | 186 |
| E67-1 | Solid Phase Synthesis | G(MOE)^G(MOE)^G(MOE)^A(MOE)^G(MOE)^A(MOE)^A(F)^U(M)^A(F)^C(M)^A(F)^ A(M)^G(F)C(M)U(F)A(M)C(F)U(M)U(F)G(M)U(F)U(M)C(F)U(M)U(F)U(M)U(F)U(M)G (F)C(M)A(F)G(M)C(F)C(M)A(F)C(M)C(F)A(F)UGG(F)ACU(F)ACA(F)AGG(F)ACG(F )ACG(F)ACG(F)ACA(F)AGA(F)UCA(F)UCG(F)ACU(F)AUA(F)AAG(F)p | 187 |
| E67-2 | Solid Phase Synthesis | ACG(F)ACG(F)ACG(F)AUA(F)AAG(F)GUG(F)GCG(F)ACU(F)AUA(F)AGG(F)ACG( F)ACG(F)ACG(F)ACA(F)AAC(F)ACC(F)ACC(F)ACC(F)ACC(F)ACC(F)ACT(MOE)^ G(MOE)^A(MOE)^A(F)^A(M)^A(F)^A(M)^A(F)^A(M)^A(F)^A(M)^A(F)^A(M)^A(F)^A( M)^A(F)^A(M)^A(F)^A(M)^A(F)^A(MOE)^A(MOE)^A(MOE) | 188 |
| E68 | Same as Example 2 | G(MOE)^G(MOE)^G(MOE)^A(MOE)^G(MOE)^A(MOE)^A(F)^U(M)^A(F)^C(M)^A(F)^ A(M)^G(F)C(M)U(F)A(M)C(F)U(M)U(F)G(M)U(F)U(M)C(F)U(M)U(F)U(M)U(F)U(M)G (F)C(M)A(F)G(M)C(F)C(M)A(F)C(M)C(F)A(F)^UGG(F)^ACU(F)^ACA(F)^AGG(F)^A CG(F)^ACG(F)^ACG(F)^ACA(F)^AGA(F)^UCA(F)^UCG(F)^ACU(F)^AUA(F)^AAG( F)ACG(F)^ACG(F)^ACG(F)^AUA(F)^AAG(F)^GUG(F)^GCG(F)^ACU(F)^AUA(F)^A GG(F)^ACG(F)^ACG(F)^ACG(F)^ACA(F)^AAC(F)^ACC(F)^ACC(F)^ACC(F)^ACC( F)^ACC(F)^ACT(MOE)^G(MOE)^A(MOE)^A(F)^A(M)^A(F)^A(M)^A(F)^A(M)^A(F)^ A(M)^A(F)^A(M)^A(F)^A(M)^A(F)^A(M)^A(F)^A(M)^A(F)^A(MOE)^A(MOE)^A(MOE) | 189 |
| E68-1 | Solid Phase Synthesis | G(MOE)^G(MOE)^G(MOE)^A(MOE)^G(MOE)^A(MOE)^A(F)^U(M)^A(F)^C(M)^A(F)^ A(M)^G(F)C(M)U(F)A(M)C(F)U(M)U(F)G(M)U(F)U(M)C(F)U(M)U(F)U(M)U(F)U(M)G (F)C(M)A(F)G(M)C(F)C(M)A(F)C(M)C(F)A(F)^UGG(F)^ACU(F)^ACA(F)^AGG(F)^A CG(F)^ACG(F)^ACG(F)^ACA(F)^AGA(F)^UCA(F)^UCG(F)^ACU(F)^AUA(F)^AAG( F)p | 190 |

**[Table 2-25]**

| | | | |
|---|---|---|---|
| E68-2 | Solid Phase Synthesis | ACG(F)^ACG(F)^ACG(F)^AUA(F)^AAG(F)^GUG(F)^GCG(F)^ACU(F)^AUA(F)^AGG (F)^ACG(F)^ACG(F)^ACG(F)^ACA(F)^AAC(F)^ACC(F)^ACC(F)^ACC(F)^ACC(F)^ ACC(F)^ACT(MOE)^G(MOE)^A(MOE)^A(F)^A(M)^A(F)^A(M)^A(F)^A(M)^A(F)^A(M )^A(F)^A(M)^A(F)^A(M)^A(F)^A(M)^A(F)^A(M)^A(F)^A(MOE)^A(MOE)^A(MOE) | 191 |
| E69 | Same as Example 1 | G(MOE)G(MOE)G(MOE)A(MOE)G(MOE)A(MOE)A(F)U(M)A(F)C(M)A(F)A(M)G(F)C (M)U(F)A(M)C(F)U(M)U(F)G(M)U(F)U(M)C(F)U(M)U(F)U(M)U(F)U(M)G(F)C(M)A(F) G(M)C(F)C(M)A(F)C(M)C(F)AUGG(F)ACU(F)ACA(F)AGG(F)ACG(F)ACG(F)ACG(F )ACA(F)AGA(F)UCA(F)UCG(F)ACU(F)AUA(F)AAGACGACG(F)ACG(F)AUA(F)AA G(F)GUG(F)GCG(F)ACU(F)AUA(F)AGG(F)ACG(F)ACG(F)ACG(F)ACA(F)AAC(F)A CC(F)ACC(F)ACC(F)ACC(F)ACC(F)ACU(MOE)G(MOE)A(MOE)AAAAAAAAAAAA AAAAAAAA | 192 |
| E69-1 | Solid Phase Synthesis | G(MOE)G(MOE)G(MOE)A(MOE)G(MOE)A(MOE)A(F)U(M)A(F)C(M)A(F)A(M)G(F)C (M)U(F)A(M)C(F)U(M)U(F)G(M)U(F)U(M)C(F)U(M)U(F)U(M)G(F)C(M)A(F) G(M)C(F)C(M)A(F)C(M)C(F)AUGG(F)ACU(F)ACA(F)AGG(F)ACG(F)ACG(F)ACG(F )ACA(F)AGA(F)UCA(F)UCG(F)ACU(F)AUA(F)AAG | 193 |
| E69-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACU(MOE)G( MOE)A(MOE)AAAAAAAAAAAAAAAAAAAA | 194 |
| E70 | Same as Example 1 | G(MOE)^G(MOE)^G(MOE)A(MOE)G(MOE)A(MOE)A(F)U(M)A(F)C(M)A(F)A(M)G(F) C(M)U(F)A(M)C(F)U(M)U(F)G(M)U(F)U(M)C(F)U(M)U(F)U(M)U(F)U(M)G(F)C(M)A( F)G(M)C(F)C(M)A(F)C(M)C(F)AUGG(F)ACU(F)ACA(F)AGG(F)ACG(F)ACG(F)ACG (F)ACA(F)AGA(F)UCA(F)UCG(F)ACU(F)AUA(F)AAGACGACG(F)ACG(F)AUA(F)A AG(F)GUG(F)GCG(F)ACU(F)AUA(F)AGG(F)ACG(F)ACG(F)ACG(F)ACA(F)AAC(F) ACC(F)ACC(F)ACC(F)ACC(F)ACC(F)ACU(MOE)^G(MOE)^A(MOE)A(F)AAA(F)AA A(F)AAA(F)AAA(F)AAA(F)AA(MOE)^A(MOE)^A(MOE) | 195 |
| E70-1 | Solid Phase Synthesis | G(MOE)^G(MOE)^G(MOE)A(MOE)G(MOE)A(MOE)A(F)U(M)A(F)C(M)A(F)A(M)G(F) C(M)U(F)A(M)C(F)U(M)U(F)G(M)U(F)U(M)C(F)U(M)U(F)U(M)U(F)U(M)G(F)C(M)A( F)G(M)C(F)C(M)A(F)C(M)C(F)AUGG(F)ACU(F)ACA(F)AGG(F)ACG(F)ACG(F)ACG (F)ACA(F)AGA(F)UCA(F)UCG(F)ACU(F)AUA(F)AAG | 196 |
| E70-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACU(MOE)^G (MOE)^A(MOE)A(F)AAA(F)AAA(F)AAA(F)AAA(F)AAA(F)AA(MOE)^A(MOE)^A(MO E) | 197 |

**[Table 2-26]**

| | | | |
|---|---|---|---|
| E71 | Solid Phase Synthesis | G(MOE)^G(MOE)^G(MOE)A(MOE)G(MOE)A(MOE)A(F)U(M)A(F)C(M)A(F)A(M)G(F) C(M)U(F)A(M)C(F)U(M)U(F)G(M)U(F)U(M)C(F)U(M)U(F)U(M)U(F)U(M)G(F)C(M)A( F)G(M)C(F)C(M)A(F)C(M)C(F)A(F)UGG(F)ACU(F)AUA(F)AGG(F)ACG(F)ACG(F)A CG(F)ACA(F)AAG(F)GUG(F)GCC(F)ACC(F)ACC(F)ACC(F)ACC(F)ACC(F)ACT(M OE)G(MOE)A(MOE) | 198 |
| E72 | Solid Phase Synthesis | G(MOE)^G(MOE)^G(MOE)A(MOE)G(MOE)A(MOE)A(F)U(M)A(F)C(M)A(F)A(M)G(F) C(M)U(F)A(M)C(F)U(M)(rSpacer)G(M)(rSpacer)U(M)(rSpacer)U(M)(rSpacer)U(M) U(F)U(M)G(F)C(M)A(F)G(M)C(F)C(M)A(F)C(M)C(F)A(F)UGG(F)ACU(F)AUA(F)AG G(F)ACG(F)ACG(F)ACG(F)ACA(F)AAG(F)GUG(F)GCC(F)ACC(F)ACC(F)ACC(F)A CC(F)ACC(F)ACT(MOE)G(MOE)A(MOE) | 199 |
| E73 | Solid Phase Synthesis | G(MOE)^G(MOE)^G(MOE)A(MOE)G(MOE)A(MOE)A(F)U(M)A(F)C(M)A(F)A(M)G(F) C(M)U(F)A(M)C(F)U(M)(dSpacer)G(M) (dSpacer)U(M) (dSpacer)U(M) (dSpacer)U(M)U(F)U(M)G(F)C(M)A(F)G(M)C(F)C(M)A(F)C(M)C(F)A(F)UGG(F)AC U(F)AUA(F)AGG(F)ACG(F)ACG(F)ACG(F)ACA(F)AAG(F)GUG(F)GCC(F)ACC(F)A CC(F)ACC(F)ACC(F)ACC(F)ACT(MOE)G(MOE)A(MOE) | 200 |
| E74 | Solid Phase Synthesis | G(MOE)^G(MOE)^G(MOE)A(MOE)G(MOE)A(MOE)A(F)U(M)A(F)C(M)A(F)A(M)G(F) C(M)U(F)A(M)C(F)U(M)(C3)G(M) (C3)U(M)(C3)U(M)(C3)U(M)U(F)U(M)G(F)C(M)A(F)G(M)C(F)C(M)A(F)C(M)C(F)A( F)UGG(F)ACU(F)AUA(F)AGG(F)ACG(F)ACG(F)ACG(F)ACA(F)AAG(F)GUG(F)GC C(F)ACC(F)ACC(F)ACC(F)ACC(F)ACC(F)ACT(MOE)G(MOE)A(MOE) | 201 |
| E75 | Solid Phase Synthesis | G(MOE)^G(MOE)^G(MOE)A(MOE)G(MOE)A(MOE)A(F)U(M)A(F)C(M)A(F)A(M)G(F) C(M)U(F)A(M)C(F)U(M)(Pyrrolidine)G(M) (Pyrrolidine )U(M)(Pyrrolidine)U(M)(Pyrrolidine)U(M)U(F)U(M)G(F)C(M)A(F)G(M) C(F)C(M)A(F)C(M)C(F)A(F)UGG(F)ACU(F)AUA(F)AGG(F)ACG(F)ACG(F)ACG(F)A CA(F)AAG(F)GUG(F)GCC(F)ACC(F)ACC(F)ACC(F)ACC(F)ACC(F)ACT(MOE)G(M OE)A(MOE) | 202 |
| E76 | Solid Phase Synthesis | G(MOE)^G(MOE)^G(MOE)A(MOE)G(MOE)A(MOE)A(F)U(M)A(F)C(M)A(F)A(M)G(F) C(M)U(F)A(M)C(F)U(M)(Ethynyl-dSpacer)G(M) (Ethynyl-dSpacer)U(M)(Ethynyl- dSpacer)U(M)(Ethynyl- dSpacer)U(M)U(F)U(M)G(F)C(M)A(F)G(M)C(F)C(M)A(F)C(M)C(F)A(F)UGG(F)ACU (F)AUA(F)AGG(F)ACG(F)ACG(F)ACG(F)ACA(F)AAG(F)GUG(F)GCC(F)ACC(F)AC C(F)ACC(F)ACC(F)ACC(F)ACT(MOE)G(MOE)A(MOE) | 203 |

**[Table 2-27]**

| | | | |
|---|---|---|---|
| E77 | Solid Phase Synthesis | G(MOE)^G(MOE)^G(MOE)A(MOE)G(MOE)A(MOE)A(F)U(M)(rSpacer)C(M)(rSpacer )A(M)(rSpacer)C(M)(rSpacer)A(M)C(F)U(M)U(F)G(M)U(F)U(M)C(F)U(M)U(F)U(M) U(F)U(M)G(F)C(M)A(F)G(M)C(F)C(M)A(F)C(M)C(F)A(F)UGG(F)ACU(F)AUA(F)AG G(F)ACG(F)ACG(F)ACG(F)ACA(F)AAG(F)GUG(F)GCC(F)ACC(F)ACC(F)ACC(F)A CC(F)ACC(F)ACT(MOE)G(MOE)A(MOE) | 204 |
| E78 | Solid Phase Synthesis | G(MOE)^G(MOE)^G(MOE)A(MOE)G(MOE)A(MOE)A(F)U(M)A(F)C(M)A(F)A(M)G(F) C(M)U(F)A(M)C(F)U(M)U(F)G(M)U(F)U(M)C(F)U(M)U(F)U(M)U(F)(rSpacer)G(F)(r Spacer)A(F)(rSpacer)C(F)(rSpacer)A(F)C(M)C(F)A(F)UGG(F)ACU(F)AUA(F)AGG (F)ACG(F)ACG(F)ACG(F)ACA(F)AAG(F)GUG(F)GCC(F)ACC(F)ACC(F)ACC(F)AC C(F)ACC(F)ACT(MOE)G(MOE)A(MOE) | 205 |
| E79 | Same as Example 2 | G(MOE)^G(MOE)^G(MOE)A(MOE)G(MOE)A(MOE)A(F)U(M)A(F)C(M)A(F)A(M)G(F) C(M)U(F)A(M)C(F)U(M)U(F)G(M)U(F)U(M)C(F)U(M)U(F)U(M)U(F)U(M)G(F)C(M)A( F)G(M)C(F)C(M)A(F)C(M)C(F)AUGG(F)ACU(F)ACA(F)AGG(F)ACG(F)ACG(F)ACG (F)ACA(F)AGA(F)UCA(F)UCG(F)ACU(F)AUA(F)AAG(F)ACG(F)ACG(F)ACG(F)AU A(F)AAG(F)GUG(F)GCG(F)ACU(F)AUA(F)AGG(F)ACG(F)ACG(F)ACG(F)ACA(F)A AC(F)ACC(F)ACC(F)ACC(F)ACC(F)ACC(F)ACT(MOE)^G(MOE)^A(MOE)^A(F)^A( M)^A(F)^A(M)^A(F)^A(M)^A(F)^A(M)^A(F)^A(M)^A(F)^A(M)^A(F)^A(M)^A(F)^A(M) ^A(F)^A(MOE)^A(MOE)^A(MOE) | 206 |
| E79-1 | Solid Phase Synthesis | G(MOE)^G(MOE)^G(MOE)A(MOE)G(MOE)A(MOE)A(F)U(M)A(F)C(M)A(F)A(M)G(F) C(M)U(F)A(M)C(F)U(M)U(F)G(M)U(F)U(M)C(F)U(M)U(F)U(M)U(F)U(M)G(F)C(M)A( F)G(M)C(F)C(M)A(F)C(M)C(F)AUGG(F)ACU(F)ACA(F)AGG(F)ACG(F)ACG(F)ACG (F)ACA(F)AGA(F)UCA(F)UCG(F)ACU(F)AUA(F)AAG(F)p | 207 |
| E79-2 | Solid Phase Synthesis | ACG(F)ACG(F)ACG(F)AUA(F)AAG(F)GUG(F)GCG(F)ACU(F)AUA(F)AGG(F)ACG( F)ACG(F)ACG(F)ACA(F)AAC(F)ACC(F)ACC(F)ACC(F)ACC(F)ACC(F)ACT(MOE)^ G(MOE)^A(MOE)^A(F)^A(M)^A(F)^A(M)^A(F)^A(M)^A(F)^A(M)^A(F)^A(M)^A(F)^A( M)^A(F)^A(M)^A(F)^A(M)^A(F)^A(MOE)^A(MOE)^A(MOE) | 208 |
| E80 | Same as Example 2 | G(MOE)^G(MOE)^G(MOE)A(MOE)G(MOE)A(MOE)A(F)U(M)A(F)C(M)A(F)A(M)G(F) C(M)U(F)A(M)C(F)U(M)U(F)G(M)U(F)U(M)C(F)U(M)U(F)U(M)U(F)U(M)G(F)C(M)A( F)G(M)C(F)C(M)A(F)C(M)C(F)AUGG(F)ACU(F)ACA(F)AGG(F)ACG(F)ACG(F)ACG (F)ACA(F)AGA(F)UCA(F)UCG(F)ACU(F)AUA(F)AAG(F)ACG(F)ACG(F)ACG(F)AU A(F)AAG(F)GUG(F)GCG(F)ACU(F)AUA(F)AGG(F)ACG(F)ACG(F)ACG(F)ACA(F)A AC(F)ACC(F)ACC(F)ACC(F)ACC(F)ACC(F)ACT(MOE)^G(MOE)^A(MOE)aaaaaaa aaaaaaaaaaaa | 209 |

**[Table 2-28]**

| | | | |
|---|---|---|---|
| E80-1 | Solid Phase Synthesis | G(MOE)^G(MOE)^G(MOE)A(MOE)G(MOE)A(MOE)A(F)U(M)A(F)C(M)A(F)A(M)G(F) C(M)U(F)A(M)C(F)U(M)U(F)G(M)U(F)U(M)C(F)U(M)U(F)U(M)U(F)U(M)G(F)C(M)A( F)G(M)C(F)C(M)A(F)C(M)C(F)AUGG(F)ACU(F)ACA(F)AGG(F)ACG(F)ACG(F)ACG (F)ACA(F)AGA(F)UCA(F)UCG(F)ACU(F)AUA(F)AAG(F)p | 210 |
| E80-2 | Solid Phase Synthesis | ACG(F)ACG(F)ACG(F)AUA(F)AAG(F)GUG(F)GCG(F)ACU(F)AUA(F)AGG(F)ACG( F)ACG(F)ACG(F)ACA(F)AAC(F)ACC(F)ACC(F)ACC(F)ACC(F)ACC(F)ACT(MOE)^ G(MOE)^A(MOE)aaaaaaaaaaaaaaaaaaaa | 211 |
| E81 | Same as Example 2 | G(MOE)^G(MOE)^G(MOE)A(MOE)G(MOE)A(MOE)A(F)U(M)A(F)C(M)A(F)A(M)G(F) C(M)U(F)A(M)C(F)U(M)U(F)G(M)U(F)U(M)C(F)U(M)U(F)U(M)U(F)U(M)G(F)C(M)A( F)G(M)C(F)C(M)A(F)C(M)C(F)AUGG(F)ACU(F)ACA(F)AGG(F)ACG(F)ACG(F)ACG (F)ACA(F)AGA(F)UCA(F)UCG(F)ACU(F)AUA(F)AAGACGACG(F)ACG(F)AUA(F)A AG(F)GUG(F)GCG(F)ACU(F)AUA(F)AGG(F)ACG(F)ACG(F)ACG(F)ACA(F)AAC(F) ACC(F)ACC(F)ACC(F)ACC(F)ACC(F)ACT(MOE)^G(MOE)^A(MOE)^a^a^a^a^a^a^a ^a^a^a^a^a^a^a^a^a^a^a^a^a | 212 |
| E81-1 | Solid Phase Synthesis | G(MOE)^G(MOE)^G(MOE)A(MOE)G(MOE)A(MOE)A(F)U(M)A(F)C(M)A(F)A(M)G(F) C(M)U(F)A(M)C(F)U(M)U(F)G(M)U(F)U(M)C(F)U(M)U(F)U(M)U(F)U(M)G(F)C(M)A( F)G(M)C(F)C(M)A(F)C(M)C(F)AUGG(F)ACU(F)ACA(F)AGG(F)ACG(F)ACG(F)ACG (F)ACA(F)AGA(F)UCA(F)UCG(F)ACU(F)AUA(F)AAG(F)p | 213 |
| E81-2 | Solid Phase Synthesis | ACG(F)ACG(F)ACG(F)AUA(F)AAG(F)GUG(F)GCG(F)ACU(F)AUA(F)AGG(F)ACG( F)ACG(F)ACG(F)ACA(F)AAC(F)ACC(F)ACC(F)ACC(F)ACC(F)ACC(F)ACT(MOE)^ G(MOE)^A(MOE)^a^a^a^a^a^a^a^a^a^a^a^a^a^a^a^a^a^a^a^a | 214 |
| E82 | Same as Example 2 | G(MOE)^G(MOE)^G(MOE)A(MOE)G(MOE)A(MOE)A(F)U(M)A(F)C(M)A(F)A(M)G(F) C(M)U(F)A(M)C(F)U(M)U(F)G(M)U(F)U(M)C(F)U(M)U(F)U(M)U(F)U(M)G(F)C(M)A( F)G(M)C(F)C(M)A(F)C(M)C(F)AUGG(F)ACU(F)ACA(F)AGG(F)ACG(F)ACG(F)ACG (F)ACA(F)AGA(F)UCA(F)UCG(F)ACU(F)AUA(F)AAGACGACG(F)ACG(F)AUA(F)A AG(F)GUG(F)GCG(F)ACU(F)AUA(F)AGG(F)ACG(F)ACG(F)ACG(F)ACA(F)AAC(F) ACC(F)ACC(F)ACC(F)ACC(F)ACC(F)ACT(MOE)^G(MOE)^A(MOE)^a^A(M)^a^A(M )^a^A(M)^a^A(M)^a^A(M)^a^A(M)^a^A(M)^a^A(M)^a^A(MOE)^A(MOE)^A(MOE) | 215 |
| E82-1 | Solid Phase Synthesis | G(MOE)^G(MOE)^G(MOE)A(MOE)G(MOE)A(MOE)A(F)U(M)A(F)C(M)A(F)A(M)G(F) C(M)U(F)A(M)C(F)U(M)U(F)G(M)U(F)U(M)C(F)U(M)U(F)U(M)U(F)U(M)G(F)C(M)A( F)G(M)C(F)C(M)A(F)C(M)C(F)AUGG(F)ACU(F)ACA(F)AGG(F)ACG(F)ACG(F)ACG (F)ACA(F)AGA(F)UCA(F)UCG(F)ACU(F)AUA(F)AAG(F)p | 216 |

**[Table 2-29]**

| | | | |
|---|---|---|---|
| E82-2 | Solid Phase Synthesis | ACG(F)ACG(F)ACG(F)AUA(F)AAG(F)GUG(F)GCG(F)ACU(F)AUA(F)AGG(F)ACG( F)ACG(F)ACG(F)ACA(F)AAC(F)ACC(F)ACC(F)ACC(F)ACC(F)ACC(F)ACT(MOE)^ G(MOE)^A(MOE)^a^A(M)^a^A(M)^a^A(M)^a^A(M)^a^A(M)^a^A(M)^a^A(M)^a^A(M) ^a^A(MOE)^A(MOE)^A(MOE) | 217 |
| E83 | Same as Example 2 | G(MOE)^G(MOE)^G(MOE)A(MOE)G(MOE)A(MOE)A(F)U(M)A(F)C(M)A(F)A(M)G(F) C(M)U(F)A(M)C(F)U(M)U(F)G(M)U(F)U(M)C(F)U(M)U(F)U(M)U(F)U(M)G(F)C(M)A( F)G(M)C(F)C(M)A(F)C(M)C(F)AUGG(F)ACU(F)ACA(F)AGG(F)ACG(F)ACG(F)ACG (F)ACA(F)AGA(F)UCA(F)UCG(F)ACU(F)AUA(F)AAGACGACG(F)ACG(F)AUA(F)A AG(F)GUG(F)GCG(F)ACU(F)AUA(F)AGG(F)ACG(F)ACG(F)ACG(F)ACA(F)AAC(F) ACC(F)ACC(F)ACC(F)ACC(F)ACC(F)ACT(MOE)^G(MOE)^A(MOE)^a^a^A(M)^a^a^ A(M)^a^a^A(M)^a^a^A(M)^a^a^A(M)^a^a^A(MOE)^A(MOE)^A(MOE) | 218 |
| E83-1 | Solid Phase Synthesis | G(MOE)^G(MOE)^G(MOE)A(MOE)G(MOE)A(MOE)A(F)U(M)A(F)C(M)A(F)A(M)G(F) C(M)U(F)A(M)C(F)U(M)U(F)G(M)U(F)U(M)C(F)U(M)U(F)U(M)U(F)U(M)G(F)C(M)A( F)G(M)C(F)C(M)A(F)C(M)C(F)AUGG(F)ACU(F)ACA(F)AGG(F)ACG(F)ACG(F)ACG (F)ACA(F)AGA(F)UCA(F)UCG(F)ACU(F)AUA(F)AAG(F)p | 219 |
| E83-2 | Solid Phase Synthesis | ACG(F)ACG(F)ACG(F)AUA(F)AAG(F)GUG(F)GCG(F)ACU(F)AUA(F)AGG(F)ACG( F)ACG(F)ACG(F)ACA(F)AAC(F)ACC(F)ACC(F)ACC(F)ACC(F)ACC(F)ACT(MOE)^ G(MOE)^A(MOE)^a^a^A(M)^a^a^A(M)^a^a^A(M)^a^a^A(M)^a^a^A(M)^a^a^A(MOE) ^A(MOE)^A(MOE) | 220 |
| E84 | Same as Example 2 | pG(MOE)^G(MOE)^G(MOE)A(MOE)G(MOE)A(MOE)A(F)U(M)A(F)C(M)A(F)A(M)G( F)C(M)U(F)A(M)C(F)U(M)U(F)G(M)U(F)U(M)C(F)U(M)U(F)U(M)U(F)U(M)G(F)C(M) A(F)G(M)C(F)C(M)A(F)C(M)C(F)AUGG(F)ACU(F)ACA(F)AGG(F)ACG(F)ACG(F)A CG(F)ACA(F)AGA(F)UCA(F)UCG(F)ACU(F)AUA(F)AAG(F)ACG(F)ACG(F)ACG(F) AUA(F)AAG(F)GUG(F)GCG(F)ACU(F)AUA(F)AGG(F)ACG(F)ACG(F)ACG(F)ACA( F)AAC(F)ACC(F)ACC(F)ACC(F)ACC(F)ACC(F)ACT(MOE)^G(MOE)^A(MOE)^A(F) ^A(M)^A(F)^A(M)^A(F)^A(M)^A(F)^A(M)^A(F)^A(M)^A(F)^A(M)^A(F)^A(M)^A(F)^A (M)^A(F)^A(MOE)^A(MOE)^A(MOE) | 221 |
| E84-1 | Solid Phase Synthesis | pG(MOE)^G(MOE)^G(MOE)A(MOE)G(MOE)A(MOE)A(F)U(M)A(F)C(M)A(F)A(M)G( F)C(M)U(F)A(M)C(F)U(M)U(F)G(M)U(F)U(M)C(F)U(M)U(F)U(M)U(F)U(M)G(F)C(M) A(F)G(M)C(F)C(M)A(F)C(M)C(F)AUGG(F)ACU(F)ACA(F)AGG(F)ACG(F)ACG(F)A CG(F)ACA(F)AGA(F)UCA(F)UCG(F)ACU(F)AUA(F)AAG(F)p | 222 |

**[Table 2-30]**

| | | | |
|---|---|---|---|
| E84-2 | Solid Phase Synthesis | ACG(F)ACG(F)ACG(F)AUA(F)AAG(F)GUG(F)GCG(F)ACU(F)AUA(F)AGG(F)ACG( F)ACG(F)ACG(F)ACA(F)AAC(F)ACC(F)ACC(F)ACC(F)ACC(F)ACC(F)ACT(MOE)^ G(MOE)^A(MOE)^A(F)^A(M)^A(F)^A(M)^A(F)^A(M)^A(F)^A(M)^A(F)^A(M)^A(F)^A( M)^A(F)^A(M)^A(F)^A(M)^A(F)^A(MOE)^A(MOE)^A(MOE) | 223 |
| E85 | Solid Phase Synthesis | G(MOE)^G(MOE)^G(MOE)A(MOE)G(MOE)A(MOE)AUACAAGCUACUUGUUCUUUU UGCAGCCACCA(F)UGG(F)ACU(F)AUA(F)AGG(F)ACG(F)ACG(F)ACG(F)ACA(F)A AG(F)GUG(F)GCC(F)ACC(F)ACC(F)ACC(F)ACC(F)ACC(F)ACT(MOE)G(MOE)A(M OE) | 224 |
| E86 | Solid Phase Synthesis | G(MOE)G(MOE)G(MOE)A(MOE)G(MOE)A(MOE)A(M)U(M)A(M)C(M)A(M)A(M)G(M) C(M)U(M)A(M)C(M)U(F)(rSpacer)G(F)(rSpacer)U(F)(rSpacer)U(F)(rSpacer)U(M)U (M)U(M)G(M)C(M)A(M)GCCA(M)CCA(F)UGG(F)ACU(F)AUA(F)AGG(F)ACG(F)AC G(F)ACG(F)ACA(F)AAG(F)GUG(F)GCC(F)ACC(F)ACC(F)ACC(F)ACC(F)ACC(F)A CT(MOE)G(MOE)A(MOE) | 225 |
| E87 | Solid Phase Synthesis | G(MOE)G(MOE)G(MOE)A(MOE)G(MOE)A(MOE)A(M)U(M)A(M)C(M)A(M)A(M)G(M) C(M)U(M)A(M)C(M)U(F)UG(F)UU(F)CU(F)UU(M)U(M)U(M)G(M)C(M)A(M)GCCA(M )CCA(F)UGG(F)ACU(F)AUA(F)AGG(F)ACG(F)ACG(F)ACG(F)ACA(F)AAG(F)GUG( F)GCC(F)ACC(F)ACC(F)ACC(F)ACC(F)ACC(F)ACT(MOE)G(MOE)A(MOE) | 226 |
| E88 | Solid Phase Synthesis | G(MOE)^G(MOE)^G(MOE)A(MOE)G(MOE)A(MOE)A(F)U(M)A(F)C(M)A(F)A(M)G(F) C(M)U(F)A(M)C(F)(rSpacer)U(F)(rSpacer)U(F)(rSpacer)C(F)(rSpacer)U(F)U(M)U( F)U(M)G(F)C(M)A(F)G(M)C(F)C(M)A(F)C(M)C(F)A(F)UGG(F)ACU(F)AUA(F)AGG( F)ACG(F)ACG(F)ACG(F)ACA(F)AAG(F)GUG(F)GCC(F)ACC(F)ACC(F)ACC(F)AC C(F)ACC(F)ACT(MOE)G(MOE)A(MOE) | 227 |
| E89 | Solid Phase Synthesis | G(MOE)^G(MOE)^G(MOE)A(MOE)G(MOE)A(MOE)A(F)U(M)(rSpacer)C(M)(rSpacer )A(M)(rSpacer)C(M)(rSpacer)A(M)C(F)U(M)(rSpacer)G(M)(rSpacer)U(M)(rSpacer )U(M)(rSpacer)U(M)U(F)U(M)G(F)C(M)A(F)G(M)C(F)C(M)A(F)C(M)C(F)A(F)UGG( F)ACU(F)AUA(F)AGG(F)ACG(F)ACG(F)ACG(F)ACA(F)AAG(F)GUG(F)GCC(F)AC C(F)ACC(F)ACC(F)ACC(F)ACC(F)ACT(MOE)G(MOE)A(MOE) | 228 |
| E90 | Solid Phase Synthesis | G(MOE)^G(MOE)^G(MOE)A(MOE)G(MOE)A(MOE)A(F)U(M)A(F)C(M)A(F)A(M)G(F) C(M)U(F)A(M)C(F)(rSpacer)(rSpacer)(rSpacer)(rSpacer)(rSpacer)(rSpacer)(rSpa cer)(rSpacer)U(M)U(F)U(M)G(F)C(M)A(F)G(M)C(F)C(M)A(F)C(M)C(F)A(F)UGG(F) ACU(F)AUA(F)AGG(F)ACG(F)ACG(F)ACG(F)ACA(F)AAG(F)GUG(F)GCC(F)ACC( F)ACC(F)ACC(F)ACC(F)ACC(F)ACT(MOE)G(MOE)A(MOE) | 229 |

**[Table 2-31]**

| | | | |
|---|---|---|---|
| E91 | Solid Phase Synthesis | G(MOE)^G(MOE)^G(MOE)A(MOE)G(MOE)A(MOE)(rSpacer)U(M)(rSpacer)C(M)(rS pacer)A(M)(rSpacer)C(M)(rSpacer)A(M)(rSpacer)U(M)(rSpacer)G(M)(rSpacer)U( M)(rSpacer)U(M)(rSpacer)U(M)(rSpacer)U(M)(rSpacer)C(M)A(F)G(M)C(F)C(M)A( F)C(M)C(F)A(F)UGG(F)ACU(F)AUA(F)AGG(F)ACG(F)ACG(F)ACG(F)ACA(F)AAG( F)GUG(F)GCC(F)ACC(F)ACC(F)ACC(F)ACC(F)ACC(F)ACT(MOE)G(MOE)A(MOE) | 230 |
| E92 | Solid Phase Synthesis | G(MOE)^G(MOE)^G(MOE)A(MOE)G(MOE)A(MOE)A(F)(rSpacer)A(F)(rSpacer)A(F) (rSpacer)G(F)(rSpacer)U(F)(rSpacer)C(F)(rSpacer)U(F)(rSpacer)U(F)(rSpacer)C( F)(rSpacer)U(F)(rSpacer)U(F)(rSpacer)G(F)(rSpacer)A(F)G(M)C(F)C(M)A(F)C(M) C(F)A(F)UGG(F)ACU(F)AUA(F)AGG(F)ACG(F)ACG(F)ACG(F)ACA(F)AAG(F)GUG (F)GCC(F)ACC(F)ACC(F)ACC(F)ACC(F)ACC(F)ACT(MOE)G(MOE)A(MOE) | 231 |
| E93 | Solid Phase Synthesis | G(MOE)G(MOE)G(MOE)A(MOE)G(MOE)A(MOE)A(F)U(M)A(F)C(M)A(F)A(M)G(F)C (M)U(F)A(M)C(F)U(M)U(F)G(M)U(F)U(M)C(F)U(M)U(F)U(M)U(F)U(M)G(F)C(M)A(F) G(M)C(F)C(M)A(F)C(M)C(F)A(F)UGG(F)ACU(F)AUA(F)AGG(F)ACG(F)ACG(F)AC G(F)ACA(F)AAG(F)GUG(F)GCC(F)ACC(F)ACC(F)ACC(F)ACC(F)ACC(F)ACT(MO E)G(MOE)A(MOE) | 232 |
| E94 | Solid Phase Synthesis | G(MOE)^G(MOE)^G(MOE)^A(MOE)^G(MOE)^A(MOE)^A(F)^U(M)^A(F)^C(M)^A(F)^ A(M)^G(F)^C(M)^U(F)^A(M)^C(F)^U(M)^U(F)^G(M)^U(F)^U(M)^C(F)^U(M)^U(F)^U( M)^U(F)^U(M)^G(F)^C(M)^A(F)^G(M)^C(F)^C(M)^A(F)^C(M)^C(F)^A(F)UGG(F)AC U(F)AUA(F)AGG(F)ACG(F)ACG(F)ACG(F)ACA(F)AAG(F)GUG(F)GCC(F)ACC(F)A CC(F)ACC(F)ACC(F)ACC(F)ACT(MOE)G(MOE)A(MOE) | 233 |
| E95 | Same as Example 2 | G(MOE)^G(MOE)^G(MOE)A(MOE)G(MOE)A(MOE)A(F)U(M)A(F)C(M)A(F)A(M)G(F) C(M)U(F)A(M)C(F)U(M)U(F)G(M)U(F)U(M)C(F)U(M)U(F)U(M)U(F)U(M)G(F)C(M)A( F)G(M)C(F)C(M)A(F)C(M)C(F)AUGG(F)ACU(F)ACA(F)AGG(F)ACG(F)ACG(F)ACG (F)ACA(F)AGA(F)UCA(F)UCG(F)ACU(F)AUA(F)AAG(F)ACG(F)ACG(F)ACG(F)AU A(F)AAG(F)GUG(F)GCG(F)ACU(F)AUA(F)AGG(F)ACG(F)ACG(F)ACG(F)ACA(F)A AC(F)ACC(F)ACC(F)ACC(F)ACC(F)ACC(F)ACT(MOE)^G(MOE)^A(MOE)^A(MOE) ^A(MOE)^A(MOE)^A(MOE)^A(MOE)^A(MOE)^A(MOE)^A(MOE)^A(MOE)^A(MOE)^ A(MOE)^A(MOE)^A(MOE)^A(MOE)^A(MOE)^A(MOE)^A(MOE)^A(MOE)^A(MOE)^A (MOE) | 234 |
| E95-1 | Solid Phase Synthesis | G(MOE)^G(MOE)^G(MOE)A(MOE)G(MOE)A(MOE)A(F)U(M)A(F)C(M)A(F)A(M)G(F) C(M)U(F)A(M)C(F)U(M)U(F)G(M)U(F)U(M)C(F)U(M)U(F)U(M)U(F)U(M)G(F)C(M)A( | 235 |
| | | F)G(M)C(F)C(M)A(F)C(M)C(F)AUGG(F)ACU(F)ACA(F)AGG(F)ACG(F)ACG(F)ACG (F)ACA(F)AGA(F)UCA(F)UCG(F)ACU(F)AUA(F)AAG(F)p | |

**[Table 2-32]**

| | | | |
|---|---|---|---|
| E95-2 | Solid Phase Synthesis | ACG(F)ACG(F)ACG(F)AUA(F)AAG(F)GUG(F)GCG(F)ACU(F)AUA(F)AGG(F)ACG( F)ACG(F)ACG(F)ACA(F)AAC(F)ACC(F)ACC(F)ACC(F)ACC(F)ACC(F)ACT(MOE)^ G(MOE)^A(MOE)^A(MOE)^A(MOE)^A(MOE)^A(MOE)^A(MOE)^A(MOE)^A(MOE)^A (MOE)^A(MOE)^A(MOE)^A(MOE)^A(MOE)^A(MOE)^A(MOE)^A(MOE)^A(MOE)^A( MOE)^A(MOE)^A(MOE)^A(MOE) | 236 |
| E96 | Solid Phase Synthesis | GGG(MOE)^AG(MOE)^A(MOE)^AU(M)^A(F)^C(M)^A(F)^AG(F)^CU(F)^A(M)^CUUG UU(M)^CUUUUUG(F)^C(M)^AG(M)^C(F)^C(M)^A(F)^C(M)^CAUGGACUAUAAGGA CGACGACGACAAAGGUGGCCACCACCACCACCACUGA | 237 |
| E97 | Solid Phase Synthesis | G(MOE)^G(MOE)^G(MOE)^A(MOE)^G(MOE)^A(MOE)^AUACAAGCUACUUGUUCU UUUUGCAGCCACCAUGGACUAUAAGGACGACGACGACAAAGGUGGCCACCAC CACCACCACCACUGA | 238 |
| E98 | Solid Phase Synthesis | G(MOE)^G(MOE)^G(MOE)A(MOE)G(MOE)A(MOE)A(F)U(M)A(F)C(M)A(F)A(M)G(F) C(M)U(F)A(M)C(F)U(M)UG(M)UU(M)CU(M)UU(M)U(F)U(M)G(F)C(M)A(F)G(M)C(F) C(M)A(F)C(M)C(F)A(F)UGG(F)ACU(F)AUA(F)AGG(F)ACG(F)ACG(F)ACG(F)ACA( F)AAG(F)GUG(F)GCC(F)ACC(F)ACC(F)ACC(F)ACC(F)ACC(F)ACT(MOE)G(MOE) A(MOE) | 239 |
| E99 | Solid Phase Synthesis | G(MOE)^G(MOE)^G(MOE)A(MOE)G(MOE)A(MOE)AUACAAGCUACUUGUUCUUUU UGCAGCCACCA(F)UGG(F)ACU(F)AUA(F)AGG(F)ACG(F)ACG(F)ACG(F)ACA(F)A AG(F)GUG(F)GCC(F)ACC(F)ACC(F)ACC(F)ACC(F)ACC(F)ACT(MOE)^G(MOE)^A (MOE) | 240 |
| E100 | Solid Phase Synthesis | G(MOE)^G(MOE)^G(MOE)A(MOE)G(MOE)A(MOE)A(F)U(M)A(F)C(M)A(F)A(M)G(F) C(M)U(F)A(M)C(F)U(M)U(F)G(M)U(F)U(M)C(F)U(M)U(F)U(M)U(F)U(M)G(F)C(M)A( F)G(M)C(F)C(M)A(F)C(M)C(F)A(F)UGG(F)ACU(F)AUA(F)AGG(F)ACG(F)ACG(F)A CG(F)ACA(F)AAG(F)GUG(F)GCC(F)ACC(F)ACC(F)ACC(F)ACC(F)ACC(F)ACT(M OE)^G(MOE)^A(MOE) | 241 |
| E101 | Solid Phase Synthesis | G(MOE)^G(MOE)^G(MOE)^A(MOE)^G(MOE)^A(MOE)^A(F)^U(M)^A(F)^C(M)^A(F)^ A(M)^G(F)^C(M)^U(F)^A(M)^C(F)^U(M)^U(F)^G(M)^U(F)^U(M)^C(F)^U(M)^U(F)^U( M)^U(F)^U(M)^G(F)^C(M)^A(F)^G(M)^C(F)^C(M)^A(F)^C(M)^C(F)^A(F)UGG(F)AC U(F)AUA(F)AGG(F)ACG(F)ACG(F)ACG(F)ACA(F)AAG(F)GUG(F)GCC(F)ACC(F)A CC(F)ACC(F)ACC(F)ACC(F)ACT(MOE)^G(MOE)^A(MOE) | 242 |

**[Table 2-33]**

| | | | |
|---|---|---|---|
| E102 | Solid Phase Synthesis | G(MOE)^G(MOE)^G(MOE)^A(MOE)^G(MOE)^A(MOE)^A(F)^U(M)^A(F)^C(M)^A(F)^ A(M)^G(F)^C(M)^U(F)^A(M)^C(F)^U(M)^U(F)^G(M)^U(F)^U(M)^C(F)^U(M)^U(F)^U( M)^U(F)^U(M)^G(F)^C(M)^A(F)^G(M)C(F)C(M)A(F)C(M)C(F)A(F)UGG(F)ACU(F)A UA(F)AGG(F)ACG(F)ACG(F)ACG(F)ACA(F)AAG(F)GUG(F)GCC(F)ACC(F)ACC(F) ACC(F)ACC(F)ACC(F)ACT(MOE)^G(MOE)^A(MOE) | 243 |
| E103 | Solid Phase Synthesis | G(MOE)^G(MOE)^G(MOE)A(MOE)G(MOE)A(MOE)A(F)U(M)A(F)C(M)A(F)A(M)G(F) C(M)U(F)A(M)C(F)U(M)U(F)G(M)U(F)U(M)C(F)U(M)U(F)U(M)U(F)U(M)G(F)C(M)A( F)G(M)^C(F)^C(M)^A(F)^C(M)^C(F)^A(F)UGG(F)ACU(F)AUA(F)AGG(F)ACG(F)AC G(F)ACG(F)ACA(F)AAG(F)GUG(F)GCC(F)ACC(F)ACC(F)ACC(F)ACC(F)ACC(F)A CT(MOE)^G(MOE)^A(MOE) | 244 |
| E104 | Solid Phase Synthesis | G(MOE)G(MOE)G(MOE)A(MOE)G(MOE)A(MOE)A(rSpacer)AC(rSpacer)AG(rSpac er)UA(rSpacer)UU(rSpacer)UU(rSpacer)UU(rSpacer)UUGCAGCCACCA(F)UGG(F )ACU(F)AUA(F)AGG(F)ACG(F)ACG(F)ACG(F)ACA(F)AAG(F)GUG(F)GCC(F)ACC( F)ACC(F)ACC(F)ACC(F)ACC(F)ACT(MOE)G(MOE)A(MOE) | 245 |
| E105 | Solid Phase Synthesis | G(MOE)G(MOE)G(MOE)A(MOE)G(MOE)A(MOE)AUACAAGCUACUUGUUCUUUUU GCA(rSpacer)CC(rSpacer)CCA(F)UGG(F)ACU(F)AUA(F)AGG(F)ACG(F)ACG(F)A CG(F)ACA(F)AAG(F)GUG(F)GCC(F)ACC(F)ACC(F)ACC(F)ACC(F)ACC(F)ACT(M OE)G(MOE)A(MOE) | 246 |
| E106 | Solid Phase Synthesis | G(MOE)G(MOE)G(MOE)A(MOE)G(MOE)A(MOE)A(dSpacer)AC(dSpacer)AG(dSpac er)UA(dSpacer)UU(dSpacer)UU(dSpacer)UU(dSpacer)UUGCAGCCACCA(F)UGG( F)ACU(F)AUA(F)AGG(F)ACG(F)ACG(F)ACG(F)ACA(F)AAG(F)GUG(F)GCC(F)AC C(F)ACC(F)ACC(F)ACC(F)ACC(F)ACT(MOE)G(MOE)A(MOE) | 247 |
| E107 | Solid Phase Synthesis | G(MOE)G(MOE)G(MOE)A(MOE)G(MOE)A(MOE)AUACAAGCUACUUGUUCUUUUU GCA(dSpacer)CC(dSpacer)CCA(F)UGG(F)ACU(F)AUA(F)AGG(F)ACG(F)ACG(F)A CG(F)ACA(F)AAG(F)GUG(F)GCC(F)ACC(F)ACC(F)ACC(F)ACC(F)ACC(F)ACT(M OE)G(MOE)A(MOE) | 248 |
| E108 | Solid Phase Synthesis | G(MOE)G(MOE)G(MOE)A(MOE)G(MOE)A(MOE)A(C3)AC(C3)AG(C3)UA(C3)UU(C 3)UU(C3)UU(C3)UUGCAGCCACCA(F)UGG(F)ACU(F)AUA(F)AGG(F)ACG(F)ACG( | 249 |
| | | F)ACG(F)ACA(F)AAG(F)GUG(F)GCC(F)ACC(F)ACC(F)ACC(F)ACC(F)ACC(F)AC T(MOE)G(MOE)A(MOE) | |
| E109 | Solid Phase Synthesis | G(MOE)G(MOE)G(MOE)A(MOE)G(MOE)A(MOE)AUACAAGCUACUUGUUCUUUUU GCA(C3)CC(C3)CCA(F)UGG(F)ACU(F)AUA(F)AGG(F)ACG(F)ACG(F)ACG(F)ACA (F)AAG(F)GUG(F)GCC(F)ACC(F)ACC(F)ACC(F)ACC(F)ACC(F)ACT(MOE)G(MOE )A(MOE) | 250 |

**[Table 2-34]**

| | | | |
|---|---|---|---|
| E110 | Solid Phase Synthesis | G(MOE)G(MOE)G(MOE)A(MOE)G(MOE)A(MOE)AU(F)ACA(F)AGC(F)UAC(F)UUG( F)UUC(F)UUU(F)UUG(F)CAG(F)CCA(F)CCA(F)UGG(F)ACU(F)AUA(F)AGG(F)AC G(F)ACG(F)ACG(F)ACA(F)AAG(F)GUG(F)GCC(F)ACC(F)ACC(F)ACC(F)ACC(F)A CC(F)ACT(MOE)G(MOE)A(MOE) | 251 |
| E111 | Solid Phase Synthesis | G(MOE)G(MOE)G(MOE)A(MOE)G(MOE)A(MOE)AUA(F)CAA(F)GCU(F)ACU(F)UG U(F)UCU(F)UUU(F)UGC(F)AGC(F)CAC(F)CA(F)UGG(F)ACU(F)AUA(F)AGG(F)AC G(F)ACG(F)ACG(F)ACA(F)AAG(F)GUG(F)GCC(F)ACC(F)ACC(F)ACC(F)ACC(F)A CC(F)ACT(MOE)G(MOE)A(MOE) | 252 |
| E112 | Solid Phase Synthesis | G(MOE)G(MOE)G(MOE)A(MOE)G(MOE)A(MOE)A(F)UAC(F)AAG(F)CUA(F)CUU(F) GUU(F)CUU(F)UUU(F)GCA(F)GCC(F)ACC(F)A(F)UGG(F)ACU(F)AUA(F)AGG(F)A CG(F)ACG(F)ACG(F)ACA(F)AAG(F)GUG(F)GCC(F)ACC(F)ACC(F)ACC(F)ACC(F) ACC(F)ACT(MOE)G(MOE)A(MOE) | 253 |
| E113 | Solid Phase Synthesis | G(MOE)G(MOE)G(MOE)A(MOE)G(MOE)A(MOE)AU(M)ACA(M)AGC(M)UAC(M)UU G(M)UUC(M)UUU(M)UUG(M)CAG(M)CCA(M)CCA(F)UGG(F)ACU(F)AUA(F)AGG(F )ACG(F)ACG(F)ACG(F)ACA(F)AAG(F)GUG(F)GCC(F)ACC(F)ACC(F)ACC(F)ACC( F)ACC(F)ACT(MOE)G(MOE)A(MOE) | 254 |
| E114 | Solid Phase Synthesis | G(MOE)G(MOE)G(MOE)A(MOE)G(MOE)A(MOE)AUA(M)CAA(M)GCU(M)ACU(M)U GU(M)UCU(M)UUU(M)UGC(M)AGC(M)CAC(M)CA(F)UGG(F)ACU(F)AUA(F)AGG(F )ACG(F)ACG(F)ACG(F)ACA(F)AAG(F)GUG(F)GCC(F)ACC(F)ACC(F)ACC(F)ACC( F)ACC(F)ACT(MOE)G(MOE)A(MOE) | 255 |
| E115 | Solid Phase Synthesis | G(MOE)G(MOE)G(MOE)A(MOE)G(MOE)A(MOE)A(M)UAC(M)AAG(M)CUA(M)CUU( M)GUU(M)CUU(M)UUU(M)GCA(M)GCC(M)ACC(M)A(F)UGG(F)ACU(F)AUA(F)AG G(F)ACG(F)ACG(F)ACG(F)ACA(F)AAG(F)GUG(F)GCC(F)ACC(F)ACC(F)ACC(F)A CC(F)ACC(F)ACT(MOE)G(MOE)A(MOE) | 256 |
| E116 | Solid Phase Synthesis | G(MOE)G(MOE)G(MOE)A(MOE)G(MOE)A(MOE)AtACaAGcUAcUUgUUcUUtUUgC AgCCaCCA(F)UGG(F)ACU(F)AUA(F)AGG(F)ACG(F)ACG(F)ACG(F)ACA(F)AAG(F )GUG(F)GCC(F)ACC(F)ACC(F)ACC(F)ACC(F)ACC(F)ACT(MOE)G(MOE)A(MOE) | 257 |
| E117 | Solid Phase Synthesis | G(MOE)G(MOE)G(MOE)A(MOE)G(MOE)A(MOE)AUaCAaGCtACtUGtUCtUUtUGcA GcCAcCA(F)UGG(F)ACU(F)AUA(F)AGG(F)ACG(F)ACG(F)ACG(F)ACA(F)AAG(F) GUG(F)GCC(F)ACC(F)ACC(F)ACC(F)ACC(F)ACC(F)ACT(MOE)G(MOE)A(MOE) | 258 |
| E118 | Solid Phase Synthesis | G(MOE)G(MOE)G(MOE)A(MOE)G(MOE)A(MOE)aUAcAAgCUaCUtGUtCUtUUtGCa GCcACcA(F)UGG(F)ACU(F)AUA(F)AGG(F)ACG(F)ACG(F)ACG(F)ACA(F)AAG(F) GUG(F)GCC(F)ACC(F)ACC(F)ACC(F)ACC(F)ACC(F)ACT(MOE)G(MOE)A(MOE) | 259 |

**[Table 2-35]**

| | | | |
|---|---|---|---|
| E119 | Solid Phase Synthesis | G(MOE)G(MOE)G(MOE)A(MOE)G(MOE)A(MOE)AT(MOE)ACA(MOE)AGC(MOE)UA C(MOE)UUG(MOE)UUC(MOE)UUT(MOE)UUG(MOE)CAG(MOE)CCA(MOE)CCA(F) UGG(F)ACU(F)AUA(F)AGG(F)ACG(F)ACG(F)ACG(F)ACA(F)AAG(F)GUG(F)GCC( F)ACC(F)ACC(F)ACC(F)ACC(F)ACC(F)ACT(MOE)G(MOE)A(MOE) | 260 |
| E120 | Solid Phase Synthesis | G(MOE)G(MOE)G(MOE)A(MOE)G(MOE)A(MOE)AT(L)ACA(L)AGC(L)UAC(L)UUG( L)UUC(L)UUT(L)UUG(L)CAG(L)CCA(L)CCA(F)UGG(F)ACU(F)AUA(F)AGG(F)ACG (F)ACG(F)ACG(F)ACA(F)AAG(F)GUG(F)GCC(F)ACC(F)ACC(F)ACC(F)ACC(F)AC C(F)ACT(MOE)G(MOE)A(MOE) | 261 |
| E121 | Solid Phase Synthesis | G(MOE)G(MOE)G(MOE)A(MOE)G(MOE)A(MOE)A(rSpacer)AC(rSpacer)AG(rSpac er)UA(rSpacer)UU(rSpacer)UU(rSpacer)UU(rSpacer)UUG(F)CAG(F)CCA(F)CCA( F)UGG(F)ACU(F)AUA(F)AGG(F)ACG(F)ACG(F)ACG(F)ACA(F)AAG(F)GUG(F)GC C(F)ACC(F)ACC(F)ACC(F)ACC(F)ACC(F)ACT(MOE)G(MOE)A(MOE) | 262 |
| E122 | Solid Phase Synthesis | G(MOE)G(MOE)G(MOE)A(MOE)G(MOE)A(MOE)AU(F)ACA(F)AGC(F)UAC(F)UUG( F)UUC(F)UUU(F)UUG(F)CA(rSpacer)CC(rSpacer)CCA(F)UGG(F)ACU(F)AUA(F)A GG(F)ACG(F)ACG(F)ACG(F)ACA(F)AAG(F)GUG(F)GCC(F)ACC(F)ACC(F)ACC(F) ACC(F)ACC(F)ACT(MOE)G(MOE)A(MOE) | 263 |
| E123 | Solid Phase Synthesis | G(MOE)G(MOE)G(MOE)A(MOE)G(MOE)A(MOE)A(dSpacer)AC(dSpacer)AG(dSpac er)UA(dSpacer)UU(dSpacer)UU(dSpacer)UU(dSpacer)UUG(F)CAG(F)CCA(F)CCA (F)UGG(F)ACU(F)AUA(F)AGG(F)ACG(F)ACG(F)ACG(F)ACA(F)AAG(F)GUG(F)GC C(F)ACC(F)ACC(F)ACC(F)ACC(F)ACC(F)ACT(MOE)G(MOE)A(MOE) | 264 |
| E124 | Solid Phase Synthesis | G(MOE)G(MOE)G(MOE)A(MOE)G(MOE)A(MOE)A(C3)AC(C3)AG(C3)UA(C3)UU(C 3)UU(C3)UU(C3)UUG(F)CAG(F)CCA(F)CCA(F)UGG(F)ACU(F)AUA(F)AGG(F)ACG (F)ACG(F)ACG(F)ACA(F)AAG(F)GUG(F)GCC(F)ACC(F)ACC(F)ACC(F)ACC(F)AC C(F)ACT(MOE)G(MOE)A(MOE) | 265 |
| E125 | Solid Phase Synthesis | G(MOE)G(MOE)G(MOE)A(MOE)G(MOE)A(MOE)AU(F)A(F)CA(F)A(F)GC(F)U(F)AC (F)U(F)UG(F)U(F)UC(F)U(F)UU(F)U(F)UG(F)C(F)AG(F)C(F)CA(F)C(F)CA(F)UGG( F)ACU(F)AUA(F)AGG(F)ACG(F)ACG(F)ACG(F)ACA(F)AAG(F)GUG(F)GCC(F)AC C(F)ACC(F)ACC(F)ACC(F)ACC(F)ACT(MOE)G(MOE)A(MOE) | 266 |
| E126 | Solid Phase Synthesis | G(MOE)G(MOE)G(MOE)A(MOE)G(MOE)A(MOE)AU(F)A(M)CA(F)A(M)GC(F)U(M)A C(F)U(M)UG(F)U(M)UC(F)U(M)UU(F)U(M)UG(F)C(M)AG(F)C(M)CA(F)C(M)CA(F)U GG(F)ACU(F)AUA(F)AGG(F)ACG(F)ACG(F)ACG(F)ACA(F)AAG(F)GUG(F)GCC(F) ACC(F)ACC(F)ACC(F)ACC(F)ACC(F)ACT(MOE)G(MOE)A(MOE) | 267 |

**[Table 2-36]**

| | | | |
|---|---|---|---|
| E127 | Solid Phase Synthesis | G(MOE)G(MOE)G(MOE)A(MOE)G(MOE)A(MOE)A(F)U(F)AC(F)A(F)AG(F)C(F)UA( F)C(F)UU(F)G(F)UU(F)C(F)UU(F)U(F)UU(F)G(F)CA(F)G(F)CC(F)A(F)CC(F)A(F)U GG(F)ACU(F)AUA(F)AGG(F)ACG(F)ACG(F)ACG(F)ACA(F)AAG(F)GUG(F)GCC(F) ACC(F)ACC(F)ACC(F)ACC(F)ACC(F)ACT(MOE)G(MOE)A(MOE) | 268 |
| E128 | Solid Phase Synthesis | G(MOE)G(MOE)G(MOE)A(MOE)G(MOE)A(MOE)A(M)U(F)AC(M)A(F)AG(M)C(F)UA (M)C(F)UU(M)G(F)UU(M)C(F)UU(M)U(F)UU(M)G(F)CA(M)G(F)CC(M)A(F)CC(M)A( F)UGG(F)ACU(F)AUA(F)AGG(F)ACG(F)ACG(F)ACG(F)ACA(F)AAG(F)GUG(F)GC C(F)ACC(F)ACC(F)ACC(F)ACC(F)ACC(F)ACT(MOE)G(MOE)A(MOE) | 269 |
| E129 | Solid Phase Synthesis | G(MOE)G(MOE)G(MOE)A(MOE)G(MOE)A(MOE)A(F)U(F)A(M)C(F)A(F)A(M)G(F)C( F)U(M)A(F)C(F)U(M)U(F)G(F)U(M)U(F)C(F)U(M)U(F)U(F)U(M)U(F)G(F)C(M)A(F)G (F)C(M)C(F)A(F)C(M)C(F)A(F)UGG(F)ACU(F)AUA(F)AGG(F)ACG(F)ACG(F)ACG( F)ACA(F)AAG(F)GUG(F)GCC(F)ACC(F)ACC(F)ACC(F)ACC(F)ACC(F)ACT(MOE) G(MOE)A(MOE) | 270 |
| E130 | Solid Phase Synthesis | G(MOE)G(MOE)G(MOE)A(MOE)G(MOE)A(MOE)A(M)U(F)A(M)C(M)A(F)A(M)G(M) C(F)U(M)A(M)C(F)U(M)U(M)G(F)U(M)U(M)C(F)U(M)U(M)U(F)U(M)U(M)G(F)C(M)A (M)G(F)C(M)C(M)A(F)C(M)C(M)A(F)UGG(F)ACU(F)AUA(F)AGG(F)ACG(F)ACG(F) ACG(F)ACA(F)AAG(F)GUG(F)GCC(F)ACC(F)ACC(F)ACC(F)ACC(F)ACC(F)ACT( MOE)G(MOE)A(MOE) | 271 |
| E131 | Solid Phase Synthesis | G(MOE)G(MOE)G(MOE)A(MOE)G(MOE)A(MOE)A(M)U(F)A(F)C(M)A(F)A(F)G(M)C (F)U(F)A(M)C(F)U(F)U(M)G(F)U(F)U(M)C(F)U(F)U(M)U(F)U(F)U(M)G(F)C(F)A(M) G(F)C(F)C(M)A(F)C(F)C(M)A(F)UGG(F)ACU(F)AUA(F)AGG(F)ACG(F)ACG(F)ACG (F)ACA(F)AAG(F)GUG(F)GCC(F)ACC(F)ACC(F)ACC(F)ACC(F)ACC(F)ACT(MOE) G(MOE)A(MOE) | 272 |
| E132 | Solid Phase Synthesis | G(MOE)G(MOE)G(MOE)A(MOE)G(MOE)A(MOE)A(F)U(F)A(F)C(F)A(F)A(F)G(F)C( F)U(F)A(F)C(F)U(F)U(F)G(F)U(F)U(F)C(F)U(F)U(F)U(F)U(F)U(F)G(F)C(F)A(F)G(F) C(F)C(F)A(F)C(F)C(F)A(F)UGG(F)ACU(F)AUA(F)AGG(F)ACG(F)ACG(F)ACG(F)A CA(F)AAG(F)GUG(F)GCC(F)ACC(F)ACC(F)ACC(F)ACC(F)ACC(F)ACT(MOE)G(M OE)A(MOE) | 273 |
| E133 | Solid Phase Synthesis | G(MOE)G(MOE)G(MOE)A(MOE)G(MOE)A(MOE)AU(M)A(F)CA(M)A(F)GC(M)U(F)A C(M)U(F)UG(M)U(F)UC(M)U(F)UU(M)U(F)UG(M)C(F)AG(M)C(F)CA(M)C(F)CA(F)U GG(F)ACU(F)AUA(F)AGG(F)ACG(F)ACG(F)ACG(F)ACA(F)AAG(F)GUG(F)GCC(F) ACC(F)ACC(F)ACC(F)ACC(F)ACC(F)ACT(MOE)G(MOE)A(MOE) | 274 |

**[Table 2-37]**

| | | | |
|---|---|---|---|
| E134 | Solid Phase Synthesis | G(MOE)G(MOE)G(MOE)A(MOE)G(MOE)A(MOE)AU(M)A(M)CA(M)A(M)GC(M)U(M) AC(M)U(M)UG(M)U(M)UC(M)U(M)UU(M)U(M)UG(M)C(M)AG(M)C(M)CA(M)C(M)CA (F)UGG(F)ACU(F)AUA(F)AGG(F)ACG(F)ACG(F)ACG(F)ACA(F)AAG(F)GUG(F)GC C(F)ACC(F)ACC(F)ACC(F)ACC(F)ACC(F)ACT(MOE)G(MOE)A(MOE) | 275 |
| E135 | Solid Phase Synthesis | G(MOE)G(MOE)G(MOE)A(MOE)G(MOE)A(MOE)A(F)U(M)AC(F)A(M)AG(F)C(M)UA (F)C(M)UU(F)G(M)UU(F)C(M)UU(F)U(M)UU(F)G(M)CA(F)G(M)CC(F)A(M)CC(F)A( F)UGG(F)ACU(F)AUA(F)AGG(F)ACG(F)ACG(F)ACG(F)ACA(F)AAG(F)GUG(F)GC C(F)ACC(F)ACC(F)ACC(F)ACC(F)ACC(F)ACT(MOE)G(MOE)A(MOE) | 276 |
| E136 | Solid Phase Synthesis | G(MOE)G(MOE)G(MOE)A(MOE)G(MOE)A(MOE)A(M)U(M)AC(M)A(M)AG(M)C(M)U A(M)C(M)UU(M)G(M)UU(M)C(M)UU(M)U(M)UU(M)G(M)CA(M)G(M)CC(M)A(M)CC( M)A(F)UGG(F)ACU(F)AUA(F)AGG(F)ACG(F)ACG(F)ACG(F)ACA(F)AAG(F)GUG(F )GCC(F)ACC(F)ACC(F)ACC(F)ACC(F)ACC(F)ACT(MOE)G(MOE)A(MOE) | 277 |
| E137 | Solid Phase Synthesis | G(MOE)G(MOE)G(MOE)A(MOE)G(MOE)A(MOE)A(F)U(M)A(M)C(F)A(M)A(M)G(F)C (M)U(M)A(F)C(M)U(M)U(F)G(M)U(M)U(F)C(M)U(M)U(F)U(M)U(M)U(F)G(M)C(M)A( F)G(M)C(M)C(F)A(M)C(M)C(F)A(F)UGG(F)ACU(F)AUA(F)AGG(F)ACG(F)ACG(F)A CG(F)ACA(F)AAG(F)GUG(F)GCC(F)ACC(F)ACC(F)ACC(F)ACC(F)ACC(F)ACT(M OE)G(MOE)A(MOE) | 278 |
| E138 | Solid Phase Synthesis | G(MOE)G(MOE)G(MOE)A(MOE)G(MOE)A(MOE)A(M)U(M)A(M)C(M)A(M)A(M)G(M) C(M)U(M)A(M)C(M)U(M)U(M)G(M)U(M)U(M)C(M)U(M)U(M)U(M)U(M)G(M)C(M )A(M)G(M)C(M)C(M)A(M)C(M)C(M)A(F)UGG(F)ACU(F)AUA(F)AGG(F)ACG(F)ACG (F)ACG(F)ACA(F)AAG(F)GUG(F)GCC(F)ACC(F)ACC(F)ACC(F)ACC(F)ACC(F)AC T(MOE)G(MOE)A(MOE) | 279 |
| E139 | Solid Phase Synthesis | G(MOE)G(MOE)G(MOE)A(MOE)G(MOE)A(MOE)A(M)U(M)A(F)C(M)A(M)A(F)G(M) C(M)U(F)A(M)C(M)U(F)U(M)G(M)U(F)U(M)C(M)U(F)U(M)U(M)U(F)U(M)G(M)C(F)A (M)G(M)C(F)C(M)A(M)C(F)C(M)A(F)UGG(F)ACU(F)AUA(F)AGG(F)ACG(F)ACG(F) ACG(F)ACA(F)AAG(F)GUG(F)GCC(F)ACC(F)ACC(F)ACC(F)ACC(F)ACC(F)ACT( MOE)G(MOE)A(MOE) | 280 |
| E140 | Solid Phase Synthesis | G(MOE)G(MOE)G(MOE)A(MOE)G(MOE)A(MOE)A(F)U(M)A(F)C(F)A(M)A(F)G(F)C( M)U(F)A(F)C(M)U(F)U(F)G(M)U(F)U(F)C(M)U(F)U(F)U(M)U(F)U(F)G(M)C(F)A(F)G (M)C(F)C(F)A(M)C(F)C(F)A(F)UGG(F)ACU(F)AUA(F)AGG(F)ACG(F)ACG(F)ACG( F)ACA(F)AAG(F)GUG(F)GCC(F)ACC(F)ACC(F)ACC(F)ACC(F)ACC(F)ACT(MOE) G(MOE)A(MOE) | 281 |

**[Table 2-38]**

| | | | |
|---|---|---|---|
| E141 | Solid Phase Synthesis | G(MOE)G(MOE)G(MOE)A(MOE)G(MOE)A(MOE)A(M)(rSpacer)A(M)C(M)(rSpacer) A(M)G(M)(rSpacer)U(M)A(M)(rSpacer)U(M)U(M)(rSpacer)U(M)U(M)(rSpacer)U(M) U(M)(rSpacer)U(M)U(M)G(F)C(M)A(M)G(F)C(M)C(M)A(F)C(M)C(M)A(F)UGG(F)AC U(F)AUA(F)AGG(F)ACG(F)ACG(F)ACG(F)ACA(F)AAG(F)GUG(F)GCC(F)ACC(F)A CC(F)ACC(F)ACC(F)ACC(F)ACT(MOE)G(MOE)A(MOE) | 282 |
| E142 | Solid Phase Synthesis | G(MOE)G(MOE)G(MOE)A(MOE)G(MOE)A(MOE)A(M)(rSpacer)A(F)C(M)(rSpacer) A(F)G(M)(rSpacer)U(F)A(M)(rSpacer)U(F)U(M)(rSpacer)U(F)U(M)(rSpacer)U(F)U( M)(rSpacer)U(F)U(M)G(F)C(F)A(M)G(F)C(F)C(M)A(F)C(F)C(M)A(F)UGG(F)ACU(F )AUA(F)AGG(F)ACG(F)ACG(F)ACG(F)ACA(F)AAG(F)GUG(F)GCC(F)ACC(F)ACC( F)ACC(F)ACC(F)ACC(F)ACT(MOE)G(MOE)A(MOE) | 283 |
| E143 | Solid Phase Synthesis | G(MOE)G(MOE)G(MOE)A(MOE)G(MOE)A(MOE)A(M)(dSpacer)A(F)C(M)(dSpacer) A(F)G(M)(dSpacer)U(F)A(M)(dSpacer)U(F)U(M)(dSpacer)U(F)U(M)(dSpacer)U(F) U(M)(dSpacer)U(F)U(M)G(F)C(F)A(M)G(F)C(F)C(M)A(F)C(F)C(M)A(F)UGG(F)ACU (F)AUA(F)AGG(F)ACG(F)ACG(F)ACG(F)ACA(F)AAG(F)GUG(F)GCC(F)ACC(F)AC C(F)ACC(F)ACC(F)ACC(F)ACT(MOE)G(MOE)A(MOE) | 284 |
| E144 | Solid Phase Synthesis | G(MOE)G(MOE)G(MOE)A(MOE)G(MOE)A(MOE)A(M)(C3)A(F)C(M)(C3)A(F)G(M)(C 3)U(F)A(M)(C3)U(F)U(M)(C3)U(F)U(M)(C3)U(F)U(M)(C3)U(F)U(M)G(F)C(F)A(M)G( F)C(F)C(M)A(F)C(F)C(M)A(F)UGG(F)ACU(F)AUA(F)AGG(F)ACG(F)ACG(F)ACG(F )ACA(F)AAG(F)GUG(F)GCC(F)ACC(F)ACC(F)ACC(F)ACC(F)ACC(F)ACT(MOE)G( MOE)A(MOE) | 285 |
| E145 | Solid Phase Synthesis | G(MOE)G(MOE)G(MOE)A(MOE)G(MOE)A(MOE)A(M)U(F)A(M)C(M)A(F)A(M)G(M) C(F)U(M)A(M)C(F)U(M)U(M)G(F)U(M)U(M)C(F)U(M)U(M)U(F)U(M)U(M)G(F)C(M)A (M)G(F)CCA(F)CCA(F)UGG(F)ACU(F)AUA(F)AGG(F)ACG(F)ACG(F)ACG(F)ACA( F)AAG(F)GUG(F)GCC(F)ACC(F)ACC(F)ACC(F)ACC(F)ACC(F)ACT(MOE)G(MOE) A(MOE) | 286 |
| E146 | Solid Phase Synthesis | G(MOE)G(MOE)G(MOE)A(MOE)G(MOE)A(MOE)A(M)U(F)A(F)C(M)A(F)A(F)G(M)C (F)U(F)A(M)C(F)U(F)U(M)G(F)U(F)U(M)C(F)U(F)U(M)U(F)U(F)U(M)G(F)C(F)A(M) G(F)CCA(F)CCA(F)UGG(F)ACU(F)AUA(F)AGG(F)ACG(F)ACG(F)ACG(F)ACA(F)A AG(F)GUG(F)GCC(F)ACC(F)ACC(F)ACC(F)ACC(F)ACC(F)ACT(MOE)G(MOE)A(M OE) | 287 |
| E147 | Solid Phase Synthesis | G(MOE)G(MOE)G(MOE)A(MOE)G(MOE)A(MOE)AU(F)ACA(F)AGC(F)UAC(F)UUG( F)UUC(F)UUU(F)UUG(F)CAG(F)C(M)C(M)A(F)C(M)C(M)A(F)UGG(F)ACU(F)AUA( F)AGG(F)ACG(F)ACG(F)ACG(F)ACA(F)AAG(F)GUG(F)GCC(F)ACC(F)ACC(F)AC C(F)ACC(F)ACC(F)ACT(MOE)G(MOE)A(MOE) | 288 |

**[Table 2-39]**

| | | | |
|---|---|---|---|
| E148 | Solid Phase Synthesis | G(MOE)G(MOE)G(MOE)A(MOE)G(MOE)A(MOE)AU(F)ACA(F)AGC(F)UAC(F)UUG( F)UUC(F)UUU(F)UUG(F)CAG(F)C(F)C(M)A(F)C(F)C(M)A(F)UGG(F)ACU(F)AUA(F )AGG(F)ACG(F)ACG(F)ACG(F)ACA(F)AAG(F)GUG(F)GCC(F)ACC(F)ACC(F)ACC( F)ACC(F)ACC(F)ACT(MOE)G(MOE)A(MOE) | 289 |
| E149 | Solid Phase Synthesis | G(MOE)G(MOE)G(MOE)A(MOE)G(MOE)A(MOE)A(M)U(F)A(M)C(M)A(F)A(M)G(M) C(F)U(M)A(M)C(F)U(M)U(M)G(F)U(M)U(M)C(F)U(M)U(M)U(F)U(M)U(M)G(F)C(M)A (M)GC(F)CAC(F)CA(F)UGG(F)ACU(F)AUA(F)AGG(F)ACG(F)ACG(F)ACG(F)ACA( F)AAG(F)GUG(F)GCC(F)ACC(F)ACC(F)ACC(F)ACC(F)ACC(F)ACT(MOE)G(MOE) A(MOE) | 290 |
| E150 | Solid Phase Synthesis | G(MOE)G(MOE)G(MOE)A(MOE)G(MOE)A(MOE)A(M)U(F)A(F)C(M)A(F)A(F)G(M)C (F)U(F)A(M)C(F)U(F)U(M)G(F)U(F)U(M)C(F)U(F)U(M)U(F)U(F)U(M)G(F)C(F)A(M) GC(F)CAC(F)CA(F)UGG(F)ACU(F)AUA(F)AGG(F)ACG(F)ACG(F)ACG(F)ACA(F)A | 291 |
| | | AG(F)GUG(F)GCC(F)ACC(F)ACC(F)ACC(F)ACC(F)ACC(F)ACC(F)ACT(MOE)G(MOE)A(M OE) | |
| E151 | Solid Phase Synthesis | G(MOE)G(MOE)G(MOE)A(MOE)G(MOE)A(MOE)A(M)U(F)A(M)C(M)A(F)A(M)G(M) C(F)U(M)A(M)C(F)U(M)U(M)G(F)U(M)U(M)C(F)U(M)U(M)U(F)U(M)U(M)G(F)C(M)A (M)GCC(F)ACC(F)A(F)UGG(F)ACU(F)AUA(F)AGG(F)ACG(F)ACG(F)ACG(F)ACA( F)AAG(F)GUG(F)GCC(F)ACC(F)ACC(F)ACC(F)ACC(F)ACC(F)ACT(MOE)G(MOE) A(MOE) | 292 |
| E152 | Solid Phase Synthesis | G(MOE)G(MOE)G(MOE)A(MOE)G(MOE)A(MOE)A(M)U(F)A(F)C(M)A(F)A(F)G(M)C (F)U(F)A(M)C(F)U(F)U(M)G(F)U(F)U(M)C(F)U(F)U(M)U(F)U(F)U(M)G(F)C(F)A(M) GCC(F)ACC(F)A(F)UGG(F)ACU(F)AUA(F)AGG(F)ACG(F)ACG(F)ACG(F)ACA(F)A AG(F)GUG(F)GCC(F)ACC(F)ACC(F)ACC(F)ACC(F)ACC(F)ACT(MOE)G(MOE)A(M OE) | 293 |
| E153 | Solid Phase Synthesis | G(MOE)G(MOE)G(MOE)A(MOE)G(MOE)A(MOE)atA(M)caA(M)gcU(M)acU(M)tgU(M )tcU(M)ttU(M)tgC(M)agC(M)caC(M)cA(F)UGG(F)ACU(F)AUA(F)AGG(F)ACG(F)A CG(F)ACG(F)ACA(F)AAG(F)GUG(F)GCC(F)ACC(F)ACC(F)ACC(F)ACC(F)ACC(F) ACT(MOE)G(MOE)A(MOE) | 294 |
| E154 | Solid Phase Synthesis | G(MOE)G(MOE)G(MOE)A(MOE)G(MOE)A(MOE)A(M)tA(M)C(M)aA(M)G(M)cU(M)A (M)cU(M)U(M)gU(M)U(M)cU(M)U(M)tU(M)U(M)gC(M)A(M)gC(M)C(M)aC(M)C(M)A( F)UGG(F)ACU(F)AUA(F)AGG(F)ACG(F)ACG(F)ACG(F)ACA(F)AAG(F)GUG(F)GC C(F)ACC(F)ACC(F)ACC(F)ACC(F)ACC(F)ACT(MOE)G(MOE)A(MOE) | 295 |
| E155 | Solid Phase Synthesis | G(MOE)G(MOE)G(MOE)A(MOE)G(MOE)A(MOE)A(M)taC(M)aaG(M)ctA(M)ctU(M)g tU(M)ctU(M)ttU(M)gcA(M)gcC(M)acC(M)A(F)UGG(F)ACU(F)AUA(F)AGG(F)ACG( F)ACG(F)ACG(F)ACA(F)AAG(F)GUG(F)GCC(F)ACC(F)ACC(F)ACC(F)ACC(F)AC C(F)ACT(MOE)G(MOE)A(MOE) | 296 |

**[Table 2-40]**

| | | | |
|---|---|---|---|
| E156 | Solid Phase Synthesis | G(MOE)G(MOE)G(MOE)A(MOE)G(MOE)A(MOE)atacaagctacttgttctttttgcagccacc A(F)UGG(F)ACU(F)AUA(F)AGG(F)ACG(F)ACG(F)ACG(F)ACA(F)AAG(F)GUG(F)G CC(F)ACC(F)ACC(F)ACC(F)ACC(F)ACC(F)ACT(MOE)G(MOE)A(MOE) | 297 |
| E157 | Solid Phase Synthesis | G(MOE)G(MOE)G(MOE)A(MOE)G(MOE)A(MOE)aU(M)A(M)cA(M)A(M)gC(M)U(M)a C(M)U(M)tG(M)U(M)tC(M)U(M)tU(M)U(M)tG(M)C(M)aG(M)C(M)cA(M)C(M)cA(F)U GG(F)ACU(F)AUA(F)AGG(F)ACG(F)ACG(F)ACG(F)ACA(F)AAG(F)GUG(F)GCC(F) ACC(F)ACC(F)ACC(F)ACC(F)ACC(F)ACT(MOE)G(MOE)A(MOE) | 298 |
| E158 | Solid Phase Synthesis | G(MOE)G(MOE)G(MOE)A(MOE)G(MOE)A(MOE)A(M)U(M)aC(M)A(M)aG(M)C(M)tA (M)C(M)tU(M)G(M)tU(M)C(M)tU(M)U(M)tU(M)G(M)cA(M)G(M)cC(M)A(M)cC(M)A( F)UGG(F)ACU(F)AUA(F)AGG(F)ACG(F)ACG(F)ACG(F)ACA(F)AAG(F)GUG(F)GC C(F)ACC(F)ACC(F)ACC(F)ACC(F)ACC(F)ACT(MOE)G(MOE)A(MOE) | 299 |
| E159 | Solid Phase Synthesis | G(MOE)G(MOE)G(MOE)A(MOE)G(MOE)A(MOE)aU(M)acA(M)agC(M)taC(M)ttG(M) ttC(M)ttU(M)ttG(M)caG(M)ccA(M)ccA(F)UGG(F)ACU(F)AUA(F)AGG(F)ACG(F)A CG(F)ACG(F)ACA(F)AAG(F)GUG(F)GCC(F)ACC(F)ACC(F)ACC(F)ACC(F)ACC(F) ACT(MOE)G(MOE)A(MOE) | 300 |
| E160 | Solid Phase Synthesis | G(MOE)G(MOE)G(MOE)A(MOE)G(MOE)A(MOE)A(M)tA(M)C(M)aA(M)G(M)cU(M)A (M)cU(M)U(M)gU(M)U(M)cU(M)U(M)tU(M)U(M)gC(M)A(M)G(F)C(M)C(M)A(F)C(M) C(M)A(F)UGG(F)ACU(F)AUA(F)AGG(F)ACG(F)ACG(F)ACG(F)ACA(F)AAG(F)GUG (F)GCC(F)ACC(F)ACC(F)ACC(F)ACC(F)ACC(F)ACT(MOE)G(MOE)A(MOE) | 301 |
| E161 | Solid Phase Synthesis | G(MOE)G(MOE)G(MOE)A(MOE)G(MOE)A(MOE)A(M)taC(M)aaG(M)ctA(M)ctU(M)g tU(M)ctU(M)ttU(M)gcA(M)G(F)C(F)C(M)A(F)C(F)C(M)A(F)UGG(F)ACU(F)AUA(F) AGG(F)ACG(F)ACG(F)ACG(F)ACA(F)AAG(F)GUG(F)GCC(F)ACC(F)ACC(F)ACC( F)ACC(F)ACC(F)ACT(MOE)G(MOE)A(MOE) | 302 |
| E162 | Solid Phase Synthesis | G(MOE)G(MOE)G(MOE)A(MOE)G(MOE)A(MOE)A(M)tA(M)C(M)aA(M)G(M)cU(M)A (M)cU(M)U(M)gU(M)U(M)cU(M)U(M)gU(M)U(M)gC(M)A(M)G(F)CCA(F)CCA(F)UGG (F)ACU(F)AUA(F)AGG(F)ACG(F)ACG(F)ACG(F)ACA(F)AAG(F)GUG(F)GCC(F)AC C(F)ACC(F)ACC(F)ACC(F)ACC(F)ACT(MOE)G(MOE)A(MOE) | 303 |
| E163 | Solid Phase Synthesis | G(MOE)G(MOE)G(MOE)A(MOE)G(MOE)A(MOE)A(M)taC(M)aaG(M)ctA(M)ctU(M)g tU(M)ctU(M)ttU(M)gcA(M)G(F)CCA(F)CCA(F)UGG(F)ACU(F)AUA(F)AGG(F)ACG( F)ACG(F)ACG(F)ACA(F)AAG(F)GUG(F)GCC(F)ACC(F)ACC(F)ACC(F)ACC(F)AC C(F)ACT(MOE)G(MOE)A(MOE) | 304 |

**[Table 2-41]**

| | | | |
|---|---|---|---|
| E164 | Same as Example 1 | G(MOE)G(MOE)G(MOE)A(MOE)G(MOE)A(MOE)A(M)U(M)A(M)C(M)A(M)A(M)G(M) C(M)U(M)A(M)C(M)U(F)U(rSpacer)G(F)U(rSpacer)U(F)C(rSpacer)U(F)U(rSpacer) U(M)U(M)U(M)G(M)C(M)A(M)GCCA(M)CCAUGG(F)ACU(F)ACA(F)AGG(F)ACG(F) ACG(F)ACG(F)ACA(F)AGA(F)UCA(F)UCG(F)ACU(F)AUA(F)AAGACGACG(F)ACG (F)AUA(F)AAG(F)GUG(F)GCG(F)ACU(F)AUA(F)AGG(F)ACG(F)ACG(F)ACG(F)AC | 305 |
| | | A(F)AAC(F)ACC(F)ACC(F)ACC(F)ACC(F)ACC(F)ACT(MOE)G(MOE)A(MOE)AAA AAAAAAAAAAAAAAAAAAAAAAAAAAAAAAAAAAAAA | |
| E164-1 | Solid Phase Synthesis | G(MOE)G(MOE)G(MOE)A(MOE)G(MOE)A(MOE)A(M)U(M)A(M)C(M)A(M)A(M)G(M) C(M)U(M)A(M)C(M)U(F)U(rSpacer)G(F)U(rSpacer)U(F)C(rSpacer)U(F)U(rSpacer) U(M)U(M)U(M)G(M)C(M)A(M)GCCA(M)CCAUGG(F)ACU(F)ACA(F)AGG(F)ACG(F) ACG(F)ACG(F)ACA(F)AGA(F)UCA(F)UCG(F)ACU(F)AUA(F)AAG | 306 |
| E164-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACT(MOE)G( MOE)A(MOE)AAAAAAAAAAAAAAAAAAAAAAAAAAAAAAAAAAAAAAAA | 307 |
| E165 | Same as Example 2 | G(MOE)^G(MOE)^G(MOE)^A(MOE)^G(MOE)^A(MOE)AUACAAGCUACUUGUUCUU UUUGCAGCCACCA(F)UGG(F)ACU(F)ACA(F)AGG(F)ACG(F)ACG(F)ACG(F)ACA( F)AGA(F)UCA(F)UCG(F)ACU(F)AUA(F)AAG(F)ACG(F)ACG(F)ACG(F)AUA(F)AAG (F)GUG(F)GCG(F)ACU(F)AUA(F)AGG(F)ACG(F)ACG(F)ACG(F)ACA(F)AAC(F)AC C(F)ACC(F)ACC(F)ACC(F)ACC(F)ACT(MOE)^G(MOE)^A(MOE)^A(F)^A(M)^A(F)^ A(M)^A(F)^A(M)^A(F)^A(M)^A(F)^A(M)^A(F)^A(M)^A(F)^A(M)^A(F)^A(M)^A(F)^A( MOE)^A(MOE)^A(MOE) | 308 |
| E165-1 | Solid Phase Synthesis | G(MOE)¨G(MOE)¨G(MOE)¨A(MOE)¨G(MOE)¨A(MOE)AUACAAGCUACUUGUUCUU UUUGCAGCCACCA(F)UGG(F)ACU(F)ACA(F)AGG(F)ACG(F)ACG(F)ACG(F)ACA( F)AGA(F)UCA(F)UCG(F)ACU(F)AUA(F)AAG(F)p | 309 |
| E165-2 | Solid Phase Synthesis | ACG(F)ACG(F)ACG(F)AUA(F)AAG(F)GUG(F)GCG(F)ACU(F)AUA(F)AGG(F)ACG( F)ACG(F)ACG(F)ACA(F)AAC(F)ACC(F)ACC(F)ACC(F)ACC(F)ACC(F)ACT(MOE)^ G(MOE)^A(MOE)^A(F)^A(M)^A(F)^A(M)^A(F)^A(M)^A(F)^A(M)^A(F)^A(M)^A(F)^A( M)^A(F)^A(M)^A(F)^A(M)^A(F)^A(MOE)^A(MOE)^A(MOE) | 310 |

**[Table 2-42]**

| | | | |
|---|---|---|---|
| E166 | Same as Example 2 | G(MOE)^G(MOE)^G(MOE)^A(MOE)^G(MOE)^A(MOE)AUACAAGCUACUUGUUCUU UUUGCAGCCACCA(F)^UGG(F)^ACU(F)^ACA(F)^AGG(F)^ACG(F)^ACG(F)^ACG( F)^ACA(F)^AGA(F)^UCA(F)^UCG(F)^ACU(F)^AUA(F)^AAG(F)ACG(F)^ACG(F)^A CG(F)^AUA(F)^AAG(F)^GUG(F)^GCG(F)^ACU(F)^AUA(F)^AGG(F)^ACG(F)^ACG( F)^ACG(F)^ACA(F)^AAC(F)^ACC(F)^ACC(F)^ACC(F)^ACC(F)^ACC(F)^ACT(MOE )^G(MOE)^A(MOE)^A(F)^A(M)^A(F)^A(M)^A(F)^A(M)^A(F)^A(M)^A(F)^A(M)^A(F)^ A(M)^A(F)^A(M)^A(F)^A(M)^A(F)^A(MOE)^A(MOE)^A(MOE) | 311 |
| E166-1 | Solid Phase Synthesis | G(MOE)^G(MOE)^G(MOE)^A(MOE)^G(MOE)^A(MOE)AUACAAGCUACUUGUUCUU UUUGCAGCCACCA(F)^UGG(F)^ACU(F)^ACA(F)^AGG(F)^ACG(F)^ACG(F)^ACG( F)^ACA(F)^AGA(F)^UCA(F)^UCG(F)^ACU(F)^AUA(F)^AAG(F)p- | 312 |
| E166-2 | Solid Phase Synthesis | ACG(F)^ACG(F)^ACG(F)^AUA(F)^AAG(F)^GUG(F)^GCG(F)^ACU(F)^AUA(F)^AGG (F)^ACG(F)^ACG(F)^ACG(F)^ACA(F)^AAC(F)^ACC(F)^ACC(F)^ACC(F)^ACC(F)^ ACC(F)^ACT(MOE)^G(MOE)^A(MOE)^A(F)^A(M)^A(F)^A(M)^A(F)^A(M)^A(F)^A(M )^A(F)^A(M)^A(F)^A(M)^A(F)^A(M)^A(F)^A(M)^A(F)^A(MOE)^A(MOE)^A(MOE) | 313 |
| E167 | Same as Example 2 | G(MOE)^G(MOE)^G(MOE)^A(MOE)^G(MOE)^A(MOE)A(F)U(M)A(F)C(M)A(F)A(M)G (F)C(M)U(F)A(M)C(F)U(M)U(F)G(M)U(F)U(M)C(F)U(M)U(F)U(M)U(F)U(M)G(F)C(M )A(F)G(M)C(F)C(M)A(F)C(M)C(F)A(F)UGG(F)ACU(F)ACA(F)AGG(F)ACG(F)ACG( F)ACG(F)ACA(F)AGA(F)UCA(F)UCG(F)ACU(F)AUA(F)AAG(F)ACG(F)ACG(F)AC G(F)AUA(F)AAG(F)GUG(F)GCG(F)ACU(F)AUA(F)AGG(F)ACG(F)ACG(F)ACG(F)A CA(F)AAC(F)ACC(F)ACC(F)ACC(F)ACC(F)ACC(F)ACT(MOE)^G(MOE)^A(MOE)^ A(F)^A(M)^A(F)^A(M)^A(F)^A(M)^A(F)^A(M)^A(F)^A(M)^A(F)^A(M)^A(F)^A(M)^A( F)^A(M)^A(F)^A(MOE)^A(MOE)^A(MOE) | 314 |
| E167-1 | Solid Phase Synthesis | G(MOE)^G(MOE)^G(MOE)^A(MOE)^G(MOE)^A(MOE)A(F)U(M)A(F)C(M)A(F)A(M)G (F)C(M)U(F)A(M)C(F)U(M)U(F)G(M)U(F)U(M)C(F)U(M)U(F)U(M)U(F)U(M)G(F)C(M )A(F)G(M)C(F)C(M)A(F)C(M)C(F)A(F)UGG(F)ACU(F)ACA(F)AGG(F)ACG(F)ACG( F)ACG(F)ACA(F)AGA(F)UCA(F)UCG(F)ACU(F)AUA(F)AAG(F)p | 315 |
| E167-2 | Solid Phase Synthesis | ACG(F)ACG(F)ACG(F)AUA(F)AAG(F)GUG(F)GCG(F)ACU(F)AUA(F)AGG(F)ACG( F)ACG(F)ACG(F)ACA(F)AAC(F)ACC(F)ACC(F)ACC(F)ACC(F)ACC(F)ACT(MOE)^ G(MOE)^A(MOE)^A(F)^A(M)^A(F)^A(M)^A(F)^A(M)^A(F)^A(M)^A(F)^A(M)^A(F)^A( M)^A(F)^A(M)^A(F)^A(M)^A(F)^A(MOE)^A(MOE)^A(MOE) | 316 |

**[Table 2-43]**

| | | | |
|---|---|---|---|
| E168 | Same as Example 2 | G(MOE)^G(MOE)^G(MOE)^A(MOE)^G(MOE)^A(MOE)A(F)U(M)A(F)C(M)A(F)A(M)G (F)C(M)U(F)A(M)C(F)U(M)U(F)G(M)U(F)U(M)C(F)U(M)U(F)U(M)U(F)U(M)G(F)C(M )A(F)G(M)C(F)C(M)A(F)C(M)C(F)A(F)^UGG(F)^ACU(F)^ACA(F)^AGG(F)^ACG(F)^ ACG(F)^ACG(F)^ACA(F)^AGA(F)^UCA(F)^UCG(F)^ACU(F)^AUA(F)^AAG(F)ACG( F)^ACG(F)^ACG(F)^AUA(F)^AAG(F)^GUG(F)^GCG(F)^ACU(F)^AUA(F)^AGG(F)^A CG(F)^ACG(F)^ACG(F)^ACA(F)^AAC(F)^ACC(F)^ACC(F)^ACC(F)^ACC(F)^ACC( | 317 |
| | | F)^ACT(MOE)^G(MOE)^A(MOE)^A(F)^A(M)^A(F)^A(M)^A(F)^A(M)^A(F)^A(M)^A(F )^A(M)^A(F)^A(M)^A(F)^A(M)^A(F)^A(M)^A(F)^A(MOE)^A(MOE)^A(MOE) | |
| E168-1 | Solid Phase Synthesis | G(MOE)^G(MOE)^G(MOE)^A(MOE)^G(MOE)^A(MOE)A(F)U(M)A(F)C(M)A(F)A(M)G (F)C(M)U(F)A(M)C(F)U(M)U(F)G(M)U(F)U(M)C(F)U(M)U(F)U(M)U(F)U(M)G(F)C(M )A(F)G(M)C(F)C(M)A(F)C(M)C(F)A(F)^UGG(F)^ACU(F)^ACA(F)^AGG(F)^ACG(F)^ ACG(F)^ACG(F)^ACA(F)^AGA(F)^UCA(F)^UCG(F)^ACU(F)^AUA(F)^AAG(F)p- | 318 |
| E168-2 | Solid Phase Synthesis | ACG(F)^ACG(F)^ACG(F)^AUA(F)^AAG(F)^GUG(F)^GCG(F)^ACU(F)^AUA(F)^AGG (F)^ACG(F)^ACG(F)^ACG(F)^ACA(F)^AAC(F)^ACC(F)^ACC(F)^ACC(F)^ACC(F)^ ACC(F)^ACT(MOE)^G(MOE)^A(MOE)^A(F)^A(M)^A(F)^A(M)^A(F)^A(M)^A(F)^A(M )^A(F)^A(M)^A(F)^A(M)^A(F)^A(M)^A(F)^A(M)^A(F)^A(MOE)^A(MOE)^A(MOE) | 319 |
| E169 | Same as Example 2 | G(MOE)^G(MOE)^G(MOE)^A(MOE)^G(MOE)^A(MOE)A(M)U(M)A(M)C(M)A(M)A(M) G(M)C(M)U(M)A(M)C(M)U(F)(rSpacer)G(F)(rSpacer)U(F)(rSpacer)U(F)(rSpacer)U (M)U(M)U(M)G(M)C(M)A(M)GCCA(M)CCA(F)UGG(F)ACU(F)ACA(F)AGG(F)ACG(F )ACG(F)ACG(F)ACA(F)AGA(F)UCA(F)UCG(F)ACU(F)AUA(F)AAG(F)ACG(F)ACG( F)ACG(F)AUA(F)AAG(F)GUG(F)GCG(F)ACU(F)AUA(F)AGG(F)ACG(F)ACG(F)AC G(F)ACA(F)AAC(F)ACC(F)ACC(F)ACC(F)ACC(F)ACC(F)ACT(MOE)^G(MOE)^A( MOE)^A(F)^A(M)^A(F)^A(M)^A(F)^A(M)^A(F)^A(M)^A(F)^A(M)^A(F)^A(M)^A(F)^A( M)^A(F)^A(M)^A(F)^A(MOE)^A(MOE)^A(MOE) | 320 |
| E169-1 | Solid Phase Synthesis | G(MOE)^G(MOE)^G(MOE)^A(MOE)^G(MOE)^A(MOE)A(M)U(M)A(M)C(M)A(M)A(M) G(M)C(M)U(M)A(M)C(M)U(F)(rSpacer)G(F)(rSpacer)U(F)(rSpacer)U(F)(rSpacer)U (M)U(M)U(M)G(M)C(M)A(M)GCCA(M)CCA(F)UGG(F)ACU(F)ACA(F)AGG(F)ACG(F )ACG(F)ACG(F)ACA(F)AGA(F)UCA(F)UCG(F)ACU(F)AUA(F)AAG(F)p | 321 |

**[Table 2-44]**

| | | | |
|---|---|---|---|
| E169-2 | Solid Phase Synthesis | ACG(F)ACG(F)ACG(F)AUA(F)AAG(F)GUG(F)GCG(F)ACU(F)AUA(F)AGG(F)ACG( F)ACG(F)ACG(F)ACA(F)AAC(F)ACC(F)ACC(F)ACC(F)ACC(F)ACC(F)ACT(MOE)^ G(MOE)^A(MOE)^A(F)^A(M)^A(F)^A(M)^A(F)^A(M)^A(F)^A(M)^A(F)^A(M)^A(F)^A( M)^A(F)^A(M)^A(F)^A(M)^A(F)^A(MOE)^A(MOE)^A(MOE) | 322 |
| E170 | Same as Example 2 | G(MOE)^G(MOE)^G(MOE)^A(MOE)^G(MOE)^A(MOE)A(M)U(M)A(M)C(M)A(M)A(M) G(M)C(M)U(M)A(M)C(M)U(F)(rSpacer)G(F)(rSpacer)U(F)(rSpacer)U(F)(rSpacer)U (M)U(M)U(M)G(M)C(M)A(M)GCCA(M)CCA(F)^UGG(F)^ACU(F)^ACA(F)^AGG(F)^A CG(F)^ACG(F)^ACG(F)^ACA(F)^AGA(F)^UCA(F)^UCG(F)^ACU(F)^AUA(F)^AAG( F)ACG(F)^ACG(F)^ACG(F)^AUA(F)^AAG(F)^GUG(F)^GCG(F)^ACU(F)^AUA(F)^A GG(F)^ACG(F)^ACG(F)^ACG(F)^ACA(F)^AAC(F)^ACC(F)^ACC(F)^ACC(F)^ACC( F)^ACC(F)^ACT(MOE)^G(MOE)^A(MOE)^A(F)^A(M)^A(F)^A(M)^A(F)^A(M)^A(F)^ A(M)^A(F)^A(M)^A(F)^A(M)^A(F)^A(M)^A(F)^A(M)^A(F)^A(MOE)^A(MOE)^A(MOE) | 323 |
| E170-1 | Solid Phase Synthesis | G(MOE)^G(MOE)^G(MOE)^A(MOE)^G(MOE)^A(MOE)A(M)U(M)A(M)C(M)A(M)A(M) G(M)C(M)U(M)A(M)C(M)U(F)(rSpacer)G(F)(rSpacer)U(F)(rSpacer)U(F)(rSpacer)U (M)U(M)U(M)G(M)C(M)A(M)GCCA(M)CCA(F)^UGG(F)^ACU(F)^ACA(F)^AGG(F)^A CG(F)^ACG(F)^ACG(F)^ACA(F)^AGA(F)^UCA(F)^UCG(F)^ACU(F)^AUA(F)^AAG( F)p | 324 |
| E170-2 | Solid Phase Synthesis | ACG(F)^ACG(F)^ACG(F)^AUA(F)^AAG(F)^GUG(F)^GCG(F)^ACU(F)^AUA(F)^AGG (F)^ACG(F)^ACG(F)^ACG(F)^ACA(F)^AAC(F)^ACC(F)^ACC(F)^ACC(F)^ACC(F)^ ACC(F)^ACT(MOE)^G(MOE)^A(MOE)^A(F)^A(M)^A(F)^A(M)^A(F)^A(M)^A(F)^A(M )^A(F)^A(M)^A(F)^A(M)^A(F)^A(M)^A(F)^A(M)^A(F)^A(MOE)^A(MOE)^A(MOE) | 325 |
| E171 | Same as Example 2 | G(MOE)^G(MOE)^G(MOE)^A(MOE)^G(MOE)^A(MOE)A(F)U(M)A(F)C(M)A(F)A(M)G (F)C(M)U(F)A(M)C(F)U(M)U(F)G(M)U(F)U(M)C(F)U(M)U(F)U(M)U(F)U(M)G(F)C(M )A(F)G(M)C(F)C(M)A(F)C(M)C(F)A(F)^UGG(F)^ACU(F)^ACA(F)^AGG(F)^ACG(F)^ ACG(F)^ACG(F)^ACA(F)^AGA(F)^UCA(F)^UCG(F)^ACU(F)^AUA(F)^AAG(F)ACG( F)^ACG(F)^ACG(F)^AUA(F)^AAG(F)^GUG(F)^GCG(F)^ACU(F)^AUA(F)^AGG(F)^A CG(F)^ACG(F)^ACG(F)^ACA(F)^AAC(F)^ACC(F)^ACC(F)^ACC(F)^ACC(F)^ACC( F)^ACT(MOE)^G(MOE)^A(MOE)^A(M)^A(M)^A(M)^A(M)^A(M)^A(M)^A(M)^A(M)^A( M)^A(M)^A(M)^A(M)^A(M)^A(M)^A(M)^A(M)^A(M)^A(M)^A(MOE)^A(MOE)^A(MOE) | 326 |

**[Table 2-45]**

| | | | |
|---|---|---|---|
| E171-1 | Solid Phase Synthesis | G(MOE)^G(MOE)^G(MOE)^A(MOE)^G(MOE)^A(MOE)A(F)U(M)A(F)C(M)A(F)A(M)G (F)C(M)U(F)A(M)C(F)U(M)U(F)G(M)U(F)U(M)C(F)U(M)U(F)U(M)U(F)U(M)G(F)C(M )A(F)G(M)C(F)C(M)A(F)C(M)C(F)A(F)^UGG(F)^ACU(F)^ACA(F)^AGG(F)^ACG(F)^ ACG(F)^ACG(F)^ACA(F)^AGA(F)^UCA(F)^UCG(F)^ACU(F)^AUA(F)^AAG(F)p- | 327 |
| E171-2 | Solid Phase Synthesis | ACG(F)^ACG(F)^ACG(F)^AUA(F)^AAG(F)^GUG(F)^GCG(F)^ACU(F)^AUA(F)^AGG (F)^ACG(F)^ACG(F)^ACG(F)^ACA(F)^AAC(F)^ACC(F)^ACC(F)^ACC(F)^ACC(F)^ ACC(F)^ACT(MOE)^G(MOE)^A(MOE)^A(M)^A(M)^A(M)^A(M)^A(M)^A(M)^A(M)^A( | 328 |
| | | M)^A(M)^A(M)^A(M)^A(M)^A(M)^A(M)^A(M)^A(M)^A(M)^A(M)^A(MOE)^A(MOE)^A(MOE) | |
| E172 | Same as Example 2 | G(MOE)G(MOE)G(MOE)A(MOE)G(MOE)A(MOE)AUACAAGCUACUUGUUCUUUUU GCAGCCACCA(F)UGG(F)ACU(F)ACA(F)AGG(F)ACG(F)ACG(F)ACG(F)ACA(F)A GA(F)UCA(F)UCG(F)ACU(F)AUA(F)AAG(F)ACG(F)ACG(F)ACG(F)AUA(F)AAG(F) GUG(F)GCG(F)ACU(F)AUA(F)AGG(F)ACG(F)ACG(F)ACG(F)ACA(F)AAC(F)ACC( F)ACC(F)ACC(F)ACC(F)ACC(F)ACT(MOE)^G(MOE)^A(MOE)^A(F)^A(M)^A(F)^A( M)^A(F)^A(M)^A(F)^A(M)^A(F)^A(M)^A(F)^A(M)^A(F)^A(M)^A(F)^A(M)^A(F)^A(M OE)^A(MOE)^A(MOE) | 329 |
| E172-1 | Solid Phase Synthesis | G(MOE)G(MOE)G(MOE)A(MOE)G(MOE)A(MOE)AUACAAGCUACUUGUUCUUUUU GCAGCCACCA(F)UGG(F)ACU(F)ACA(F)AGG(F)ACG(F)ACG(F)ACG(F)ACA(F)A GA(F)UCA(F)UCG(F)ACU(F)AUA(F)AAG(F)p | 330 |
| E172-2 | Solid Phase Synthesis | ACG(F)ACG(F)ACG(F)AUA(F)AAG(F)GUG(F)GCG(F)ACU(F)AUA(F)AGG(F)ACG( F)ACG(F)ACG(F)ACA(F)AAC(F)ACC(F)ACC(F)ACC(F)ACC(F)ACC(F)ACT(MOE)^ G(MOE)^A(MOE)^A(F)^A(M)^A(F)^A(M)^A(F)^A(M)^A(F)^A(M)^A(F)^A(M)^A(F)^A( M)^A(F)^A(M)^A(F)^A(M)^A(F)^A(MOE)^A(MOE)^A(MOE) | 331 |
| E173 | Same as Example 2 | G(MOE)G(MOE)G(MOE)A(MOE)G(MOE)A(MOE)A(F)U(M)A(F)C(M)A(F)A(M)G(F)C (M)U(F)A(M)C(F)U(M)U(F)G(M)U(F)U(M)C(F)U(M)U(F)U(M)U(F)U(M)G(F)C(M)A(F) G(M)C(F)C(M)A(F)C(M)C(F)A(F)UGG(F)ACU(F)ACA(F)AGG(F)ACG(F)ACG(F)AC G(F)ACA(F)AGA(F)UCA(F)UCG(F)ACU(F)AUA(F)AAG(F)ACG(F)ACG(F)ACG(F)A UA(F)AAG(F)GUG(F)GCG(F)ACU(F)AUA(F)AGG(F)ACG(F)ACG(F)ACG(F)ACA(F) AAC(F)ACC(F)ACC(F)ACC(F)ACC(F)ACC(F)ACT(MOE)^G(MOE)^A(MOE)^A(F)^A (M)^A(F)^A(M)^A(F)^A(M)^A(F)^A(M)^A(F)^A(M)^A(F)^A(M)^A(F)^A(M)^A(F)^A(M )^A(F)^A(MOE)^A(MOE)^A(MOE) | 332 |

**[Table 2-46]**

| | | | |
|---|---|---|---|
| E173-1 | Solid Phase Synthesis | G(MOE)G(MOE)G(MOE)A(MOE)G(MOE)A(MOE)A(F)U(M)A(F)C(M)A(F)A(M)G(F)C (M)U(F)A(M)C(F)U(M)U(F)G(M)U(F)U(M)C(F)U(M)U(F)U(M)U(F)U(M)G(F)C(M)A(F) G(M)C(F)C(M)A(F)C(M)C(F)A(F)UGG(F)ACU(F)ACA(F)AGG(F)ACG(F)ACG(F)AC G(F)ACA(F)AGA(F)UCA(F)UCG(F)ACU(F)AUA(F)AAG(F)p | 333 |
| E173-2 | Solid Phase Synthesis | ACG(F)ACG(F)ACG(F)AUA(F)AAG(F)GUG(F)GCG(F)ACU(F)AUA(F)AGG(F)ACG( F)ACG(F)ACG(F)ACA(F)AAC(F)ACC(F)ACC(F)ACC(F)ACC(F)ACC(F)ACT(MOE)^ G(MOE)^A(MOE)^A(F)^A(M)^A(F)^A(M)^A(F)^A(M)^A(F)^A(M)^A(F)^A(M)^A(F)^A( M)^A(F)^A(M)^A(F)^A(M)^A(F)^A(MOE)^A(MOE)^A(MOE) | 334 |
| E174 | Same as Example 2 | G(MOE)G(MOE)G(MOE)A(MOE)G(MOE)A(MOE)A(M)U(M)A(M)C(M)A(M)A(M)G(M) C(M)U(M)A(M)C(M)U(F)(rSpacer)G(F)(rSpacer)U(F)(rSpacer)U(F)(rSpacer)U(M)U (M)U(M)G(M)C(M)A(M)GCCA(M)CCA(F)UGG(F)ACU(F)ACA(F)AGG(F)ACG(F)AC G(F)ACG(F)ACA(F)AGA(F)UCA(F)UCG(F)ACU(F)AUA(F)AAG(F)ACG(F)ACG(F)A CG(F)AUA(F)AAG(F)GUG(F)GCG(F)ACU(F)AUA(F)AGG(F)ACG(F)ACG(F)ACG(F) ACA(F)AAC(F)ACC(F)ACC(F)ACC(F)ACC(F)ACC(F)ACT(MOE)^G(MOE)^A(MOE) ^A(F)^A(M)^A(F)^A(M)^A(F)^A(M)^A(F)^A(M)^A(F)^A(M)^A(F)^A(M)^A (F)^A(M)^A(F)^A(MOE)^A(MOE)^A(MOE) | 335 |
| E174-1 | Solid Phase Synthesis | G(MOE)G(MOE)G(MOE)A(MOE)G(MOE)A(MOE)A(M)U(M)A(M)C(M)A(M)A(M)G(M) C(M)U(M)A(M)C(M)U(F)(rSpacer)G(F)(rSpacer)U(F)(rSpacer)U(F)(rSpacer)U(M)U (M)U(M)G(M)C(M)A(M)GCCA(M)CCA(F)UGG(F)ACU(F)ACA(F)AGG(F)ACG(F)AC G(F)ACG(F)ACA(F)AGA(F)UCA(F)UCG(F)ACU(F)AUA(F)AAG(F)p | 336 |
| E174-2 | Solid Phase Synthesis | ACG(F)ACG(F)ACG(F)AUA(F)AAG(F)GUG(F)GCG(F)ACU(F)AUA(F)AGG(F)ACG( F)ACG(F)ACG(F)ACA(F)AAC(F)ACC(F)ACC(F)ACC(F)ACC(F)ACC(F)ACT(MOE)^ G(MOE)^A(MOE)^A(F)^A(M)^A(F)^A(M)^A(F)^A(M)^A(F)^A(M)^A(F)^A(M)^A(F)^A( M)^A(F)^A(M)^A(F)^A(M)^A(F)^A(MOE)^A(MOE)^A(MOE) | 337 |
| E175 | Purchased from GeneDesign | G(MOE)^G(MOE)^G(MOE)^A(MOE)^G(MOE)^A(MOE)AUACAAGCUACUUGUUCUU UUUGCAGCCACCAUGGACUACAAGGACGACGACGACAAGAUCAUCGACUAUA AAGACGACGACGAUAAAGGUGGCGACUAUAAGGACGACGACGACAAACACCA CCACCACCACCACUGAAAAAAAAAAAAAAAAAAAAA | 338 |
| E176 | Purchased from GeneDesign | G(MOE)^G(MOE)^G(MOE)^A(MOE)^G(MOE)^A(MOE)A(F)U(M)A(F)C(M)A(F)A(M)G (F)C(M)U(F)A(M)C(F)U(M)U(F)G(M)U(F)U(M)C(F)U(M)U(F)U(M)U(F)U(M)G(F)C(M )A(F)G(M)C(F)C(M)A(F)C(M)C(F)AUGGACUACAAGGACGACGACGACAAGAUCA UCGACUAUAAAGACGACGACGAUAAAGGUGGCGACUAUAAGGACGACGACGA CAAACACCACCACCACCACCACUGAAAAAAAAAAAAAAAAAAAAA | 339 |

**[Table 2-47]**

| | | | |
|---|---|---|---|
| E177 | Purchased from GeneDesign | GGGAGAAUACAAGCUACUUGUUCUUUUUGCAGCCACCAUGGACUACAAGGAC GACGACGACAAGAUCAUCGACUAUAAAGACGACGACGAUAAAGGUGGCGACU AUAAGGACGACGACGACAAACACCACCACCACCACCACUGAA(F)^A(M)^A(F)^A | 340 |
| | | (M)^A(F)^A(M)^A(F)^A(M)^A(F)^A(M)^A(F)^A(M)^A(F)^A(M)^A(F)^A(M)^A(F)^A(M OE)^A(MOE)^A(MOE) | |
| E178 | Purchased from GeneDesign | G(MOE)^G(MOE)^G(MOE)^A(MOE)^G(MOE)^A(MOE)AUACAAGCUACUUGUUCUU UUUGCAGCCACCAUGGACUACAAGGACGACGACGACAAGAUCAUCGACUAUA AAGACGACGACGAUAAAGGUGGCGACUAUAAGGACGACGACGACAAACACCA CCACCACCACCACUGAA(F)^A(M)^A(F)^A(M)^A(F)^A(M)^A(F)^A(M)^A(F)^A(M)^ A(F)^A(M)^A(F)^A(M)^A(F)^A(M)^A(F)^A(MOE)^A(MOE)^A(MOE) | 341 |
| E179 | Purchased from GeneDesign | G(MOE)^G(MOE)^G(MOE)^A(MOE)^G(MOE)^A(MOE)A(F)U(M)A(F)C(M)A(F)A(M)G (F)C(M)U(F)A(M)C(F)U(M)U(F)G(M)U(F)U(M)C(F)U(M)U(F)U(M)U(F)U(M)G(F)C(M )A(F)G(M)C(F)C(M)A(F)C(M)C(F)AUGGACUACAAGGACGACGACGACAAGAUCA UCGACUAUAAAGACGACGACGAUAAAGGUGGCGACUAUAAGGACGACGACGA CAAACACCACCACCACCACCACUGAA(F)^A(M)^A(F)^A(M)^A(F)^A(M)^A(F)^A(M )^A(F)^A(M)^A(F)^A(M)^A(F)^A(M)^A(F)^A(M)^A(F)^A(MOE)^A(MOE)^A(MOE) | 342 |
| E180 | Same as Example 2 | GGGAGAAUACAAGCUACUUGUUCUUUUUGCAGCCACCA(F)UGG(F)ACU(F)ACA (F)AGG(F)ACG(F)ACG(F)ACG(F)ACA(F)AGA(F)UCA(F)UCG(F)ACU(F)AUA(F)AA G(F)ACG(F)ACG(F)ACG(F)AUA(F)AAG(F)GUG(F)GCG(F)ACU(F)AUA(F)AGG(F)A CG(F)ACG(F)ACG(F)ACA(F)AAC(F)ACC(F)ACC(F)ACC(F)ACC(F)ACC(F)ACT(M OE)^G(MOE)^A(MOE)^AAAAAAAAAAAAAAAAAAA | 343 |
| E180-1 | Solid Phase Synthesis | GGGAGAAUACAAGCUACUUGUUCUUUUUGCAGCCACCA(F)UGG(F)ACU(F)ACA (F)AGG(F)ACG(F)ACG(F)ACG(F)ACA(F)AGA(F)UCA(F)UCG(F)ACU(F)AUA(F)AA G(F)p | 344 |
| E180-2 | Solid Phase Synthesis | ACG(F)ACG(F)ACG(F)AUA(F)AAG(F)GUG(F)GCG(F)ACU(F)AUA(F)AGG(F)ACG( F)ACG(F)ACG(F)ACA(F)AAC(F)ACC(F)ACC(F)ACC(F)ACC(F)ACC(F)ACT(MOE)^ G(MOE)^A(MOE)^AAAAAAAAAAAAAAAAAAAA | 345 |
| E181 | Same as Example 2 | GGGAGAAUACAAGCUACUUGUUCUUUUUGCAGCCACCA(F)^UGG(F)^ACU(F)^A CA(F)^AGG(F)^ACG(F)^ACG(F)^ACG(F)^ACA(F)^AGA(F)^UCA(F)^UCG(F)^ACU( F)^AUA(F)^AAG(F)ACG(F)^ACG(F)^ACG(F)^AUA(F)^AAG(F)^GUG(F)^GCG(F)^A CU(F)^AUA(F)^AGG(F)^ACG(F)^ACG(F)^ACG(F)^ACA(F)^AAC(F)^ACC(F)^ACC( F)^ACC(F)^ACC(F)^ACC(F)^ACT(MOE)^G(MOE)^A(MOE)^AAAAAAAAAAAAAA AAAAA | 346 |

**[Table 2-48]**

| | | | |
|---|---|---|---|
| E181-1 | Solid Phase Synthesis | GGGAGAAUACAAGCUACUUGUUCUUUUUGCAGCCACCA(F)^UGG(F)^ACU(F)^A CA(F)^AGG(F)^ACG(F)^ACG(F)^ACG(F)^ACA(F)^AGA(F)^UCA(F)^UCG(F)^ACU( F)^AUA(F)^AAG(F)p | 347 |
| E181-2 | Solid Phase Synthesis | ACG(F)^ACG(F)^ACG(F)^AUA(F)^AAG(F)^GUG(F)^GCG(F)^ACU(F)^AUA(F)^AGG (F)^ACG(F)^ACG(F)^ACG(F)^ACA(F)^AAC(F)^ACC(F)^ACC(F)^ACC(F)^ACC(F)^ ACC(F)^ACT(MOE)^G(MOE)^A(MOE)^AAAAAAAAAAAAAAAAAAAA | 348 |
| E182 | Purchased from GeneDesign | GGGAAAUAAGAGAGAAAAGAAGAGUAAGAAGAAAUAUAAGAGCCACCAUGGAC UACAAGGACGACGACGACAAGAUCAUCGACUAUAAAGACGACGACGAUAAAG GUGGCGACUAUAAGGACGACGACGACAAACACCACCACCACCACCACUGAAA AAAAAAAAAAAAAAAAAA | 349 |
| E183 | Same as Example 2 | G(MOE)^G(MOE)^G(MOE)^A(MOE)^A(MOE)^A(MOE)UAAGAGAGAAAAGAAGAGU AAGAAGAAAUAUAAGAGCCACCA(F)UGG(F)ACU(F)ACA(F)AGG(F)ACG(F)ACG( F)ACG(F)ACA(F)AGA(F)UCA(F)UCG(F)ACU(F)AUA(F)AAG(F)ACG(F)ACG(F)AC G(F)AUA(F)AAG(F)GUG(F)GCG(F)ACU(F)AUA(F)AGG(F)ACG(F)ACG(F)ACG(F)A CA(F)AAC(F)ACC(F)ACC(F)ACC(F)ACC(F)ACC(F)ACT(MOE)^G(MOE)^A(MOE)^ A(F)^A(M)^A(F)^A(M)^A(F)^A(M)^A(F)^A(M)^A(F)^A(M)^A(F)^A(M)^A(F)^A(M)^A( F)^A(M)^A(F)^A(MOE)^A(MOE)^A(MOE) | 350 |
| E183-1 | Solid Phase Synthesis | G(MOE)^G(MOE)^G(MOE)^A(MOE)^A(MOE)^A(MOE)UAAGAGAGAAAAGAAGAGU AAGAAGAAAUAUAAGAGCCACCA(F)UGG(F)ACU(F)ACA(F)AGG(F)ACG(F)ACG( F)ACG(F)ACA(F)AGA(F)UCA(F)UCG(F)ACU(F)AUA(F)AAG(F)p | 351 |
| E183-2 | Solid Phase Synthesis | ACG(F)ACG(F)ACG(F)AUA(F)AAG(F)GUG(F)GCG(F)ACU(F)AUA(F)AGG(F)ACG( F)ACG(F)ACG(F)ACA(F)AAC(F)ACC(F)ACC(F)ACC(F)ACC(F)ACC(F)ACT(MOE)^ G(MOE)^A(MOE)^A(F)^A(M)^A(F)^A(M)^A(F)^A(M)^A(F)^A(M)^A(F)^A(M)^A(F)^A( M)^A(F)^A(M)^A(F)^A(M)^A(F)^A(MOE)^A(MOE)^A(MOE) | 352 |
| E184 | Same as Example 2 | G(MOE)^G(MOE)^G(MOE)^A(MOE)^A(MOE)^A(MOE)U(F)A(M)A(F)G(M)A(F)G(M)A (F)G(M)A(F)A(M)A(F)A(M)G(F)A(M)A(F)G(M)A(F)G(M)U(F)A(M)A(F)G(M)A(F)A(M) G(F)A(M)A(F)A(M)U(F)A(M)U(F)A(M)A(F)G(M)A(F)G(M)C(F)C(M)A(F)C(M)C(F)A( F)UGG(F)ACU(F)ACA(F)AGG(F)ACG(F)ACG(F)ACG(F)ACA(F)AGA(F)UCA(F)UC G(F)ACU(F)AUA(F)AAG(F)ACG(F)ACG(F)ACG(F)AUA(F)AAG(F)GUG(F)GCG(F)A CU(F)AUA(F)AGG(F)ACG(F)ACG(F)ACG(F)ACA(F)AAC(F)ACC(F)ACC(F)ACC(F) ACC(F)ACC(F)ACT(MOE)^G(MOE)^A(MOE)^A(F)^A(M)^A(F)^A(M)^A(F)^A(M)^A( F)^A(M)^A(F)^A(M)^A(F)^A(M)^A(F)^A(M)^A(F)^A(M)^A(F)^A(MOE)^A(MOE)^A(M OE) | 353 |

**[Table 2-49]**

| | | | |
|---|---|---|---|
| E184-1 | Solid Phase Synthesis | G(MOE)^G(MOE)^G(MOE)^A(MOE)^A(MOE)^A(MOE)U(F)A(M)A(F)G(M)A(F)G(M)A (F)G(M)A(F)A(M)A(F)A(M)G(F)A(M)A(F)G(M)A(F)G(M)U(F)A(M)A(F)G(M)A(F)A(M) G(F)A(M)A(F)A(M)U(F)A(M)U(F)A(M)A(F)G(M)A(F)G(M)C(F)C(M)A(F)C(M)C(F)A( F)UGG(F)ACU(F)ACA(F)AGG(F)ACG(F)ACG(F)ACG(F)ACA(F)AGA(F)UCA(F)UC G(F)ACU(F)AUA(F)AAG(F)p | 354 |
| E184-2 | Solid Phase Synthesis | ACG(F)ACG(F)ACG(F)AUA(F)AAG(F)GUG(F)GCG(F)ACU(F)AUA(F)AGG(F)ACG( F)ACG(F)ACG(F)ACA(F)AAC(F)ACC(F)ACC(F)ACC(F)ACC(F)ACC(F)ACT(MOE)^ G(MOE)^A(MOE)^A(F)^A(M)^A(F)^A(M)^A(F)^A(M)^A(F)^A(M)^A(F)^A(M)^A(F)^A( M)^A(F)^A(M)^A(F)^A(M)^A(F)^A(MOE)^A(MOE)^A(MOE) | 355 |
| E185 | Same as Example 2 | G(MOE)G(MOE)G(MOE)A(MOE)A(MOE)A(MOE)U(F)A(M)A(F)G(M)A(F)G(M)A(F)G (M)A(F)A(M)A(F)A(M)G(F)A(M)A(F)G(M)A(F)G(M)U(F)A(M)A(F)G(M)A(F)A(M)G(F) A(M)A(F)A(M)U(F)A(M)U(F)A(M)A(F)G(M)A(F)G(M)C(F)C(M)A(F)C(M)C(F)A(F)UG G(F)ACU(F)ACA(F)AGG(F)ACG(F)ACG(F)ACG(F)ACA(F)AGA(F)UCA(F)UCG(F)A CU(F)AUA(F)AAG(F)ACG(F)ACG(F)ACG(F)AUA(F)AAG(F)GUG(F)GCG(F)ACU(F) AUA(F)AGG(F)ACG(F)ACG(F)ACG(F)ACA(F)AAC(F)ACC(F)ACC(F)ACC(F)ACC( F)ACC(F)ACT(MOE)^G(MOE)^A(MOE)^A(F)^A(M)^A(F)^A(M)^A(F)^A(M)^A(F)^A( M)^A(F)^A(M)^A(F)^A(M)^A(F)^A(M)^A(F)^A(M)^A(F)^A(MOE)^A(MOE)^A(MOE) | 356 |
| E185-1 | Solid Phase Synthesis | G(MOE)G(MOE)G(MOE)A(MOE)A(MOE)A(MOE)U(F)A(M)A(F)G(M)A(F)G(M)A(F)G (M)A(F)A(M)A(F)A(M)G(F)A(M)A(F)G(M)A(F)G(M)U(F)A(M)A(F)G(M)A(F)A(M)G(F) A(M)A(F)A(M)U(F)A(M)U(F)A(M)A(F)G(M)A(F)G(M)C(F)C(M)A(F)C(M)C(F)A(F)UG G(F)ACU(F)ACA(F)AGG(F)ACG(F)ACG(F)ACG(F)ACA(F)AGA(F)UCA(F)UCG(F)A CU(F)AUA(F)AAG(F)p | 357 |
| E185-2 | Solid Phase Synthesis | ACG(F)ACG(F)ACG(F)AUA(F)AAG(F)GUG(F)GCG(F)ACU(F)AUA(F)AGG(F)ACG( F)ACG(F)ACG(F)ACA(F)AAC(F)ACC(F)ACC(F)ACC(F)ACC(F)ACC(F)ACT(MOE)^ G(MOE)^A(MOE)^A(F)^A(M)^A(F)^A(M)^A(F)^A(M)^A(F)^A(M)^A(F)^A(M)^A(F)^A( M)^A(F)^A(M)^A(F)^A(M)^A(F)^A(MOE)^A(MOE)^A(MOE) | 358 |
| E186 | Purchased from GeneDesign | GGGAGACCUCUUCUGGUCCCCACAGACUCAGAGAGAACCCACCGGCCACCAU GGACUACAAGGACGACGACGACAAGAUCAUCGACUAUAAAGACGACGACGAU AAAGGUGGCGACUAUAAGGACGACGACGACAAACACCACCACCACCACCACU GAAAAAAAAAAAAAAAAAAAAA | 359 |

**[Table 2-50]**

| | | | |
|---|---|---|---|
| E187 | Same as Example 2 | G(MOE)^G(MOE)^G(MOE)^A(MOE)^G(MOE)^A(MOE)C(M)C(F)U(M)C(F)U(M)U(F) C(M)U(F)G(M)G(F)U(M)C(F)C(M)C(F)C(M)A(F)C(M)A(F)G(M)A(F)C(M)U(F)C(M)A( F)G(M)A(F)G(M)A(F)G(M)A(F)A(M)C(F)C(M)C(F)A(M)C(F)C(M)G(F)G(M)C(F)C(M) A(F)C(M)C(F)A(F)UGG(F)ACU(F)ACA(F)AGG(F)ACG(F)ACG(F)ACG(F)ACA(F)AG A(F)UCA(F)UCG(F)ACU(F)AUA(F)AAG(F)ACG(F)ACG(F)ACG(F)AUA(F)AAG(F)G UG(F)GCG(F)ACU(F)AUA(F)AGG(F)ACG(F)ACG(F)ACG(F)ACA(F)AAC(F)ACC(F) ACC(F)ACC(F)ACC(F)ACC(F)ACT(MOE)^G(MOE)^A(MOE)^A(F)^A(M)^A(F)^A(M) ^A(F)^A(M)^A(F)^A(M)^A(F)^A(M)^A(F)^A(M)^A(F)^A(M)^A(F)^A(M)^A(F)^A(MOE) ^A(MOE)^A(MOE) | 360 |
| E187-1 | Solid Phase Synthesis | G(MOE)^G(MOE)^G(MOE)^A(MOE)^G(MOE)^A(MOE)C(M)C(F)U(M)C(F)U(M)U(F) C(M)U(F)G(M)G(F)U(M)C(F)C(M)C(F)C(M)A(F)C(M)A(F)G(M)A(F)C(M)U(F)C(M)A( F)G(M)A(F)G(M)A(F)G(M)A(F)A(M)C(F)C(M)C(F)A(M)C(F)C(M)G(F)G(M)C(F)C(M) A(F)C(M)C(F)A(F)UGG(F)ACU(F)ACA(F)AGG(F)ACG(F)ACG(F)ACG(F)ACA(F)AG A(F)UCA(F)UCG(F)ACU(F)AUA(F)AAG(F)p | 361 |
| E187-2 | Solid Phase Synthesis | ACG(F)ACG(F)ACG(F)AUA(F)AAG(F)GUG(F)GCG(F)ACU(F)AUA(F)AGG(F)ACG( F)ACG(F)ACG(F)ACA(F)AAC(F)ACC(F)ACC(F)ACC(F)ACC(F)ACC(F)ACT(MOE)^ G(MOE)^A(MOE)^A(F)^A(M)^A(F)^A(M)^A(F)^A(M)^A(F)^A(M)^A(F)^A(M)^A(F)^A( M)^A(F)^A(M)^A(F)^A(M)^A(F)^A(MOE)^A(MOE)^A(MOE) | 362 |
| E188 | Same as Example 2 | G(MOE)G(MOE)G(MOE)A(MOE)G(MOE)A(MOE)C(M)C(F)U(M)C(F)U(M)U(F)C(M)U (F)G(M)G(F)U(M)C(F)C(M)C(F)C(M)A(F)C(M)A(F)G(M)A(F)C(M)U(F)C(M)A(F)G(M )A(F)G(M)A(F)G(M)A(F)A(M)C(F)C(M)C(F)A(M)C(F)C(M)G(F)G(M)C(F)C(M)A(F)C (M)C(F)A(F)UGG(F)ACU(F)ACA(F)AGG(F)ACG(F)ACG(F)ACG(F)ACA(F)AGA(F)U CA(F)UCG(F)ACU(F)AUA(F)AAG(F)ACG(F)ACG(F)ACG(F)AUA(F)AAG(F)GUG(F) GCG(F)ACU(F)AUA(F)AGG(F)ACG(F)ACG(F)ACG(F)ACA(F)AAC(F)ACC(F)ACC( F)ACC(F)ACC(F)ACC(F)ACT(MOE)^G(MOE)^A(MOE)^A(F)^A(M)^A(F)^A(M)^A(F) ^A(M)^A(F)^A(M)^A(F)^A(M)^A(F)^A(M)^A(F)^A(M)^A(F)^A(M)^A(F)^A(MOE)^A(M OE)^A(MOE) | 363 |
| E188-1 | Solid Phase Synthesis | G(MOE)G(MOE)G(MOE)A(MOE)G(MOE)A(MOE)C(M)C(F)U(M)C(F)U(M)U(F)C(M)U (F)G(M)G(F)U(M)C(F)C(M)C(F)C(M)A(F)C(M)A(F)G(M)A(F)C(M)U(F)C(M)A(F)G(M )A(F)G(M)A(F)G(M)A(F)A(M)C(F)C(M)C(F)A(M)C(F)C(M)G(F)G(M)C(F)C(M)A(F)C (M)C(F)A(F)UGG(F)ACU(F)ACA(F)AGG(F)ACG(F)ACG(F)ACG(F)ACA(F)AGA(F)U CA(F)UCG(F)ACU(F)AUA(F)AAG(F)p | 364 |

**[Table 2-51]**

| | | | |
|---|---|---|---|
| E188-2 | Solid Phase Synthesis | ACG(F)ACG(F)ACG(F)AUA(F)AAG(F)GUG(F)GCG(F)ACU(F)AUA(F)AGG(F)ACG( F)ACG(F)ACG(F)ACA(F)AAC(F)ACC(F)ACC(F)ACC(F)ACC(F)ACC(F)ACT(MOE)^ G(MOE)^A(MOE)^A(F)^A(M)^A(F)^A(M)^A(F)^A(M)^A(F)^A(M)^A(F)^A(M)^A(F)^A( M)^A(F)^A(M)^A(F)^A(M)^A(F)^A(MOE)^A(MOE)^A(MOE) | 365 |
| E189 | Purchased from GeneDesign | GGGAGCCACCAUGGACUACAAGGACGACGACGACAAGAUCAUCGACUAUAAA GACGACGACGAUAAAGGUGGCGACUAUAAGGACGACGACGACAAACACCACC ACCACCACCACUGAAAAAAAAAAAAAAAAAAAAA | 366 |
| E190 | Same as Example 2 | G(MOE)^G(MOE)G^(MOE)A(F)G(M)C(F)C(M)A(F)C(M)C(F)A(F)UGG(F)ACU(F)AC A(F)AGG(F)ACG(F)ACG(F)ACG(F)ACA(F)AGA(F)UCA(F)UCG(F)ACU(F)AUA(F)A AG(F)ACG(F)ACG(F)ACG(F)AUA(F)AAG(F)GUG(F)GCG(F)ACU(F)AUA(F)AGG(F) ACG(F)ACG(F)ACG(F)ACA(F)AAC(F)ACC(F)ACC(F)ACC(F)ACC(F)ACC(F)ACT( MOE)^G(MOE)^A(MOE)^A(F)^A(M)^A(F)^A(M)^A(F)^A(M)^A(F)^A(M)^A(F)^A(M)^ A(F)^A(M)^A(F)^A(M)^A(F)^A(M)^A(F)^A(MOE)^A(MOE)^A(MOE) | 367 |
| E190-1 | Solid Phase Synthesis | G(MOE)^G(MOE)^G(MOE)A(F)G(M)C(F)C(M)A(F)C(M)C(F)A(F)UGG(F)ACU(F)AC A(F)AGG(F)ACG(F)ACG(F)ACG(F)ACA(F)AGA(F)UCA(F)UCG(F)ACU(F)AUA(F)A AG(F)p | 368 |
| E190-2 | Solid Phase Synthesis | ACG(F)ACG(F)ACG(F)AUA(F)AAG(F)GUG(F)GCG(F)ACU(F)AUA(F)AGG(F)ACG( F)ACG(F)ACG(F)ACA(F)AAC(F)ACC(F)ACC(F)ACC(F)ACC(F)ACC(F)ACT(MOE)^ G(MOE)^A(MOE)^A(F)^A(M)^A(F)^A(M)^A(F)^A(M)^A(F)^A(M)^A(F)^A(M)^A(F)^A( M)^A(F)^A(M)^A(F)^A(M)^A(F)^A(MOE)^A(MOE)^A(MOE) | 369 |
| E191 | Same as Example 2 | G(MOE)G(MOE)G(MOE)A(F)G(M)C(F)C(M)A(F)C(M)C(F)A(F)UGG(F)ACU(F)ACA( F)AGG(F)ACG(F)ACG(F)ACG(F)ACA(F)AGA(F)UCA(F)UCG(F)ACU(F)AUA(F)AA G(F)ACG(F)ACG(F)ACG(F)AUA(F)AAG(F)GUG(F)GCG(F)ACU(F)AUA(F)AGG(F)A CG(F)ACG(F)ACG(F)ACA(F)AAC(F)ACC(F)ACC(F)ACC(F)ACC(F)ACC(F)ACT(M OE)^G(MOE)^A(MOE)^A(F)^A(M)^A(F)^A(M)^A(F)^A(M)^A(F)^A(M)^A(F)^A(M)^A( F)^A(M)^A(F)^A(M)^A(F)^A(M)^A(F)^A(MOE)^A(MOE)^A(MOE) | 370 |
| E191-1 | Solid Phase Synthesis | G(MOE)G(MOE)G(MOE)A(F)G(M)C(F)C(M)A(F)C(M)C(F)A(F)UGG(F)ACU(F)ACA( F)AGG(F)ACG(F)ACG(F)ACG(F)ACA(F)AGA(F)UCA(F)UCG(F)ACU(F)AUA(F)AA G(F)p | 371 |
| E191-2 | Solid Phase Synthesis | ACG(F)ACG(F)ACG(F)AUA(F)AAG(F)GUG(F)GCG(F)ACU(F)AUA(F)AGG(F)ACG( F)ACG(F)ACG(F)ACA(F)AAC(F)ACC(F)ACC(F)ACC(F)ACC(F)ACC(F)ACT(MOE)^ G(MOE)^A(MOE)^A(F)^A(M)^A(F)^A(M)^A(F)^A(M)^A(F)^A(M)^A(F)^A(M)^A(F)^A( M)^A(F)^A(M)^A(F)^A(M)^A(F)^A(MOE)^A(MOE)^A(MOE) | 372 |

**[Table 2-52]**

| | | | |
|---|---|---|---|
| E192 | Purchased from GeneDesign | GGGAGAAUACAAGCUACUUGUUCUUUUUGCAGCCACCAUGGACUACAAGGAC GACGACGACAAGAUCAUCGACUAUAAAGACGACGACGAUAAAGGUGGCGACU AUAAGGACGACGACGACAAACACCACCACCACCACCACUGAAAAAAAAAAAA AAAAAAAAAAAAAAAAAAAAAAAAAAAA | 373 |
| E193 | Same as Example 2 | G(MOE)G(MOE)G(MOE)A(MOE)G(MOE)A(MOE)AU(F)ACA(F)AGC(F)UAC(F)UUG( F)UUC(F)UUU(F)UUG(F)CAG(F)CCA(F)CCA(F)UGG(F)ACU(F)ACA(F)AGG(F)AC G(F)ACG(F)ACG(F)ACA(F)AGA(F)UCA(F)UCG(F)ACU(F)AUA(F)AAG(F)ACG(F)A CG(F)ACG(F)AUA(F)AAG(F)GUG(F)GCG(F)ACU(F)AUA(F)AGG(F)ACG(F)ACG(F) ACG(F)ACA(F)AAC(F)ACC(F)ACC(F)ACC(F)ACC(F)ACC(F)ACT(MOE)G(MOE)A( MOE)A(F)AAA(F)AAA(F)AAA(F)AAA(F)AAA(F)AA(MOE)A(MOE)A(MOE) | 374 |
| E193-1 | Solid Phase Synthesis | G(MOE)G(MOE)G(MOE)A(MOE)G(MOE)A(MOE)AU(F)ACA(F)AGC(F)UAC(F)UUG( F)UUC(F)UUU(F)UUG(F)CAG(F)CCA(F)CCA(F)UGG(F)ACU(F)ACA(F)AGG(F)AC G(F)ACG(F)ACG(F)ACA(F)AGA(F)UCA(F)UCG(F)ACU(F)AUA(F)AAG(F)p | 375 |
| E193-2 | Solid Phase Synthesis | ACG(F)ACG(F)ACG(F)AUA(F)AAG(F)GUG(F)GCG(F)ACU(F)AUA(F)AGG(F)ACG( F)ACG(F)ACG(F)ACA(F)AAC(F)ACC(F)ACC(F)ACC(F)ACC(F)ACC(F)ACT(MOE) G(MOE)A(MOE)A(F)AAA(F)AAA(F)AAA(F)AAA(F)AAA(F)AA(MOE)A(MOE)A(MOE ) | 376 |
| E194 | Same as Example 2 | G(MOE)G(MOE)G(MOE)A(MOE)G(MOE)A(MOE)A(rSpacer)AC(rSpacer)AG(rSpac er)UA(rSpacer)UU(rSpacer)UU(rSpacer)UU(rSpacer)UUG(F)CAG(F)CCA(F)CCA( F)UGG(F)ACU(F)ACA(F)AGG(F)ACG(F)ACG(F)ACG(F)ACA(F)AGA(F)UCA(F)UC G(F)ACU(F)AUA(F)AAG(F)ACG(F)ACG(F)ACG(F)AUA(F)AAG(F)GUG(F)GCG(F)A CU(F)AUA(F)AGG(F)ACG(F)ACG(F)ACG(F)ACA(F)AAC(F)ACC(F)ACC(F)ACC(F) ACC(F)ACC(F)ACT(MOE)G(MOE)A(MOE)A(F)AAA(F)AAA(F)AAA(F)AAA(F)AAA( F)AA(MOE)A(MOE)A(MOE) | 377 |
| E194-1 | Solid Phase Synthesis | G(MOE)G(MOE)G(MOE)A(MOE)G(MOE)A(MOE)A(rSpacer)AC(rSpacer)AG(rSpac er)UA(rSpacer)UU(rSpacer)UU(rSpacer)UU(rSpacer)UUG(F)CAG(F)CCA(F)CCA( F)UGG(F)ACU(F)ACA(F)AGG(F)ACG(F)ACG(F)ACG(F)ACA(F)AGA(F)UCA(F)UC | 378 |
| | | G(F)ACU(F)AUA(F)AAG(F)p | |
| E194-2 | Solid Phase Synthesis | ACG(F)ACG(F)ACG(F)AUA(F)AAG(F)GUG(F)GCG(F)ACU(F)AUA(F)AGG(F)ACG( F)ACG(F)ACG(F)ACA(F)AAC(F)ACC(F)ACC(F)ACC(F)ACC(F)ACC(F)ACT(MOE) G(MOE)A(MOE)A(F)AAA(F)AAA(F)AAA(F)AAA(F)AAA(F)AA(MOE)A(MOE)A(MOE ) 379 | 379 |

**[Table 2-53]**

| | | | |
|---|---|---|---|
| E195 | Same as Example 2 | G(MOE)G(MOE)G(MOE)A(MOE)G(MOE)A(MOE)AU(F)ACA(F)AGC(F)UAC(F)UUG( F)UUC(F)UUU(F)UUG(F)CA(rSpacer)CC(rSpacer)CCA(F)UGG(F)ACU(F)ACA(F)A GG(F)ACG(F)ACG(F)ACG(F)ACA(F)AGA(F)UCA(F)UCG(F)ACU(F)AUA(F)AAG(F) ACG(F)ACG(F)ACG(F)AUA(F)AAG(F)GUG(F)GCG(F)ACU(F)AUA(F)AGG(F)ACG( F)ACG(F)ACG(F)ACA(F)AAC(F)ACC(F)ACC(F)ACC(F)ACC(F)ACC(F)ACT(MOE) G(MOE)A(MOE)A(F)AAA(F)AAA(F)AAA(F)AAA(F)AAA(F)AA(MOE)A(MOE)A(MOE ) | 380 |
| E195-1 | Solid Phase Synthesis | G(MOE)G(MOE)G(MOE)A(MOE)G(MOE)A(MOE)AU(F)ACA(F)AGC(F)UAC(F)UUG( F)UUC(F)UUU(F)UUG(F)CA(rSpacer)CC(rSpacer)CCA(F)UGG(F)ACU(F)ACA(F)A GG(F)ACG(F)ACG(F)ACG(F)ACA(F)AGA(F)UCA(F)UCG(F)ACU(F)AUA(F)AAG(F) p | 381 |
| E195-2 | Solid Phase Synthesis | ACG(F)ACG(F)ACG(F)AUA(F)AAG(F)GUG(F)GCG(F)ACU(F)AUA(F)AGG(F)ACG( F)ACG(F)ACG(F)ACA(F)AAC(F)ACC(F)ACC(F)ACC(F)ACC(F)ACC(F)ACT(MOE) G(MOE)A(MOE)A(F)AAA(F)AAA(F)AAA(F)AAA(F)AAA(F)AA(MOE)A(MOE)A(MOE ) | 382 |
| E196 | Same as Example 2 | G(MOE)G(MOE)G(MOE)A(MOE)G(MOE)A(MOE)AU(F)ACA(F)AGC(F)UAC(F)UUG( F)UUC(F)UUU(F)UUG(F)CAG(F)CCA(F)CC(rSpacer)UG(rSpacer)ACU(F)ACA(F)A GG(F)ACG(F)ACG(F)ACG(F)ACA(F)AGA(F)UCA(F)UCG(F)ACU(F)AUA(F)AAG(F) ACG(F)ACG(F)ACG(F)AUA(F)AAG(F)GUG(F)GCG(F)ACU(F)AUA(F)AGG(F)ACG( F)ACG(F)ACG(F)ACA(F)AAC(F)ACC(F)ACC(F)ACC(F)ACC(F)ACC(F)ACT(MOE) G(MOE)A(MOE)A(F)AAA(F)AAA(F)AAA(F)AAA(F)AAA(F)AA(MOE)A(MOE)A(MOE ) | 383 |
| E196-1 | Solid Phase Synthesis | G(MOE)G(MOE)G(MOE)A(MOE)G(MOE)A(MOE)AU(F)ACA(F)AGC(F)UAC(F)UUG( F)UUC(F)UUU(F)UUG(F)CAG(F)CCA(F)CC(rSpacer)UG(rSpacer)ACU(F)ACA(F)A GG(F)ACG(F)ACG(F)ACG(F)ACA(F)AGA(F)UCA(F)UCG(F)ACU(F)AUA(F)AAG(F) p | 384 |
| E196-2 | Solid Phase Synthesis | ACG(F)ACG(F)ACG(F)AUA(F)AAG(F)GUG(F)GCG(F)ACU(F)AUA(F)AGG(F)ACG( F)ACG(F)ACG(F)ACA(F)AAC(F)ACC(F)ACC(F)ACC(F)ACC(F)ACC(F)ACT(MOE) G(MOE)A(MOE)A(F)AAA(F)AAA(F)AAA(F)AAA(F)AAA(F)AA(MOE)A(MOE)A(MOE ) | 385 |
| E197 | Same as Example 2 | G(MOE)G(MOE)G(MOE)A(MOE)G(MOE)A(MOE)AU(F)ACA(F)AGC(F)UAC(F)UUG( F)UUC(F)UUU(F)UUG(F)CAG(F)CCA(F)CCA(F)UGG(F)ACU(F)ACA(F)AGG(F)AC G(F)ACG(F)ACG(F)ACA(F)AGA(F)UCA(F)UCG(F)ACU(F)AUA(F)AAG(F)ACG(F)A CG(F)ACG(F)AUA(F)AAG(F)GUG(F)GCG(F)ACU(F)AUA(F)AG(rSpacer)AC(rSpac er)ACG(F)ACG(F)ACA(F)AAC(F)ACC(F)ACC(F)ACC(F)ACC(F)ACC(F)ACT(MOE) G(MOE)A(MOE)A(F)AAA(F)AAA(F)AAA(F)AAA(F)AAA(F)AA(MOE)A(MOE)A(MOE ) | 386 |
| E197-1 | Solid Phase Synthesis | G(MOE)G(MOE)G(MOE)A(MOE)G(MOE)A(MOE)AU(F)ACA(F)AGC(F)UAC(F)UUG( F)UUC(F)UUU(F)UUG(F)CAG(F)CCA(F)CCA(F)UGG(F)ACU(F)ACA(F)AGG(F)AC G(F)ACG(F)ACG(F)ACA(F)AGA(F)UCA(F)UCG(F)ACU(F)AUA(F)AAG(F)p | 387 |

**[Table 2-54]**

| | | | |
|---|---|---|---|
| E197-2 | Solid Phase Synthesis | ACG(F)ACG(F)ACG(F)AUA(F)AAG(F)GUG(F)GCG(F)ACU(F)AUA(F)AG(rSpacer) AC(rSpacer)ACG(F)ACG(F)ACA(F)AAC(F)ACC(F)ACC(F)ACC(F)ACC(F)ACC(F)A CT(MOE)G(MOE)A(MOE)A(F)AAA(F)AAA(F)AAA(F)AAA(F)AAA(F)AA(MOE)A(MO E)A(MOE) | 388 |
| E198 | Same as Example 2 | G(MOE)G(MOE)G(MOE)A(MOE)G(MOE)A(MOE)AU(F)ACA(F)AGC(F)UAC(F)UUG( F)UUC(F)UUU(F)UUG(F)CAG(F)CCA(F)CCA(F)UGG(F)ACU(F)ACA(F)AGG(F)AC G(F)ACG(F)ACG(F)ACA(F)AGA(F)UCA(F)UCG(F)ACU(F)AUA(F)AAG(F)ACG(F)A CG(F)ACG(F)AUA(F)AAG(F)GUG(F)GCG(F)ACU(F)AUA(F)AGG(F)ACG(F)ACG(F) ACG(F)ACA(F)AAC(F)ACC(F)ACC(F)ACC(F)ACC(F)ACC(F)ACT(MOE)G(MOE)A( MOE)A(F)AAA(F)AAA(F)AAA(F)AA(rSpacer)AA(rSpacer)AA(MOE)A(MOE)A(MOE ) | 389 |
| E198-1 | Solid Phase Synthesis | G(MOE)G(MOE)G(MOE)A(MOE)G(MOE)A(MOE)AU(F)ACA(F)AGC(F)UAC(F)UUG( F)UUC(F)UUU(F)UUG(F)CAG(F)CCA(F)CCA(F)UGG(F)ACU(F)ACA(F)AGG(F)AC G(F)ACG(F)ACG(F)ACA(F)AGA(F)UCA(F)UCG(F)ACU(F)AUA(F)AAG(F)p | 390 |
| E198-2 | Solid Phase Synthesis | ACG(F)ACG(F)ACG(F)AUA(F)AAG(F)GUG(F)GCG(F)ACU(F)AUA(F)AGG(F)ACG( F)ACG(F)ACG(F)ACA(F)AAC(F)ACC(F)ACC(F)ACC(F)ACC(F)ACC(F)ACT(MOE) G(MOE)A(MOE)A(F)AAA(F)AAA(F)AAA(F)AA(rSpacer)AA(rSpacer)AA(MOE)A(M OE)A(MOE) | 391 |
| E199 | Same as Example 2 | G(MOE)G(MOE)G(MOE)A(MOE)G(MOE)A(MOE)AU(F)ACA(F)AGC(F)UAC(F)UUG( F)UUC(F)UUU(F)UUG(F)CAG(F)CCA(F)CCA(F)UGG(F)ACU(F)ACA(F)AGG(F)AC G(F)ACG(F)ACG(F)ACA(F)AGA(F)UCA(F)UCG(F)ACU(F)AUA(F)AAG(F)ACG(F)A CG(F)ACG(F)AUA(F)AAG(F)GUG(F)GCG(F)ACU(F)AUA(F)AGG(F)ACG(F)ACG(F) ACG(F)ACA(F)AAC(F)ACC(F)ACC(F)ACC(F)ACC(F)ACC(F)ACT(MOE)G(MOE)A( MOE)(rSpacer)AA(rSpacer)AA(rSpacer)AA(rSpacer)AA(rSpacer)AA(rSpacer)AA( MOE)A(MOE)A(MOE) | 392 |
| E199-1 | Solid Phase Synthesis | G(MOE)G(MOE)G(MOE)A(MOE)G(MOE)A(MOE)AU(F)ACA(F)AGC(F)UAC(F)UUG( F)UUC(F)UUU(F)UUG(F)CAG(F)CCA(F)CCA(F)UGG(F)ACU(F)ACA(F)AGG(F)AC G(F)ACG(F)ACG(F)ACA(F)AGA(F)UCA(F)UCG(F)ACU(F)AUA(F)AAG(F)p | 393 |
| E199-2 | Solid Phase Synthesis | ACG(F)ACG(F)ACG(F)AUA(F)AAG(F)GUG(F)GCG(F)ACU(F)AUA(F)AGG(F)ACG( F)ACG(F)ACG(F)ACA(F)AAC(F)ACC(F)ACC(F)ACC(F)ACC(F)ACC(F)ACT(MOE) G(MOE)A(MOE)(rSpacer)AA(rSpacer)AA(rSpacer)AA(rSpacer)AA(rSpacer)AA(rS pacer)AA(MOE)A(MOE)A(MOE) | 394 |

**[Table 2-55]**

| | | | |
|---|---|---|---|
| E200 | Solid Phase Synthesis | G(MOE)^G(MOE)^G(MOE)A(MOE)G(MOE)A(MOE)A(F)U(M)A(F)C(M)A(F)A(M)G(F) C(M)U(F)A(M)C(F)U(M)(C2)G(M)U(F)U(M)C(F)U(M)U(F)U(M)U(F)U(M)G(F)C(M)A( F)G(M)C(F)C(M)A(F)C(M)C(F)A(F)UGG(F)ACU(F)AUA(F)AGG(F)ACG(F)ACG(F)A CG(F)ACA(F)AAG(F)GUG(F)GCC(F)ACC(F)ACC(F)ACC(F)ACC(F)ACC(F)ACT(M OE)G(MOE)A(MOE) | 395 |
| E201 | Solid Phase Synthesis | G(MOE)^G(MOE)^G(MOE)A(MOE)G(MOE)A(MOE)A(F)U(M)A(F)C(M)A(F)A(M)G(F) C(M)U(F)A(M)C(F)U(M)(C2)G(M)U(F)U(M)(C2)U(M)U(F)U(M)U(F)U(M)G(F)C(M)A( F)G(M)C(F)C(M)A(F)C(M)C(F)A(F)UGG(F)ACU(F)AUA(F)AGG(F)ACG(F)ACG(F)A CG(F)ACA(F)AAG(F)GUG(F)GCC(F)ACC(F)ACC(F)ACC(F)ACC(F)ACC(F)ACT(M OE)G(MOE)A(MOE) | 396 |
| E202 | Solid Phase Synthesis | G(MOE)^G(MOE)^G(MOE)A(MOE)G(MOE)A(MOE)A(F)U(M)A(F)C(M)A(F)A(M)G(F) C(M)U(F)A(M)C(F)U(M)(Spacer9)U(F)U(M)C(F)U(M)U(F)U(M)U(F)U(M)G(F)C(M)A( F)G(M)C(F)C(M)A(F)C(M)C(F)A(F)UGG(F)ACU(F)AUA(F)AGG(F)ACG(F)ACG(F)A CG(F)ACA(F)AAG(F)GUG(F)GCC(F)ACC(F)ACC(F)ACC(F)ACC(F)ACC(F)ACT(M OE)G(MOE)A(MOE) | 397 |
| E203 | Solid Phase Synthesis | G(MOE)^G(MOE)^G(MOE)A(MOE)G(MOE)A(MOE)A(F)U(M)A(F)C(M)A(F)A(M)G(F) C(M)U(F)A(M)C(F)U(M)U(F)G(M)U(F)U(M)(Spacer9)U(F)U(M)U(F)U(M)G(F)C(M)A (F)G(M)C(F)C(M)A(F)C(M)C(F)A(F)UGG(F)ACU(F)AUA(F)AGG(F)ACG(F)ACG(F) ACG(F)ACA(F)AAG(F)GUG(F)GCC(F)ACC(F)ACC(F)ACC(F)ACC(F)ACC(F)ACT( MOE)G(MOE)A(MOE) | 398 |
| E204 | Solid Phase Synthesis | GG(MOE)^G(MOE)A(MOE)G(MOE)A(MOE)A(F)U(M)A(F)C(M)A(F)A(M)G(F)C(M)U( F)A(M)C(F)U(M)U(F)G(M)U(F)U(M)C(F)U(M)U(F)U(M)U(F)U(M)G(F)C(M)A(F)G(M) C(F)C(M)A(F)C(M)C(F)A(F)UGG(F)ACU(F)AUA(F)AGG(F)ACG(F)ACG(F)ACG(F)A CA(F)AAG(F)GUG(F)GCC(F)ACC(F)ACC(F)ACC(F)ACC(F)ACC(F)ACT(MOE)G(M OE)A(MOE) | 399 |
| E205 | Solid Phase Synthesis | GGGA(MOE)G(MOE)A(MOE)A(F)U(M)A(F)C(M)A(F)A(M)G(F)C(M)U(F)A(M)C(F)U( M)U(F)G(M)U(F)U(M)C(F)U(M)U(F)U(M)U(F)U(M)G(F)C(M)A(F)G(M)C(F)C(M)A(F) C(M)C(F)A(F)UGG(F)ACU(F)AUA(F)AGG(F)ACG(F)ACG(F)ACG(F)ACA(F)AAG(F) GUG(F)GCC(F)ACC(F)ACC(F)ACC(F)ACC(F)ACC(F)ACT(MOE)G(MOE)A(MOE) | 400 |
| E206 | Solid Phase Synthesis | G^G^G^A(MOE)G(MOE)A(MOE)A(F)U(M)A(F)C(M)A(F)A(M)G(F)C(M)U(F)A(M)C(F )U(M)U(F)G(M)U(F)U(M)C(F)U(M)U(F)U(M)U(F)U(M)G(F)C(M)A(F)G(M)C(F)C(M)A (F)C(M)C(F)A(F)UGG(F)ACU(F)AUA(F)AGG(F)ACG(F)ACG(F)ACG(F)ACA(F)AAG (F)GUG(F)GCC(F)ACC(F)ACC(F)ACC(F)ACC(F)ACC(F)ACT(MOE)G(MOE)A(MOE ) | 401 |

**[Table 2-56]**

| | | | |
|---|---|---|---|
| E207 | Solid Phase Synthesis | G(MOE)^G(MOE)^G(MOE)A(MOE)G(MOE)A(MOE)A(F)U(M)A(F)C(M)A(F)A(M)G(F) C(M)U(F)A(M)C(F)U(M)U(F)G(M)U(F)U(M)C(F)U(M)U(F)U(M)U(F)U(M)G(F)C(M)A( F)G(M)C(F)C(M)A(F)C(M)C(F)A(F)UGG(F)ACU(F)AUA(F)AGG(F)ACG(F)ACG(F)A CG(F)ACA(F)AAG(F)GUG(F)GCC(F)ACC(F)ACC(F)ACC(F)ACC(F)ACC(F)ACUGA | 402 |
| E208 | Solid Phase Synthesis | GGGA(MOE)G(MOE)A(MOE)A(F)U(M)A(F)C(M)A(F)A(M)G(F)C(M)U(F)A(M)C(F)U( M)U(F)G(M)U(F)U(M)C(F)U(M)U(F)U(M)U(F)U(M)G(F)C(M)A(F)G(M)C(F)C(M)A(F) C(M)C(F)A(F)UGG(F)ACU(F)AUA(F)AGG(F)ACG(F)ACG(F)ACG(F)ACA(F)AAG(F) GUG(F)GCC(F)ACC(F)ACC(F)ACC(F)ACC(F)ACC(F)ACUGA | 403 |
| E209 | Solid Phase Synthesis | GG(MOE)G(MOE)A(MOE)G(MOE)A(MOE)A(F)U(F)AC(F)A(F)AG(F)C(F)UA(F)C(F) UU(F)G(F)UU(F)C(F)UU(F)U(F)UU(F)G(F)CA(F)G(F)CC(F)A(F)CC(F)A(F)UGG(F) ACU(F)AUA(F)AGG(F)ACG(F)ACG(F)ACG(F)ACA(F)AAG(F)GUG(F)GCC(F)ACC( F)ACC(F)ACC(F)ACC(F)ACC(F)ACT(MOE)G(MOE)A(MOE) | 404 |
| E210 | Solid Phase Synthesis | GGGA(MOE)G(MOE)A(MOE)A(F)U(F)AC(F)A(F)AG(F)C(F)UA(F)C(F)UU(F)G(F)UU (F)C(F)UU(F)U(F)UU(F)G(F)CA(F)G(F)CC(F)A(F)CC(F)A(F)UGG(F)ACU(F)AUA(F )AGG(F)ACG(F)ACG(F)ACG(F)ACA(F)AAG(F)GUG(F)GCC(F)ACC(F)ACC(F)ACC( F)ACC(F)ACC(F)ACT(MOE)G(MOE)A(MOE) | 405 |
| E211 | Solid Phase Synthesis | G^G^G^A(MOE)G(MOE)A(MOE)A(F)U(F)AC(F)A(F)AG(F)C(F)UA(F)C(F)UU(F)G(F) UU(F)C(F)UU(F)U(F)UU(F)G(F)CA(F)G(F)CC(F)A(F)CC(F)A(F)UGG(F)ACU(F)AU A(F)AGG(F)ACG(F)ACG(F)ACG(F)ACA(F)AAG(F)GUG(F)GCC(F)ACC(F)ACC(F)A CC(F)ACC(F)ACC(F)ACT(MOE)G(MOE)A(MOE) | 406 |
| E212 | Solid Phase Synthesis | G(MOE)G(MOE)G(MOE)A(MOE)G(MOE)A(MOE)A(F)U(F)AC(F)A(F)AG(F)C(F)UA( F)C(F)UU(F)G(F)UU(F)C(F)UU(F)U(F)UU(F)G(F)CA(F)G(F)CC(F)A(F)CC(F)A(F)U GG(F)ACU(F)AUA(F)AGG(F)ACG(F)ACG(F)ACG(F)ACA(F)AAG(F)GUG(F)GCC(F) ACC(F)ACC(F)ACC(F)ACC(F)ACC(F)ACUGA | 407 |
| E213 | Solid Phase Synthesis | GGGA(MOE)G(MOE)A(MOE)A(F)U(F)AC(F)A(F)AG(F)C(F)UA(F)C(F)UU(F)G(F)UU (F)C(F)UU(F)U(F)UU(F)G(F)CA(F)G(F)CC(F)A(F)CC(F)A(F)UGG(F)ACU(F)AUA(F )AGG(F)ACG(F)ACG(F)ACG(F)ACA(F)AAG(F)GUG(F)GCC(F)ACC(F)ACC(F)ACC( F)ACC(F)ACC(F)ACUGA | 408 |
| E214 | Solid Phase Synthesis | GGG(MOE)G(MOE)G(MOE)A(MOE)G(MOE)A(MOE)A(F)U(F)AC(F)A(F)AG(F)C(F)U A(F)C(F)UU(F)G(F)UU(F)C(F)UU(F)U(F)UU(F)G(F)CA(F)G(F)CC(F)A(F)CC(F)A(F) UGG(F)ACU(F)AUA(F)AGG(F)ACG(F)ACG(F)ACG(F)ACA(F)AAG(F)GUG(F)GCC( F)ACC(F)ACC(F)ACC(F)ACC(F)ACC(F)ACT(MOE)G(MOE)A(MOE)AA | 409 |

**[Table 2-57]**

| | | | |
|---|---|---|---|
| E215 | Same as Example 2 | G(MOE)^G(MOE)^G(MOE)^A(MOE)^G(MOE)^A(MOE)^A(M)^t^A(M)^C(M)^a^A(M)^ G(M)cU(M)A(M)cU(M)U(M)gU(M)U(M)cU(M)U(M)tU(M)U(M)gC(M)A(M)gC(M)C(M)a C(M)C(M)A(F)UGG(F)ACU(F)ACA(F)AGG(F)ACG(F)ACG(F)ACG(F)ACA(F)AGA(F )UCA(F)UCG(F)ACU(F)AUA(F)AAG(F)ACG(F)ACG(F)ACG(F)AUA(F)AAG(F)GUG( F)GCG(F)ACU(F)AUA(F)AGG(F)ACG(F)ACG(F)ACG(F)ACA(F)AAC(F)ACC(F)AC C(F)ACC(F)ACC(F)ACC(F)ACT(MOE)^G(MOE)^A(MOE)^A(M)^A(M)^A(M)^A(M)^A (M)^A(M)^A(M)^A(M)^A(M)^A(M)^A(M)^A(M)^A(M)^A(M)^A(M)^A(M)^A(M)^A (M)^A(M) | 410 |
| E215-1 | Solid Phase Synthesis | G(MOE)^G(MOE)^G(MOE)^A(MOE)^G(MOE)^A(MOE)^A(M)^t^A(M)^C(M)^a^A(M)^ G(M)cU(M)A(M)cU(M)U(M)gU(M)U(M)cU(M)U(M)tU(M)U(M)gC(M)A(M)gC(M)C(M)a C(M)C(M)A(F)UGG(F)ACU(F)ACA(F)AGG(F)ACG(F)ACG(F)ACG(F)ACA(F)AGA(F )UCA(F)UCG(F)ACU(F)AUA(F)AAG(F)p | 411 |
| E215-2 | Solid Phase Synthesis | ACG(F)ACG(F)ACG(F)AUA(F)AAG(F)GUG(F)GCG(F)ACU(F)AUA(F)AGG(F)ACG( F)ACG(F)ACG(F)ACA(F)AAC(F)ACC(F)ACC(F)ACC(F)ACC(F)ACC(F)ACT(MOE)^ G(MOE)^A(MOE)^A(M)^A(M)^A(M)^A(M)^A(M)^A(M)^A(M)^A(M)^A(M)^A(M)^A(M) ^A(M)^A(M)^A(M)^A(M)^A(M)^A(M)^A(M)^A(M)^A(M)^A(M) | 412 |
| E216 | Same as Example 2 | G(MOE)^G(MOE)^G(MOE)^A(MOE)^G(MOE)^A(MOE)^a^t^A(M)^c^a^A(M)^gcU(M) acU(M)tgU(M)tcU(M)ttU(M)tgC(M)agC(M)caC(M)cA(F)UGG(F)ACU(F)ACA(F)AGG (F)ACG(F)ACG(F)ACG(F)ACA(F)AGA(F)UCA(F)UCG(F)ACU(F)AUA(F)AAG(F)AC G(F)ACG(F)ACG(F)AUA(F)AAG(F)GUG(F)GCG(F)ACU(F)AUA(F)AGG(F)ACG(F)A CG(F)ACG(F)ACA(F)AAC(F)ACC(F)ACC(F)ACC(F)ACC(F)ACC(F)ACT(MOE)^G( MOE)^A(MOE)^A(M)^A(M)^A(M)^A(M)^A(M)^A(M)^A(M)^A(M)^A(M)^A(M)^A(M)^A( M)^A(M)^A(M)^A(M)^A(M)^A(M)^A(M)^A(M)^A(M) | 413 |
| E216-1 | Solid Phase Synthesis | G(MOE)^G(MOE)^G(MOE)^A(MOE)^G(MOE)^A(MOE)^a^t^A(M)^c^a^A(M)^gcU(M) acU(M)tgU(M)tcU(M)ttU(M)tgC(M)agC(M)caC(M)cA(F)UGG(F)ACU(F)ACA(F)AGG (F)ACG(F)ACG(F)ACG(F)ACA(F)AGA(F)UCA(F)UCG(F)ACU(F)AUA(F)AAG(F)p | 414 |
| E216-2 | Solid Phase Synthesis | ACG(F)ACG(F)ACG(F)AUA(F)AAG(F)GUG(F)GCG(F)ACU(F)AUA(F)AGG(F)ACG( F)ACG(F)ACG(F)ACA(F)AAC(F)ACC(F)ACC(F)ACC(F)ACC(F)ACC(F)ACT(MOE)^ G(MOE)^A(MOE)^A(M)^A(M)^A(M)^A(M)^A(M)^A(M)^A(M)^A(M)^A(M)^A(M)^A(M) ^A(M)^A(M)^A(M)^A(M)^A(M)^A(M)^A(M)^A(M)^A(M) | 415 |

**[Table 2-58]**

| | | | |
|---|---|---|---|
| E220 | Same as Example 1 | GGGAGAAUACAAGCUACUUGUUCUUUUUGCAGCCACCAUGGACUACAAGGAC GACGACGACAAGAUCAUCGACUAUAAAGACGACGACGAUAAAGGUGGCGACU AUAAGGACGACGACGACAAACACCACCACCACCACCACUGAAAAAA | 448 |
| E220-1 | Solid Phase Synthesis | GGGAGAAUACAAGCUACUUGUUCUUUUUGCAGCCACCAUGGACUACAAGGAC GACGACGACAAGAUCAUCGACUAUAAAG | 449 |
| E220-2 | Solid Phase Synthesis | pACGACGACGAUAAAGGUGGCGACUAUAAGGACGACGACGACAAACACCACCA CCACCACCACUGAAAAAA | 450 |
| E221 | Same as Example 1 | GGGAGAAUACAAGCUACUUGUUCUUUUUGCAGCCACCAUGGACUACAAGGAC GACGACGACAAGAUCAUCGACUAUAAAGACGACGACGAUAAAGGUGGCGACU AUAAGGACGACGACGACAAACACCACCACCACCACCACUGAAAAAAAAAA | 451 |
| E221-1 | Solid Phase Synthesis | GGGAGAAUACAAGCUACUUGUUCUUUUUGCAGCCACCAUGGACUACAAGGAC GACGACGACAAGAUCAUCGACUAUAAAG | 452 |
| E221-2 | Solid Phase Synthesis | pACGACGACGAUAAAGGUGGCGACUAUAAGGACGACGACGACAAACACCACCA CCACCACCACUGAAAAAAAAAAA | 453 |
| E222 | Same as Example 2 | G(MOE)G(MOE)G(MOE)A(MOE)G(MOE)A(MOE)A(F)U(M)A(F)C(M)A(F)A(M)G(F)C (M)U(F)A(M)C(F)U(M)U(F)G(M)U(F)U(M)C(F)U(M)U(F)U(M)U(F)U(M)G(F)C(M)A(F) G(M)C(F)C(M)A(F)C(M)C(F)A(F)UGG(F)ACU(F)ACA(F)AGG(F)ACG(F)ACG(F)AC G(F)ACA(F)AGA(F)UCA(F)UCG(F)ACU(F)AUA(F)AAG(F)ACG(F)ACG(F)ACG(F)A UA(F)AAG(F)GUG(F)GCG(F)ACU(F)AUA(F)AGG(F)ACG(F)ACG(F)ACG(F)ACA(F) AAC(F)ACC(F)ACC(F)ACC(F)ACC(F)ACC(F)ACT(MOE)^G(MOE)^A(MOE)^A(M)^ A(M)^A(MOE)^A(MOE)^A(MOE) | 454 |
| E222-1 | Solid Phase Synthesis | G(MOE)G(MOE)G(MOE)A(MOE)G(MOE)A(MOE)A(F)U(M)A(F)C(M)A(F)A(M)G(F)C (M)U(F)A(M)C(F)U(M)U(F)G(M)U(F)U(M)C(F)U(M)U(F)U(M)U(F)U(M)G(F)C(M)A(F) G(M)C(F)C(M)A(F)C(M)C(F)A(F)UGG(F)ACU(F)ACA(F)AGG(F)ACG(F)ACG(F)AC G(F)ACA(F)AGA(F)UCA(F)UCG(F)ACU(F)AUA(F)AAG(F)p | 455 |
| E222-2 | Solid Phase Synthesis | ACG(F)ACG(F)ACG(F)AUA(F)AAG(F)GUG(F)GCG(F)ACU(F)AUA(F)AGG(F)ACG( F)ACG(F)ACG(F)ACA(F)AAC(F)ACC(F)ACC(F)ACC(F)ACC(F)ACC(F)ACT(MOE)^ G(MOE)^A(MOE)^A(M)^A(M)^A(MOE)^A(MOE)^A(MOE) | 456 |
| E223 | Same as Example 2 | G(MOE)G(MOE)G(MOE)A(MOE)G(MOE)A(MOE)A(F)U(M)A(F)C(M)A(F)A(M)G(F)C (M)U(F)A(M)C(F)U(M)U(F)G(M)U(F)U(M)C(F)U(M)U(F)U(M)G(F)C(M)A(F) G(M)C(F)C(M)A(F)C(M)C(F)A(F)UGG(F)ACU(F)ACA(F)AGG(F)ACG(F)ACG(F)AC G(F)ACA(F)AGA(F)UCA(F)UCG(F)ACU(F)AUA(F)AAG(F)ACG(F)ACG(F)ACG(F)A UA(F)AAG(F)GUG(F)GCG(F)ACU(F)AUA(F)AGG(F)ACG(F)ACG(F)ACG(F)ACA(F) AAC(F)ACC(F)ACC(F)ACC(F)ACC(F)ACC(F)ACT(MOE)^G(MOE)^A(MOE)^A(M)^ A(M)^A(M)^A(M)^A(M)^A(M)^A(M)^A(MOE)^A(MOE)^A(MOE) | 457 |
| E223-1 | Solid Phase Synthesis | G(MOE)G(MOE)G(MOE)A(MOE)G(MOE)A(MOE)A(F)U(M)A(F)C(M)A(F)A(M)G(F)C (M)U(F)A(M)C(F)U(M)U(F)G(M)U(F)U(M)C(F)U(M)U(F)U(M)U(F)U(M)G(F)C(M)A(F) G(M)C(F)C(M)A(F)C(M)C(F)A(F)UGG(F)ACU(F)ACA(F)AGG(F)ACG(F)ACG(F)AC G(F)ACA(F)AGA(F)UCA(F)UCG(F)ACU(F)AUA(F)AAG(F)p | 458 |
| E223-2 | Solid Phase Synthesis | ACG(F)ACG(F)ACG(F)AUA(F)AAG(F)GUG(F)GCG(F)ACU(F)AUA(F)AGG(F)ACG( F)ACG(F)ACG(F)ACA(F)AAC(F)ACC(F)ACC(F)ACC(F)ACC(F)ACC(F)ACT(MOE)^ G(MOE)^A(MOE)^A(M)^A(M)^A(M)^A(M)^A(M)^A(M)^A(M)^A(MOE)^A(MOE)^A(M OE) | 459 |

**[Table 2-59]**

| | | | |
|---|---|---|---|
| E224 | Same as Example 2 | G(MOE)G(MOE)G(MOE)A(MOE)G(MOE)A(MOE)A(F)U(M)A(F)C(M)A(F)A(M)G(F)C (M)U(F)A(M)C(F)U(M)U(F)G(M)U(F)U(M)C(F)U(M)U(F)U(M)U(F)U(M)G(F)C(M)A(F) G(M)C(F)C(M)A(F)C(M)C(F)A(F)UGG(F)ACU(F)ACA(F)AGG(F)ACG(F)ACG(F)AC G(F)ACA(F)AGA(F)UCA(F)UCG(F)ACU(F)AUA(F)AAG(F)ACG(F)ACG(F)ACG(F)A UA(F)AAG(F)GUG(F)GCG(F)ACU(F)AUA(F)AGG(F)ACG(F)ACG(F)ACG(F)ACA(F) AAC(F)ACC(F)ACC(F)ACC(F)ACC(F)ACC(F)ACT(MOE)^G(MOE)^A(MOE)^A(F)^A (M)^A(F)^A(M)^A(F)^A(M)^A(F)^A(M)^A(F)^A(M)^A(F)^A(M)^A(F)^A(M)^A(F)^A(M )^A(F)^A(MOE)^A(MOE)^A(MOE) | 460 |
| E224-1 | Solid Phase Synthesis | G(MOE)G(MOE)G(MOE)A(MOE)G(MOE)A(MOE)A(F)U(M)A(F)C(M)A(F)A(M)G(F)C (M)U(F)A(M)C(F)U(M)U(F)G(M)U(F)U(M)C(F)U(M)U(M)U(F)U(M)G(F)C(M)A(F) G(M)C(F)C(M)A(F)C(M)C(F)A(F)UGG(F)ACU(F)ACA(F)AGG(F)ACG(F)ACG(F)AC G(F)ACA(F)AGA(F)UCA(F)UCG(F)ACU(F)AUA(F)AAG(F)p | 461 |
| E224-2 | Solid Phase Synthesis | ACG(F)ACG(F)ACG(F)AUA(F)AAG(F)GUG(F)GCG(F)ACU(F)AUA(F)AGG(F)ACG( F)ACG(F)ACG(F)ACA(F)AAC(F)ACC(F)ACC(F)ACC(F)ACC(F)ACC(F)ACT(MOE)^ G(MOE)^A(MOE)^A(F)^A(M)^A(F)^A(M)^A(F)^A(M)^A(F)^A(M)^A(F)^A(M)^A(F)^A( M)^A(F)^A(M)^A(F)^A(M)^A(F)^A(MOE)^A(MOE)^A(MOE) | 462 |
| E225 | Same as Example 1 | G(MOE)G(MOE)G(MOE)A(MOE)G(MOE)A(MOE)A(F)U(M)A(F)C(M)A(F)A(M)G(F)C (M)U(F)A(M)C(F)U(M)U(F)G(M)U(F)U(M)C(F)U(M)U(F)U(M)U(F)U(M)G(F)C(M)A(F) G(M)C(F)C(M)A(F)C(M)C(F)AUGG(F)ACU(F)ACA(F)AGG(F)ACG(F)ACG(F)ACG(F )ACA(F)AGA(F)UCA(F)UCG(F)ACU(F)AUA(F)AAGACGACG(F)ACG(F)AUA(F)AA G(F)GUG(F)GCG(F)ACU(F)AUA(F)AGG(F)ACG(F)ACG(F)ACG(F)ACA(F)AAC(F)A CC(F)ACC(F)ACC(F)ACC(F)ACC(F)ACT(MOE)^G(MOE)^A(MOE)^A(M)^A(M)^A(M )^A(M)^A(M)^A(M)^A(M)^A(M)^A(M)^A(M)^A(M)^A(M)^A(M)^A(M)^A(M)^A(M)^A(M )^A(MOE)^A(MOE)^A(MOE) | 463 |
| E225-1 | Solid Phase Synthesis | G(MOE)G(MOE)G(MOE)A(MOE)G(MOE)A(MOE)A(F)U(M)A(F)C(M)A(F)A(M)G(F)C (M)U(F)A(M)C(F)U(M)U(F)G(M)U(F)U(M)C(F)U(M)U(F)U(M)U(F)U(M)G(F)C(M)A(F) | 464 |
| | | G(M)C(F)C(M)A(F)C(M)C(F)AUGG(F)ACU(F)ACA(F)AGG(F)ACG(F)ACG(F)ACG(F )ACA(F)AGA(F)UCA(F)UCG(F)ACU(F)AUA(F)AAG | |
| E225-2 | Solid Phase Synthesis | pACGACG(F)ACG(F)AUA(F)AAG(F)GUG(F)GCG(F)ACU(F)AUA(F)AGG(F)ACG(F) ACG(F)ACG(F)ACA(F)AAC(F)ACC(F)ACC(F)ACC(F)ACC(F)ACC(F)ACT(MOE)^G (MOE)^A(MOE)^A(M)^A(M)^A(M)^A(M)^A(M)^A(M)^A(M)^A(M)^A(M)^A(M)^A(M)^A (M)^A(M)^A(M)^A(M)^A(M)^A(M)^A(MOE)^A(MOE)^A(MOE) | 465 |
| E226 | Solid Phase Synthesis | GGGAGCCACCAUGGACUAUAAGGACGACGACGACAAAGGUGGCAGCCACCAC CACCACCACCACUGA | 466 |
| E227 | Solid Phase Synthesis | G(MOE)G(MOE)G(MOE)AGCCACCAUGGACUAUAAGGACGACGACGACAAAGGU GGCAGCCACCACCACCACCACCACT(MOE)G(MOE)A(MOE) | 467 |
| E228 | Solid Phase Synthesis | G(MOE)G(MOE)G(MOE)AGCCACCA(F)UGG(F)ACU(F)AUA(F)AGG(F)ACG(F)ACG (F)ACG(F)ACA(F)AAG(F)GUG(F)GCA(F)GCC(F)ACC(F)ACC(F)ACC(F)ACC(F)AC C(F)ACT(MOE)G(MOE)A(MOE) | 468 |
| E229 | Solid Phase Synthesis | G(MOE)G(MOE)G(MOE)AGCCACCAU(F)GGA(F)CUA(F)UAA(F)GGA(F)CGA(F)CG A(F)CGA(F)CAA(F)AGG(F)UGG(F)CAG(F)CCA(F)CCA(F)CCA(F)CCA(F)CCA(F)C CA(F)CT(MOE)G(MOE)A(MOE) | 469 |
| E230 | Solid Phase Synthesis | G(M)G(M)G(M)A(M)G(M)A(M)AUACAAGCUACUUGUUCUUUUUGCAGCCACCAUG GACUAUAAGGACGACGACGACAAAGGUGGCCACCACCACCACCACCACU(M)G( M)A(M) | 470 |
| E231 | Solid Phase Synthesis | G(M)G(M)G(M)A(M)G(M)A(M)AUACAAGCUACUUGUUCUUUUUGCAGCCACCA(F) UGG(F)ACU(F)AUA(F)AGG(F)ACG(F)ACG(F)ACG(F)ACA(F)AAG(F)GUG(F)GCC( F)ACC(F)ACC(F)ACC(F)ACC(F)ACC(F)ACU(M)G(M)A(M) | 471 |
| E232 | Solid Phase Synthesis | G(M)G(M)G(M)A(M)G(M)A(M)AUACAAGCUACUUGUUCUUUUUGCAGCCACCAU( F)GGA(F)CUA(F)UAA(F)GGA(F)CGA(F)CGA(F)CGA(F)CAA(F)AGG(F)UGG(F)CC A(F)CCA(F)CCA(F)CCA(F)CCA(F)CCA(F)CU(M)G(M)A(M) | 472 |

**[Table 2-60]**

| | | | |
|---|---|---|---|
| E233 | Solid Phase Synthesis | G(M)G(M)G(M)A(M)G(M)A(M)AUACAAGCUACUUGUUCUUUUUGCAGCCACCAUG (F)GAC(F)UAU(F)AAG(F)GAC(F)GAC(F)GAC(F)GAC(F)AAA(F)GGU(F)GGC(F)CA C(F)CAC(F)CAC(F)CAC(F)CAC(F)CAC(F)U(M)G(M)A(M) | 473 |
| E234 | Solid Phase Synthesis | G(M)G(M)G(M)A(M)G(M)A(M)AUACAAGCUACUUGUUCUUUUUGCAGCCACCaUG gACtAUaAGgACgACgACgACaAAgGUgGCcACcACcACcACcACcACU(M)G(M)A(M ) | 474 |
| E235 | Solid Phase Synthesis | G(MOE)G(MOE)G(MOE)A(MOE)G(MOE)A(MOE)AUACAAGCUACUUGUUCUUUUU GCAGCCACCAUGGACUAUAAGGACGACGACGACAAAGGUGGCCACCACCACC ACCACCACT(MOE)G(MOE)A(MOE) | 475 |
| E236 | Solid Phase Synthesis | G(F)G(F)G(F)A(F)G(F)A(F)AUACAAGCUACUUGUUCUUUUUGCAGCCACCAUGG ACUAUAAGGACGACGACGACAAAGGUGGCCACCACCACCACCACCACU(F)G(F) A(F) | 476 |
| E237 | Solid Phase Synthesis | gggagaAUACAAGCUACUUGUUCUUUUUGCAGCCACCAUGGACUAUAAGGACGA CGACGACAAAGGUGGCCACCACCACCACCACCACtga | 477 |
| E238 | Solid Phase Synthesis | G(M)^G(M)^G(M)A(M)G(M)A(M)AUACAAGCUACUUGUUCUUUUUGCAGCCACCA UGGACUAUAAGGACGACGACGACAAAGGUGGCCACCACCACCACCACCACU(M )^G(M)^A(M) | 478 |
| E239 | Solid Phase Synthesis | G(MOE)^G(MOE)^G(MOE)A(MOE)G(MOE)A(MOE)AUACAAGCUACUUGUUCUUUU UGCAGCCACCAUGGACUAUAAGGACGACGACGACAAAGGUGGCCACCACCAC CACCACCACT(MOE)^G(MOE)^A(MOE) | 479 |
| E240 | Solid Phase Synthesis | g^g^gagaAUACAAGCUACUUGUUCUUUUUGCAGCCACCAUGGACUAUAAGGACG ACGACGACAAAGGUGGCCACCACCACCACCACCACt^g^a | 480 |

Table 3-1 to Table 3-14:

**[Table 3-1]**

| Compound Name | SEQ ID NO: | Yield (%) | MS (found value) | MS (calculated value) |
|---|---|---|---|---|
| E3 | 8 | 42 | | |
| E3-1 | 9 | | 25741.90 | 25738.44 |
| E3-2 | 10 | | 21023.11 | 21020.70 |
| E4 | 11 | 35 | | |
| E4-1 | 12 | | 25742.31 | 25738.44 |
| E4-2 | 13 | | 27607.82 | 27604.90 |
| E5 | 14 | 35 | | |
| E5-1 | 15 | | 25851.38 | 25848.62 |
| E5-2 | 16 | | 21104.22 | 21100.79 |
| E6 | 17 | 38 | | |
| E6-1 | 18 | | 26032.49 | 26028.98 |
| E6-2 | 19 | | 21023.19 | 21020.70 |
| E7 | 20 | 34 | | |
| E7-1 | 21 | | 25826.35 | 25822.62 |
| E7-2 | 22 | | 27650.91 | 27646.99 |
| E8 | 23 | 39 | | |
| E8-1 | 24 | | 25826.58 | 25822.62 |
| E8-2 | 25 | | 27769.59 | 27765.23 |
| E9 | 26 | 35 | | |
| E9-1 | 27 | | 25826.44 | 25822.62 |
| E9-2 | 28 | | 27752.63 | 27747.17 |
| E10 | 29 | 34 | | |
| E10-1 | 30 | | 26116.33 | 26112.92 |
| E10-2 | 31 | | 21249.39 | 21246.97 |
| E11 | 32 | 31 | | |
| E11-1 | 33 | | 26116.06 | 26112.92 |
| E11-2 | 34 | | 27834.62 | 27831.17 |
| E12 | 35 | 31 | | |
| E12-1 | 36 | | 26148.32 | 26145.06 |
| E12-2 | 37 | | 28088.14 | 28081.69 |

**[Table 3-2]**

| | | | | |
|---|---|---|---|---|
| E13 | 38 | 34 | | |
| E13-1 | 39 | | 25851.92 | 25848.62 |
| E13-2 | 40 | | 27688.31 | 27684.99 |
| E14 | 41 | 34 | | |
| E14-1 | 42 | | 25851.92 | 25848.62 |
| E14-2 | 43 | | 27728.18 | 27724.99 |
| E15 | 44 | 34 | | |
| E15-1 | 45 | | 25851.99 | 25848.62 |
| E15-2 | 46 | | 27968.93 | 27965.59 |
| E16 | 47 | 26 | | |
| E16-1 | 48 | | 25852.02 | 25848.62 |
| E16-2 | 49 | | 27848.67 | 27845.29 |
| E17 | 50 | 27 | | |
| E17-1 | 51 | | 25851.88 | 25848.62 |
| E17-2 | 52 | | 28169.48 | 28166.69 |
| E18 | 53 | 36 | | |
| E18-1 | 54 | | 26203.38 | 26199.37 |
| E18-2 | 55 | | 21103.36 | 21100.79 |
| E19 | 56 | 40 | | |
| E19-1 | 57 | | 26315.40 | 26311.53 |
| E19-2 | 58 | | 21103.32 | 21100.79 |
| E20 | 59 | 42 | | |
| E20-1 | 60 | | 26218.93 | 26215.29 |
| E20-2 | 61 | | 21103.24 | 21100.79 |
| E21 | 62 | 40 | | |
| E21-1 | 63 | | 25968.41 | 25964.74 |
| E21-2 | 64 | | 21103.18 | 21100.79 |
| E22 | 65 | 37 | | |
| E22-1 | 66 | | 25985.36 | 25980.74 |
| E22-2 | 67 | | 21103.28 | 21100.79 |
| E23 | 68 | 17 | | |
| E23-1 | 69 | | 26287.55 | 26283.55 |
| E23-2 | 70 | | 21103.34 | 21100.79 |

**[Table 3-3]**

| | | | | |
|---|---|---|---|---|
| E24 | 71 | 27 | | |
| E24-1 | 72 | | 26413.24 | 26409.73 |
| E24-2 | 73 | | 21103.10 | 21100.79 |
| E25 | 74 | 42 | | |
| E25-1 | 75 | | 26304.89 | 26301.46 |
| E25-2 | 76 | | 21103.24 | 21100.79 |
| E26 | 77 | 36 | | |
| E26-1 | 78 | | 25822.79 | 25822.62 |
| E26-2 | 79 | | 27745.91 | 27745.17 |
| E27 | 80 | 35 | | |
| E27-1 | 81 | | 25823.15 | 25822.62 |
| E27-2 | 82 | | 27735.83 | 27735.14 |
| E28 | 83 | 33 | | |
| E28-1 | 84 | | 25822.86 | 25822.62 |
| E28-2 | 85 | | 27723.52 | 27723.11 |
| E29 | 86 | 24 | | |
| E29-1 | 87 | | 25822.85 | 25822.62 |
| E29-2 | 88 | | 27763.43 | 27763.23 |
| E30 | 89 | | 29201.49 | 29198.50 |
| E31 | 90 | | | 29375.25 |
| E32 | 91 | 23 | | |
| E32-1 | 92 | | 26092.95 | 26091.07 |
| E32-2 | 93 | | 21101.35 | 21100.79 |
| E33 | 94 | 38 | | |
| E33-1 | 95 | | 26128.89 | 26127.16 |
| E33-2 | 96 | | 21101.26 | 21100.79 |
| E34 | 97 | 32 | | |
| E34-1 | 98 | | | 26199.34 |
| E34-2 | 99 | | | 21100.79 |
| E35 | 100 | 40 | | |
| E35-1 | 101 | | | 26247.46 |
| E35-2 | 102 | | | 21100.79 |

**[Table 3-4]**

| | | | | |
|---|---|---|---|---|
| E36 | 103 | 41 | | |
| E36-1 | 104 | | 26357.11 | 26355.37 |
| E36-2 | 105 | | 21248.01 | 21246.97 |
| E37 | 106 | 38 | | |
| E37-1 | 107 | | | 26683.82 |
| E37-2 | 108 | | | 21246.97 |
| E38 | 109 | 38 | | |
| E38-1 | 110 | | | 27410.87 |
| E38-2 | 111 | | | 21246.97 |
| E39 | 112 | 37 | | |
| E39-1 | 113 | | | 27837.49 |
| E39-2 | 114 | | | 21246.97 |
| E40 | 115 | 38 | | |
| E40-1 | 116 | | | 27002.21 |
| E40-2 | 117 | | | 21100.79 |
| E41 | 118 | 41 | | |
| E41-1 | 119 | | | 27246.77 |
| E41-2 | 120 | | | 21100.79 |
| E42 | 121 | 37 | | |
| E42-1 | 122 | | 26060.05 | 26054.98 |
| E42-2 | 123 | | 21101.34 | 21100.79 |
| E43 | 124 | 30 | | |
| E43-1 | 125 | | 26151.59 | 26151.40 |
| E43-2 | 126 | | 21101.31 | 21100.79 |
| E44 | 127 | 20 | | |
| E44-1 | 128 | | 26247.81 | 26247.82 |
| E44-2 | 129 | | 21101.31 | 21100.79 |
| E45 | 130 | 34 | | |
| E45-1 | 131 | | | 25848.62 |
| E45-2 | 132 | | | 27756.99 |
| E46 | 133 | 32 | | |
| E46-1 | 134 | | 25849.38 | 25848.62 |
| E46-2 | 135 | | 28847.69 | 28846.59 |

**[Table 3-5]**

| | | | | |
|---|---|---|---|---|
| E47 | 136 | | | 47580.38 |
| E48 | 137 | | | 47628.47 |
| E49 | 138 | | | 47658.53 |
| E50 | 139 | | | 47692.59 |
| E51 | 140 | 35 | | |
| E51-1 | 141 | | | 25934.17 |
| E51-2 | 142 | | | 21246.97 |
| E52 | 143 | 22 | | |
| E52-1 | 144 | | | 26711.98 |
| E52-2 | 145 | | | 21246.97 |
| E53 | 146 | 13 | | |
| E53-1 | 147 | | 26150.66 | 26145.06 |
| E53-2 | 148 | | 29341.39 | 29346.31 |
| E54 | 149 | 25 | | |
| E54-1 | 150 | | 26145.49 | 26145.06 |
| E54-2 | 151 | | 28582.21 | 28597.46 |
| E55 | 152 | 25 | | |
| E55-1 | 153 | | 26145.43 | 26145.06 |
| E55-2 | 154 | | 28491.66 | 28489.19 |
| E56 | 155 | 24 | | |
| E56-1 | 156 | | 26390.31 | 26387.51 |
| E56-2 | 157 | | 28490.44 | 28489.19 |
| E57 | 158 | 24 | | |
| E57-1 | 159 | | | 26688.90 |
| E57-2 | 160 | | | 28489.19 |
| E58 | 161 | 29 | | |
| E58-1 | 162 | | 26113.95 | 26112.92 |
| E58-2 | 163 | | 34417.04 | 34415.37 |
| E59 | 164 | 17 | | |
| E59-1 | 165 | | | 26145.06 |
| E59-2 | 166 | | | 35555.09 |

**[Table 3-6]**

| | | | | |
|---|---|---|---|---|
| E60 | 167 | 23 | | |
| E60-1 | 168 | | | 26592.48 |
| E60-2 | 169 | | | 28794.52 |
| E61 | 170 | | 29760.87 | 29769.25 |
| E62 | 171 | 34 | | |
| E62-1 | 172 | | | 26329.37 |
| E62-2 | 173 | | | 28097.65 |
| E63 | 174 | 33 | | |
| E63-1 | 175 | | | 26522.21 |
| E63-2 | 176 | | | 28451.19 |
| E64 | 177 | 19 | | |
| E64-1 | 178 | | 26438.71 | 26437.35 |
| E64-2 | 179 | | 28059.72 | 28057.67 |
| E65 | 180 | 17 | | |
| E65-1 | 181 | | 26438.81 | 26439.35 |
| E65-2 | 182 | | 28059.00 | 28057.67 |
| E66 | 183 | 34 | | |
| E66-1 | 184 | | 26482.87 | 26481.44 |
| E66-2 | 185 | | 28115.38 | 28113.79 |
| E67 | 186 | 35 | | |
| E67-1 | 187 | | 26633.16 | 26632.19 |
| E67-2 | 188 | | 28413.40 | 28411.21 |
| E68 | 189 | 28 | | |
| E68-1 | 190 | | 26858.18 | 26857.17 |
| E68-2 | 191 | | 28731.35 | 28732.61 |
| E69 | 192 | 35 | | |
| E69-1 | 193 | | 26357.11 | 26355.37 |
| E69-2 | 194 | | 27834.62 | 27831.17 |
| E70 | 195 | 28 | | |
| E70-1 | 196 | | 26390.31 | 26387.51 |
| E70-2 | 197 | | 28088.14 | 28081.69 |
| E71 | 198 | | 30044.72 | 30043.84 |
| E72 | 199 | | 28597.28 | 29596.55 |

**[Table 3-7]**

| | | | | |
|---|---|---|---|---|
| E73 | 200 | | 29533.43 | 29532.55 |
| E74 | 201 | | 29365.19 | 29364.39 |
| E75 | 202 | | 29529.48 | 29524.55 |
| E76 | 203 | | 29629.31 | 29628.63 |
| E77 | 204 | | | 29510.45 |
| E78 | 205 | | | 29510.39 |
| E79 | 206 | 41 | | |
| E79-1 | 207 | | 26473.07 | 26469.49 |
| E79-2 | 208 | | 28413.80 | 28411.21 |
| E80 | 209 | 39 | | |
| E80-1 | 210 | | 26473.07 | 26469.49 |
| E80-2 | 211 | | 27468.04 | 27465.33 |
| E81 | 212 | 35 | | |
| E81-1 | 213 | | 26473.07 | 26469.49 |
| E81-2 | 214 | | 27788.89 | 27786.73 |
| E82 | 215 | 39 | | |
| E82-1 | 216 | | 26473.07 | 26469.49 |
| E82-2 | 217 | | 28251.61 | 28249.21 |
| E83 | 218 | 36 | | |
| E83-1 | 219 | | 26473.07 | 26469.49 |
| E83-2 | 220 | | 28162.29 | 28159.12 |
| E84 | 221 | 34 | | |
| E84-1 | 222 | | 26551.48 | 26549.47 |
| E84-2 | 223 | | 28413.80 | 28411.21 |
| E85 | 224 | | | 29801.39 |
| E86 | 225 | | | 29590.50 |
| E87 | 226 | | | 30029.79 |
| E88 | 227 | | | 29508.41 |
| E89 | 228 | | | 29063.16 |
| E90 | 229 | | | 29061.12 |
| E91 | 230 | | | 28553.80 |
| E92 | 231 | | | 28472.50 |
| E93 | 232 | | | 30011.70 |

**[Table 3-8]**

| | | | | |
|---|---|---|---|---|
| E94 | 233 | | | 30606.29 |
| E95 | 234 | 37 | | |
| E95-1 | 235 | | 26473.07 | 26469.49 |
| E95-2 | 236 | | 29271.38 | 29268.33 |
| E96 | 237 | | 29789.00 | 29788.24 |
| E97 | 238 | | 29644.33 | 29643.40 |
| E98 | 239 | | 30036.67 | 30035.84 |
| E99 | 240 | | | 29833.53 |
| E100 | 241 | | | 30075.98 |
| E101 | 242 | | | 30638.43 |
| E102 | 243 | | | 30542.01 |
| E103 | 244 | | | 30172.40 |
| E104 | 245 | | 28940.81 | 28939.66 |
| E105 | 246 | | | 29487.04 |
| E106 | 247 | | 28829.04 | 28827.66 |
| E107 | 248 | | | 29455.04 |
| E108 | 249 | | 28534.84 | 28533.38 |
| E109 | 250 | | | 29370.96 |
| E110 | 251 | | | 29789.25 |
| E111 | 252 | | | 29789.25 |
| E112 | 253 | | | 29791.25 |
| E113 | 254 | | | 29909.55 |
| E114 | 255 | | | 29909.55 |
| E115 | 256 | | | 29923.58 |
| E116 | 257 | | | 29637.34 |
| E117 | 258 | | | 29679.40 |
| E118 | 259 | | | 29649.38 |
| E119 | 260 | | | 30420.20 |
| E120 | 261 | | | 29959.40 |
| E121 | 262 | | | 28945.66 |
| E122 | 263 | | | 29503.04 |
| E123 | 264 | | | 28833.66 |
| E124 | 265 | | | 28539.38 |

**[Table 3-9]**

| | | | | |
|---|---|---|---|---|
| E125 | 266 | | | 29809.25 |
| E126 | 267 | | | 29929.55 |
| E127 | 268 | | 29812.73 | 29811.25 |
| E128 | 269 | | | 29943.58 |
| E129 | 270 | | | 29951.55 |
| E130 | 271 | | 30081.52 | 30083.88 |
| E131 | 272 | | 29964.62 | 29963.58 |
| E132 | 273 | | | 29831.25 |
| E133 | 274 | | | 29929.55 |
| E134 | 275 | | | 30049.85 |
| E135 | 276 | | | 29931.55 |
| E136 | 277 | | | 30063.88 |
| E137 | 278 | | | 30071.85 |
| E138 | 279 | | | 30204.18 |
| E139 | 280 | | | 30083.88 |
| E140 | 281 | | | 29951.55 |
| E141 | 282 | | 29241.57 | 29240.29 |
| E142 | 283 | | 29121.04 | 29119.99 |
| E143 | 284 | | 29008.69 | 29007.99 |
| E144 | 285 | | 28714.90 | 28713.71 |
| E145 | 286 | | | 30027.76 |
| E146 | 287 | | | 29931.52 |
| E147 | 288 | | | 29845.37 |
| E148 | 289 | | | 29821.31 |
| E149 | 290 | | | 30027.76 |
| E150 | 291 | | | 29931.52 |
| E151 | 292 | | | 30027.76 |
| E152 | 293 | | | 29931.52 |
| E153 | 294 | | | 29657.77 |
| E154 | 295 | | 29933.20 | 29931.97 |
| E155 | 296 | | 29702.81 | 29701.82 |
| E156 | 297 | | | 29427.62 |
| E157 | 298 | | | 29929.98 |

**[Table 3-10]**

| | | | | |
|---|---|---|---|---|
| E158 | 299 | | | 29974.03 |
| E159 | 300 | | | 29699.83 |
| E160 | 301 | | | 29967.96 |
| E161 | 302 | | | 29773.81 |
| E162 | 303 | | | 29936.85 |
| E163 | 304 | | | 29741.75 |
| E164 | 305 | 29 | | |
| E164-1 | 306 | | 25935.53 | 25934.17 |
| E164-2 | 307 | | 34415.78 | 34415.37 |
| E165 | 308 | 38 | | |
| E165-1 | 309 | | 26277.38 | 26277.25 |
| E165-2 | 310 | | 28410.45 | 28411.21 |
| E166 | 311 | 22 | | |
| E166-1 | 312 | | 26502.04 | 26502.23 |
| E166-2 | 313 | | 28732.35 | 28732.61 |
| E167 | 314 | 25 | | |
| E167-1 | 315 | | 26519.56 | 26519.70 |
| E167-2 | 316 | | 28410.62 | 28411.21 |
| E168 | 317 | 30 | | |
| E168-1 | 318 | | 26744.10 | 26744.68 |
| E168-2 | 319 | | 28732.31 | 28732.61 |
| E169 | 320 | 9 | | |
| E169-1 | 321 | | 26098.47 | 26098.50 |
| E169-2 | 322 | | 28411.47 | 28411.21 |
| E170 | 323 | 11 | | |
| E170-1 | 324 | | 26324.51 | 26323.48 |
| E170-2 | 325 | | 28733.32 | 28732.61 |
| E171 | 326 | 18 | | |
| E171-1 | 327 | | 26745.90 | 26744.68 |
| E171-2 | 328 | | 28841.81 | 28840.88 |
| E172 | 329 | 14 | | |
| E172-1 | 330 | | 26197.62 | 26196.90 |
| E172-2 | 331 | | 28412.69 | 28411.21 |

**[Table 3-11]**

| | | | | |
|---|---|---|---|---|
| E173 | 332 | 13 | | |
| E173-1 | 333 | | 26439.77 | 28439.35 |
| E173-2 | 334 | | 28411.59 | 28411.21 |
| E174 | 335 | 10 | | |
| E174-1 | 336 | | 26018.71 | 26018.15 |
| E174-2 | 337 | | 28410.90 | 28411.21 |
| E175 | 338 | | | |
| E176 | 339 | | | |
| E177 | 340 | | | |
| E178 | 341 | | | |
| E179 | 342 | | | |
| E180 | 343 | 39 | | |
| E 180-1 | 344 | | 25848.81 | 25848.42 |
| E180-2 | 345 | | 27801.90 | 27801.40 |
| E181 | 346 | 38 | | |
| E181-1 | 347 | | 26072.74 | 26073.40 |
| E181-2 | 348 | | 28122.75 | 28122.80 |
| E182 | 349 | | | |
| E183 | 350 | 28 | | |
| E183-1 | 351 | | 29930.55 | 29930.73 |
| E183-2 | 352 | | 28410.81 | 28411.21 |
| E184 | 353 | 38 | | |
| E184-1 | 354 | | 30252.99 | 30253.33 |
| E184-2 | 355 | | 28410.44 | 28411.21 |
| E185 | 356 | 37 | | |
| E185-1 | 357 | | 30172.51 | 30172.98 |
| E185-2 | 358 | | 28410.83 | 28411.21 |
| E186 | 359 | | | |
| E187 | 360 | 26 | | |
| E187-1 | 361 | | 30824.36 | 30824.47 |
| E187-2 | 362 | | 28410.35 | 28411.21 |

**[Table 3-12]**

| | | | | |
|---|---|---|---|---|
| E188 | 363 | 33 | | |
| E188-1 | 364 | | 30743.93 | 30744.12 |
| E188-2 | 365 | | 28410.48 | 28411.21 |
| E189 | 366 | | | |
| E190 | 367 | 25 | | |
| E190-1 | 368 | | 17525.61 | 17525.85 |
| E190-2 | 369 | | 28410.36 | 28411.21 |
| E191 | 370 | 26 | | |
| E191-1 | 371 | | 17493.47 | 17493.71 |
| E191-2 | 372 | | 28410.96 | 28411.21 |
| E192 | 373 | | | |
| E193 | 374 | 20 | | |
| E193-1 | 375 | | 26216.77 | 26216.90 |
| E193-2 | 376 | | 27940.07 | 27939.43 |
| E194 | 377 | 32 | | |
| E194-1 | 378 | | 25373.27 | 25373.31 |
| E194-2 | 379 | | 27940.10 | 27939.43 |
| E195 | 380 | 32 | | |
| E195-1 | 381 | | 25930.83 | 25930.69 |
| E195-2 | 382 | | 27940.27 | 27939.43 |
| E196 | 383 | 30 | | |
| E196-1 | 384 | | 25930.59 | 25930.69 |
| E196-2 | 385 | | 27940.25 | 27939.43 |
| E197 | 386 | 32 | | |
| E197-1 | 387 | | 26216.56 | 26216.90 |
| E197-2 | 388 | | 27637.91 | 27637.23 |
| E198 | 389 | 37 | | |
| E198-1 | 390 | | 26216.96 | 26216.90 |
| E198-2 | 391 | | 27669.13 | 27669.21 |
| E199 | 392 | 44 | | |
| E199-1 | 393 | | 26216.79 | 26216.90 |
| E199-2 | 394 | | 27129.39 | 27128.77 |
| E200 | 395 | | 29860.16 | 29859.70 |

**[Table 3-13]**

| | | | | |
|---|---|---|---|---|
| E201 | 396 | | 29677.75 | 29676.50 |
| E202 | 397 | | 29589.55 | 29588.58 |
| E203 | 398 | | 29629.67 | 29628.60 |
| E204 | 399 | | | 29969.69 |
| E205 | 400 | | | 29837.46 |
| E206 | 401 | | | 29885.67 |
| E207 | 402 | | | 29855.57 |
| E208 | 403 | | | 29649.19 |
| E209 | 404 | | | 29753.17 |
| E210 | 405 | | 29637.84 | 29637.01 |
| E211 | 406 | | 29685.10 | 29685.22 |
| E212 | 407 | | 29623.80 | 29622.98 |
| E213 | 408 | | 29450.09 | 29448.74 |
| E214 | 409 | | 31162.10 | 31160.07 |
| E215 | 410 | | | |
| E215-1 | 411 | | | |
| E215-2 | 412 | | | |
| E216 | 413 | | | |
| E216-1 | 414 | | | |
| E216-2 | 415 | | | |

**[Table 3-14]**

| | | | | | |
|---|---|---|---|---|---|
| E220 | 448 | 51 | | | |
| E220-1 | 449 | | 25738.08 | | 25738.44 |
| E220-2 | 450 | | 22666.65 | | 22666.75 |
| E221 | 451 | 42 | | | |
| E221-1 | 452 | | 25738.02 | | 25738.44 |
| E221-2 | 453 | | 24312.63 | | 24312.80 |
| E222 | 454 | 35 | | | |
| E222-1 | 455 | | 26438.06 | | 26439.35 |
| E222-2 | 456 | | 23128.13 | | 23129.83 |
| E223 | 457 | 32 | | | |
| E223-1 | 458 | | 26438.10 | | 26439.35 |
| E223-2 | 459 | | 24925.92 | | 24926.38 |
| E224 | 460 | 31 | | | |
| E224-1 | 461 | | 26438.89 | | 26439.35 |
| E224-2 | 462 | | 28409.93 | | 28411.21 |
| E225 | 463 | 34 | | | |
| E225-1 | 464 | | 26354.29 | | 26355.37 |
| E225-2 | 465 | | 28583.28 | | 28597.46 |
| E226 | 466 | | 21600.06 | | 21599.05 |
| E227 | 467 | | 21684.46 | | 21683.23 |
| E228 | 468 | | 21720.56 | | 21719.23 |
| E229 | 469 | | 21719.88 | | 21719.23 |
| E230 | 470 | | 29325.79 | | 29324.77 |
| E231 | 471 | | 29360.60 | | 29358.77 |
| E232 | 472 | | 29359.83 | | 29358.77 |
| E233 | 473 | | 29359.41 | | 29358.77 |
| E234 | 474 | | 29067.47 | | 29066.87 |
| E235 | 475 | | 29736.14 | | 29735.25 |
| E236 | 476 | | 29217.34 | | 29216.50 |
| E237 | 477 | | 29069.55 | | 29068.58 |
| E238 | 478 | | 29389.66 | | 29389.05 |
| E239 | 479 | | 29800.31 | | 29799.53 |
| E240 | 480 | | 29133.00 | | 29132.86 |

Tables 4-1 to 4-3 below show sequence information of compounds (polynucleotides) used in Example 4.

Table 4-1 to Table 4-3:

**[Table 4-1]**

| Compound Name | Sequence (5' to 3') | SEQ ID NO: |
|---|---|---|
| E217 | CAUAAACCCUGGCGCGCUCGCGGGCCGGCACUCUUCUGGUCCCCACAGACU CAGAGAGAACCCACCAUGGACUACAAGGACGACGACGACAAGAUCAUCGAC UAUAAAGACGACGACGAUAAAGGUGGCGACUAUAAGGACGACGACGACAAA CACCACCACCACCACCACUGAAAAAAAAAAAAAAAAAAAAA | 416 |
| E217-1 | CAUAAACCCUGGCGCGCUCGCGGGCCGGCACUCUUCUGGUCCCCACAGACU CAGAGAGAACCCACCAUGGACUACAAGGAC | 417 |
| E217-2 | pGACGACGACAAGAUCAUCGACUAUAAAGACGACGACGAUAAAGGUGGCGAC UAUAAG | 418 |
| E217-3 | pGACGACGACGACAAACACCACCACCACCACCACUGAAAAAAAAAAAAAAAA AAAAA | 419 |
| Template DNA 2 | gatcttgtcgtcgtcgtccttgtagtccat | 420 |
| Template DNA 3 | tttgtcgtcgtcgtccttatagtcgccacc | 421 |

**[Table 4-2]**

| | | |
|---|---|---|
| E218 | C(MOE)^A(MOE)^T(MOE)^A(MOE)^A(MOE)^A(MOE)C(M)C(F)C(M)U(F)G(M)G(F) C(M)G(F)C(M)G(F)C(M)U(F)C(M)G(F)C(M)G(F)G(M)G(F)C(M)C(F)G(M)G(F)C(M) A(F)C(M)U(F)C(M)U(F)U(M)C(F)U(M)G(F)G(M)U(F)C(M)C(F)C(M)C(F)A(M)C(F)A (M)G(F)A(M)C(F)U(M)C(F)A(M)G(F)A(M)G(F)A(M)G(F)A(M)A(F)C(M)C(F)C(M)A( F)C(M)C(F)A(F)UGG(F)ACU(F)ACA(F)AGGACGACG(F)ACG(F)ACA(F)AGA(F)U CA(F)UCG(F)ACU(F)AUA(F)AAG(F)ACG(F)ACG(F)ACG(F)AUA(F)AAG(F)GUG(F )GCG(F)ACU(F)AUAAGGACG(F)ACG(F)ACG(F)ACA(F)AAC(F)ACC(F)ACC(F)A CC(F)ACC(F)ACC(F)ACT(MOE)^G(MOE)^A(MOE)^A(F)^A(M)^A(F)^A(M)^A(F)^A (M)^A(F)^A(M)^A(F)^A(M)^A(F)^A(M)^A(F)^A(M)^A(F)^A(M)^A(F)^A(MOE)^A(MO E)^A(MOE) | 422 |
| E218-1 | C(MOE)^A(MOE)^T(MOE)^A(MOE)^A(MOE)^A(MOE)C(M)C(F)C(M)U(F)G(M)G(F) C(M)G(F)C(M)G(F)C(M)U(F)C(M)G(F)C(M)G(F)G(M)G(F)C(M)C(F)G(M)G(F)C(M) A(F)C(M)U(F)C(M)U(F)U(M)C(F)U(M)G(F)G(M)U(F)C(M)C(F)C(M)C(F)A(M)C(F)A (M)G(F)A(M)C(F)U(M)C(F)A(M)G(F)A(M)G(F)A(M)G(F)A(M)A(F)C(M)C(F)C(M)A( F)C(M)C(F)A(F)UGG(F)ACU(F)ACA(F)AGGAC | 423 |
| E218-2 | pGACG(F)ACG(F)ACA(F)AGA(F)UCA(F)UCG(F)ACU(F)AUA(F)AAG(F)ACG(F)A CG(F)ACG(F)AUA(F)AAG(F)GUG(F)GCG(F)ACU(F)AUAAG | 424 |
| E218-3 | pGACG(F)ACG(F)ACG(F)ACA(F)AAC(F)ACC(F)ACC(F)ACC(F)ACC(F)ACC(F)A CT(MOE)^G(MOE)^A(MOE)^A(F)^A(M)^A(F)^A(M)^A(F)^A(M)^A(F)^A(M)^A(F)^A (M)^A(F)^A(M)^A(F)^A(M)^A(F)^A(M)^A(F)^A(MOE)^A(MOE)^A(MOE) | 425 |

**[Table 4-3]**

| | | |
|---|---|---|
| E219 | C(MOE)A(MOE)T(MOE)A(MOE)A(MOE)A(MOE)C(M)C(F)C(M)U(F)G(M)G(F)C(M) G(F)C(M)G(F)C(M)U(F)C(M)G(F)C(M)G(F)G(M)G(F)C(M)C(F)G(M)G(F)C(M)A(F) C(M)U(F)C(M)U(F)U(M)C(F)U(M)G(F)G(M)U(F)C(M)C(F)C(M)C(F)A(M)C(F)A(M) G(F)A(M)C(F)U(M)C(F)A(M)G(F)A(M)G(F)A(M)G(F)A(M)A(F)C(M)C(F)C(M)A(F)C (M)C(F)A(F)UGG(F)ACU(F)ACA(F)AGGACGACG(F)ACG(F)ACA(F)AGA(F)UCA( F)UCG(F)ACU(F)AUA(F)AAG(F)ACG(F)ACG(F)ACG(F)AUA(F)AAG(F)GUG(F)GC G(F)ACU(F)AUAAGGACG(F)ACG(F)ACG(F)ACA(F)AAC(F)ACC(F)ACC(F)ACC( F)ACC(F)ACC(F)ACT(MOE)^G(MOE)^A(MOE)^A(F)^A(M)^A(F)^A(M)^A(F)^A(M) ^A(F)^A(M)^A(F)^A(M)^A(F)^A(M)^A(F)^A(M)^A(F)^A(M)^A(F)^A(MOE)^A(MOE)^ A(MOE) | 426 |
| E219-1 | C(MOE)A(MOE)T(MOE)A(MOE)A(MOE)A(MOE)C(M)C(F)C(M)U(F)G(M)G(F)C(M) G(F)C(M)G(F)C(M)U(F)C(M)G(F)C(M)G(F)G(M)G(F)C(M)C(F)G(M)G(F)C(M)A(F) C(M)U(F)C(M)U(F)U(M)C(F)U(M)G(F)G(M)U(F)C(M)C(F)C(M)C(F)A(M)C(F)A(M) G(F)A(M)C(F)U(M)C(F)A(M)G(F)A(M)G(F)A(M)G(F)A(M)A(F)C(M)C(F)C(M)A(F)C (M)C(F)A(F)UGG(F)ACU(F)ACA(F)AGGAC | 427 |
| E219-2 | pGACG(F)ACG(F)ACA(F)AGA(F)UCA(F)UCG(F)ACU(F)AUA(F)AAG(F)ACG(F)A CG(F)ACG(F)AUA(F)AAG(F)GUG(F)GCG(F)ACU(F)AUAAG | 428 |
| E219-3 | pGACG(F)ACG(F)ACG(F)ACA(F)AAC(F)ACC(F)ACC(F)ACC(F)ACC(F)ACC(F)A CT(MOE)^G(MOE)^A(MOE)^A(F)^A(M)^A(F)^A(M)^A(F)^A(M)^A(F)^A(M)^A(F)^A (M)^A(F)^A(M)^A(F)^A(M)^A(F)^A(M)^A(F)^A(MOE)^A(MOE)^A(MOE) | 429 |

### Example 4 (Linking of Three Fragments with Enzyme)

An RNA ligation product E217 (8.9 nmol, yield: 45%) was obtained in the same manner as in Example 1 except that RNA fragments E217-1, E217-2, and E217-3 obtained by solid phase synthesis, and templates DNA2 and DNA3 were simultaneously used.

An RNA ligation product E218 (2.6 nmol, yield: 13%) was obtained in the same manner as in Example 1 except that RNA fragments E218-1, E218-2, and E218-3 obtained by solid phase synthesis, and templates DNA2 and DNA3 were simultaneously used.

An RNA ligation product E219 (1.4 nmol, yield: 7%) was obtained in the same manner as in Example 1 except that RNA fragments E219-1, E219-2, and E219-3 obtained by solid phase synthesis, and templates DNA2 and DNA3 were simultaneously used.

### Test Example 1

### (Translation Reaction Test of mRNA Sample with Hela Cell Lysate)

Respective mRNAs shown in Tables 5-1 to 5-25 below were evaluated for translation activity in a human cell system with 1-Step Human Coupled IVT Kit (manufactured by Thermo Fisher Scientific K.K., Catalog No. 88882). First, each mRNA diluted to a final concentration of 0.3 µM with THE RNA storage solution (Thermo Fisher Scientific K.K., Catalog No. AM7001) was dispensed into a 96 well PCR plate (manufactured by As One Corporation) by 1 µL each. Subsequently, a master mix was prepared by mixing 5.0 µL per reaction of Hela Lysate, 1.0 µL per reaction of Accessory Proteins, 2.0 µL per reaction of Reaction Mix, 0.2 µL per reaction of RNase Inhibitor, Murine (manufactured by New England BioLabs, Inc., Catalog No. M0314), and 0.8 µL per reaction of purified water, and the resultant was dispensed by 9 µL each into the PCR plate to which the mRNA sample had been added, and after addition and mixture, the resultant was allowed to stand still at 37°C for 45 minutes to perform a translation reaction.

A translation product in a reaction solution after the translation reaction was detected by the following sandwich ELISA: First, 6*His, His-Tag antibody (Proteintech Group, Inc., Catalog No. 66005-1-Ig) was diluted with 0.1 M carbonate buffer (pH 9.4) to 3 µg/mL, and the resultant was dispensed into a 96 well ELISA plate (manufactured by Nunc Inc.) by 50 µL per well, and allowed to stand still at 4°C overnight, and thus, a plate in which the antibody was immobilized was produced. Subsequently, the plate was washed with Tris Buffered Saline with Tween 20 (Santa Cruz Biotechnology, Catalog No. sc-24953) diluted 1x concentration with purified water (hereinafter referred to as the washing solution), and then, a washing solution obtained by diluting bovine serum albumin (Wako Pure Chemical Industries Ltd., Catalog No. 017-22231) to a final concentration of 3% (hereinafter referred to as the blocking solution) was dispensed thereinto by 200 µL per well, and the resultant was allowed to stand still at room temperature for 1 hour. After washing the plate with the washing solution, the translation reaction solution diluted with the blocking solution was dispensed thereinto by 50 µL per well, and the resultant was allowed to stand still at room temperature for 1 hour. At this point, a translation product polypeptide preparation described below (manufactured by Cosmo Bio Co., Ltd.) was similarly diluted to each concentration with the blocking solution, and the resultant was dispensed into the plate. After washing the plate with the washing solution, Monoclonal ANTI-FLAG M2-Peroxidase (HRP) Ab produced in mouse (manufactured by SIGMA, Catalog Antibody A8592-1MG) diluted 10,000 fold with the blocking solution was dispensed thereinto by 50 µL per well, and the resultant was allowed to stand still at room temperature for 1 hour. After washing the plate with the washing solution, 1-Step Ultra TMB-ELISA (Thermo Fisher Scientific K.K., Catalog No. 34028) was dispensed thereinto by 50 µL per well, and the resultant was allowed to stand still at room temperature for several minutes. Thereafter, 0.5 M sulfuric acid (manufactured by Wako Pure Chemical Industries Ltd.) was dispensed thereinto by 50 µL per well to stop the reaction, and then, absorbances at a measurement wavelength of 450 nm and a reference wavelength of 570 nm were measured with an absorptiometer (manufactured by BIORAD). A translation product concentration (nM) in each translation reaction solution quantitatively determined with a calibration curve created based on the absorbances of the polypeptide preparation, and a relative amount of the translation product calculated assuming that the amount obtained from compound E3 having no sugar modification is 1 are shown in the following Table 5:
Translation product polypeptide preparation:
NH₂-MDYKDDDDKIIDYKDDDDKGGDYKDDDDKHHHHHH-COOH (SEQ ID NO: 430)

Table 5-1 to Table 5-25:

**[Table 5-1]**

| Translation product concentration obtained from each mRNA | | | |
|---|---|---|---|
| mRNA Name | SEQ ID NO: | Translation Product Concentration (nM) | Relative Amount of Translation Product |
| E5 | 14 | 2.047 | 1.84 |
| E13 | 38 | 7.100 | 6.38 |
| E14 | 41 | 7.700 | 6.92 |
| E15 | 44 | 6.713 | 6.03 |
| E16 | 47 | 7.420 | 6.66 |
| E17 | 50 | 11.187 | 10.05 |
| E3 | 8 | 1.113 | 1.00 |

**[Table 5-2]**

| Translation product concentration obtained from each mRNA | | | |
|---|---|---|---|
| mRNA Name | SEQ ID NO: | Translation Product Concentration (nM) | Relative Amount of Translation Product |
| E5 | 14 | 2.420 | 2.16 |
| E18 | 53 | 0.860 | 0.77 |
| E19 | 56 | 1.693 | 1.51 |
| E20 | 59 | 1.613 | 1.44 |
| E3 | 8 | 1.120 | 1.00 |

**[Table 5-3]**

| Translation product concentration obtained from each mRNA | | | |
|---|---|---|---|
| mRNA Name | SEQ ID NO: | Translation Product Concentration (nM) | Relative Amount of Translation Product |
| E5 | 14 | 0.867 | 8.67 |
| E21 | 62 | 2.653 | 26.53 |
| E22 | 65 | 3.293 | 32.93 |
| E3 | 8 | 0.100 | 1.00 |

**[Table 5-4]**

| Translation product concentration obtained from each mRNA | | | |
|---|---|---|---|
| mRNA Name | SEQ ID NO: | Translation Product Concentration (nM) | Relative Amount of Translation Product |
| E5 | 14 | 0.980 | 8.17 |
| E18 | 53 | 0.333 | 2.78 |
| E20 | 59 | 0.727 | 6.06 |
| E23 | 68 | 0.133 | 1.11 |
| E24 | 71 | 0.420 | 3.50 |
| E25 | 74 | 0.407 | 3.39 |
| E3 | 8 | 0.120 | 1.00 |

**[Table 5-5]**

| Translation product concentration obtained from each mRNA | | | |
|---|---|---|---|
| mRNA Name | SEQ ID NO: | Translation Product Concentration (nM) | Relative Amount of Translation Product |
| E4 | 11 | 1.973 | 1.48 |
| E7 | 20 | 3.807 | 2.86 |
| E26 | 77 | 4.607 | 3.46 |
| E27 | 80 | 7.433 | 5.58 |
| E28 | 83 | 3.727 | 2.80 |
| E29 | 86 | 7.140 | 5.36 |
| E3 | 8 | 1.333 | 1.00 |

**[Table 5-6]**

| Translation product concentration obtained from each mRNA | | | |
|---|---|---|---|
| mRNA Name | SEQ ID NO: | Translation Product Concentration (nM) | Relative Amount of Translation Product |
| E5 | 14 | 3.740 | 3.17 |
| E23 | 68 | 0.687 | 0.58 |
| E32 | 91 | 1.240 | 1.05 |
| E33 | 94 | 1.800 | 1.53 |
| E34 | 97 | 0.960 | 0.81 |
| E35 | 100 | 0.767 | 0.65 |
| E3 | 8 | 1.180 | 1.00 |

**[Table 5-7]**

| Translation product concentration obtained from each mRNA | | | |
|---|---|---|---|
| mRNA Name | SEQ ID NO: | Translation Product Concentration (nM) | Relative Amount of Translation Product |
| E10 | 29 | 2.813 | 1.91 |
| E32 | 91 | 1.040 | 0.71 |
| E36 | 103 | 0.953 | 0.65 |
| E37 | 106 | 0.313 | 0.21 |
| E38 | 109 | 0.027 | 0.02 |
| E39 | 112 | 0.020 | 0.01 |
| E3 | 8 | 1.473 | 1.00 |

**[Table 5-8]**

| Translation product concentration obtained from each mRNA | | | |
|---|---|---|---|
| mRNA Name | SEQ ID NO: | Translation Product Concentration (nM) | Relative Amount of Translation Product |
| E6 | 17 | 0.720 | 0.96 |
| E40 | 115 | 0.287 | 0.38 |
| E41 | 118 | 0.073 | 0.10 |
| E42 | 121 | 1.487 | 1.99 |
| E43 | 124 | 3.467 | 4.64 |
| E44 | 127 | 3.800 | 5.09 |
| E3 | 8 | 0.747 | 1.00 |

**[Table 5-9]**

| Translation product concentration obtained from each mRNA | | | |
|---|---|---|---|
| mRNA Name | SEQ ID NO: | Translation Product Concentration (nM) | Relative Amount of Translation Product |
| E10 | 29 | 3.087 | 2.91 |
| E36 | 103 | 0.993 | 0.94 |
| E47 | 136 | 0.373 | 0.35 |
| E48 | 137 | 0.227 | 0.21 |
| E49 | 138 | 0.953 | 0.90 |
| E50 | 139 | 0.620 | 0.58 |
| E3 | 8 | 1.060 | 1.00 |

**[Table 5-10]**

| Translation product concentration obtained from each mRNA | | | |
|---|---|---|---|
| mRNA Name | SEQ ID NO: | Translation Product Concentration (nM) | Relative Amount of Translation Product |
| E4 | 11 | 3.133 | 15.67 |
| E15 | 44 | 67.533 | 337.67 |
| E45 | 130 | 35.400 | 177.00 |
| E46 | 133 | 77.333 | 386.67 |
| E3 | 8 | 0.200 | 1.00 |

**[Table 5-11]**

| Translation product concentration obtained from each mRNA | | | |
|---|---|---|---|
| mRNA Name | SEQ ID NO: | Translation Product Concentration (nM) | Relative Amount of Translation Product |
| E4 | 11 | 0.933 | 4.67 |
| E12 | 35 | 119.467 | 597.33 |
| E55 | 152 | 84.067 | 420.33 |
| E56 | 155 | 34.800 | 174.00 |
| E57 | 158 | 76.200 | 381.00 |
| E60 | 167 | 51.800 | 259.00 |
| E3 | 8 | 0.200 | 1.00 |

**[Table 5-12]**

| Translation product concentration obtained from each mRNA | | | |
|---|---|---|---|
| mRNA Name | SEQ ID NO: | Translation Product Concentration (nM) | Relative Amount of Translation Product |
| E11 | 32 | 40.667 | 203.33 |
| E55 | 152 | 73.600 | 368.00 |
| E58 | 161 | 41.933 | 209.67 |
| E59 | 164 | 72.867 | 364.33 |
| E3 | 8 | 0.200 | 1.00 |

**[Table 5-13]**

| Translation product concentration obtained from each mRNA | | | |
|---|---|---|---|
| mRNA Name | SEQ ID NO: | Translation Product Concentration (nM) | Relative Amount of Translation Product |
| E10 | 29 | 6.333 | 12.67 |
| E36 | 103 | 0.667 | 1.33 |
| E47 | 136 | 0.333 | 0.67 |
| E49 | 138 | 0.500 | 1.00 |
| E51 | 140 | 16.667 | 33.33 |
| E52 | 143 | 0.833 | 1.67 |
| E3 | 8 | 0.500 | 1.00 |

**[Table 5-14]**

| Translation product concentration obtained from each mRNA | | | |
|---|---|---|---|
| mRNA Name | SEQ ID NO: | Translation Product Concentration (nM) | Relative Amount of Translation Product |
| E10 | 29 | 5.500 | 11.00 |
| E11 | 32 | 39.000 | 78.00 |
| E12 | 35 | 118.167 | 236.33 |
| E53 | 146 | 78.000 | 156.00 |
| E54 | 149 | 104.167 | 208.33 |
| E55 | 152 | 83.167 | 166.33 |
| E3 | 8 | 0.500 | 1.00 |

**[Table 5-15]**

| Translation product concentration obtained from each mRNA | | | |
|---|---|---|---|
| mRNA Name | SEQ ID NO: | Translation Product Concentration (nM) | Relative Amount of Translation Product |
| E4 | 11 | 1.833 | 3.67 |
| E12 | 35 | 228.167 | 456.33 |
| E55 | 152 | 87.000 | 174.00 |
| E56 | 155 | 49.333 | 98.67 |
| E70 | 195 | 72.333 | 144.67 |
| E3 | 8 | 0.500 | 1.00 |

**[Table 5-16]**

| Translation product concentration obtained from each mRNA | | | |
|---|---|---|---|
| mRNA Name | SEQ ID NO: | Translation Product Concentration (nM) | Relative Amount of Translation Product |
| E4 | 11 | 1.833 | 2.75 |
| E11 | 32 | 75.000 | 112.50 |
| E69 | 192 | 15.167 | 22.75 |
| E3 | 8 | 0.667 | 1.00 |

**[Table 5-17]**

| Translation product concentration obtained from each mRNA | | | |
|---|---|---|---|
| mRNA Name | SEQ ID NO: | Translation Product Concentration (nM) | Relative Amount of Translation Product |
| E4 | 111 | 1.167 | 2.33 |
| E56 | 155 | 51.500 | 103.00 |
| E62 | 171 | 99.333 | 198.67 |
| E63 | 174 | 98.333 | 196.67 |
| E64 | 177 | 43.833 | 87.67 |
| E65 | 180 | 48.833 | 97.67 |
| E3 | 8 | 0.500 | 1.00 |

**[Table 5-18]**

| Translation product concentration obtained from each mRNA | | | |
|---|---|---|---|
| mRNA Name | SEQ ID NO: | Translation Product Concentration (nM) | Relative Amount of Translation Product |
| E4 | 11 | 1.333 | 2.67 |
| E10 | 29 | 6.167 | 12.33 |
| E47 | 136 | 0.500 | 1.00 |
| E51 | 140 | 19.667 | 39.33 |
| E58 | 161 | 59.000 | 118.00 |
| E164 | 305 | 109.000 | 218.00 |
| E3 | 8 | 0.500 | 1.00 |

**[Table 5-19]**

| Translation product concentration obtained from each mRNA | | | |
|---|---|---|---|
| mRNA Name | SEQ ID NO: | Translation Product Concentration (nM) | Relative Amount of Translation Product |
| E4 | 11 | 1.500 | 3.00 |
| E64 | 177 | 25.833 | 51.67 |
| E65 | 180 | 23.333 | 46.67 |
| E66 | 183 | 14.833 | 29.67 |
| E67 | 186 | 25.000 | 50.00 |
| E68 | 189 | 10.667 | 21.33 |
| E3 | 8 | 0.500 | 1.00 |

**[Table 5-20]**

| Translation product concentration obtained from each mRNA | | | |
|---|---|---|---|
| mRNA Name | SEQ ID NO: | Translation Product Concentration (nM) | Relative Amount of Translation Product |
| E4 | 11 | 1.500 | 3.00 |
| E56 | 155 | 32.500 | 65.00 |
| E2 | 5 | 22.000 | 44.00 |
| E67 | 188 | 21.333 | 42.67 |
| E79 | 206 | 16.833 | 33.67 |
| E95 | 234 | 20.833 | 41.67 |
| E3 | 8 | 0.500 | 1.00 |

**[Table 5-21]**

| Translation product concentration obtained from each mRNA | | | |
|---|---|---|---|
| mRNA Name | SEQ ID NO: | Translation Product Concentration (nM) | Relative Amount of Translation Product |
| E4 | 11 | 1.500 | 3.00 |
| E79 | 206 | 22.500 | 45.00 |
| E80 | 209 | 12.667 | 25.33 |
| E81 | 212 | 19.500 | 39.00 |
| E82 | 215 | 21.833 | 43.67 |
| E83 | 218 | 23.167 | 46.33 |
| E3 | 8 | 0.500 | 1.00 |

**[Table 5-22]**

| Translation product concentration obtained from each mRNA | | | |
|---|---|---|---|
| mRNA Name | SEQ ID NO: | Translation Product Concentration (nM) | Relative Amount of Translation Product |
| E4 | 11 | 1.000 | - |
| E165 | 308 | 42.000 | - |
| E166 | 311 | 29.667 | - |
| E167 | 314 | 19.167 | - |
| E169 | 320 | 37.167 | - |
| E172 | 329 | 28.833 | - |
| E3 | 8 | 0.000 | - |

**[Table 5-23]**

| Translation product concentration obtained from each mRNA | | | |
|---|---|---|---|
| mRNA Name | SEQ ID NO: | Translation Product Concentration (nM) | Relative Amount of Translation Product |
| E4 | 11 | 1.167 | - |
| E166 | 311 | 35.833 | - |
| E167 | 314 | 22.000 | - |
| E168 | 317 | 8.833 | - |
| E171 | 326 | 9.667 | - |
| E173 | 332 | 7.333 | - |
| E3 | 8 | 0.000 | - |

**[Table 5-24]**

| Translation product concentration obtained from each mRNA | | | |
|---|---|---|---|
| mRNA Name | SEQ ID NO: | Translation Product Concentration (nM) | Relative Amount of Translation Product |
| E4 | 11 | 1.667 | 5.00 |
| E165 | 308 | 63.667 | 191.00 |
| E167 | 314 | 21.833 | 65.50 |
| E169 | 320 | 55.500 | 166.50 |
| E170 | 323 | 35.833 | 107.50 |
| E174 | 335 | 38.167 | 114.50 |
| E3 | 8 | 0.333 | 1.00 |

**[Table 5-25]**

| Translation product concentration obtained from each mRNA | | | |
|---|---|---|---|
| mRNA Name | SEQ ID NO: | Translation Product Concentration (nM) | Relative Amount of Translation Product |
| E4 | 11 | 1.000 | 2.00 |
| E84 | 221 | 11.167 | 22.33 |
| E3 | 8 | 0.500 | 1.00 |

As is obvious from the test results shown in Tables 5-1 to 5-25, each mRNA having sugar modification produced, after being added to the Hela cell lysate, a polypeptide encoded by a gene sequence in the eukaryotic cell translation system.

### Test Example 2

### (in vitro Translation Reaction Test of mRNA Sample with Hela Cell Line)

The respective mRNAs shown in Tables 6-1 to 6-9 below were evaluated for translation activity *in vitro* with Hela cell line. First, a Hela cell suspended in RPMI medium (manufactured by Nacalai Tesque, Inc.) containing 10% fetal bovine serum was seeded in a 96 well adherent cell culture plate at 10,000 cells/100 µL per well, and the resultant was cultured at 37°C under 5% CO2 condition overnight. A culture supernatant was removed from the cell cultured overnight, RPMI medium containing 40 µL of 10% fetal bovine serum per well was added thereto, and each mRNA and Lipofectamin Messenger MAX Transfection Reagent (manufactured by Thermo Fisher Scientific K.K., Catalog No: LMRNA008) at a final concentration of 0.3% were diluted and mixed with Opti-MEM (manufactured by Thermo Fisher Scientific K.K., Catalog No: 31985-070) to a final concentration of 3 nM, 10 nM, and 30 nM of each mRNA, the resultant mixture was added to each culture plate in an amount of 10 µL per well, and the resultant was cultured at 37°C under 5% CO2 condition for 5 hours. A culture supernatant was removed from the cell cultured for 5 hours, the resultant was washed once with ice cooled D-PBS(-) (manufactured by Nacalai Tesque, Inc.), iScript RT-qPCR Sample Preparation Reagent (BIORAD, 1708898) containing 2% protease inhibitor cocktail (for an animal cell extract, manufactured by Nacalai Tesque, Inc.) was added in an amount of 20 µL per well, and the resultant was vigorously shaken for 30 seconds for cell lysis.

A translation product in a cell lysate thus obtained was detected by the sandwich ELISA method described in Test Example 1. As results of the measurement, a translation product concentration (nM) in each translation reaction solution quantitatively determined with a calibration curve created based on the absorbances of the polypeptide preparation is shown in the following Table 6:

Table 6-1 to Table 6-9:

**[Table 6-1]**

| Translation product concentration obtained from each mRNA | | | | |
|---|---|---|---|---|
| mRNA Name | SEQ ID NO: | Translation Product Concentration (nM) | | |
| | | mRNA 3 nM | mRNA10 nM | mRNA30 nM |
| E4 | 11 | 0.000 | 0.010 | 0.020 |
| E13 | 38 | 0.010 | 0.083 | 0.317 |
| E15 | 44 | 0.120 | 0.717 | 1.173 |
| E46 | 133 | 0.127 | 0.550 | 0.857 |

**[Table 6-2]**

| Translation product concentration obtained from each mRNA | | | | |
|---|---|---|---|---|
| mRNA Name | SEQ ID NO: | Translation Product Concentration (nM) | | |
| | | mRNA 3 nM | mRNA10 nM | mRNA30 nM |
| E12 | 35 | 0.077 | 1.030 | 1.367 |
| E55 | 152 | 0.183 | 0.893 | 1.697 |
| E56 | 155 | 0.233 | 1.113 | 1.847 |
| E70 | 195 | 0.053 | 0.580 | 1.123 |
| E4 | 11 | | | 0.010 |

**[Table 6-3]**

| Translation product concentration obtained from each mRNA | | | | |
|---|---|---|---|---|
| mRNA Name | SEQ ID NO: | Translation Product Concentration (nM) | | |
| | | mRNA 3 nM | mRNA10 nM | mRNA30 nM |
| E62 | 171 | 0.073 | 0.453 | 0.780 |
| E63 | 174 | 0.033 | 0.410 | 0.960 |
| E65 | 180 | 0.063 | 0.757 | 0.823 |
| E66 | 183 | 0.030 | 0.273 | 0.593 |

**[Table 6-4]**

| Translation product concentration obtained from each mRNA | | | | |
|---|---|---|---|---|
| mRNA Name | SEQ ID NO: | Translation Product Concentration (nM) | | |
| | | mRNA 3 nM | mRNA10 nM | mRNA30 nM |
| E10 | 29 | 0.000 | 0.010 | 0.010 |
| E51 | 140 | 0.000 | 0.010 | 0.013 |
| E58 | 161 | 0.010 | 0.180 | 0.347 |
| E164 | 305 | 0.010 | 0.160 | 0.343 |
| E4 | 11 | | | 0.010 |

**[Table 6-5]**

| Translation product concentration obtained from each mRNA | | | | |
|---|---|---|---|---|
| mRNA Name | SEQ ID NO: | Translation Product Concentration (nM) | | |
| | | mRNA 3 nM | mRNA10 nM | mRNA30 nM |
| E79 | 206 | 0.140 | 0.780 | 1.483 |
| E80 | 209 | 0.010 | 0.040 | 0.073 |
| E81 | 212 | 0.107 | 0.787 | 1.243 |
| E95 | 234 | 0.053 | 0.710 | 1.313 |
| E4 | 11 | | | 0.020 |

**[Table 6-6]**

| Translation product concentration obtained from each mRNA | | | | |
|---|---|---|---|---|
| mRNA Name | SEQ ID NO: | Translation Product Concentration (nM) | | |
| | | mRNA 3 nM | mRNA10 nM | mRNA30 nM |
| E79 | 206 | 0.117 | 0.803 | 1.503 |
| E81 | 212 | 0.100 | 0.787 | 1.223 |
| E82 | 215 | 0.120 | 0.757 | 1.350 |
| E83 | 218 | 0.130 | 0.780 | 1.377 |
| E4 | 11 | | | 0.020 |

**[Table 6-7]**

| Translation product concentration obtained from each mRNA | | | | |
|---|---|---|---|---|
| mRNA Name | SEQ ID NO: | Translation Product Concentration (nM) | | |
| | | mRNA 3 nM | mRNA10 nM | mRNA30 nM |
| E63 | 174 | 0.107 | 0.757 | 1.673 |
| E65 | 180 | 0.067 | 0.430 | 0.733 |
| E67 | 186 | 0.050 | 0.577 | 1.047 |
| E68 | 189 | 0.010 | 0.190 | 0.397 |
| E4 | 11 | 0.000 | 0.000 | 0.010 |

**[Table 6-8]**

| Translation product concentration obtained from each mRNA | | | | |
|---|---|---|---|---|
| mRNA Name | SEQ ID NO: | Translation Product Concentration (nM) | | |
| | | mRNA 3 nM | mRNA10 nM | mRNA30 nM |
| E56 | 155 | 0.143 | 0.947 | 1.610 |
| E64 | 177 | 0.117 | 0.553 | 0.670 |
| E2 | 5 | 0.063 | 0.483 | 0.920 |
| E79 | 206 | 0.063 | 0.300 | 0.903 |
| E4 | 11 | 0.000 | 0.000 | 0.010 |

**[Table 6-9]**

| Translation product concentration obtained from each mRNA | | | | |
|---|---|---|---|---|
| mRNA Name | SEQ ID NO: | Translation Product Concentration (nM) | | |
| | | mRNA 3 nM | mRNA10 nM | mRNA30 nM |
| E47 | 136 | 0.000 | 0.000 | 0.000 |
| E51 | 140 | 0.000 | 0.000 | 0.010 |
| E58 | 161 | 0.023 | 0.327 | 0.447 |
| E164 | 305 | 0.020 | 0.300 | 0.353 |
| E4 | 11 | 0.000 | 0.000 | 0.010 |

As is obvious from the test results shown in Tables 6-1 to 6-9, each mRNA having sugar modification produced, after being added to the Hela cell, a polypeptide encoded by a gene sequence, and the translation level was more excellent than that of an mRNA having no sugar modification.

### Test Example 3

### (in vitro Translation Reaction Test of mRNA Sample with Hela Cell Line)

Respective mRNAs shown in Tables 7-1 to 7-4 below were evaluated for persistence of translation activity *in vitro* with Hela cell line. The culture of cells and introduction of the mRNAs were performed in the same manner as in Test Example 2 except that each mRNA was prepared to a final concentration of 30 nM. A culture supernatant was removed from the cell cultured for 4 hours after adding each mRNA, RPMI medium (manufactured by Nacalai Tesque, Inc.) containing 50 µL of 10% fetal bovine serum per well was added thereto, and the culture was continued at 37°C under 5% CO2 condition. A culture supernatant was removed from each of the cells cultured respectively for 5 hours, 8 hours, and 24 hours after the addition of the mRNA, and the cells were lysed in the same manner as in Test Example 2.

A translation product in the thus obtained cell lysate was detected in the same manner as in the sandwich ELISA method described in Test Example 1. As results of the measurement, a translation product concentration (nM) in each translation reaction solution quantitatively determined with a calibration curve created based on the absorbances of the polypeptide preparation is shown in the following Table 7:
Table 7-1 to Table 7-4:

**[Table 7-1]**

| Translation product concentration obtained from each mRNA | | | | |
|---|---|---|---|---|
| mRNA Name | SEQ ID NO: | Translation Product Concentration (nM) | | |
| | | 5 Hours After Adding mRNA | 8 Hours After Adding mRNA | 24 Hours After Adding mRNA |
| E4 | 11 | 0.013 | 0.010 | 0.010 |
| E13 | 38 | 0.263 | 0.117 | 0.003 |
| E15 | 44 | 1.347 | 1.037 | 0.150 |
| E16 | 47 | 1.133 | 0.683 | 0.040 |
| E17 | 50 | 2.713 | 2.570 | 0.970 |
| E46 | 133 | 1.217 | 0.927 | 0.153 |

**[Table 7-2]**

| Translation product concentration obtained from each mRNA | | | | |
|---|---|---|---|---|
| mRNA Name | SEQ ID NO: | Translation Product Concentration (nM) | | |
| | | 5 Hours After Adding mRNA | 8 Hours After Adding mRNA | 24 Hours After Adding mRNA |
| E4 | 11 | 0.013 | 0.010 | 0.010 |
| E12 | 35 | 2.553 | 2.523 | 1.130 |
| E53 | 146 | 2.267 | 2.557 | 1.747 |
| E54 | 149 | 3.263 | 3.187 | 2.627 |
| E55 | 152 | 2.723 | 2.847 | 2.087 |
| E56 | 155 | 2.383 | 2.663 | 2.093 |
| E4 | 11 | 0.013 | 0.010 | 0.010 |

**[Table 7-3]**

| Translation product concentration obtained from each mRNA | | | | |
|---|---|---|---|---|
| mRNA Name | SEQ ID NO: | Translation Product Concentration (nM) | | |
| | | 5 Hours After Adding mRNA | 8 Hours After Adding mRNA | 24 Hours After Adding mRNA |
| E4 | 11 | 0.010 | 0.003 | 0.000 |
| E79 | 206 | 0.843 | 1.020 | 0.597 |
| E95 | 234 | 0.813 | 1.017 | 0.670 |

**[Table 7-4]**

| Translation product concentration obtained from each mRNA | | | | |
|---|---|---|---|---|
| mRNA Name | SEQ ID NO: | Translation Product Concentration (nM) | | |
| | | 5 Hours After Adding mRNA | 8 Hours After Adding mRNA | 24 Hours After Adding mRNA |
| E4 | 11 | 0.010 | 0.000 | 0.000 |
| E58 | 161 | 0.373 | 0.490 | 0.043 |
| E67 | 186 | 0.817 | 0.983 | 0.547 |
| E68 | 189 | 0.333 | 0.473 | 0.283 |
| E164 | 305 | 0.367 | 0.517 | 0.200 |

As is obvious from the test results shown in Tables 7-1 to 7-4, each mRNA having sugar modification produced, after being added to the Hela cell, a polypeptide encoded by a gene sequence, and the translation level was more excellent than that of an mRNA having no sugar modification.

### Test Example 4

### (in vitro Translation Reaction Test of mRNA Sample with Hela Cell Line)

The respective mRNAs shown in Tables 8-1 to 8-8 below were evaluated for translation activity *in vitro* with Hela cell line.

First, each mRNA was diluted with THE RNA Storage Solution (manufactured by Thermo Fisher Scientific K.K., Catalog No. AM7000) to a concentration of 19 µM. The Hela cell line was suspended in Opti-MEM I Reduced Serum Media (manufactured by Thermo Fisher Scientific K.K., Catalog No. 31985070) containing bovine serum albumin (manufactured by Wako Pure Chemical Industries Ltd., Catalog No. 017-22231) in a final concentration of 1%, the resultant was centrifuged at 90 xg at room temperature for 10 minutes, a supernatant was carefully removed, and then the resultant was suspended in a mixture of SE Cell Line Nucleofector Solution attached to 1% SE Cell Line 96-well Nucleofector Kit (manufactured by Lonza, Catalog No. V4SC-1096) and Supplement 1 in a concentration of 200,000 cells/19 µL. The mRNA solution and the Hela cell suspension thus prepared were mixed in a volume ratio of 1:19, and the resultant was subjected to electroporation with Nucleofector(TM) 96-well Shuttle system (Lonza) under pulse condition FF-150. The resultant cell obtained 10 minutes after the electroporation was suspended in RPMI medium (manufactured by Nacalai Tesque, Inc.) containing 10% fetal bovine serum, and the resultant was seeded in a 96 well adherent cell culture plate at 50,000 cells/145 µL per well, followed by culturing at 37°C under 5% CO2 condition. A culture supernatant was removed from each of the cells cultured respectively for 3 hours, 8 hours, and 24 hours, the resultant was washed once with ice cooled D-PBS(-) (manufactured by Nacalai Tesque, Inc.), iScript RT-qPCR Sample Preparation Reagent (BIORAD, 1708898) containing 2% protease inhibitor cocktail (for an animal cell extract, manufactured by Nacalai Tesque, Inc.) was added thereto in an amount of 20 µL per well, and the resultant was vigorously shaken for 30 seconds for cell lysis.

A translation product in a cell lysate thus obtained was detected in the same manner as in the sandwich ELISA method described in Test Example 1. As results of the measurement, a translation product concentration (nM) in each translation reaction solution quantitatively determined with a calibration curve created based on the absorbances of the polypeptide preparation is shown in Table 8 below.

Table 8-1 to Table 8-8:

**[Table 8-1]**

| Translation product concentration obtained from each mRNA | | | | |
|---|---|---|---|---|
| mRNA Name | SEQ ID NO: | Translation Product Concentration (nM) | | |
| | | 3 Hours After Introducing mRNA | 8 Hours After Introducing mRNA | 24 Hours After Introducing mRNA |
| E4 | 11 | 0.000 | 0.000 | 0.000 |
| E12 | 35 | 0.627 | 0.318 | 0.020 |
| E55 | 180 | 0.633 | 0.577 | 0.370 |
| E56 | 183 | 0.730 | 0.733 | 0.663 |
| E70 | 195 | 0.403 | 0.240 | 0.037 |

**[Table 8-2]**

| Translation product concentration obtained from each mRNA | | | | |
|---|---|---|---|---|
| mRNA Name | SEQ ID NO: | Translation Product Concentration (nM) | | |
| | | 3 Hours After Introducing mRNA | 8 Hours After Introducing mRNA | 24 Hours After Introducing mRNA |
| E4 | 11 | 0.010 | 0.000 | 0.010 |
| E62 | 171 | 1.270 | 0.997 | 0.203 |
| E63 | 174 | 1.027 | 0.873 | 0.453 |
| E64 | 177 | 0.840 | 0.747 | 0.283 |
| E67 | 186 | 1.017 | 0.890 | 0.597 |
| E68 | 189 | 0.460 | 0.477 | 0.237 |

**[Table 8-3]**

| Translation product concentration obtained from each mRNA | | | | |
|---|---|---|---|---|
| mRNA Name | SEQ ID NO: | Translation Product Concentration (nM) | | |
| | | 3 Hours After | 8 Hours After | 24 Hours After |
| | | Introducing mRNA | Introducing mRNA | Introducing mRNA |
| E4 | 11 | 0.010 | 0.000 | 0.007 |
| E79 | 206 | 0.543 | 0.540 | 0.300 |
| E80 | 209 | 0.047 | 0.027 | 0.010 |
| E81 | 212 | 0.620 | 0.500 | 0.280 |
| E82 | 215 | 0.507 | 0.507 | 0.273 |
| E95 | 234 | 0.640 | 0.567 | 0.297 |

**[Table 8-4]**

| Translation product concentration obtained from each mRNA | | | | |
|---|---|---|---|---|
| mRNA Name | SEQ ID NO: | Translation Product Concentration (nM) | | |
| | | 3 Hours After Introducing mRNA | 8 Hours After Introducing mRNA | 24 Hours After Introducing mRNA |
| E4 | 11 | 0.010 | 0.010 | 0.010 |
| E13 | 38 | 0.037 | 0.010 | 0.007 |
| E14 | 41 | 0.010 | 0.010 | 0.007 |
| E15 | 44 | 0.517 | 0.183 | 0.020 |
| E16 | 47 | 0.050 | 0.010 | 0.000 |
| E17 | 50 | 0.670 | 0.380 | 0.037 |

**[Table 8-5]**

| Translation product concentration obtained from each mRNA | | | | |
|---|---|---|---|---|
| mRNA Name | SEQ ID NO: | Translation Product Concentration (nM) | | |
| | | 3 Hours After Introducing mRNA | 8 Hours After Introducing mRNA | 24 Hours After Introducing mRNA |
| E4 | 11 | 0.000 | 0.000 | 0.000 |
| E7 | 20 | 0.043 | 0.000 | 0.000 |
| E26 | 77 | 0.537 | 0.183 | 0.000 |
| E27 | 80 | 0.317 | 0.050 | 0.000 |
| E28 | 83 | 0.183 | 0.040 | 0.000 |
| E29 | 86 | 0.093 | 0.017 | 0.000 |

**[Table 8-6]**

| Translation product concentration obtained from each mRNA | | | | |
|---|---|---|---|---|
| mRNA Name | SEQ ID NO: | Translation | | |
| | | Product Concentration (nM) | | |
| | | 3 Hours After Introducing mRNA | 8 Hours After Introducing mRNA | 24 Hours After Introducing mRNA |
| E4 | 11 | 0.000 | 0.000 | 0.000 |
| E7 | 20 | 0.023 | 0.000 | 0.000 |
| E9 | 26 | 0.413 | 0.123 | 0.000 |
| E26 | 77 | 0.397 | 0.117 | 0.000 |
| E27 | 80 | 0.230 | 0.037 | 0.000 |
| E28 | 83 | 0.137 | 0.030 | 0.000 |

**[Table 8-7]**

| Translation product concentration obtained from each mRNA | | | | |
|---|---|---|---|---|
| mRNA Name | SEQ ID NO: | Translation Product Concentration (nM) | | |
| | | 3 Hours After Introducing mRNA | 8 Hours After Introducing mRNA | 24 Hours After Introducing mRNA |
| E4 | 11 | 0.000 | 0.000 | 0.000 |
| E56 | 155 | 0.613 | 0.537 | 0.343 |
| E79 | 206 | 0.360 | 0.373 | 0.190 |
| E95 | 234 | 0.407 | 0.447 | 0.203 |

**[Table 8-8]**

| Translation product concentration obtained from each mRNA | | | | |
|---|---|---|---|---|
| mRNA Name | SEQ ID NO: | Translation Product Concentration (nM) | | |
| | | 3 Hours After Introducing mRNA | 8 Hours After Introducing mRNA | 24 Hours After Introducing mRNA |
| E58 | 161 | 0.130 | 0.100 | 0.000 |
| E164 | 305 | 0.143 | 0.147 | 0.010 |

As is obvious from the test results shown in Table 8-1 to Table 8-8 above, each mRNA having sugar modification produced, after being electroporated into the Hela cell, a polypeptide encoded by a gene sequence, and the activity was more excellent than that of an mRNA having no sugar modification. In particular, it was revealed, based on the test results shown in Table 8-5, that E26, E27, and E28 in each of which 65% or more of nucleotides contained in the poly A chain were sugar modified exhibit more excellent translation activity than E29 in which 50% of nucleotides contained in the poly A chain were sugar modified.

### Test Example 5

### (in vitro Translation Reaction Test of mRNA Sample with Human Aortic Smooth Muscle Cell)

Respective mRNAs shown in Tables 9-1 to 9-3 were evaluated for translation activity *in vitro* with Human Aortic Smooth Muscle Cells (manufactured by Lonza, CC-2571; hereinafter referred to also as hAoSMC). First, hAoSMC cultured with SmGM-2 BulletKit medium (manufactured by Lonza, CC-3182) in accordance with a manual provided by the manufacturer was used, and hAoSMC suspended in SmGM-2 BulletKit medium was seeded in a 96 well adherent cell culture plate at 10,000 cells/100 µL per well, and the resultant was cultured at 37°C under 5% CO2 condition overnight. A culture supernatant was removed from the cell cultured overnight, 40 µL per well of SmGM-2 BulletKit medium was added thereto, and each mRNA and Lipofectamin Messenger MAX Transfection Reagent in a final concentration of 1% (manufactured by Thermo Fisher Scientific K.K., Catalog No: LMRNA008) were diluted with Opti-MEM (manufactured by Thermo Fisher Scientific K.K., Catalog No: 31985-070) to a final concentration of each mRNA of 3 nM, 10 nM, or 30 nM to be mixed, the resultant mixture was added to each culture plate in an amount of 10 µL per well, and the resultant was cultured at 37°C under 5% CO2 condition for 5 hours. A culture supernatant was removed from the cell cultured for 5 hours, the resultant was washed once with ice cooled D-PBS(-) (manufactured by Nacalai Tesque, Inc.), iScript RT-qPCR Sample Preparation Reagent (BIORAD, 1708898) containing 2% protease inhibitor cocktail (for an animal cell extract, manufactured by Nacalai Tesque, Inc.) was added thereto in an amount of 20 µL per well, and the resultant was vigorously shaken for 30 seconds for cell lysis.

A translation product in a cell lysate thus obtained was detected in the same manner as in the sandwich ELISA method described in Test Example 1. As results of the measurement, a translation product concentration (nM) in each translation reaction solution quantitatively determined with a calibration curve created based on the absorbances of the polypeptide preparation is shown in Table 9 below.

Table 9-1 to Table 9-3:

**[Table 9-1]**

| Translation product concentration obtained from each mRNA | | | | |
|---|---|---|---|---|
| mRNA Name | SEQ ID NO: | Translation Product Concentration (nM) | | |
| | | mRNA 3 nM | mRNA10 nM | mRNA30 nM |
| E4 | 11 | 0.000 | 0.000 | 0.010 |
| E12 | 35 | 0.023 | 0.333 | 0.247 |
| E55 | 152 | 0.047 | 0.347 | 0.437 |
| E56 | 155 | 0.030 | 0.327 | 0.517 |

**[Table 9-2]**

| Translation product concentration obtained from each mRNA | | | | |
|---|---|---|---|---|
| mRNA Name | SEQ ID NO: | Translation Product Concentration (nM) | | |
| | | mRNA 3 nM | mRNA10 nM | mRNA30 nM |
| E4 | 11 | 0.000 | 0.007 | 0.010 |
| E79 | 206 | 0.017 | 0.233 | 0.350 |
| E81 | 212 | 0.013 | 0.157 | 0.367 |
| E95 | 234 | 0.023 | 0.243 | 0.457 |

**[Table 9-3]**

| Translation product concentration obtained from each mRNA | | | | |
|---|---|---|---|---|
| mRNA Name | SEQ ID NO: | Translation Product Concentration (nM) | | |
| | | mRNA 3 nM | mRNA10 nM | mRNA30 nM |
| E4 | 11 | 0.000 | 0.010 | 0.010 |
| E65 | 180 | 0.010 | 0.110 | 0.177 |
| E67 | 186 | 0.020 | 0.243 | 0.210 |
| E68 | 189 | 0.007 | 0.043 | 0.060 |

As is obvious from the test results shown in Table 9-1 to Table 9-3 above, each mRNA having sugar modification produced, after being added to the hAoSMC, a polypeptide encoded by a gene sequence, and the translation level was more excellent than that of an mRNA having no sugar modification.

### Test Example 6

### (in vitro Translation Reaction Test of mRNA Sample with Human Aortic Smooth Muscle Cell)

Respective mRNAs shown in Tables 10-1 to 10-5 below were evaluated for translation activity *in vitro* with human aortic smooth muscle cell.

First, each mRNA was diluted with THE RNA Storage Solution (manufactured by Thermo Fisher Scientific K.K., Catalog No. AM7000) to a concentration of 19 µM. hAoSMC was suspended in Opti-MEM I Reduced Serum Media (manufactured by Thermo Fisher Scientific K.K., Catalog No. 31985070) containing bovine serum albumin (manufactured by Wako Pure Chemical Industries Ltd., Catalog No. 017-22231) in a final concentration of 1%, the resultant was centrifuged at 90 xg at room temperature for 10 minutes, a supernatant was carefully removed, and then the resultant was suspended in a mixture of P1 Primary Cell Nucleofector Solution attached to P1 Primary Cell 96-well Nucleofector Kit (manufactured by Lonza, Catalog No. V4SP-1096) and Supplement 1 in a concentration of 100,000 cells/19 µL. The mRNA solution and the hAoSMC suspension thus prepared were mixed in a volume ratio of 1:19, and the resultant mixture was subjected to electroporation with Nucleofector^{(™)} 96-well Shuttle system (manufactured by Lonza) under pulse condition FF-130. The resultant cell electroporated for 10 minutes was suspended in SmGM-2 BulletKit medium (manufactured by Lonza, CC-3182), and was seeded in a 96 well adherent cell culture plate at 20,000 cells/145 µL per well, followed by culturing at 37°C under 5% CO2 condition. A culture supernatant was removed from the cells cultured respectively for 4 hours, 8 hours, and 24 hours, the resultant was washed once with ice cooled D-PBS(-) (manufactured by Nacalai Tesque, Inc.), iScript RT-qPCR Sample Preparation Reagent (BIORAD, 1708898) containing 2% protease inhibitor cocktail (for an animal cell extract, manufactured by Nacalai Tesque, Inc.) was added thereto in an amount of 20 µL per well, and the resultant was vigorously shaken for 30 seconds for cell lysis.

A translation product in a cell lysate thus obtained was detected in the same manner as in the sandwich ELISA method described in Test Example 1. As results of the measurement, a translation product concentration (nM) in each translation reaction solution quantitatively determined with a calibration curve created based on the absorbances of the polypeptide preparation is shown in Table 10 below.

Table 10-1 to Table 10-5:

**[Table 10-1]**

| Translation product concentration obtained from each mRNA | | | | |
|---|---|---|---|---|
| mRNA Name | SEQ ID NO: | Translation Product Concentration (nM) | | |
| | | 4 Hours After Introducing mRNA | 8 Hours After Introducing mRNA | 24 Hours After Introducing mRNA |
| E4 | 11 | 0.003 | 0.000 | 0.000 |
| E13 | 38 | 0.007 | 0.003 | 0.000 |
| E14 | 41 | 0.003 | 0.000 | 0.000 |
| E15 | 44 | 0.023 | 0.027 | 0.010 |
| E16 | 47 | 0.023 | 0.023 | 0.003 |
| E17 | 50 | 0.017 | 0.017 | 0.007 |

**[Table 10-2]**

| Translation product concentration obtained from each mRNA | | | | |
|---|---|---|---|---|
| mRNA Name | SEQ ID NO: | Translation Product Concentration (nM) | | |
| | | 4 Hours After Introducing mRNA | 8 Hours After Introducing mRNA | 24 Hours After Introducing mRNA |
| E4 | 11 | 0.000 | 0.000 | 0.000 |
| E12 | 35 | 0.027 | 0.033 | 0.010 |
| E55 | 152 | 0.033 | 0.050 | 0.027 |
| E56 | 155 | 0.027 | 0.043 | 0.043 |
| E70 | 195 | 0.023 | 0.033 | 0.013 |

**[Table 10-3]**

| Translation product concentration obtained from each mRNA | | | | |
|---|---|---|---|---|
| mRNA Name | SEQ ID NO: | Translation Product | | |
| | | Concentration (nM) | | |
| | | 4 Hours After Introducing mRNA | 8 Hours After Introducing mRNA | 24 Hours After Introducing mRNA |
| E4 | 11 | 0.000 | 0.000 | 0.000 |
| E65 | 180 | 0.043 | 0.077 | 0.047 |
| E66 | 183 | 0.040 | 0.083 | 0.060 |
| E68 | 189 | 0.010 | 0.010 | 0.010 |
| E67 | 186 | 0.020 | 0.030 | 0.023 |

**[Table 10-4]**

| Translation product concentration obtained from each mRNA | | | | |
|---|---|---|---|---|
| mRNA Name | SEQ ID NO: | Translation Product Concentration (nM) | | |
| | | 4 Hours After Introducing mRNA | 8 Hours After Introducing mRNA | 24 Hours After Introducing mRNA |
| E4 | 11 | 0.003 | 0.003 | 0.000 |
| E79 | 206 | 0.124 | 0.122 | 0.079 |
| E80 | 209 | 0.030 | 0.023 | 0.007 |
| E81 | 212 | 0.127 | 0.142 | 0.085 |
| E95 | 234 | 0.139 | 0.170 | 0.094 |

**[Table 10-5]**

| Translation product concentration obtained from each mRNA | | | | |
|---|---|---|---|---|
| mRNA Name | SEQ ID NO: | Translation Product Concentration (nM) | | |
| | | 4 Hours After Introducing mRNA | 8 Hours After Introducing mRNA | 24 Hours After Introducing mRNA |
| E4 | 11 | 0.000 | 0.000 | 0.000 |
| E56 | 155 | 0.197 | 0.220 | 0.163 |
| E79 | 206 | 0.103 | 0.120 | 0.093 |

As is obvious from the test results shown in Table 10-1 to Table 10-5 above, each mRNA having sugar modification produced, after being electroporated into the hAoSMC, a polypeptide encoded by a gene sequence, and the activity was more excellent than that of an mRNA having no sugar modification in the translated region.

### Test Example 7

### (Test of Stability in Serum of mRNA Sample)

The respective mRNAs shown in Table 11 below were evaluated for nucleic acid stability in serum with a commercially available mouse serum (Kohjin Bio, Catalog No. 12081001). First, the mouse serum was diluted 50 fold with UltraPure DNase/RNase-Free Distilled Water (DW) (Invitrogen, Catalog No. 10977-015) to prepare a diluted serum solution. Each mRNA was diluted to a concentration of 5 µM with THE RNA storage solution (Thermo Fisher Scientific K.K., Catalog No. AM7001).

As a sample before enzymatic reaction (0 min), 10.5 µL of a mixed solution of 8 µL of the diluted serum solution and 2.5 µL of 6 U/µL Ribonuclease Inhibitor (Takara Bio Inc., Catalog No. 2311B), and 2 µL of 5 µM mRNA were added to another 96 well PCR plate, and the resultant was stored at -30°C. As a sample for enzymatic reaction, 8 µL of the diluted serum solution and 2 µL of 5 µM mRNA were added to and well mixed in another 96 well PCR plate. A reaction was caused in the respective PCR plates at 37°C respectively for prescribed times (15 min, 30 min, and 60 min), 2.5 µL of 6 U/µL Rnase inhibitor was added thereto, and the resultant was stored at -30°C until measurement.

A remaining amount of mRNA in a reaction solution after the enzymatic reaction was detected by RT-qPCR method as follows. A calibration curve was created for each of the evaluated mRNAs, and dilution series were produced by obtaining 11 concentrations from 4 µM with 4-fold dilution with THE RNA storage solution. 2.5 µL of each of samples for the calibration curve and after the enzymatic reaction was diluted 1071 fold by using DW to which Ribonuclease Inhibitor had been added to a final concentration of 0.2 U/mL. A reverse transcription product cDNA was produced using 5 µL of the diluted sample and 1 µL of 2 µM RT primer (Sigma Aldrich Co.) with a TaqMan Micro RNA RT kit (Thermo Fisher Scientific K.K., Catalog No. 4366597). The reaction was performed at a reaction temperature of 16°C for 30 minutes, then at 42°C for 30 minutes, and then at 85°C for 5 minutes. 5 µL of cDNA, 10 µL of TaqMan Gene Expression Master Mix, 0.28 µL of Fw primer (Sigma Aldrich Co.), 0.33 µL of Rv primer (Sigma Aldrich Co.), 0.38 µL of TaqMan MGB Probe (Thermo Fisher Scientific K.K., Catalog No. 4316033), and 4.01 µL of distilled water were mixed to perform qPCR measurement. As an apparatus, Quantstudio12K Flex (Applied Biosystems) was used.

The DNA sequences of the used primers and Taqman MGB probe were as follows. As results of the measurement, a concentration of each mRNA in each sample was quantitatively determined by using a calibration curve based on a CT value of a preparation, and a relative remaining amount with respect to the amount before the enzymatic reaction (0 min) was calculated, which is shown in Table 11 below.
RT primer: 5'-TCAGTGGTGGTGGTGGTGGTGTTTG-3' (SEQ ID NO: 431)
Fw primer: 5'-ATCTTGTCGTCGTCGTCCTT-3' (SEQ ID NO: 432)
Rv primer: 5'-GAATACAAGCTACTTGTTCTTTT-3' (SEQ ID NO: 433)
Taqman MGB Probe: 5'-CAGCCACCATG-3' (SEQ ID NO: 434)

Table 11:

**[Table 11]**

| Relative remaining amount of each mRNA at each reaction time point with respect to that before enzymatic reaction (0 min) | | | | | |
|---|---|---|---|---|---|
| mRNA Name | SEQ ID NO: | 0 min | 15 min | 30 min | 60 min |
| E4 | 11 | 1.000 | 0.387 | 0.199 | 0.062 |
| E12 | 35 | 1.000 | 0.516 | 0.330 | 0.149 |
| E55 | 152 | 1.000 | 0.788 | 0.644 | 0.550 |
| E56 | 155 | 1.000 | 0.854 | 0.805 | 0.772 |

As is obvious from the test results shown in Table 11 above, an mRNA having sugar modification was improved in degradation resistance in serum as compared with an mRNA having no sugar modification.

### Test Example 8

### (Translation Reaction Test of mRNA Sample with Hela Cell Lysate)

The respective mRNAs shown in Table 12-1 to Table 12-7 below were evaluated for translation activity in a human cell system with 1-Step Human Coupled IVT Kit (manufactured by Thermo Fisher Scientific K.K., Catalog No. 88882). The translation reaction was performed in the same manner as in Test Example 1 under condition of a final concentration of mRNA of 1 µM.

A translation product in a reaction solution after the translation reaction was detected in the same manner as in the sandwich ELISA method described in Test Example 1 except that a peptide (manufactured by Cosmo Bio Co., Ltd.) shown below was used as a translation product polypeptide preparation. A translation product concentration (nM) in each translation reaction solution quantitatively determined with a calibration curve created based on the absorbances of the polypeptide preparation, and a relative amount of the translation product calculated assuming that the amount obtained from E30 having no sugar modification is 1 are shown in Table 12 below.

Translation product polypeptide preparation: NH₂-MDYKDDDDKGGHHHHHH-COOH (SEQ ID NO: 435)

Table 12-1 to Table 12-7:

**[Table 12-1]**

| mRNA Name | SEQ ID NO: | Translation Product Concentration (nM) | Relative Amount of Translation Product |
|---|---|---|---|
| E31 | 90 | 25.993 | 4.71 |
| E71 | 198 | 10.892 | 1.97 |
| E72 | 199 | 19.436 | 3.52 |
| E73 | 200 | 20.622 | 3.73 |
| E74 | 201 | 19.338 | 3.50 |
| E75 | 202 | 8.980 | 1.63 |
| E76 | 203 | 15.411 | 2.79 |
| E77 | 204 | 15.536 | 2.81 |
| E78 | 205 | 10.471 | 1.90 |
| E85 | 224 | 19.894 | 3.60 |
| E86 | 225 | 13.435 | 2.43 |
| E87 | 226 | 11.132 | 2.02 |
| E88 | 227 | 12.522 | 2.27 |
| E89 | 228 | 18.310 | 3.32 |
| E90 | 229 | 11.413 | 2.07 |
| E91 | 230 | 14.883 | 2.69 |
| E92 | 231 | 21.319 | 3.86 |
| E30 | 89 | 5.523 | 1.00 |
| E61 | 170 | 16.168 | 2.93 |

**[Table 12-2]**

| mRNA Name | SEQ ID NO: | Translation Product Concentration (nM) | Relative Amount of Translation Product |
|---|---|---|---|
| E93 | 232 | 3.464 | 0.61 |
| E94 | 233 | 6.309 | 1.11 |
| E96 | 237 | 14.861 | 2.60 |
| E97 | 238 | 22.251 | 3.90 |
| E98 | 239 | 9.450 | 1.66 |
| E99 | 240 | 14.573 | 2.55 |
| E100 | 241 | 8.723 | 1.53 |
| E101 | 242 | 13.510 | 2.37 |
| E102 | 243 | 11.197 | 1.96 |
| E103 | 244 | 4.959 | 0.87 |
| E104 | 245 | 25.710 | 4.50 |
| E105 | 246 | 12.564 | 2.20 |
| E106 | 247 | 16.057 | 2.81 |
| E107 | 248 | 13.531 | 2.37 |
| E108 | 249 | 20.671 | 3.62 |
| E30 | 89 | 5.709 | 1.00 |
| E61 | 170 | 11.057 | 1.94 |

**[Table 12-3]**

| mRNA Name | SEQ ID NO: | Translation Product Concentration (nM) | Relative Amount of Translation Product |
|---|---|---|---|
| E019 | 250 | 18.672 | 4.04 |
| E110 | 251 | 12.976 | 2.81 |
| E111 | 252 | 13.883 | 3.00 |
| E112 | 253 | 11.138 | 2.41 |
| E113 | 254 | 13.333 | 2.88 |
| E114 | 255 | 11.570 | 2.50 |
| E115 | 256 | 13.348 | 2.89 |
| E116 | 257 | 20.125 | 4.35 |
| E117 | 258 | 14.713 | 3.18 |
| E118 | 259 | 19.148 | 4.14 |
| E119 | 260 | 7.051 | 1.52 |
| E120 | 261 | 1.294 | 0.28 |
| E121 | 262 | 22.516 | 4.87 |
| E122 | 263 | 16.092 | 3.48 |
| E123 | 264 | 22.733 | 4.92 |
| E124 | 265 | 23.290 | 5.04 |
| E125 | 266 | 10.663 | 2.31 |
| E30 | 89 | 4.625 | 1.00 |
| E61 | 170 | 13.887 | 3.00 |

**[Table 12-4]**

| mRNA Name | SEQ ID NO: | Translation Product Concentration (nM) | Relative Amount of Translation Product |
|---|---|---|---|
| E126 | 267 | 7.770 | 1.84 |
| E127 | 268 | 7.617 | 1.81 |
| E128 | 269 | 6.520 | 1.55 |
| E129 | 270 | 4.105 | 0.97 |
| E130 | 271 | 4.843 | 1.15 |
| E131 | 272 | 3.846 | 0.91 |
| E132 | 273 | 2.425 | 0.58 |
| E133 | 274 | 6.694 | 1.59 |
| E134 | 275 | 8.231 | 1.95 |
| E135 | 276 | 6.688 | 1.59 |
| E136 | 277 | 7.887 | 1.87 |
| E137 | 278 | 6.767 | 1.61 |
| E138 | 279 | 4.118 | 0.98 |
| E139 | 280 | 2.913 | 0.69 |
| E140 | 281 | 6.503 | 1.54 |
| E141 | 282 | 17.884 | 4.24 |
| E142 | 283 | 18.111 | 4.30 |
| E30 | 89 | 4.216 | 1.00 |
| E61 | 170 | 9.462 | 2.24 |

**[Table 12-5]**

| mRNA Name | SEQ ID NO: | Translation Product Concentration (nM) | Relative Amount of Translation Product |
|---|---|---|---|
| E143 | 284 | 11.167 | 3.13 |
| E144 | 285 | 15.514 | 4.34 |
| E145 | 286 | 5.484 | 1.53 |
| E146 | 287 | 5.053 | 1.41 |
| E147 | 288 | 13.516 | 3.78 |
| E148 | 289 | 12.278 | 3.44 |
| E149 | 290 | 4.911 | 1.37 |
| E150 | 291 | 4.017 | 1.12 |
| E151 | 293 | 3.751 | 1.05 |
| E152 | 293 | 3.476 | 0.97 |
| E153 | 294 | 16.010 | 4.48 |
| E154 | 295 | 12.791 | 3.58 |
| E155 | 296 | 14.127 | 3.95 |
| E156 | 297 | 15.781 | 4.42 |
| E157 | 298 | 10.380 | 2.90 |
| E158 | 299 | 10.570 | 2.96 |
| E159 | 300 | 16.523 | 4.62 |
| E30 | 89 | 3.574 | 1.00 |
| E61 | 170 | 10.746 | 3.01 |

**[Table 12-6]**

| mRNA Name | SEQ ID NO: | Translation Product Concentration (nM) | Relative Amount of Translation Product |
|---|---|---|---|
| E160 | 301 | 9.461 | 3.65 |
| E161 | 302 | 10.070 | 3.88 |
| E162 | 303 | 8.835 | 3.41 |
| E163 | 304 | 13.862 | 5.35 |
| E30 | 89 | 2.593 | 1.00 |
| E61 | 170 | 7.277 | 2.81 |

**[Table 12-7]**

| mRNA Name | SEQ ID NO: | Translation Product Concentration (nM) | Relative Amount of Translation Product |
|---|---|---|---|
| E71 | 198 | 11.680 | 2.31 |
| E72 | 199 | 20.707 | 4.10 |
| E74 | 201 | 22.307 | 4.41 |
| E85 | 224 | 24.987 | 4.94 |
| E98 | 239 | 10.253 | 2.03 |
| E200 | 395 | 19.160 | 3.79 |
| E201 | 396 | 19.480 | 3.85 |
| E202 | 397 | 16.533 | 3.27 |
| E203 | 394 | 15.347 | 3.04 |
| E30 | 89 | 5.053 | 1.00 |

As is obvious from the test results shown in Table 12-1 to Table 12-7 above, each mRNA produced, after being added to the Hela cell lysate, a polypeptide encoded by a gene sequence in the eukaryotic cell translation system.

### Example 5

### (Synthesis of mRNA Translating VEGF)

Sequence information of materials (polynucleotides) used in synthesis of mRNAs to be translated to VEGF protein is as follows.

Table 13:

**[Table 13]**

| Compound Name | Sequence (5' to 3') | SEQ ID NO: |
|---|---|---|
| 5' End Polynucleotide Sequence N1 | GGGAAAUAAGAGAGAAAAGAAGAGUAAGAAGAAAUAUAAGAGCCAC CAUGAACUUUCUGCUGUCUU | 436 |
| 5' End Polynucleotide Sequence N2 | G(MOE)^G(MOE)^G(MOE)^A(MOE)^A(MOE)^A(MOE)U(F)A(M)A(F)G(M) A(F)G(M)A(F)G(M)A(F)A(M)A(F)A(M)G(F)A(M)A(F)G(M)A(F)G(M)U(F)A( M)A(F)G(M)A(F)A(M)G(F)A(M)A(F)A(M)U(F)A(M)U(F)A(M)A(F)G(M)A(F) G(M)C(F)C(M)A(F)C(M)C(F)A(F)UGA(F)ACU(F)UUC(F)UGC(F)UGU(F)C UU | 437 |
| 5' End Polynucleotide Sequence N3 | G(MOE)^G(MOE)^G(MOE)^A(MOE)^A(MOE)^A(MOE)U(F)Am6(M)Am6(F )G(M)Am6(F)G(M)Am6(F)G(M)Am6(F)Am6(M)Am6(F)Am6(M)G(F)Am6( M)Am6(F)G(M)Am6(F)G(M)U(F)Am6(M)Am6(F)G(M)Am6(F)Am6(M)G(F) Am6(M)Am6(F)Am6(M)U(F)Am6(M)U(F)Am6(M)Am6(F)G(M)Am6(F)G(M) C(F)C(M)Am6(F)C(M)C(F)A(F)UGA(F)ACU(F)UUC(F)UGC(F)UGU(F)CU U | 438 |
| Artificially Synthesized Gene Sequence GN | AATTCAGTACTTAATACGACTCACTATAGGGTGCATTGGAGCCTTGC CTTGCTGCTCTACCTCCACCATGCCAAGTGGTCCCAGGCTGCACCC ATGGCAGAAGGAGGAGGGCAGAATCATCACGAAGTGGTGAAGTTCA TGGATGTCTATCAGCGCAGCTACTGCCATCCAATCGAGACCCTGGT GGACATCTTCCAGGAGTACCCTGATGAGATCGAGTACATCTTCAAG CCATCCTGTGTGCCCCTGATGCGATGCGGGGGCTGCTGCAATGACG AGGGCCTGGAGTGTGTGCCCACTGAGGAGTCCAACATCACCATGCA GATTATGCGGATCAAACCTCACCAAGGCCAGCACATAGGAGAGATG AGCTTCCTACAGCACAACAAATGTGAATGCAGACCAAAGAAAGATA GAGCAAGACAAGAAAATCCCTGTGGGCCTTGCTCAGAGCGGAGAAA GCATTTGTTTGTACAAGATCCGCAGACGTGTAAATGTTCCTGCAAAA ACACAGACTCGCGTTGCAAGGCGAGGCAGCTTGAGTTAAACGAACG TACTTGCAGATGTGACAAGCCGAGGCGGTGATAATAGGCTGGAGCC TCGGTGGCCATGCTTCTTGCCCCTTGGGCCTCCCCCCAGCCCCTCC TCCCCTTCCTGCACCCGTACCCCCGTGGTCTTTGAATAAAGTCTGA GTGGGCGGCAAAAAAAAAAAAAAAAAAAAAAAAAAAAAAAAAAAAAA AAAAAAAAAAAAAAAAAAAAAAAAAAAAAAAAAAAAAAAAAAAAAAA AAAAAAAAAAAAAAAT | 439 |
| Template DNA-4 | dGdGdCdTdCdCdAdAdTdGdCdAdCdCdCdAdAdGdAdCdAdGdCdAdGdAdAdAdG dT | 440 |

Through the following series of operations, mRNAs (VEGF-1, VEGF-2, and VEGF-3) were obtained.

### (Step 1: Preparation of Linearized Plasmid DNA and Preparation of RNA Fragment by in vitro Transcription)

As a plasmid DNA, one obtained by inserting, into EcoRV site and Xbal site of a commercially available pUC19 vector, an artificially synthesized gene sequence GN shown in Table 13 was used (manufactured by Genewiz Inc.). The plasmid DNA was linearized with restriction enzyme Xbal. Final concentrations in the resultant reaction solution were: the plasmid DNA: 20 ng/µL, BSA: 0.01%, Xba I: 0.15 U/µL (Takara 1093A), and attached buffer: 1 x. The resultant was incubated at 37°C for 2 hours, and the resultant was subjected to phenol chloroform extraction and isopropanol precipitation to obtain a crude product of a linearized plasmid. The thus obtained template DNA and T7 RNA polymerase were used to perform a transcription reaction. Final concentrations in the resultant reaction solution were as follows: template DNA: 10 ng/µL, DTT: 5mM, ATP: 2 mM, CTP: 2 mM, UTP: 2mM, GMP: 2 mM, GTP: 0.5 mM, Murine RNase inhibitor: 0.2 U/µL (NEB, M0314), T7 RNA polymerase (Takara 2540A): 2.5 U/µL, and attached buffer: 1 x. The resultant was incubated at 37°C for 2 hours, and then DNase (Takara, 2270A) was added thereto to a final concentration of 0.1 U/ µL, followed by incubation at the same temperature for 30 minutes. The resultant was subjected to phenol chloroform extraction, a treatment with Amicon 10K (Merck Millipore), and isopropanol precipitation to obtain a crude product of a transcriptional product. After subjecting the resultant to electrophoresis in modified polyacrylamide gel, a corresponding band was cut out, and subjected to extraction with MQ water, purification with Amicon, and isopropanol precipitation to obtain a purified RNA. Subsequently, a terminal triphosphate form was converted into a monophosphate form by a treatment with RNA 5 ' Pyrophosphohydrolase (RppH). Final concentrations in the resultant reaction solution were as follows:
RNA: 0.1 µg/ µL, RppH: 0.1 U/µL, Murine RNase inhibitor: 1 U/µL (NEB, M0314), and NEBuffer 2 (NEB, B7992S): 1x. The resultant was incubated at 37°C for 30 minutes, and then subjected to phenol chloroform extraction and isopropanol precipitation to obtain a crude product of a target polynucleotide that is a 3' end side polynucleotide fragment.

### (Step 2: Preparation of RNA Ligation Product by RNA Ligation)

A ligation reaction with RNA ligase 2 was performed using 5' end side polynucleotide fragments (N1, N2, and N3) obtained by chemical synthesis in accordance with an ordinary method shown in Table 13, a 3' end side polynucleotide fragment obtained by the *in vitro* transcription of Step 1, and a template DNA-4. Final concentrations were as follows: 5' end side RNA: 2 µM, 3' end side RNA: 1 µM, template DNA: 4 µM, PEG 8000: 10%, T4 RNA ligase 2: 1 U/µL (NEB, M0239), attached buffer: 1 x, and Murine RNase inhibitor: 1 U/µL (NEB, M0314). A mixture before adding the enzyme and PEG was heated at 90°C for 3 minutes, and was gradually returned to room temperature, and the enzyme and PEG were added thereto, followed by incubation at 45°C for 1 hour. The resultant was subjected to phenol chloroform extraction, a treatment with Amicon 10K (Merck Millipore Corp.), and isopropanol precipitation to obtain a crude product of a transcription product. After subjecting the resultant to electrophoresis in modified polyacrylamide gel, a corresponding band was cut out, and subjected to extraction with MQ water, purification with Amicon, and isopropanol precipitation to obtain a purified RNA.

### Test Example 9

### (Translation Reaction of mRNA Sample)

Sequence information of mRNAs obtained in Example 5 described above is shown in Table 14-1 to Table 14-2.

Table 14-1 to Table 14-2:

**[Table 14-1]**

| Compound Name | Sequence (5' to 3') | SEQ ID NO: |
|---|---|---|
| VEGF-1 | G(MOE)^G(MOE)^G(MOE)^A(MOE)^A(MOE)^A(MOE)U(F)A(M)A(F)G(M)A(F )G(M)A(F)G(M)A(F)A(M)A(F)A(M)G(F)A(M)A(F)G(M)A(F)G(M)U(F)A(M)A(F) G(M)A(F)A(M)G(F)A(M)A(F)A(M)U(F)A(M)U(F)A(M)A(F)G(M)A(F)G(M)C(F) C(M)A(F)C(M)C(F)A(F)UGA(F)ACU(F)UUC(F)UGC(F)UGU(F)CUUGGGUGC AUUGGAGCCUUGCCUUGCUGCUCUACCUCCACCAUGCCAAGUGGUCCC AGGCUGCACCCAUGGCAGAAGGAGGAGGGCAGAAUCAUCACGAAGUG GUGAAGUUCAUGGAUGUCUAUCAGCGCAGCUACUGCCAUCCAAUCGAG ACCCUGGUGGACAUCUUCCAGGAGUACCCUGAUGAGAUCGAGUACAUC UUCAAGCCAUCCUGUGUGCCCCUGAUGCGAUGCGGGGGCUGCUGCAA UGACGAGGGCCUGGAGUGUGUGCCCACUGAGGAGUCCAACAUCACCA UGCAGAUUAUGCGGAUCAAACCUCACCAAGGCCAGCACAUAGGAGAGA UGAGCUUCCUACAGCACAACAAAUGUGAAUGCAGACCAAAGAAAGAUA GAGCAAGACAAGAAAAUCCCUGUGGGCCUUGCUCAGAGCGGAGAAAGC AUUUGUUUGUACAAGAUCCGCAGACGUGUAAAUGUUCCUGCAAAAACA CAGACUCGCGUUGCAAGGCGAGGCAGCUUGAGUUAAACGAACGUACUU GCAGAUGUGACAAGCCGAGGCGGUGAUAAUAGGCUGGAGCCUCGGUG GCCAUGCUUCUUGCCCCUUGGGCCUCCCCCCAGCCCCUCCUCCCCUU CCUGCACCCGUACCCCCGUGGUCUUUGAAUAAAGUCUGAGUGGGCGG CAAAAAAAAAAAAAAAAAAAAAAAAAAAAAAAAAAAAAAAAAAAAAAAAAA AAAAAAAAAAAAAAAAAAAAAAAAAAAAAAAAAAAAAAAAAAAAAAAAAAA AAAUCUAG | 441 |
| VEGF-2 | G(MOE)^G(MOE)^G(MOE)^A(MOE)^A(MOE)^A(MOE)U(F)Am6(M)Am6(F)G( M)Am6(F)G(M)Am6(F)G(M)Am6(F)Am6(M)Am6(F)Am6(M)G(F)Am6(M)Am6( F)G(M)Am6(F)G(M)U(F)Am6(M)Am6(F)G(M)Am6(F)Am6(M)G(F)Am6(M)Am 6(F)Am6(M)U(F)Am6(M)U(F)Am6(M)Am6(F)G(M)Am6(F)G(M)C(F)C(M)Am6 (F)C(M)C(F)A(F)UGA(F)ACU(F)UUC(F)UGC(F)UGU(F)CUUGGGUGCAUUG GAGCCUUGCCUUGCUGCUCUACCUCCACCAUGCCAAGUGGUCCCAGG CUGCACCCAUGGCAGAAGGAGGAGGGCAGAAUCAUCACGAAGUGGUG AAGUUCAUGGAUGUCUAUCAGCGCAGCUACUGCCAUCCAAUCGAGACC CUGGUGGACAUCUUCCAGGAGUACCCUGAUGAGAUCGAGUACAUCUUC AAGCCAUCCUGUGUGCCCCUGAUGCGAUGCGGGGGCUGCUGCAAUGA CGAGGGCCUGGAGUGUGUGCCCACUGAGGAGUCCAACAUCACCAUGC AGAUUAUGCGGAUCAAACCUCACCAAGGCCAGCACAUAGGAGAGAUGA GCUUCCUACAGCACAACAAAUGUGAAUGCAGACCAAAGAAAGAUAGAG CAAGACAAGAAAAUCCCUGUGGGCCUUGCUCAGAGCGGAGAAAGCAUU UGUUUGUACAAGAUCCGCAGACGUGUAAAUGUUCCUGCAAAAACACAG ACUCGCGUUGCAAGGCGAGGCAGCUUGAGUUAAACGAACGUACUUGCA GAUGUGACAAGCCGAGGCGGUGAUAAUAGGCUGGAGCCUCGGUGGCC AUGCUUCUUGCCCCUUGGGCCUCCCCCCAGCCCCUCCUCCCCUUCCU GCACCCGUACCCCCGUGGUCUUUGAAUAAAGUCUGAGUGGGCGGCAAA AAAAAAAAAAAAAAAAAAAAAAAAAAAAAAAAAAAAAAAAAAAAAAAAA AAAAAAAAAAAAAAAAAAAAAAAAAAAAAAAAAAAAAAAAAAAAAAAAU CUAG | 442 |

**[Table 14-2]**

| | | |
|---|---|---|
| VEGF-3 | GGGAAAUAAGAGAGAAAAGAAGAGUAAGAAGAAAUAUAAGAGCCACCA UGAACUUUCUGCUGUCUUGGGUGCAUUGGAGCCUUGCCUUGCUGCUCU ACCUCCACCAUGCCAAGUGGUCCCAGGCUGCACCCAUGGCAGAAGGA GGAGGGCAGAAUCAUCACGAAGUGGUGAAGUUCAUGGAUGUCUAUCAG | 443 |
| | CGCAGCUACUGCCAUCCAAUCGAGACCCUGGUGGACAUCUUCCAGGAG UACCCUGAUGAGAUCGAGUACAUCUUCAAGCCAUCCUGUGUGCCCCUG AUGCGAUGCGGGGGCUGCUGCAAUGACGAGGGCCUGGAGUGUGUGCC CACUGAGGAGUCCAACAUCACCAUGCAGAUUAUGCGGAUCAAACCUCA CCAAGGCCAGCACAUAGGAGAGAUGAGCUUCCUACAGCACAACAAAUG UGAAUGCAGACCAAAGAAAGAUAGAGCAAGACAAGAAAAUCCCUGUGG GCCUUGCUCAGAGCGGAGAAAGCAUUUGUUUGUACAAGAUCCGCAGAC GUGUAAAUGUUCCUGCAAAAACACAGACUCGCGUUGCAAGGCGAGGCA GCUUGAGUUAAACGAACGUACUUGCAGAUGUGACAAGCCGAGGCGGUG AUAAUAGGCUGGAGCCUCGGUGGCCAUGCUUCUUGCCCCUUGGGCCU CCCCCCAGCCCCUCCUCCCCUUCCUGCACCCGUACCCCCGUGGUCUU UGAAUAAAGUCUGAGUGGGCGGCAAAAAAAAAAAAAAAAAAAAAAAAA AAAAAAAAAAAAAAAAAAAAAAAAAAAAAAAAAAAAAAAAAAAAAAAAAA AAAAAAAAAAAAAAAAAAAAAAAAAUCUAG | |

Each mRNA was evaluated for translation activity *in vitro* with Hela cell line. First, a Hela cell suspended in RPMI medium (manufactured by Nacalai Tesque, Inc.) containing 10% fetal bovine serum was seeded in a 96 well adherent cell culture plate at 10,000 cells/100 µL per well, and the resultant was cultured at 37°C under 5% CO2 condition overnight. A culture supernatant was removed from the cell cultured overnight, RPMI medium containing 40 µL of 10% fetal bovine serum per well was added thereto, and each compound and Lipofectamin Messenger MAX Transfection Reagent (manufactured by Thermo Fisher Scientific K.K., Catalog No: LMRNA008) at a final concentration of 0.3% were diluted and mixed with Opti-MEM (manufactured by Thermo Fisher Scientific K.K., Catalog No: 31985-070) to a final concentration of 0.3, 1, 3 or 10 nM of each compound, the resultant mixture was added to each culture plate in an amount of 10 µL per well, and the resultant was cultured at 37°C under 5% CO2 condition for 24 hours. A culture supernatant was recovered from the cell cultured for 24 hours, and an amount of VEGF protein in the thus obtained culture supernatant was measured with Human VEGE Quantikine ELISA (manufactured by R & D, Catalog No. DVE00) in accordance with a manual attached to the kit. A VEGF protein concentration (ng/mL) in each culture supernatant quantitatively determined as a result of the measurement is shown in Table 15 below.

Table 15:

**[Table 15]**

| Translation product concentration obtained from each mRNA | | | | |
|---|---|---|---|---|
| mRNA Name | Translation Product Concentration (nM) | | | |
| | mRNA 0.3 nM | mRNA 1 nM | mRNA 3 nM | mRNA 10 nM |
| VEGF-1 | 1.38 | 1.57 | 3.08 | 6.10 |
| VEGF-2 | 1.47 | 1.52 | 3.31 | 12.57 |
| VEGF-3 | 1.52 | 1.33 | 2.18 | 3.88 |

As is obvious from the test results shown in Table 15 above, each mRNA having sugar modification produced, after being added to the Hela cell, VEGF protein encoded by a gene sequence, and the efficiency was more excellent than that of an mRNA having no sugar modification.

### Example 6

### (Synthesis of mRNA by IVT)

Sequence information of materials (polynucleotides) used in mRNA synthesis by IVT is as follows.

Table 16:

**[Table 16]**

| Compound Name | Sequence (5' to 3') | SEQ ID NO: |
|---|---|---|
| Artificially Synthesized Gene Sequence GO | GATCCGAAATTAATACGACTCACTATAGATGGACTACAAGGACGACGAC GACAAGATCATCGACTATAAAGACGACGACGATAAAGGTGGCGACTAT AAGGACGACGACGACAAACACCACCACCACCACCACGCTGCAATCAGT CTGATTGCGGCGTTAGCGGTAGATCGCGTTATCGGCATGGAAAACGCC ATGCCGTGGAACCTGCCTGCCGATCTCGCCTGGTTTAAACGCAACACC TTAAATAAACCCGTGATTATGGGCCGCCATACCTGGGAATCAATCGGTC GTCCGTTGCCAGGACGCAAAAATATTATCCTCAGCAGTCAACCGGGTA CGGACGATCGCGTAACGTGGGTGAAGTCGGTGGATGAAGCCATCGCGG CGTGTGGTGACGTACCAGAAATCATGGTGATTGGCGGCGGTCGCGTTT ATGAACAGTTCTTGCCAAAAGCGCAAAAACTGTATCTGACGCATATCGA CGCAGAAGTGGAAGGCGACACCCATTTCCCGGATTACGAGCCGGATGA CTGGGAATCGGTATTCAGCGAATTCCACGATGCTGATGCGCAGAACTCT CACAGCTATTGCTTTGAGATTCTGGAGCGGCGGTAATGAATAACTAATC CCTGCA | 444 |
| Primer P1 | dTdTdGdGdAdCdCdCdTdCdGdTdAdCdAdGdAdAdGdCdTdAdAdTdAdCdGd AdCdTdCdAdCdTdAdTdAdGdGdGdAdGdAdAdTdAdCdAdAdGdCdTdAdCd TdTdGdTdTdCdTdTdTdTdTdGdCdAdGdCdCdAdCdCdAdTdGdGdAdCdTdA dCdAdAdGdGdAdCdG | 445 |
| Primer P2 | dTdTdTdTdTdTdTdTdTdTdTdTdTdTdTdTdTdTdTdTdCdAdGdTdGdGdT dGdGdTdGdGdTdGdGdTdGdGdTdGdTdTdTdG | 446 |

Through the following series of operations, an mRNA (IVT-1) was obtained.

### (Step 1: Preparation of Linearized DNA)

As a plasmid DNA, one obtained by inserting, into BamHI site and PstI site of a commercially available pUC57 vector, an artificially synthesized gene sequence GO shown in Table 16 was used. The plasmid DNA was used to perform PCR reaction as follows. Specifically, the plasmid DNA in a final concentration of 250 ng/µL, primers P1 and P2 each in a final concentration of 250 nM, and 200 µL of Primestar MAX (manufactured by Takara Bio Inc., Catalog No. R045B) were mixed, the resultant was prepared to 400 µL with Nuclease-free water, a thermal cycler was used to heat the resultant at 98°C for 30 seconds, then a cycle of heating at 98°C for 10 seconds, 55°C for 5 seconds, and at 72°C for 5 seconds was repeated 30 times, and thereafter, the resultant was heated at 72°C for 5 minutes, followed by cooling at 4°C. To 400 µL of the PCR product thus obtained, 10 µL of Dpn I (manufactured by New England BioLab, Catalog No. R0176L), 50 µL of CutSmart Buffer (manufactured by New England Biolab), and 40 µL of Nuclease-free water were added to be mixed, and the resultant was allowed to stand still at 37°C for 30 minutes. The reaction solution thus obtained was subjected to electrophoresis in 3.0% agarose-TAE gel, a corresponding band was cut out, the thus obtained PCR product was purified with NucleoSpin Gel and PCR Clean-up Midi (manufactured by MACHEREY-NAGEL, Catalog No. 740986.20), and then the resultant was subjected to phenol chloroform extraction and ethanol precipitation to obtain a PCR DNA.

### (Step 2: Preparation of RNA Fragment by in vitro Transcription))

The thus obtained PCR DNA was used to perform a transcription reaction. Final concentrations in the resultant reaction solution were as follows: PCR DNA: 4 ng/µL, ATP, CTP, UTP, and GTP: 9 mM each, T7 Enzyme attached to MEGAScript T7 Transcription Kit (manufactured by Invitrogen, Catalog No. AMB13345): 10%, and T7 Reaction Buffer attached to MEGAScript T7 Transcription Kit: 10%. The reaction solution was prepared to 400 µL, and incubated at 37°C for 6 hours. Subsequently, Trubo DNase attached to MEGAScript T7 Transcription Kit was added thereto in an amount corresponding to 1/20 in volume of the reaction solution to be mixed, followed by shaking at 37°C for 15 minutes. The resultant was roughly purified by phenol chloroform extraction and ethanol precipitation to obtain an RNA fragment. The thus obtained RNA fragment was used to perform a capping reaction as follows with Vaccinia Capping System (manufactured by New England Biolab, Catalog No. MB2080S), and ScriptCap 2'-O-Methyltransferase Kit (manufactured by CELLSCRIPT, Catalog No. C-SCMT0625). Final concentrations in the resultant reaction solution were as follows: RNA fragment: 500 ng/µL, Capping Buffer: 10%, GTP: 0.5 mM, SAM: 0.2 mM, Vaccinia capping enzyme: 0.5 U/µL, RNase inhibitor: 1 U/µL, and 2'-O-Methyltransferase: 2.5 U/µL. The reaction solution was prepared to 2000 µL with Nuclease-free water, and the resultant was allowed to stand still at 37°C for 1 hour. The resultant was purified by phenol chloroform extraction and ethanol precipitation to obtain a capped RNA fragment. Thereafter, to 105 µL of the thus obtained capped RNA fragment, 7 µL of Antarctic Phosphatase (manufactured by New England Biolab, Catalog No. M0289S), 14 µL of 10 x Antarctic Phosphatase Buffer, and 14 µL of Nuclease-free water were added to be mixed, and the resultant was allowed to stand still at 37°C for 1 hour to perform alkali phosphatase reaction. A reaction sample thus obtained was purified in the same manner as in Example 1 (Purification of RNA Fragment with dPAGE) to obtain 3.04 nmol of a capped mRNA IVT-1. A sequence of the thus obtained IVT-1 is shown in Table 17.

In Table 17, each nucleotide N (upper case) indicates an RNA, N(M) indicates a 2'-O-methyl modified RNA, and m7Gppp indicates the following structural formula:

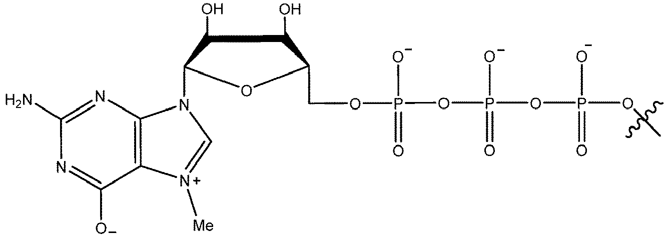

**[Table 17]**

| Compound Name | Sequence (5' to 3') | SEQ ID NO: |
|---|---|---|
| IVT-1 | m7GpppG(M)GGAGAAUACAAGCUACUUGUUCUUUUUGCAGCCACCAUG GACUACAAGGACGACGACGACAAGAUCAUCGACUAUAAAGACGACGAC GAUAAAGGUGGCGACUAUAAGGACGACGACGACAAACACCACCACCAC CACCACUGAAAAAAAAAAAAAAAAAAAAA | 447 |

### Test Example 10

### (Translation Reaction Test of mRNA Sample with Hela Cell Lysate)

Respective mRNAs shown in Table 18-1 to Table 18-10 below were evaluated for translation activity in a human cell system in the same manner as in Test Example 1. A translation product concentration (nM) in a translation reaction solution in which each mRNA was added in a concentration of 0.3 µM is shown in Table 18 below.

Table 18-1 to Table 18-10:

**[Table 18-1]**

| Translation product concentration obtained from each mRNA | | |
|---|---|---|
| mRNA Name | SEQ ID NO: | Translation Product Concentration (nM) |
| E4 | 11 | 2.167 |
| E193 | 374 | 50.167 |
| E194 | 377 | 71.333 |
| E195 | 380 | 90.833 |
| E196 | 383 | 4.500 |
| E197 | 386 | 2.167 |

**[Table 18-2]**

| Translation product concentration obtained from each mRNA | | |
|---|---|---|
| mRNA Name | SEQ ID NO: | Translation Product Concentration (nM) |
| E4 | 11 | 0.500 |
| E193 | 374 | 27.833 |
| E194 | 377 | 44.333 |
| E195 | 380 | 37.667 |
| E198 | 389 | 49.000 |
| E199 | 392 | 43.833 |

**[Table 18-3]**

| Translation product concentration obtained from each mRNA | | |
|---|---|---|
| mRNA Name | SEQ ID NO: | Translation Product Concentration (nM) |
| E4 | 11 | 0.833 |
| E65 | 180 | 24.500 |
| E67 | 186 | 20.667 |
| E68 | 189 | 8.167 |
| E180 | 343 | 13.500 |
| E181 | 346 | 21.833 |

**[Table 18-4]**

| Translation product concentration obtained from each mRNA | | |
|---|---|---|
| mRNA Name | SEQ ID NO: | Translation Product Concentration (nM) |
| E4 | 11 | 1.000 |
| E175 | 338 | 17.333 |
| E177 | 340 | 15.167 |
| E178 | 341 | 17.333 |
| E180 | 343 | 21.167 |
| E165 | 308 | 70.500 |

**[Table 18-5]**

| Translation product concentration obtained from each mRNA | | |
|---|---|---|
| mRNA Name | SEQ ID NO: | Translation Product Concentration (nM) |
| E4 | 11 | 1.000 |
| E176 | 339 | 5.500 |
| E177 | 340 | 17.333 |
| E179 | 342 | 8.167 |
| E180 | 343 | 22.000 |
| E167 | 314 | 25.667 |

**[Table 18-6]**

| Translation product concentration obtained from each mRNA | | |
|---|---|---|
| mRNA Name | SEQ ID NO: | Translation Product Concentration (nM) |
| E58 | 161 | 61.167 |
| E59 | 164 | 66.000 |
| E164 | 305 | 108.167 |
| E192 | 373 | 1.333 |

**[Table 18-7]**

| Translation product concentration obtained from each mRNA | | |
|---|---|---|
| mRNA Name | SEQ ID NO: | Translation Product Concentration (nM) |
| E182 | 349 | 1.167 |
| E183 | 350 | 57.167 |
| E184 | 353 | 75.167 |
| E185 | 356 | 69.000 |

**[Table 18-8]**

| Translation product concentration obtained from each mRNA | | |
|---|---|---|
| mRNA Name | SEQ ID NO: | Translation Product Concentration (nM) |
| E186 | 359 | 0.000 |
| E187 | 360 | 0.500 |
| E188 | 363 | 0.500 |

**[Table 18-9]**

| Translation product concentration obtained from each mRNA | | |
|---|---|---|
| mRNA Name | SEQ ID NO: | Translation Product Concentration (nM) |
| E189 | 366 | 1.667 |
| E190 | 367 | 10.000 |
| E191 | 370 | 6.333 |

**[Table 18-10]**

| Translation product concentration obtained from each mRNA | | |
|---|---|---|
| mRNA Name | SEQ ID NO: | Translation Product Concentration (nM) |
| E217 | 416 | 0.000 |
| E218 | 422 | 1.000 |
| E219 | 426 | 1.000 |

As is obvious from the test results shown in Table 18-1 to Table 18-10 above, each mRNA having sugar modification produced, after being added to the Hela cell lysate, a polypeptide encoded by a gene sequence in the eukaryotic cell translation system.

### Test Example 11

### (in vitro Translation Reaction Test of mRNA Sample with Hela Cell Line)

Respective mRNAs shown in Table 19-1 to Table 19-13 below were evaluated for translation activity *in vitro* with Hela cell line of a human cell system in the same manner as in Test Example 2. A translation product concentration (nM) in a cell lysate obtained from a cell 5 hours after adding each mRNA in a concentration of 3 to 30 nm is shown in Table 19 below.

Table 19-1 to Table 19-13:

**[Table 19-1]**

| Translation product concentration obtained from each mRNA | | | | |
|---|---|---|---|---|
| mRNA Name | SEQ ID NO: | Translation Product Concentration (nM) | | |
| | | mRNA 3 nM | mRNA10 nM | mRNA30 nM |
| E4 | 11 | 0.000 | 0.003 | 0.010 |
| E165 | 308 | 0.097 | 0.667 | 1.363 |
| E166 | 311 | 0.027 | 0.390 | 0.920 |
| E167 | 314 | 0.070 | 0.637 | 1.170 |
| E169 | 320 | 0.073 | 0.527 | 1.070 |
| E172 | 329 | 0.050 | 0.487 | 1.087 |

**[Table 19-2]**

| Translation product concentration obtained from each mRNA | | | | |
|---|---|---|---|---|
| mRNA Name | SEQ ID NO: | Translation Product Concentration (nM) | | |
| | | mRNA 3 nM | mRNA10 nM | mRNA30 nM |
| E4 | 11 | 0.000 | 0.010 | 0.010 |
| E166 | 311 | 0.030 | 0.517 | 1.100 |
| E167 | 314 | 0.070 | 0.707 | 1.263 |
| E168 | 317 | 0.023 | 0.390 | 0.650 |
| E171 | 326 | 0.017 | 0.233 | 0.570 |
| E173 | 332 | 0.040 | 0.513 | 0.813 |

**[Table 19-3]**

| Translation product concentration obtained from each mRNA | | | | |
|---|---|---|---|---|
| mRNA Name | SEQ ID NO: | Translation Product Concentration (nM) | | |
| | | mRNA 3 nM | mRNA10 nM | mRNA30 nM |
| E4 | 11 | 0.000 | 0.010 | 0.010 |
| E165 | 308 | 0.090 | 0.887 | 2.093 |
| E167 | 314 | 0.060 | 0.667 | 1.620 |
| E169 | 320 | 0.060 | 0.520 | 1.123 |
| E170 | 323 | 0.030 | 0.363 | 0.933 |
| E174 | 335 | 0.060 | 0.513 | 0.963 |

**[Table 19-4]**

| Translation product concentration obtained from each mRNA | | | | |
|---|---|---|---|---|
| mRNA Name | SEQ ID NO: | Translation Product Concentration (nM) | | |
| | | mRNA 3 nM | mRNA10 nM | mRNA30 nM |
| E4 | 11 | 0.000 | 0.010 | 0.010 |
| E193 | 374 | 0.010 | 0.073 | 0.187 |
| E194 | 377 | 0.010 | 0.153 | 0.227 |
| E195 | 380 | 0.010 | 0.090 | 0.143 |
| E196 | 383 | 0.000 | 0.000 | 0.000 |
| E197 | 386 | 0.000 | 0.000 | 0.000 |

**[Table 19-5]**

| Translation product concentration obtained from each mRNA | | | | |
|---|---|---|---|---|
| mRNA Name | SEQ ID NO: | Translation Product Concentration (nM) | | |
| | | mRNA 3 nM | mRNA10 nM | mRNA30 nM |
| E4 | 11 | 0.000 | 0.000 | 0.003 |
| E193 | 374 | 0.010 | 0.087 | 0.143 |
| E194 | 377 | 0.013 | 0.123 | 0.210 |
| E195 | 380 | 0.010 | 0.077 | 0.123 |
| E198 | 389 | 0.060 | 0.370 | 0.497 |
| E199 | 392 | 0.057 | 0.353 | 0.453 |

**[Table 19-6]**

| Translation product concentration obtained from each mRNA | | | | |
|---|---|---|---|---|
| mRNA Name | SEQ ID NO: | Translation Product Concentration (nM) | | |
| | | mRNA 3 nM | mRNA10 nM | mRNA30 nM |
| E4 | 11 | 0.000 | 0.010 | 0.010 |
| E65 | 180 | 0.087 | 0.657 | 0.940 |
| E67 | 186 | 0.057 | 0.700 | 0.570 |
| E68 | 189 | 0.020 | 0.337 | 0.190 |
| E180 | 343 | 0.000 | 0.050 | 0.207 |
| E181 | 346 | 0.000 | 0.153 | 0.413 |

**[Table 19-7]**

| Translation product concentration obtained from each mRNA | | | | |
|---|---|---|---|---|
| mRNA Name | SEQ ID NO: | Translation Product Concentration (nM) | | |
| | | mRNA 3 nM | mRNA10 nM | mRNA30 nM |
| E4 | 11 | 0.000 | 0.000 | 0.010 |
| E175 | 338 | 0.000 | 0.010 | 0.040 |
| E177 | 340 | 0.023 | 0.133 | 0.323 |
| E178 | 341 | 0.010 | 0.037 | 0.130 |
| E180 | 343 | 0.000 | 0.020 | 0.113 |
| E165 | 308 | 0.053 | 0.327 | 0.940 |

**[Table 19-8]**

| Translation product concentration obtained from each mRNA | | | | |
|---|---|---|---|---|
| mRNA Name | SEQ ID NO: | Translation Product Concentration (nM) | | |
| | | mRNA 3 nM | mRNA10 nM | mRNA30 nM |
| E4 | 11 | 0.000 | 0.000 | 0.010 |
| E176 | 339 | 0.000 | 0.000 | 0.050 |
| E177 | 340 | 0.017 | 0.150 | 0.360 |
| E179 | 342 | 0.000 | 0.020 | 0.097 |
| E180 | 343 | 0.000 | 0.020 | 0.077 |
| E167 | 314 | 0.033 | 0.370 | 0.957 |

**[Table 19-9]**

| Translation product concentration obtained from each mRNA | | | | |
|---|---|---|---|---|
| mRNA Name | SEQ ID NO: | Translation Product Concentration (nM) | | |
| | | mRNA 3 nM | mRNA10 nM | mRNA30 nM |
| E58 | 161 | 0.010 | 0.153 | 0.177 |
| E59 | 164 | 0.090 | 0.737 | 1.147 |
| E164 | 305 | 0.010 | 0.143 | 0.167 |
| E192 | 373 | 0.020 | 0.117 | 0.177 |

**[Table 19-10]**

| Translation product concentration obtained from each mRNA | | | | |
|---|---|---|---|---|
| mRNA Name | SEQ ID NO: | Translation Product Concentration (nM) | | |
| | | mRNA 3 nM | mRNA10 nM | mRNA30 nM |
| E182 | 349 | 0.000 | 0.000 | 0.010 |
| E183 | 350 | 0.013 | 0.153 | 0.930 |
| E184 | 353 | 0.020 | 0.163 | 1.223 |
| E185 | 356 | 0.020 | 0.160 | 0.983 |

**[Table 19-11]**

| Translation product concentration obtained from each mRNA | | | | |
|---|---|---|---|---|
| mRNA Name | SEQ ID NO: | Translation Product Concentration (nM) | | |
| | | mRNA 3 nM | mRNA10 nM | mRNA30 nM |
| E186 | 359 | 0.000 | 0.000 | 0.000 |
| E187 | 360 | 0.000 | 0.010 | 0.037 |
| E188 | 363 | 0.000 | 0.010 | 0.020 |

**[Table 19-12]**

| Translation product concentration obtained from each mRNA | | | | |
|---|---|---|---|---|
| mRNA Name | SEQ ID NO: | Translation Product Concentration (nM) | | |
| | | mRNA 3 nM | mRNA10 nM | mRNA30 nM |
| E189 | 366 | 0.000 | 0.000 | 0.010 |
| E190 | 367 | 0.040 | 0.283 | 1.170 |
| E191 | 370 | 0.033 | 0.300 | 1.070 |

**[Table 19-13]**

| Translation product concentration obtained from each mRNA | | | | |
|---|---|---|---|---|
| mRNA Name | SEQ ID NO: | Translation Product Concentration (nM) | | |
| | | mRNA 3 nM | mRNA10 nM | mRNA30 nM |
| E217 | 416 | 0.000 | 0.007 | 0.010 |
| E218 | 422 | 0.010 | 0.073 | 0.320 |
| E219 | 426 | 0.010 | 0.077 | 0.287 |

As is obvious from the test results shown in Table 19-1 to Table 19-13 above, each mRNA having sugar modification produced, after being added to the Hela cell, a polypeptide encoded by a gene sequence, and the translation level was more excellent than that of an mRNA having no sugar modification.

### Test Example 12

### (in vitro Translation Reaction Test of mRNA Sample with Hela Cell Line)

Respective mRNAs shown in Table 20-1 to Table 20-8 below were evaluated for persistence of translation activity *in vitro* with Hela cell line in the same manner as in Test Example 3. A translation product concentration (nM) in a cell lysate obtained from a cell in which each mRNA was added in a concentration of 30 nM is shown in Table 20 below.

Table 20-1 to Table 20-8:

**[Table 20-1]**

| Translation product concentration obtained from each mRNA | | | | |
|---|---|---|---|---|
| mRNA Name | SEQ ID NO: | Translation Product Concentration (nM) | | |
| | | 5 Hours After Adding mRNA | 8 Hours After Adding mRNA | 24 Hours After Adding mRNA |
| E4 | 11 | 0.010 | 0.010 | 0.000 |
| E167 | 314 | 1.263 | 1.513 | 1.047 |
| E169 | 320 | 1.270 | 1.453 | 0.933 |
| E170 | 323 | 1.000 | 1.127 | 0.770 |
| E174 | 335 | 0.940 | 1.043 | 0.717 |

**[Table 20-2]**

| Translation product concentration obtained from each mRNA | | | | |
|---|---|---|---|---|
| mRNA Name | SEQ ID NO: | Translation Product Concentration (nM) | | |
| | | 5 Hours After Adding mRNA | 8 Hours After Adding mRNA | 24 Hours After Adding mRNA |
| E172 | 329 | 0.983 | 1.147 | 0.673 |
| E173 | 332 | 0.830 | 1.010 | 0.693 |

**[Table 20-3]**

| Translation product concentration obtained from each mRNA | | | | |
|---|---|---|---|---|
| mRNA Name | SEQ ID NO: | Translation Product Concentration (nM) | | |
| | | 5 Hours After Adding mRNA | 8 Hours After Adding mRNA | 24 Hours After Adding mRNA |
| E4 | 11 | 0.010 | 0.010 | 0.003 |
| E175 | 338 | 0.070 | 0.050 | 0.010 |
| E177 | 340 | 0.347 | 0.477 | 0.403 |
| E178 | 341 | 0.203 | 0.253 | 0.130 |
| E180 | 343 | 0.300 | 0.127 | 0.000 |
| E165 | 308 | 1.437 | 1.440 | 0.817 |

**[Table 20-4]**

| Translation product concentration obtained from each mRNA | | | | |
|---|---|---|---|---|
| mRNA Name | SEQ ID NO: | Translation Product Concentration (nM) | | |
| | | 5 Hours After Adding mRNA | 8 Hours After Adding mRNA | 24 Hours After Adding mRNA |
| E4 | 11 | 0.010 | 0.010 | 0.010 |
| E176 | 339 | 0.060 | 0.067 | 0.030 |
| E177 | 340 | 0.303 | 0.307 | 0.187 |
| E179 | 342 | 0.103 | 0.080 | 0.030 |
| E180 | 343 | 0.233 | 0.130 | 0.010 |
| E167 | 314 | 1.220 | 1.357 | 0.767 |

**[Table 20-5]**

| Translation product concentration obtained from each mRNA | | | | |
|---|---|---|---|---|
| mRNA Name | SEQ ID NO: | Translation Product Concentration (nM) | | |
| | | 5 Hours After Adding mRNA | 8 Hours After Adding mRNA | 24 Hours After Adding mRNA |
| E182 | 349 | 0.010 | 0.010 | 0.000 |
| E184 | 353 | 1.483 | 1.663 | 0.933 |
| E185 | 356 | 1.030 | 1.230 | 0.790 |

**[Table 20-6]**

| Translation product concentration obtained from each mRNA | | | | |
|---|---|---|---|---|
| mRNA Name | SEQ ID NO: | Translation Product Concentration (nM) | | |
| | | 5 Hours After Adding mRNA | 8 Hours After Adding mRNA | 24 Hours After Adding mRNA |
| E217 | 416 | 0.003 | 0.007 | 0.000 |
| E218 | 422 | 0.360 | 0.537 | 0.317 |
| E219 | 426 | 0.307 | 0.463 | 0.317 |

**[Table 20-7]**

| Translation product concentration obtained from each mRNA | | | | |
|---|---|---|---|---|
| mRNA Name | SEQ ID NO: | Translation Product Concentration (nM) | | |
| | | 5 Hours After Adding mRNA | 8 Hours After Adding mRNA | 24 Hours After Adding mRNA |
| E189 | 366 | 0.017 | 0.010 | 0.010 |
| E190 | 367 | 1.717 | 1.820 | 1.260 |
| E191 | 370 | 1.303 | 1.390 | 0.933 |

**[Table 20-8]**

| Translation product concentration obtained from each mRNA | | | | |
|---|---|---|---|---|
| mRNA Name | SEQ ID NO: | Translation Product Concentration (nM) | | |
| | | 5 Hours After Adding mRNA | 8 Hours After Adding mRNA | 24 Hours After Adding mRNA |
| E79 | 206 | 0.910 | 1.313 | 0.873 |
| IVT-1 | 447 | 0.010 | 0.010 | 0.000 |

As is obvious from the test results shown in Table 20-1 to Table 20-8 above, each mRNA having sugar modification produced, after being added to the Hela cell, a polypeptide encoded by a gene sequence, and the translation level was more excellent than that of an mRNA having no sugar modification.

### Test Example 13

### (in vitro Translation Reaction Test of mRNA Sample with Hela Cell Line)

Respective mRNAs shown in Table 21-1 to Table 21-4 below were evaluated for translation activity *in vitro* with Hela cell line in the same manner as in Test Example 4. A translation product concentration (nM) in a cell lysate obtained from a cell in which each mRNA was added is shown in Table 21 below.

Table 21-1 to Table 21-4:

**[Table 21-1]**

| Translation product concentration obtained from each mRNA | | | | |
|---|---|---|---|---|
| mRNA Name | SEQ ID NO: | Translation Product Concentration (nM) | | |
| | | 3 Hours After Introducing mRNA | 8 Hours After Introducing mRNA | 24 Hours After Introducing mRNA |
| E4 | 11 | 0.000 | 0.000 | 0.000 |
| E165 | 308 | 0.323 | 0.337 | 0.117 |
| E167 | 314 | 0.343 | 0.403 | 0.207 |
| E169 | 320 | 0.300 | 0.350 | 0.130 |
| E173 | 332 | 0.220 | 0.300 | 0.147 |
| E174 | 335 | 0.187 | 0.223 | 0.097 |

**[Table 21-2]**

| Translation product concentration obtained from each mRNA | | | | |
|---|---|---|---|---|
| mRNA Name | SEQ ID NO: | Translation Product Concentration (nM) | | |
| | | 3 Hours After Introducing mRNA | 8 Hours After Introducing mRNA | 24 Hours After Introducing mRNA |
| E4 | 11 | 0.000 | 0.000 | 0.000 |
| E165 | 308 | 0.440 | 0.447 | 0.187 |
| E166 | 311 | 0.367 | 0.397 | 0.150 |
| E172 | 329 | 0.317 | 0.363 | 0.133 |
| E180 | 343 | 0.007 | 0.000 | 0.000 |
| E181 | 346 | 0.010 | 0.000 | 0.000 |

**[Table 21-3]**

| Translation product concentration obtained from each mRNA | | | | |
|---|---|---|---|---|
| mRNA Name | SEQ ID NO: | Translation Product Concentration (nM) | | |
| | | 3 Hours After Introducing mRNA | 8 Hours After Introducing mRNA | 24 Hours After Introducing mRNA |
| E4 | 11 | 0.000 | 0.000 | 0.000 |
| E175 | 338 | 0.020 | 0.010 | 0.000 |
| E177 | 340 | 0.113 | 0.290 | 0.207 |
| E178 | 341 | 0.063 | 0.097 | 0.057 |
| E180 | 343 | 0.013 | 0.000 | 0.000 |
| E165 | 308 | 0.460 | 0.497 | 0.233 |
| E181 | 346 | 0.010 | 0.000 | 0.000 |

**[Table 21-4]**

| Translation product concentration obtained from each mRNA | | | | |
|---|---|---|---|---|
| mRNA Name | SEQ ID NO: | Translation Product Concentration (nM) | | |
| | | 3 Hours After Introducing mRNA | 8 Hours After Introducing mRNA | 24 Hours After Introducing mRNA |
| E79 | 206 | 0.437 | 0.533 | 0.343 |
| IVT-1 | 447 | 0.000 | 0.000 | 0.000 |

As is obvious from the test results shown in Table 21-1 to Table 21-4 above, each mRNA having sugar modification produced, after being electroporated into the Hela cell, a polypeptide encoded by a gene sequence, and the activity was more excellent than that of an mRNA having no sugar modification in the translated region.

### Test Example 14

### (Translation Reaction Test of mRNA Sample with Hela Cell Lysate)

Respective mRNAs shown in Table 22-1 to Table 22-2 below were evaluated for translation activity in a human cell system in the same manner as in Test Example 8. A translation product concentration (nM) in a translation reaction solution obtained by adding each mRNA in a concentration of 1 µM is shown in Table 22 below.

Table 22-1 to Table 22-2:

**[Table 22-1]**

| Translation product concentration obtained from each mRNA | | | |
|---|---|---|---|
| mRNA Name | SEQ ID NO: | Translation Product Concentration (nM) | Relative Amount of Translation Product |
| E61 | 170 | 24.693 | 5.02 |
| E71 | 198 | 15.920 | 3.24 |
| E85 | 224 | 33.347 | 6.78 |
| E204 | 399 | 15.040 | 3.06 |
| E205 | 400 | 16.013 | 3.25 |
| E206 | 401 | 20.107 | 4.09 |
| E207 | 402 | 14.013 | 2.85 |
| E208 | 403 | 10.827 | 2.20 |
| E30 | 89 | 4.920 | 1.00 |

**[Table 22-2]**

| Translation product concentration obtained from each mRNA | | | |
|---|---|---|---|
| mRNA Name | SEQ ID NO: | Translation Product Concentration (nM) | Relative Amount of Translation Product |
| E61 | 170 | 25.067 | 5.47 |
| E127 | 268 | 25.253 | 5.51 |
| E209 | 404 | 27.840 | 6.07 |
| E210 | 405 | 16.213 | 3.53 |
| E211 | 406 | 36.120 | 7.88 |
| E212 | 407 | 14.000 | 3.05 |
| E213 | 408 | 10.493 | 2.29 |
| E214 | 409 | 14.840 | 3.24 |
| E30 | 89 | 4.587 | 1.00 |

As is obvious from the test results shown in Table 22-1 to Table 22-2 above, each mRNA produced, after being added to the Hela cell lysate, a polypeptide encoded by a gene sequence in the eukaryotic cell translation system.

### Test Example 15

### (Test of Intracellular Nucleic Acid Stability of mRNA Sample with Hela Cell Line)

Respective mRNAs shown in Table 23 below were evaluated for intracellular nucleic acid stability with Hela cell line. The culture of cell and introduction of the mRNA were performed in the same manner as in Test Example 3 with each mRNA introduced to be prepared to a final concentration of 30 nM. A culture supernatant was removed from the cell cultured for 4 hours after adding each mRNA, RPMI medium (manufactured by Nacalai Tesque, Inc.) containing 50 µL of 10% fetal bovine serum per well was added thereto, and the culture was continued at 37°C under 5% CO2 condition. For the cells obtained respectively after 4 hours, 8 hours, and 24 hours after the addition of the mRNA, a cell lysis operation was performed as follows. Specifically, after removing a culture supernatant from the cell, the resultant was washed once with ice cooled D-PBS(-) (manufactured by Nacalai Tesque, Inc.), iScript RT-qPCR Sample Preparation Reagent (BIORAD, 1708898) containing 2% protease inhibitor cocktail (for an animal cell extract, manufactured by Nacalai Tesque, Inc.) was added thereto in an amount of 20 µL per well, and the resultant was vigorously shaken for 30 seconds for cell lysis.

A remaining amount of mRNA in the thus obtained cell lysate was detected by RT-qPCR method as follows: First, DW was prepared as a sample dilution solution by adding Ribonuclease Inhibitor thereto to a final concentration of 0.2 U/mL. A calibration curve was created for each of mRNAs evaluated, and dilution series were produced by each mRNA with a solution obtained by diluting the cell lysate prepared from a cell in which no nucleic acid was added with the sample dilution solution 10 fold to obtain 11 concentrations from 1 µM with 4-fold dilution. Each cell lysate to be measured was diluted 10 fold with the sample dilution solution. The calibration curve and the cell lysate to be measured were diluted 1071 fold with the DW to which Ribonuclease Inhibitor was added to a final concentration of 0.2 U/mL. A reverse transcription reaction and RT-qPCR reaction thereafter were performed in the same manner as in Test Example 7. As results of the measurement, a concentration of each mRNA in each sample was quantitatively determined by using a calibration curve based on a CT value of a preparation, which is shown in Table 23 below.

Table 23:

**[Table 23]**

| mRNA Remaining Concentration in Cell Lysate at Each Time Point | | | | |
|---|---|---|---|---|
| mRNA Name | SEQ ID NO: | mRNA Remaining Concentration (nM) | | |
| | | 4 Hours After Introducing mRNA | 8 Hours After Introducing mRNA | 24 Hours After Introducing mRNA |
| E79 | 206 | 12.990 | 22.005 | 11.253 |
| E95 | 234 | 10.980 | 12.803 | 9.763 |
| IVT-1 | 447 | 60.214 | 0.433 | 0.129 |

As is obvious from the test results shown in Table 23 above, an mRNA having sugar modification was improved in degradation resistance in cell as compared with an mRNA prepared by IVT.

### Test Example 16

### (Translation Reaction Test of mRNA Sample with Hela Cell Lysate)

Respective mRNAs shown in Table 24 below were evaluated for translation activity in a human cell system in the same manner as in Test Example 1. A translation product concentration (nM) in a translation reaction solution obtained by adding each mRNA in a concentration of 0.3 µM is shown in Table 24 below.

**[Table 24]**

| Translation product concentration obtained from each mRNA | | |
|---|---|---|
| mRNA Name | SEQ ID NO: | Translation Product Concentration (nM) |
| E3 | 8 | 0.500 |
| E36 | 103 | 0.500 |
| E220 | 448 | 0.333 |
| E222 | 454 | 0.833 |
| E221 | 451 | 0.500 |
| E223 | 457 | 11.833 |
| E4 | 11 | 1.333 |
| E65 | 180 | 29.333 |
| E224 | 460 | 7.167 |
| E225 | 463 | 32.000 |

As is obvious from the test results shown in Table 24, each mRNA having sugar modification produced, after being added to the Hela cell lysate, a polypeptide encoded by a gene sequence in the eukaryotic cell translation system.

### Test Example 17

### (in vitro Translation Reaction Test of mRNA Sample with Hela Cell Line)

Respective mRNAs shown in Table 25 below were evaluated for *in vitro* translation activity with Hela cell line of a human cell system in the same manner as in Test Example 2. A translation product concentration (nM) in a cell lysate obtained from a cell 5 hours after adding each mRNA in a concentration of 3 to 30 nM is shown in Table 25 below.

**[Table 25]**

| Translation product concentration obtained from each mRNA | | | | |
|---|---|---|---|---|
| mRNA Name | SEQ ID NO: | Translation Product Concentration (nM) | | |
| | | mRNA 3 nM | mRNA10 nM | mRNA30 nM |
| E3 | 8 | 0.000 | 0.000 | 0.000 |
| E36 | 103 | 0.000 | 0.000 | 0.000 |
| E220 | 448 | 0.000 | 0.000 | 0.000 |
| E222 | 454 | 0.000 | 0.000 | 0.010 |
| E221 | 451 | 0.000 | 0.000 | 0.000 |
| E223 | 457 | 0.010 | 0.083 | 0.203 |
| E4 | 11 | 0.000 | 0.000 | 0.010 |
| E65 | 180 | 0.063 | 0.353 | 0.450 |
| E224 | 460 | 0.020 | 0.127 | 0.300 |
| E225 | 463 | 0.053 | 0.400 | 0.797 |
| E192 | 373 | 0.010 | 0.073 | 0.103 |
| E59 | 164 | 0.080 | 0.517 | 0.747 |

E222 having a length of sugar modified poly A chain of 5, E223 having a length of sugar modified poly A chain of 10, E65, E224, and E225 having a length of sugar modified poly A chain of 20, and E59 having a length of sugar modified poly A chain of 40 exhibited more excellent translation potential respectively than mRNAs containing unmodified poly A chains having the same lengths.

### Test Example 18

### (in vitro Translation Reaction Test of mRNA Sample with Hela Cell Line)

Respective mRNAs shown in Table 26 below were evaluated for persistence of translation activity *in vitro* with Hela cell line in the same manner as in Test Example 3. A translation product concentration (nM) in a cell lysate obtained from a cell in which each mRNA was added in a concentration of 30 nM is shown in Table 26 below.

**[Table 26]**

| Translation product concentration obtained from each mRNA | | | | |
|---|---|---|---|---|
| mRNA Name | SEQ ID NO: | Translation Product Concentration (nM) | | |
| | | 5 Hours After Adding mRNA | 8 Hours After Adding mRNA | 24 Hours After Adding mRNA |
| E3 | 8 | 0.000 | 0.000 | 0.000 |
| E36 | 103 | 0.000 | 0.000 | 0.000 |
| E220 | 448 | 0.000 | 0.000 | 0.000 |
| E222 | 454 | 0.010 | 0.010 | 0.010 |
| E221 | 451 | 0.000 | 0.000 | 0.000 |
| E223 | 457 | 0.310 | 0.380 | 0.263 |
| E4 | 11 | 0.000 | 0.000 | 0.000 |
| E65 | 180 | 0.560 | 0.693 | 0.460 |
| E224 | 460 | 0.370 | 0.507 | 0.337 |
| E225 | 463 | 0.983 | 1.307 | 0.907 |
| E192 | 373 | 0.107 | 0.120 | 0.050 |
| E59 | 164 | 0.803 | 0.997 | 0.623 |

As is obvious from the test results shown in Table 26 above, each mRNA having sugar modification produced, after being added to the Hela cell, a polypeptide encoded by a gene sequence, and the translation level was more excellent in accordance with the length of the poly A chain than that of an mRNA having no sugar modification.

### Test Example 19

### (Translation Reaction Test of mRNA Sample with Hela Cell Lysate)

Respective mRNAs shown in Table 27-1 to Table 27-5 below were evaluated for translation activity in a human cell system in the same manner as in Test Example 8. A translation product concentration (nM) in a translation reaction solution obtained by adding each mRNA in a concentration of 1 µM, and a relative amount of the translation product calculated assuming that the amount obtained from E226 or E30, that is, an mRNA having no sugar modification, is 1 are shown in Table 27-1 to Table 27-5 below.

Table 27-1 to Table 27-5:

**[Table 27-1]**

| mRNA Name | SEQ ID NO: | Translation Product Concentration (nM) | Relative Amount of Translation Product |
|---|---|---|---|
| E226 | 466 | 0.987 | 1.00 |
| E227 | 467 | 1.787 | 1.81 |
| E228 | 468 | 2.560 | 2.59 |
| E229 | 469 | 2.347 | 2.38 |

**[Table 27-2]**

| mRNA Name | SEQ ID NO: | Translation Product Concentration (nM) | Relative Amount of Translation Product |
|---|---|---|---|
| E30 | 89 | 0.333 | 1.00 |
| E230 | 470 | 1.347 | 4.04 |
| E231 | 471 | 1.520 | 4.56 |
| E232 | 472 | 1.173 | 3.52 |
| E233 | 473 | 0.640 | 1.92 |

**[Table 27-3]**

| mRNA Name | SEQ ID NO: | Translation Product Concentration (nM) | Relative Amount of Translation Product |
|---|---|---|---|
| E30 | 89 | 0.680 | 1.00 |
| E230 | 470 | 2.347 | 3.45 |
| E231 | 471 | 2.080 | 3.06 |
| E234 | 474 | 1.333 | 1.96 |

**[Table 27-4]**

| mRNA Name | SEQ ID NO: | Translation Product Concentration (nM) | Relative Amount of Translation Product |
|---|---|---|---|
| E30 | 89 | 0.720 | 1.00 |
| E230 | 470 | 2.307 | 3.20 |
| E235 | 475 | 1.867 | 2.59 |
| E236 | 476 | 1.000 | 1.39 |
| E237 | 477 | 2.107 | 2.93 |

**[Table 27-5]**

| mRNA Name | SEQ ID NO: | Translation Product Concentration (nM) |
|---|---|---|
| E230 | 470 | 2.227 |
| E235 | 475 | 1.453 |
| E237 | 477 | 2.040 |
| E238 | 478 | 2.253 |
| E239 | 479 | 1.587 |
| E240 | 480 | 2.413 |

As is obvious from the test results shown in Table 27-1 to Table 27-5 above, each mRNA produced, after being added to the Hela cell lysate, a polypeptide encoded by a gene sequence in the eukaryotic cell translation system, and the translation level was more excellent than that of an mRNA having no sugar modification.

## Claims

1. A polynucleotide, comprising:
a translated region from a start codon to a stop codon,
a 5' untranslated region, and
a poly A chain,
wherein 65% or more of nucleotides contained in the poly A chain are sugar modified nucleotides.

2. The polynucleotide according to claim 1, wherein all the nucleotides contained in the poly A chain are sugar modified nucleotides.

3. The polynucleotide according to claim 1 or 2, wherein modified sugar portions of the sugar modified nucleotides are each independently selected from the following structures:

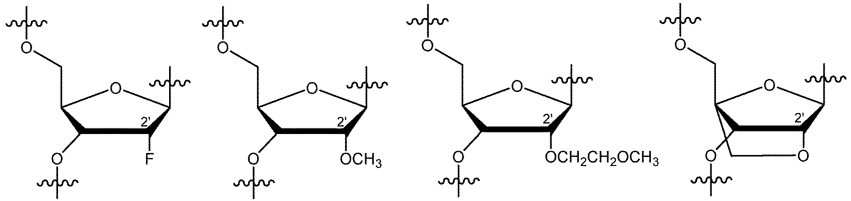

4. The polynucleotide according to any one of claims 1 to 3, wherein modified sugar portions of the sugar modified nucleotides are each independently selected from the following structures:

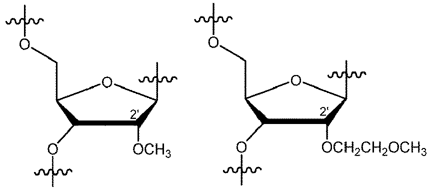

5. The polynucleotide according to any one of claims 1 to 4, wherein the poly A chain contains at least one phosphate modified nucleotide.

6. The polynucleotide according to any one of claims 1 to 5, wherein first to second nucleotides, first to third nucleotides, first to fourth nucleotides, or first to fifth nucleotides from the 3' end of the poly A chain are linked to one another via phosphorothioate.

7. The polynucleotide according to any one of claims 1 to 6, wherein all the nucleotides contained in the poly A chain are linked to one another via phosphorothioate.

8. The polynucleotide according to any one of claims 1 to 7, wherein the poly A chain has a length of 2 to 40 bases.

9. The polynucleotide according to any one of claims 1 to 8, wherein nucleotides of the 5' untranslated region are each independently selected from a 2'-deoxyribonucleotide, and a spacer modified or sugar modified nucleotide.

10. The polynucleotide according to any one of claims 1 to 9, wherein first to sixth nucleotides from the 5' end of the 5 untranslated region are sugar modified nucleotides, and modified sugar portions of the sugar modified nucleotides have the following structure:

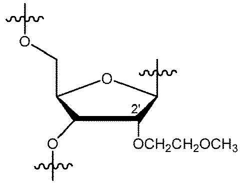

11. The polynucleotide according to claim 10, further comprising, on the 5' side of the 5' end of the 5' untranslated region, a portion containing 1 to 10 non-sugar modified nucleotides.

12. The polynucleotide according to any one of claims 1 to 11, wherein nucleotides excluding first to sixth nucleotides from the 5' end of the 5' untranslated region include a 2'-deoxyribonucleotide and/or spacer modification.

13. The polynucleotide according to claim 9 or 12,
wherein the 5' untranslated region includes spacer modifications, and the spacer modifications are each independently selected from the following structures: 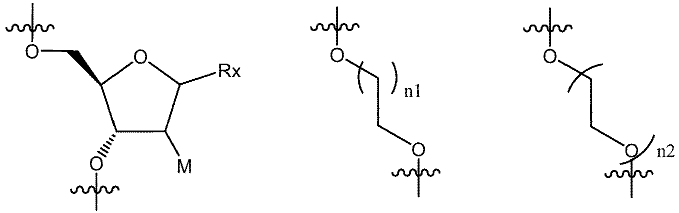
wherein Rx is ethynyl, a hydrogen atom, or OH,
M is a hydrogen atom or OH,
n1 is 1, 2, or 5, and
n2 is 1, 2, or 3.

14. The polynucleotide according to any one of claims 1 to 13,
wherein first to second nucleotides, first to third nucleotides, first to fourth nucleotides, or first to fifth nucleotides from the 5' end of the 5' untranslated region are linked to one another via phosphorothioate.

15. The polynucleotide according to any one of claims 1 to 14,
wherein the 5' untranslated region contains a base modified nucleotide, and a modified base portion of the base modified nucleotide has the following structure: 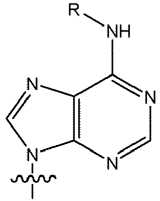
wherein R is an alkyl group having 1 to 6 carbon atoms.

16. The polynucleotide according to any one of claims 1 to 15, wherein the translated region contains at least two codons in which a first nucleotide is a sugar modified nucleotide.

17. The polynucleotide according to any one of claims 1 to 16, wherein the translated region contains four or more codons, and first nucleotides of all the codons are sugar modified nucleotides.

18. The polynucleotide according to any one of claims 1 to 16, wherein in the translated region, first nucleotides are sugar modified nucleotides in all codons excluding the stop codon, and modified sugar portions of the sugar modified nucleotides have the following structure:

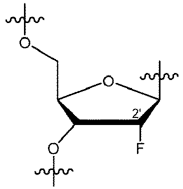

19. The polynucleotide according to any one of claims 1 to 18, wherein the translated region contains 2000 or less codons.

20. The polynucleotide according to any one of claims 1 to 19, wherein all nucleotides of the stop codon are sugar modified nucleotides.

21. The polynucleotide according to any one of claims 1 to 20, comprising the following structure: 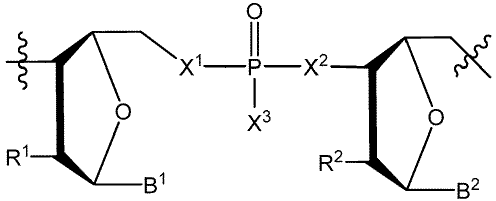
wherein R¹ and R² are each independently H, OH, F, OCH₂CH₂OCH₃ or OCH₃,
B¹ and B² are each independently a base portion,
X¹ is O, S, or NH, and
X² is O, S, NH, or the following structure: 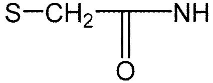
wherein X³ is OH, SH, or a salt thereof, and
X¹ and X² are not simultaneously O.

22. A pharmaceutical composition, comprising the polynucleotide according to any one of claims 1 to 21.
